# EUROPEAN PATENT APPLICATION

(11) **EP 4 710 946 A1**
(43) Date of publication of application: **18.03.2026**
(21) Application number: 24806479.2
(22) Date of filing: 10.05.2024
(51) Int. Cl.: A61K 47/68, A61K 47/65, A61K 47/54, A61K 45/00, A61K 31/4745, A61K 31/357, A61P 35/00, C07K 16/32, C07K 16/28

(54) **MULTI-PROJECTILE ANTIBODY-DRUG CONJUGATE, AND PREPARATION METHOD THEREFOR AND USE THEREOF**

(30) Priority: 12.05.2023 CN 202310535777; 28.07.2023 CN 202310943484; 21.11.2023 CN 202311558666
(71) Applicant: Medilink Therapeutics (Suzhou) Co., Ltd., Suzhou, Jiangsu 215000 (CN)
(72) Inventor: CAI, Jiaqiang, Suzhou, Jiangsu 215000 (CN); SHAN, Ailin, Suzhou, Jiangsu 215000 (CN); DENG, Hanwen, Suzhou, Jiangsu 215000 (CN); DENG, Chun, Suzhou, Jiangsu 215000 (CN); SONG, Shuai, Suzhou, Jiangsu 215000 (CN); ZOU, Peng, Suzhou, Jiangsu 215000 (CN); XIAO, Liang, Suzhou, Jiangsu 215000 (CN); XUE, Tongtong, Suzhou, Jiangsu 215000 (CN)
(74) Representative: Greaves Brewster LLP
(86) International application number: PCT/CN2024/092419
(87) International publication number: WO 2024/235127

(57) **Abstract**

A multi-projectile antibody-drug conjugate as represented by formula (I), and a preparation method therefor and the use thereof in the prevention and/or treatment of diseases related to abnormal cell activity, including, but not limited to, the use in the prevention and/or treatment of tumor diseases.

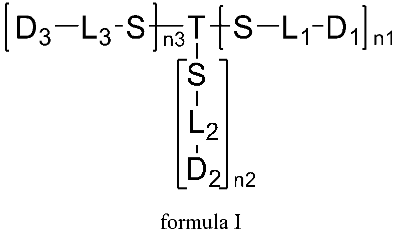

## Description

The present disclosure claims priority to Chinese Patent Application No. 2023105357777 filed on May 12, 2023, Chinese Patent Application No. 2023115586664 filed on November 21, 2023, and Chinese Patent Application No. 2023109434842 filed on July 28, 2023, which are incorporated herein by reference in their entirety.

### TECHNICAL FIELD

The present disclosure belongs to the field of pharmaceutical technology, and relates to a multi-warhead antibody-drug conjugate, a compound, a drug-linker conjugate, a preparation method therefor, and use thereof in the prevention and/or treatment of a disease associated with abnormal cell activity, including but not limited to use thereof in the prevention and/or treatment of a tumor disease.

### BACKGROUND

The improvement of the efficacy of cytotoxic drugs by antibody-drug conjugates (ADCs) has achieved great success. At present, 12 ADC drugs containing single small-molecule toxins have been approved for marketing globally. However, tumors are highly heterogeneous, and toxins with a single mechanism of action have limited therapeutic effects; furthermore, tumors can also develop drug resistance during treatment, including using PgP, ABCB1, and ABCG2 protein pumps to pump toxin molecules out of cells, thereby escaping being killed. Although the combined administration of two ADCs containing different toxins may overcome these problems, the administration is extremely inconvenient, and the patient compliance is poor; moreover, ADCs using the same antibody may exhibit antagonism between each other, so that the therapeutic efficacy is greatly reduced. Therefore, the development of ADCs capable of overcoming drug resistance is very attractive for overcoming the sensitivity to different drugs in heterogeneous tumor cell populations.

In order to improve the efficacy of ADC drugs, there have been patents that disclose double-toxin ADC molecules, and most of them use branched linker technology (in which a linker is split into two or even more linkers) to conjugate identical drugs. However, this only increases the drug antibody ratio (DAR) and cannot achieve synergy mechanistically. Moreover, no double-toxin ADC has been clinically successful at present. Therefore, it is difficult to find a multi-toxin ADC capable of having a synergistic effect on anti-tumor activity. Due to differences in the mechanism of action, pharmacokinetic properties, onset dose, and toxic dose of toxins, the two different toxins in such double-toxin ADCs are mostly in a ratio of 1:1 or other fixed ratios. It is apparent that the two toxins are not in a suitable ratio in terms of efficacy and toxicology, such that the expected double-toxin effect cannot be achieved. It is of great significance for tumor treatment to use a novel conjugation technology to flexibly adjust the ratio of two or more toxins, so that the two or more toxins are capable of producing the optimal efficacy at the same ADC dose while reducing the toxicity and enabling the ADC to have an optimal safety window.

Known antibody-drug conjugates generally bind to tumor cell surface antigens through the antibodies in the ADCs. Then, they enter endosomes through endocytosis and are trafficked from the endosomes to lysosomes, and the bioactive molecules (toxins or payloads) are dissociated from the ADCs by the action of hydrolases in the lysosomes, enter the cytoplasm from the lysosomes, and then kill the tumor cells. The bioactive molecules that escape from the killed tumor cells can further kill neighboring tumor cells that do not express the antigens or lowly express the antigens (i.e., a so-called bystander effect). In the whole process, changes in any link can cause drug resistance to the ADCs, thereby leading to the loss of the therapeutic efficacy, such as changes in antigen expression, weakened endocytosis or even loss of endocytosis, changes in functions of endosomes or lysosomes, etc., during treatment. Therefore, an ADC that can remain stable in the blood circulation system in the body and can be extracellularly cleaved in the tumor microenvironment, releasing the toxins to kill tumors, without the need for endocytosis, will be able to overcome a number of mechanisms of drug resistance to conventional ADCs and thus will be of great significance in clinical treatment.

### SUMMARY

In order to resolve the above problems, the present invention provides a multi-warhead antibody-drug conjugate, in which an antibody or an antigen-binding fragment thereof is used as a starting material and is conjugated to an appropriate number of toxin-linkers (drug-linkers) of other targets via active sites, wherein the toxin-linkers comprise two or more bioactive molecules with different mechanisms of action, thereby forming a novel antibody conjugate.

For this purpose, in a first aspect of the present disclosure, the present disclosure provides a multi-warhead antibody-drug conjugate of formula I, 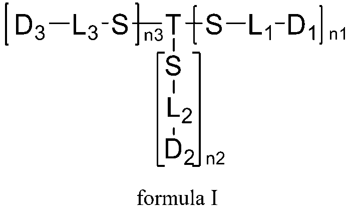
or a stereoisomer of the multi-warhead antibody-drug conjugate, a prodrug thereof, a pharmaceutically acceptable salt thereof, or a pharmaceutically acceptable solvate thereof,
wherein
T is an antibody or an antigen-binding fragment thereof;
L₁, L₂, and L₃ are linkers that covalently bind to T and corresponding D₁, D₂, and D₃, and L₁, L₂, and L₃ may be identical or different;
D₁, D₂, and D₃ are drug units, and D₁, D₂, and D₃ may be identical or different; and when D₁, D₂, and D₃ are identical, L₁, L₂, and L₃ are different;
n1, n2, and n3 are each independently selected from any numerical value between 0 and 8, and when one of n1, n2, and n3 is 0, the other two are not 0.

In some embodiments, n1, n2, and n3 are each independently selected from any numerical value between 0 and 8; and one of n1, n2, and n3 is 0, and the other two are not 0.

In some embodiments, an S atom attached to T in formula I is not an additional extraneous sulfur atom, but is -S- formed by attaching a sulfhydryl group contained in T itself after a disulfide bond is opened to the linker.

In some embodiments, the S atoms attached to T in formula I are all derived from disulfide bonds in the antibody T.

In some embodiments, T is an antibody or an antigen-binding fragment thereof, and the antibody or the antigen-binding fragment thereof comprises: a Fab, a Fab', a F(ab')2, an Fd, an Fv (for example, scFv), a dAb, a complementarity determining region fragment, a non-human antibody, a humanized antibody, a chimeric antibody, a fully human antibody, a probody, a monoclonal antibody, a bispecific antibody, or a multispecific antibody.

In some embodiments, T is a non-engineered antibody.

In some embodiments, L₁, L₂, and L₃ are linkers that covalently bind to T and corresponding D₁, D₂, and D₃, and L₁, L₂, and L₃ may be identical or different;
D₁, D₂, and D₃ are drug units, and D₁, D₂, and D₃ are different.

In some embodiments, L₁, L₂, and L₃ are linkers that covalently bind to T and corresponding D₁, D₂, and D₃, and L₁, L₂, and L₃ are different;
D₁, D₂, and D₃ are drug units, and D₁, D₂, and D₃ are identical.

In some embodiments, n1 is selected from integers or decimals from 0 to 1, from 1 to 2, from 2 to 3, from 3 to 4, from 4 to 5, from 5 to 6, from 6 to 7, and from 7 to 8.

In some embodiments, n2 is selected from integers or decimals from 0 to 1, from 1 to 2, from 2 to 3, from 3 to 4, from 4 to 5, from 5 to 6, from 6 to 7, and from 7 to 8.

In some embodiments, n3 is selected from integers or decimals from 0 to 1, from 1 to 2, from 2 to 3, from 3 to 4, from 4 to 5, from 5 to 6, from 6 to 7, and from 7 to 8.

In some embodiments, provided is a multi-warhead antibody-drug conjugate of formula I or a stereoisomer of the multi-warhead antibody-drug conjugate, a prodrug thereof, a pharmaceutically acceptable salt thereof, or a pharmaceutically acceptable solvate thereof,
wherein
T is a non-engineered antibody;
L₁, L₂, and L₃ are linkers that covalently bind to T and corresponding D₁, D₂, and D₃, and L₁, L₂, and L₃ may be identical or different;
D₁, D₂, and D₃ are drug units, and D₁, D₂, and D₃ are different;
n1, n2, and n3 are each independently selected from any numerical value between 0 and 8, and when one of n1, n2, and n3 is 0, the other two are not 0.

In some embodiments, provided is a multi-warhead antibody-drug conjugate of formula I or a stereoisomer of the multi-warhead antibody-drug conjugate, a prodrug thereof, a pharmaceutically acceptable salt thereof, or a pharmaceutically acceptable solvate thereof,
wherein
T is a non-engineered antibody;
L₁, L₂, and L₃ are linkers that covalently bind to T and corresponding D₁, D₂, and D₃, and L₁, L₂, and L₃ are different;
D₁, D₂, and D₃ are drug units, and D₁, D₂, and D₃ are identical;
n1, n2, and n3 are each independently selected from any numerical value between 0 and 8, and when one of n1, n2, and n3 is 0, the other two are not 0.

In some embodiments, L₁, L₂, and L₃ are selected from cleavable linkers and non-cleavable linkers.

In some embodiments, L₁, L₂, and L₃ are selected from linkers that are cleavable in a tumor microenvironment.

In some embodiments, provided is a multi-warhead antibody-drug conjugate of formula I or a stereoisomer of the multi-warhead antibody-drug conjugate, a prodrug thereof, a pharmaceutically acceptable salt thereof, or a pharmaceutically acceptable solvate thereof,
wherein
T is a non-engineered antibody;
at least one of L₁, L₂, and L₃ is a linker that is cleavable in the tumor microenvironment and covalently binds to T and corresponding D₁, D₂, and D₃, and L₁, L₂, and L₃ may be identical or different;
D₁, D₂, and D₃ are drug units, and D₁, D₂, and D₃ are different;
n1, n2, and n3 are each independently selected from any numerical value between 0 and 8, and when one of n1, n2, and n3 is 0, the other two are not 0;
the S atoms attached to T in formula I are all derived from disulfide bonds in the antibody.

In some embodiments, provided is a multi-warhead antibody-drug conjugate of formula I or a stereoisomer of the multi-warhead antibody-drug conjugate, a prodrug thereof, a pharmaceutically acceptable salt thereof, or a pharmaceutically acceptable solvate thereof,
wherein
T is a non-engineered antibody;
at least one of L₁ and L₂ is a linker that is cleavable in the tumor microenvironment and covalently binds to T and corresponding D₁ and D₂, and L₁ and L₂ may be identical or different;
D₁ and D₂ are drug units, and D₁ and D₂ are different;
n1 and n2 are each independently selected from any numerical value between 0 and 8, and neither n1 nor n2 is 0;
the S atoms attached to T in formula I are all derived from disulfide bonds in the antibody.

In some embodiments, provided is a multi-warhead antibody-drug conjugate of formula I or a stereoisomer of the multi-warhead antibody-drug conjugate, a prodrug thereof, a pharmaceutically acceptable salt thereof, or a pharmaceutically acceptable solvate thereof,
wherein
T is a non-engineered antibody;
at least one of L₁ and L₃ is a linker that is cleavable in the tumor microenvironment and covalently binds to T and corresponding D₁ and D₃, and L₁ and L₃ may be identical or different;
D₁ and D₃ are drug units, and D₁ and D₃ are different;
n1 and n3 are each independently selected from any numerical value between 0 and 8, and neither n1 nor n3 is 0;
the S atoms attached to T in formula I are all derived from disulfide bonds in the antibody.

In some embodiments, provided is a multi-warhead antibody-drug conjugate of formula I or a stereoisomer of the multi-warhead antibody-drug conjugate, a prodrug thereof, a pharmaceutically acceptable salt thereof, or a pharmaceutically acceptable solvate thereof,
wherein
T is a non-engineered antibody;
at least one of L₁ and L₂ is a linker that is cleavable in the tumor microenvironment and covalently binds to T and corresponding D₁ and D₂, and L₁ and L₂ may be identical or different;
D₁ and D₂ are drug units, and D₁ and D₂ are different;
n1 and n2 are each independently selected from any numerical value between 0 and 8, and neither n1 nor n2 is 0;
n3 is 0;
the S atoms attached to T in formula I are all derived from disulfide bonds in the antibody.

In some embodiments, provided is a multi-warhead antibody-drug conjugate of formula I or a stereoisomer of the multi-warhead antibody-drug conjugate, a prodrug thereof, a pharmaceutically acceptable salt thereof, or a pharmaceutically acceptable solvate thereof,
wherein
T is a non-engineered antibody;
at least one of L₁ and L₃ is a linker that is cleavable in the tumor microenvironment and covalently binds to T and corresponding D₁ and D₃, and L₁ and L₃ may be identical or different;
D₁ and D₃ are drug units, and D₁ and D₃ are different;
n1 and n3 are each independently selected from any numerical value between 0 and 8, and neither n1 nor n3 is 0;
n2 is 0;
the S atoms attached to T in formula I are all derived from disulfide bonds in the antibody.

In some embodiments, the linker comprises an amino acid residue or a short peptide consisting of 2-10 amino acid residues. Preferably, the linker comprises a dipeptide, a tripeptide, or a tetrapeptide.

In some embodiments, the amino acid residue is selected from Val, Ala, Gly, Cit, Arg, Phe, Lys, Asn, and substituted forms of the above amino acid residues.

In some embodiments, the dipeptide is selected from Val-Cit, Val-Ala, Ala-Ala, Val-Lys, and substituted forms of the above dipeptides.

In some embodiments, the tripeptide is selected from Ala-Ala-Ala, Ala-Ala-Asn, Val-Lys-Gly, Ala-Lys-Gly, Gly-Lys-Gly, Val-Lys-Ala, Ala-Lys-Ala, Gly-Lys-Ala, and substituted forms of the above tripeptides.

In some embodiments, the tetrapeptide is selected from Gly-Gly-Phe-Gly, Gly-Gly-Val-Ala, and substituted forms of the above tetrapeptides.

In some embodiments, the linker is selected from the following structure: 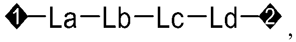 wherein
La is a linker unit that covalently binds to T and Lb;
Lb is a connector unit that covalently binds to La and Lc;
Lc is an amino acid residue, a polypeptide consisting of 2-10 amino acid residues, 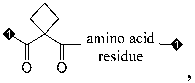 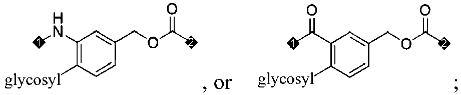 the amino acid residue is selected from a natural amino acid residue, a non-natural amino acid residue, a substituted amino acid residue, and a stereoisomer thereof;
Lc covalently binds to Lb and Ld;
Ld is a direct bond or a spacer unit that covalently binds to Lc and a drug unit.

In some embodiments, La is selected from heteroaryl, partially saturated heteroaryl, heteroarylbiaryl, heteroarylbiheteroaryl, heterocyclyl, oxoheterocyclyl, maleimide, cycloalkenyl, alkylamido, cycloalkylcarbonyl, heterocycloalkylcarbonyl, alkenylamido, 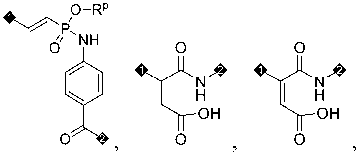 and 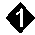 -S- 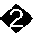 , wherein R^{p} is selected from C1-6 alkyl, -(CH₂CH₂O)_{q}H, and -(CH₂CH₂O)_{q}-C1-6 alkyl; q is selected from any integer between 0 and 30; position 1 is attached to an S atom, and position 2 is attached to Lb.

In some embodiments, Lₐ is selected from 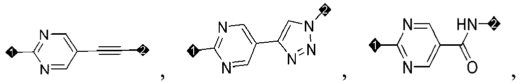 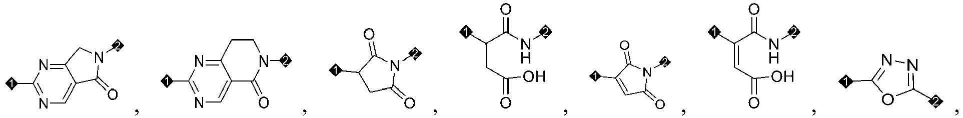 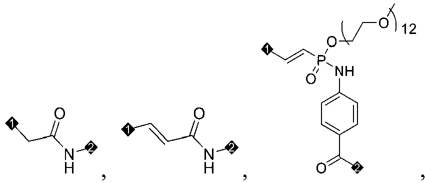 and 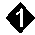 -S- 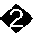 , wherein position 1 is attached to an S atom, and position 2 is attached to Lb.

In some embodiments, La is selected from 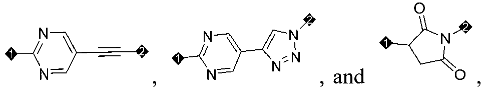 wherein position 1 is attached to an S atom, and position 2 is attached to Lb.

In some embodiments, La is selected from 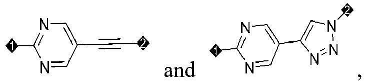 wherein position 1 is attached to an S atom, and position 2 is attached to Lb.

In some embodiments, La is selected from 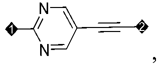 wherein position 1 is attached to an S atom, and position 2 is attached to Lb.

In some embodiments, Lb is 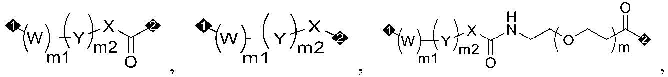 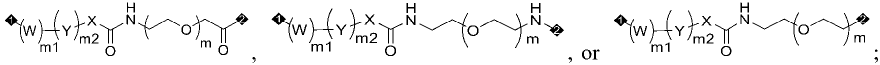 position 1 is attached to La, and position 2 is attached to Lc;
wherein
W is selected from -CR^{x1}R^{x2}-;
R^{x1} and R^{x2} are each independently selected from hydrogen, C1-6 alkyl, C2-6 alkenyl, C2-6 alkynyl, C3-10 cycloalkyl, and 4-10 membered heterocyclyl;
or R^{x1} and R^{x2}, together with the carbon atom to which they are attached, form a 3-6 membered carbocyclic ring or 3-6 membered heterocyclic ring;
each Y is independently selected from -O-, -CH₂-, -OCH₂CH₂-, -CH₂CH₂O-, and any combination of one or more of the above groups;
X is selected from -CR^{x3}R^{x4}- and -NR^{x5}-;
R^{x3}, R^{x4}, and R^{x5} are each independently selected from hydrogen, C1-6 alkyl, C2-6 alkenyl, C2-6 alkynyl, C3-10 cycloalkyl, and 4-10 membered heterocyclyl;
or R^{x3} and R^{x4}, together with the carbon atom to which they are attached, form a 3-6 membered carbocyclic ring or 3-6 membered heterocyclic ring;
m1 and m2 are each independently selected from 0, 1, 2, 3, 4, and 5;
m is selected from any integer between 0 and 10; preferably, m is selected from 1, 2, 3, 4, 5, 6, 7, and 8.

In some embodiments, W is selected from -CH₂-.

In some embodiments, X is selected from -CR^{x3}R^{x4}-, wherein R^{x3} and R^{x4} are each independently selected from hydrogen and methyl.

In some embodiments, Lb is

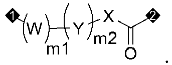

In some embodiments, Lb is selected from 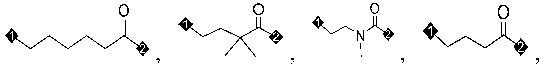 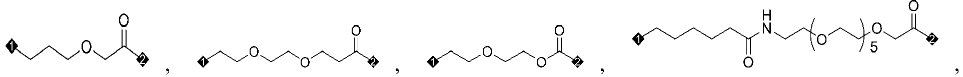 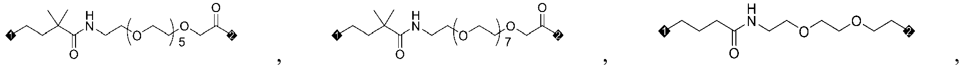 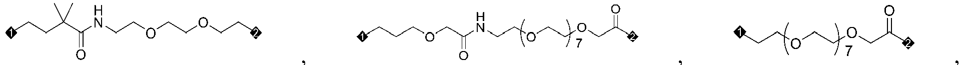 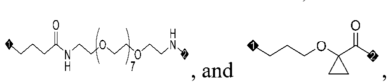 wherein position 1 is attached to La, and position 2 is attached to Lc.

In some embodiments, Lb is selected from 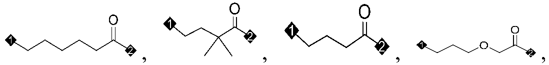 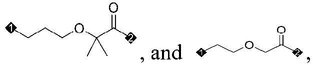 wherein position 1 is attached to La, and position 2 is attached to Lc.

In some embodiments, Lb is selected from 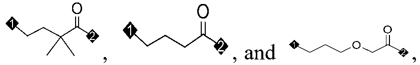 wherein position 1 is attached to La, and position 2 is attached to Lc.

In some embodiments, Lb is selected from 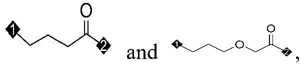 wherein position 1 is attached to La, and position 2 is attached to Lc.

In some embodiments, Lc is an amino acid residue or a short peptide consisting of 2-10 amino acid residues. The amino acid residue is selected from a natural amino acid residue, a non-natural amino acid residue, a substituted amino acid residue, and a stereoisomer thereof. A dipeptide, a tripeptide, or a tetrapeptide is preferred.

In some embodiments, Lc is selected from Val-Cit, Val-Ala, Val-Lys, Ala-Ala-Ala, Ala-Ala-Asn, Val-Lys-Gly, Gly-Gly-Phe-Gly, and substituted forms of the above dipeptides, tripeptides, or tetrapeptides.

In some embodiments, Lc is selected from: 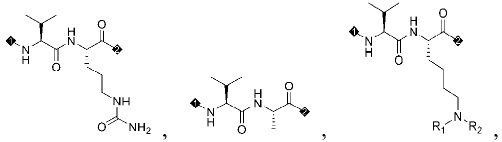 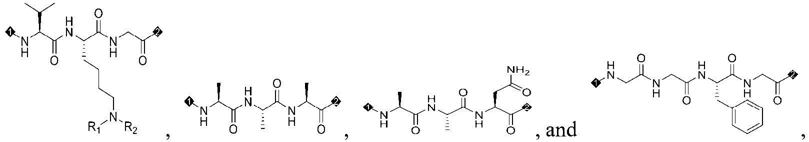 wherein R₁ and R₂ are each independently selected from hydrogen, C1-6 alkyl (for example, methyl, ethyl, or propyl), and C3-6 cycloalkyl (for example, cyclopropyl); preferably, R₁ and R₂ are both methyl, ethyl, or propyl; position 1 is attached to Lb, and position 2 is attached to Ld.

In some embodiments, Lc is selected from 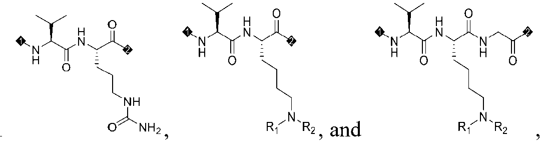 wherein R₁ and R₂ are each independently selected from hydrogen, C1-6 alkyl (for example, methyl, ethyl, or propyl), and C3-6 cycloalkyl (for example, cyclopropyl); preferably, R₁ and R₂ are both methyl, ethyl, or propyl; position 1 is attached to Lb, and position 2 is attached to Ld.

In some embodiments, Lc is a fragment that is cleavable in the tumor microenvironment, preferably 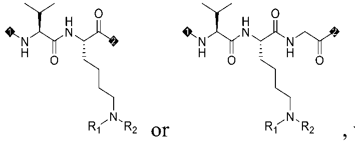 wherein position 1 is attached to Lb, and position 2 is attached to Ld.

In some embodiments, Lc is selected from 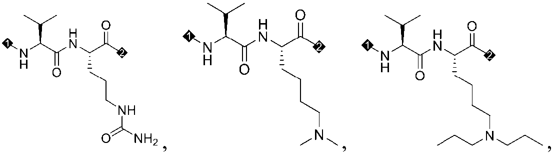 and 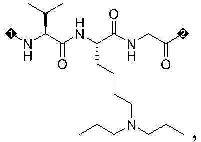 wherein position 1 is attached to Lb, and position 2 is attached to Ld.

In some embodiments, Lc is selected from 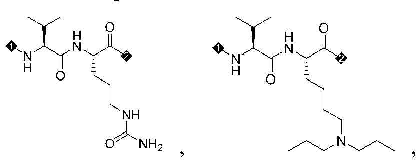 and 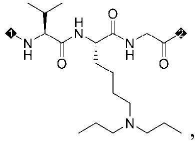 wherein position 1 is attached to Lb, and position 2 is attached to Ld.

In some embodiments, Lc is a fragment that is cleavable in the tumor microenvironment, preferably 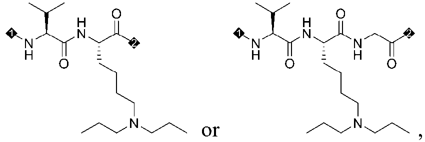 wherein position 1 is attached to Lb, and position 2 is attached to Ld.

In some embodiments, Ld is selected from a direct bond, 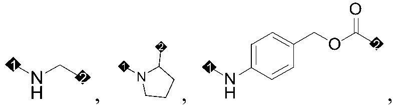 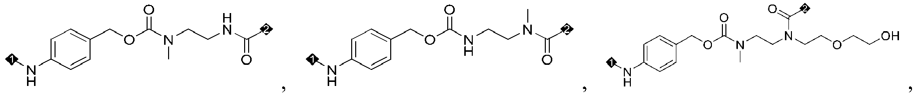 and 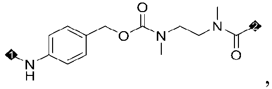 wherein position 1 is attached to Lc, and position 2 is attached to a drug unit.

In some embodiments, Ld is selected from a direct bond, 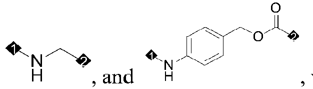 wherein position 1 is attached to Lc, and position 2 is attached to a drug unit.

In some embodiments, L₁, L₂, and L₃ are each independently selected from the following structures: 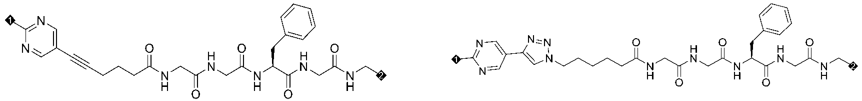 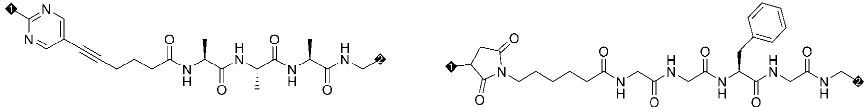 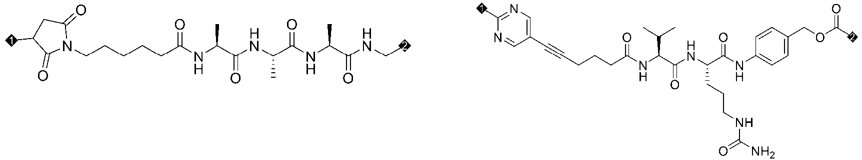 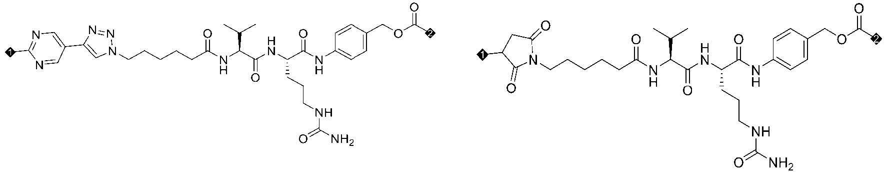 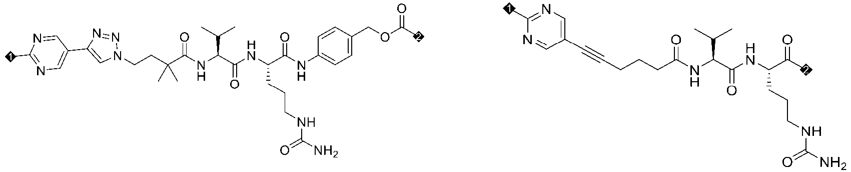 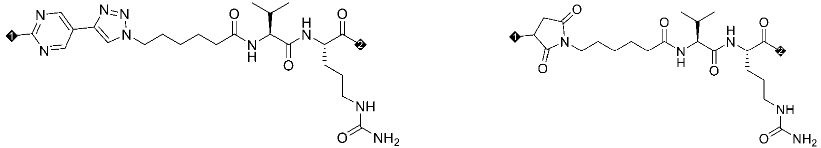 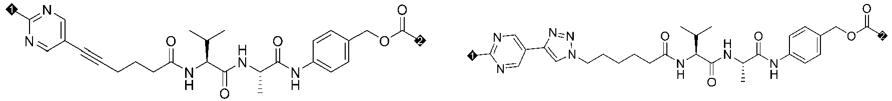 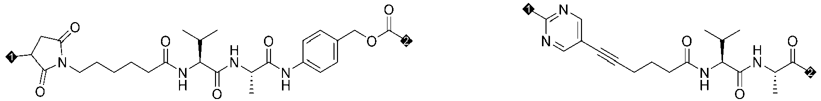 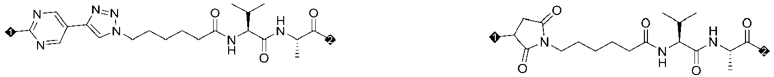 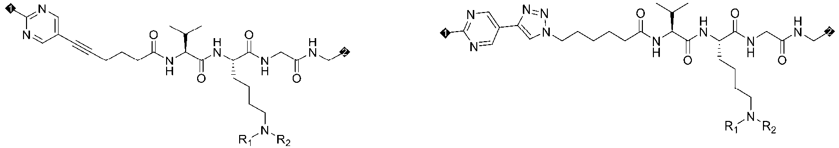 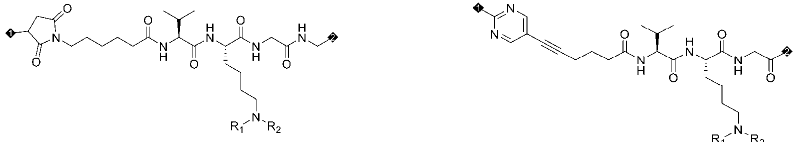 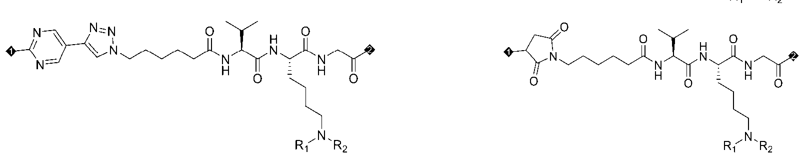 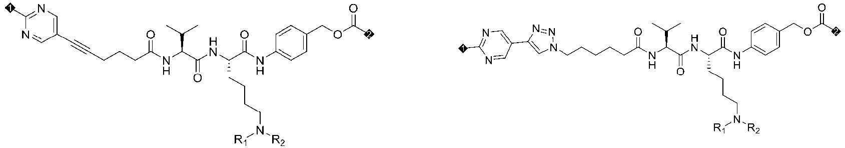 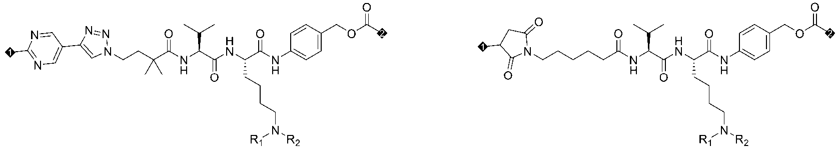 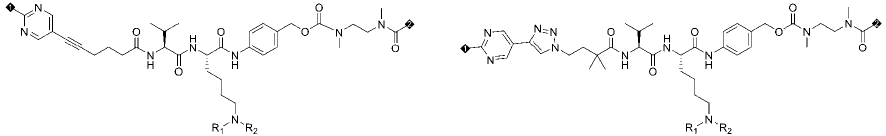 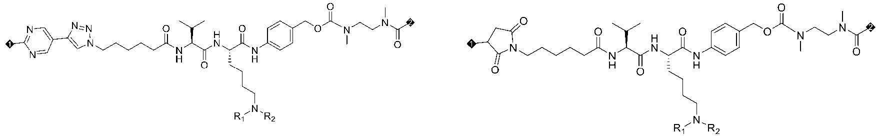 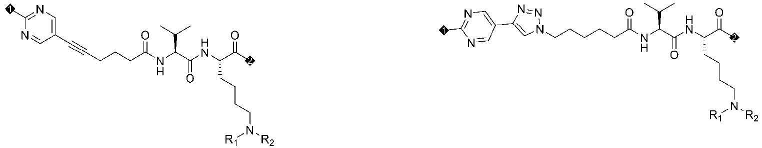 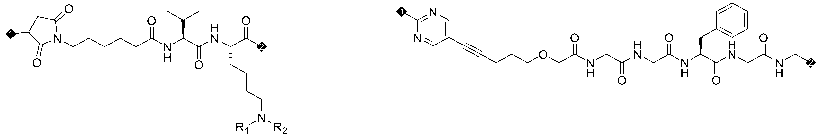 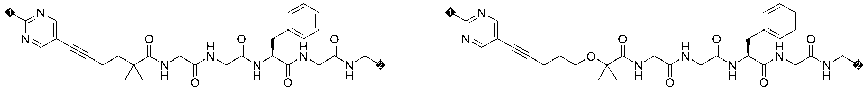 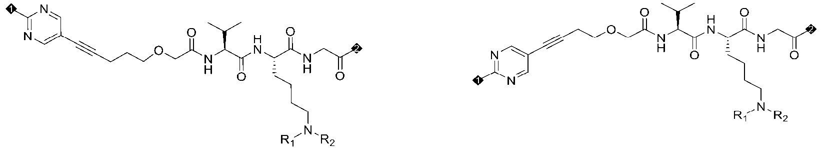 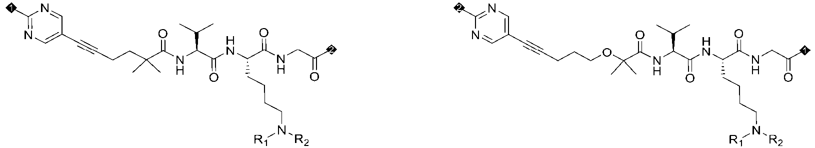 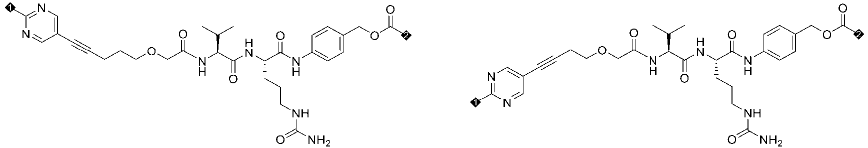 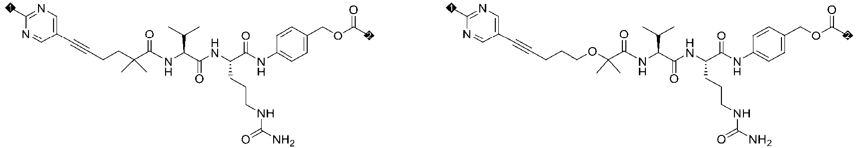 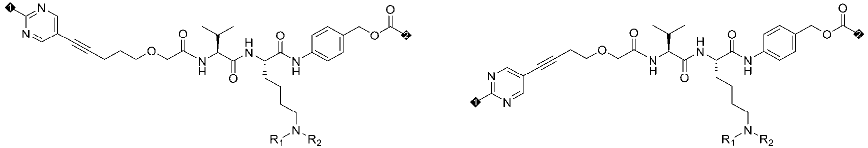 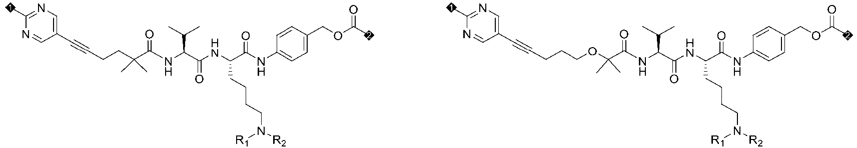 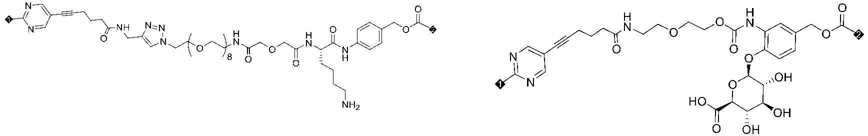 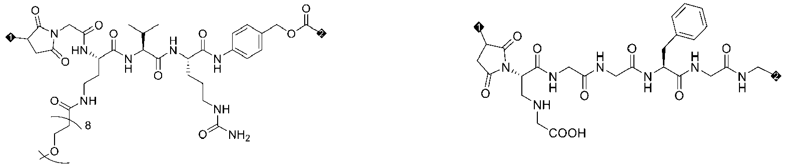 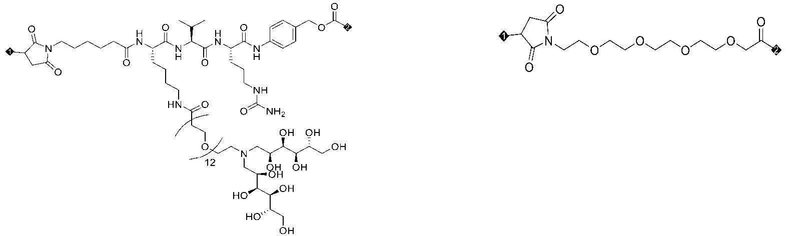 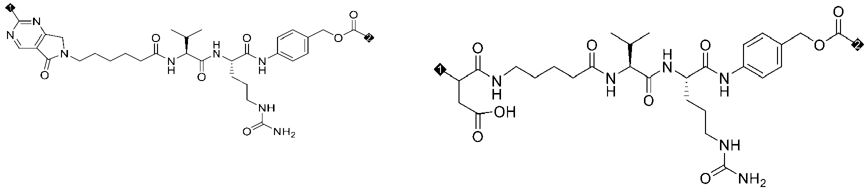 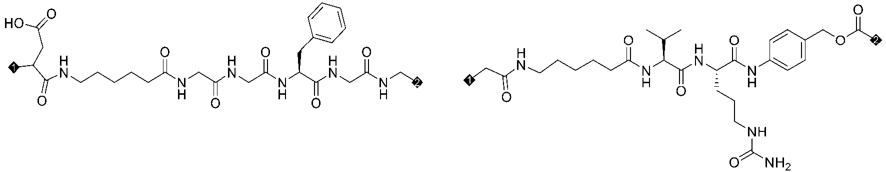 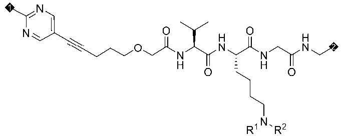 wherein position 1 is attached to an S atom, and position 2 is attached to a drug unit; R₁ and R₂ are each independently selected from hydrogen, C1-6 alkyl (for example, methyl, ethyl, or propyl), and C3-6 cycloalkyl (for example, cyclopropyl); preferably, R₁ and R₂ are both methyl, ethyl, or propyl.

In some preferred embodiments, L₁, L₂, and L₃ are each independently selected from the following structures: 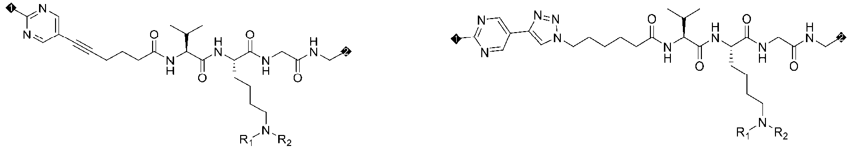 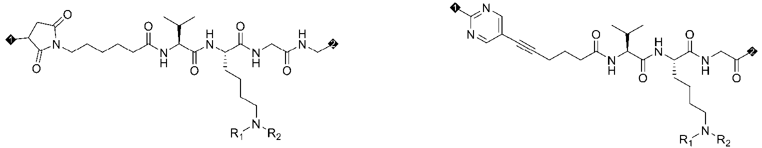 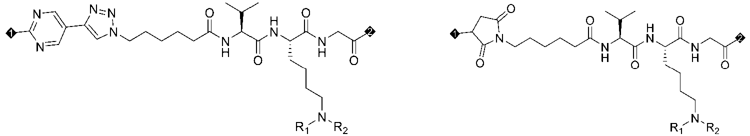 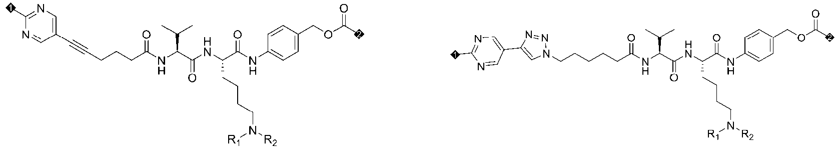 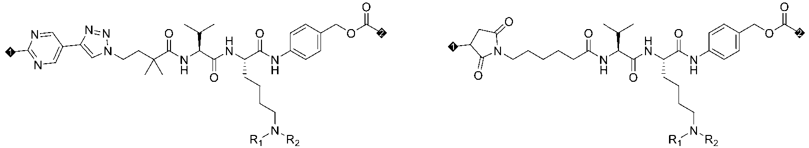 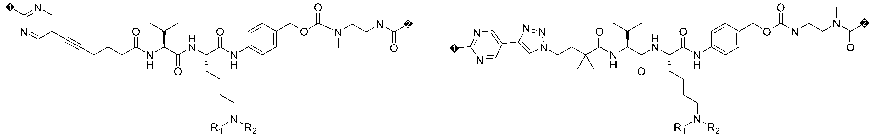 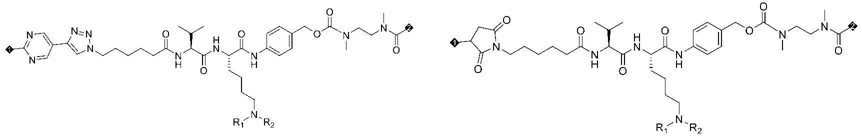 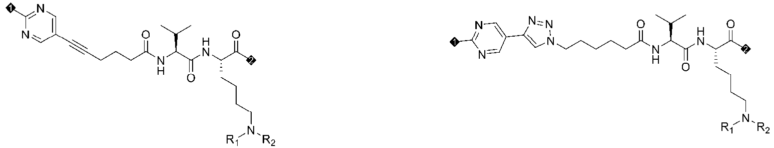 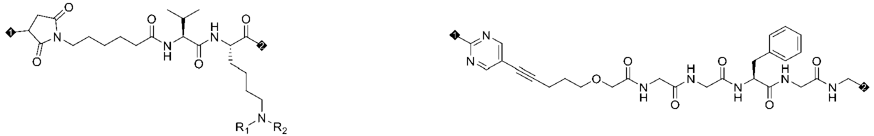 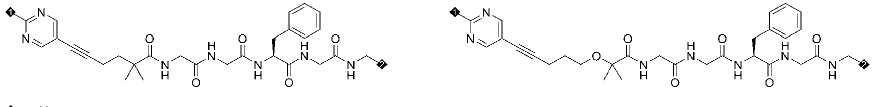 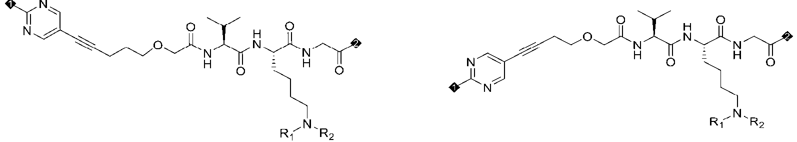 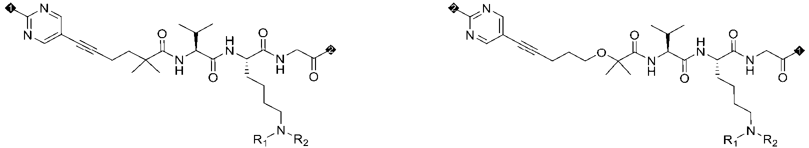 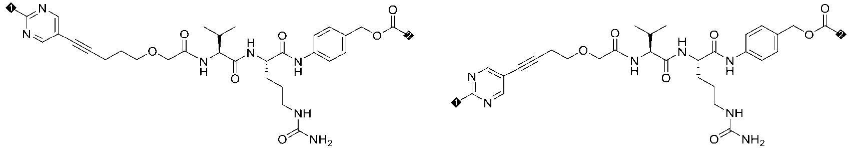 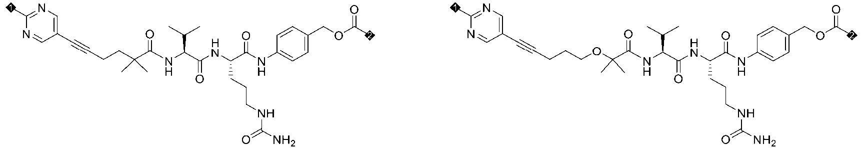 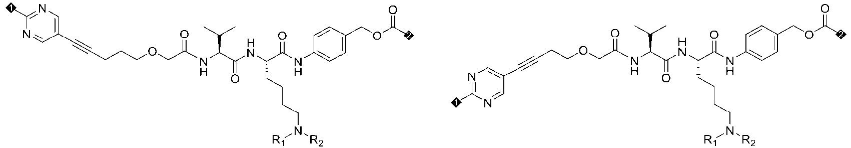 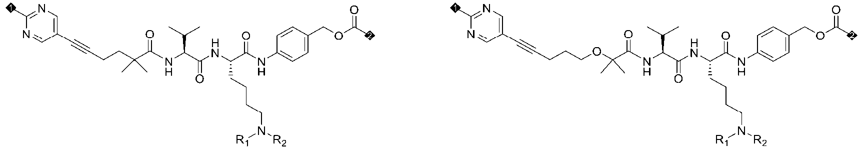 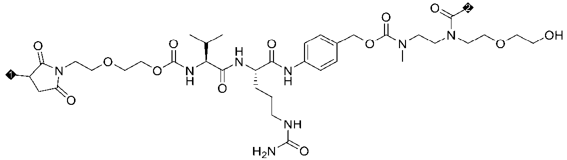 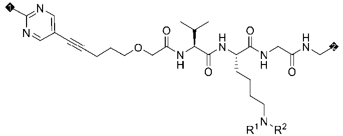 wherein position 1 is attached to an S atom, and position 2 is attached to a drug unit; R₁ and R₂ are each independently selected from H, C1-6 alkyl (for example, methyl, ethyl, or propyl), and C3-6 cycloalkyl (for example, cyclopropyl); preferably, R₁ and R₂ are both methyl, ethyl, or propyl.

In some preferred embodiments, L₁, L₂, and L₃ are each independently selected from the following structures: 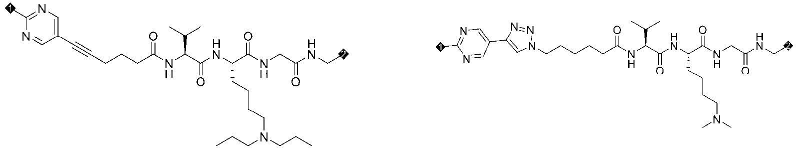 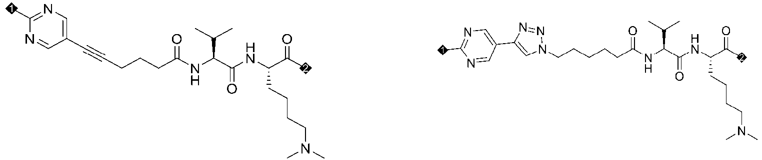 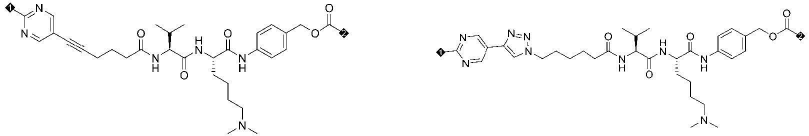 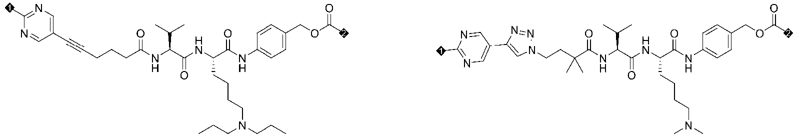 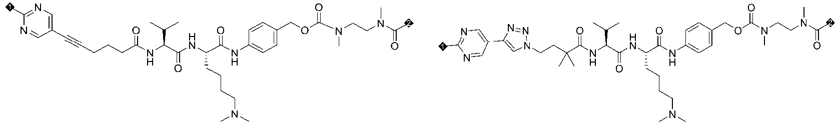 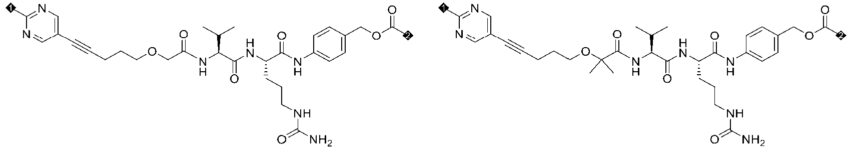 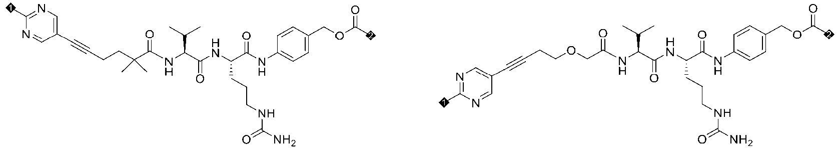 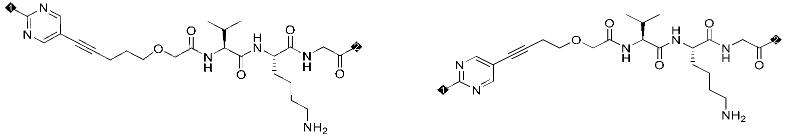 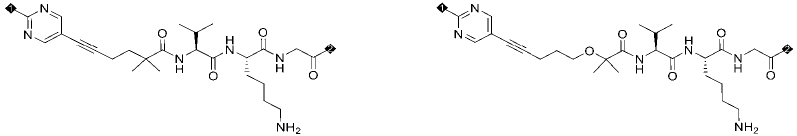 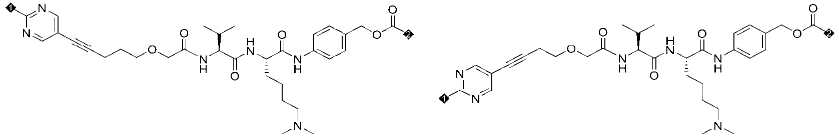 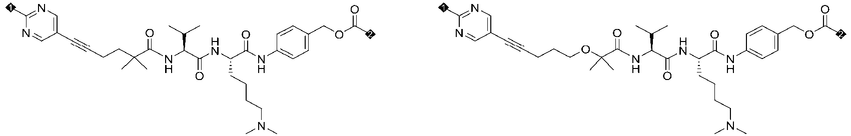 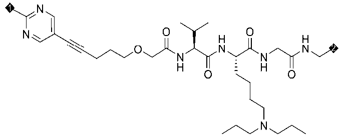 wherein position 1 is attached to an S atom, and position 2 is attached to a drug unit.

In some embodiments, L₁ is 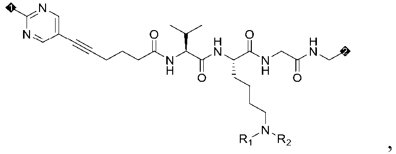 preferably

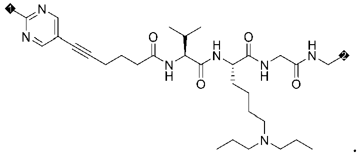

In some embodiments, L₂ is 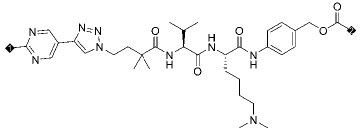 or 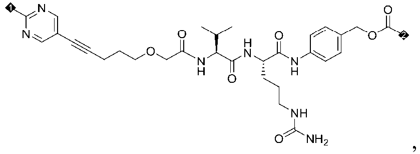 preferably

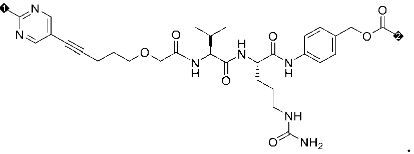

In some embodiments, L₃ is 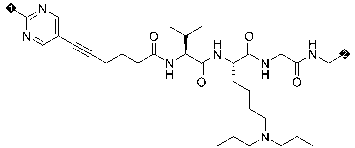 or 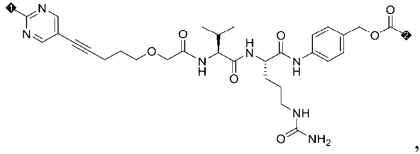 preferably

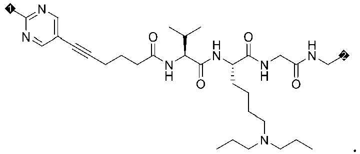

In some embodiments, D₁, D₂, and D₃ are bioactive molecular fragments.

In some embodiments, the bioactive molecule is selected from, but is not limited to, radioisotopes, such as At 211, I 131, I 125, Y 90, Re 186, Re 188, Sm 153, Bi 212, P 32, Pb 212, and radioisotopes of Lu; metal complexes, such as metal platinum complexes (for example, oxaliplatin) or metal gold complexes; glycopeptide antibiotics, such as bleomycin or pingyangmycin; glucocorticoid drugs, such as prednisone, prednisolone, or dexamethasone; calcineurin inhibitors, such as ciclosporin or tacrolimus; alkylating agents, such as nitrogen mustard, chlorambucil, cyclophosphamide, busulfan, mitomycin C, or melphalan; DNA topoisomerase inhibitors, including topoisomerase I inhibitors, such as camptothecin derivatives; topoisomerase II inhibitors, such as actinomycin D, adriamycin, doxorubicin, duocarmycin, daunorubicin, mitoxantrone, podophyllotoxin, nemorubicin, PNU-159682, or etoposide; drugs that interfere with DNA synthesis, such as methotrexate, 5-fluorouracil, cytarabine, gemcitabine, mercaptopurine, pentostatin, fludarabine, cladribine, nelarabine, pyrrolobenzodiazepine dimers (PBDs), or duocarmycin or calicheamicin and derivatives thereof; drugs that act on structural proteins, including tubulin inhibitors, such as vinca alkaloids, vincristine, eribulin and derivatives thereof, vinblastine, tubulysins, maytansine derivatives, MMAE/MMAF and derivatives thereof, paclitaxel, epothilone, docetaxel or cabazitaxel, or combretastatin and derivatives thereof; tumor signaling pathway inhibitors, such as serine/threonine kinase inhibitors, tyrosine kinase inhibitors, aspartate kinase inhibitors, or histidine kinase inhibitors; proteasome inhibitors, such as bortezomib, ixazomib, or carfilzomib; protease inhibitors, such as carmaphycin A or carmaphycin B; histone deacetylase inhibitors; tumor angiogenesis inhibitors; cyclin inhibitors; nucleic acid drugs, such as antisense oligonucleotides (ASOs), small interfering RNA (siRNA), microRNA, aptamers, or messenger RNA (mRNA); immunomodulators, such as thymosin, levamisole, Toll-like receptor modulators, or STING modulators; toxins, such as small-molecule toxins: amatoxin, or enzymatically active toxins of bacterial, fungal, plant, or animal origin, including fragments and/or variants thereof; steroidal saponins; thailanstatin and analogs thereof, such as thailanstatin A; BCL-xl/BCL2 inhibitors; and other active substances that inhibit tumor cell growth and promote tumor cell apoptosis or necrosis.

In some embodiments, D₁, D₂, and D₃ are each independently selected from, but are not limited to, a tubulin inhibitor, such as MMAE/MMAF and a derivative thereof, eribulin and a derivative thereof, or a maytansine derivative; a topoisomerase inhibitor, such as a camptothecin derivative; and a drug that interferes with DNA synthesis, such as duocarmycin or calicheamicin and a derivative thereof.

In some embodiments, D₁, D₂, and D₃ are each independently selected from MMAE/MMAF and a derivative thereof, eribulin and a derivative thereof, a camptothecin derivative, and duocarmycin or calicheamicin and a derivative thereof.

In some preferred embodiments, the camptothecin derivative is selected from the following structures:

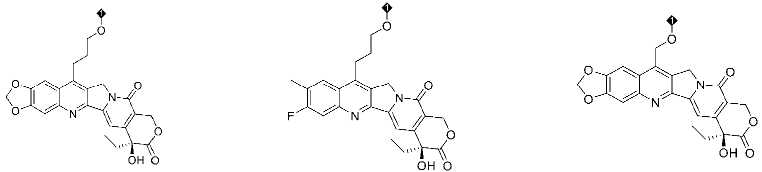

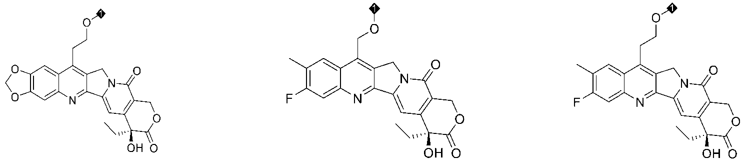

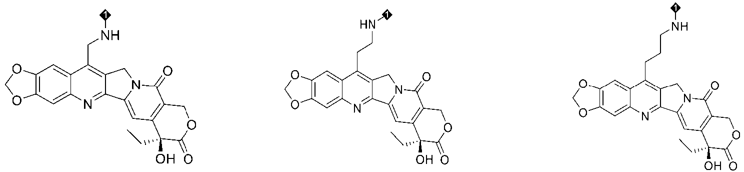

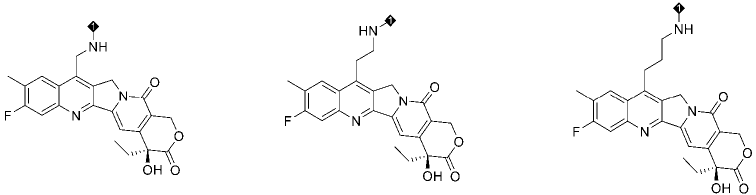

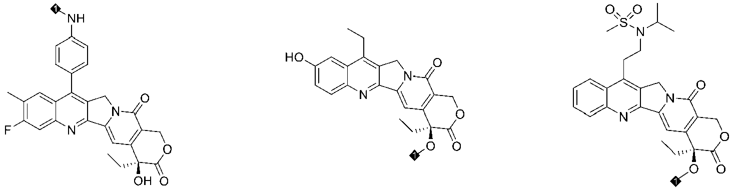

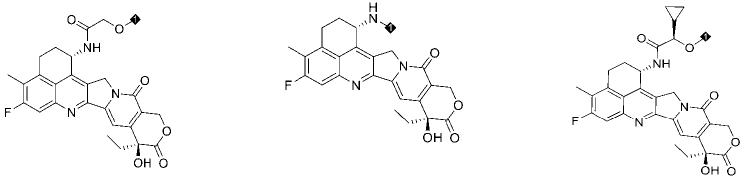

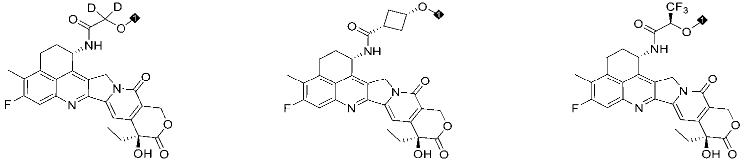

In some preferred embodiments, the camptothecin derivative is selected from the following structures:

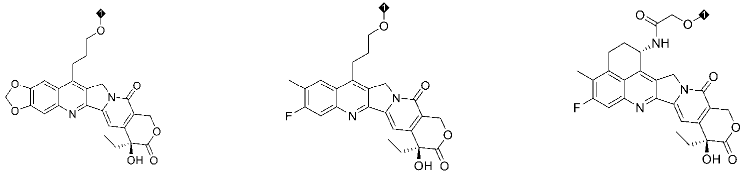

In some embodiments, the camptothecin derivative is

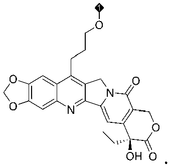

In some embodiments, D₁ is a camptothecin derivative.

In some embodiments, the eribulin derivative is selected from the following structures:

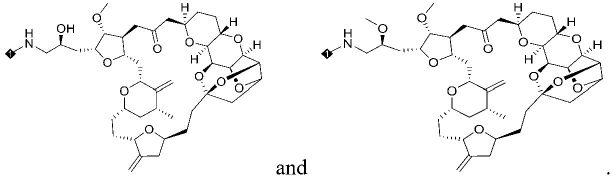

In some preferred embodiments, the eribulin derivative is selected from the following structure:

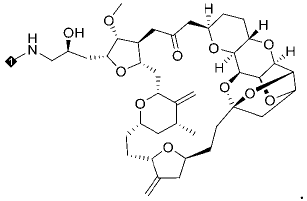

In some embodiments, the duocarmycin or calicheamicin and the derivative thereof are selected from the following structures:

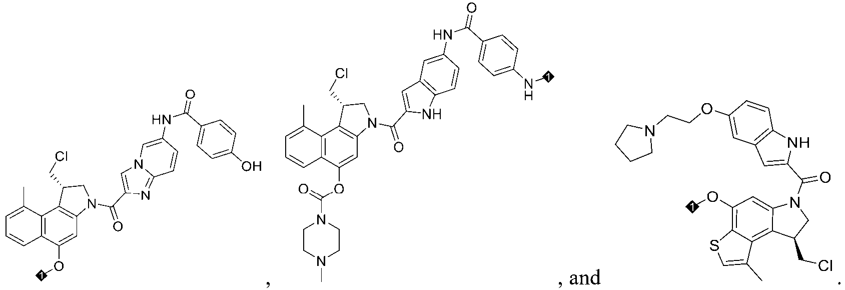

In some preferred embodiments, the duocarmycin or calicheamicin and the derivative thereof are selected from the following structure:

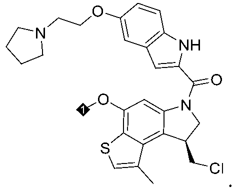

In some embodiments, D₂ is eribulin and a derivative thereof, or duocarmycin or calicheamicin and a derivative thereof.

In some embodiments, D₂ is eribulin and a derivative thereof.

In some embodiments, D₂ is duocarmycin or calicheamicin and a derivative thereof.

In some embodiments, D₂ is duocarmycin and a derivative thereof.

In some embodiments, D₂ is calicheamicin and a derivative thereof.

In some embodiments, the MMAE/MMAF and the derivative thereof are selected from the following structures:

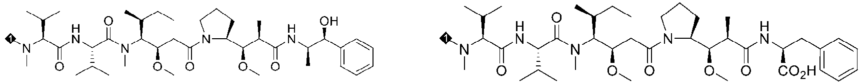

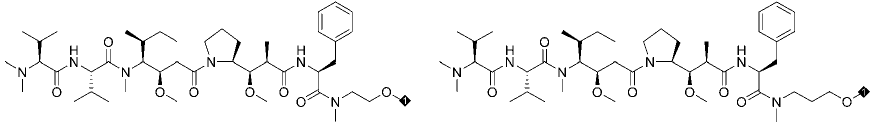

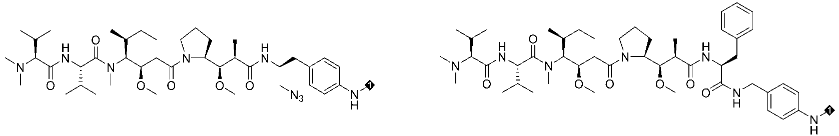

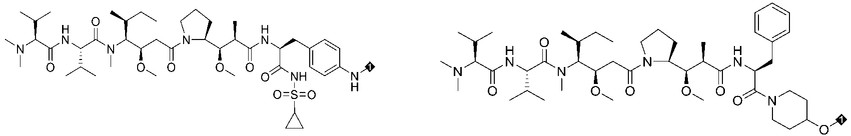

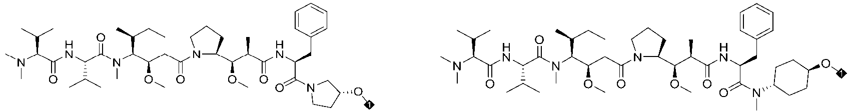

In some preferred embodiments, the MMAE/MMAF and the derivative thereof are selected from the following structures:

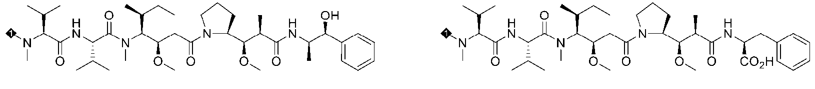

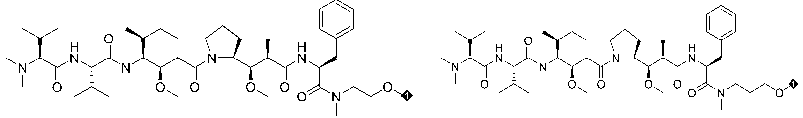

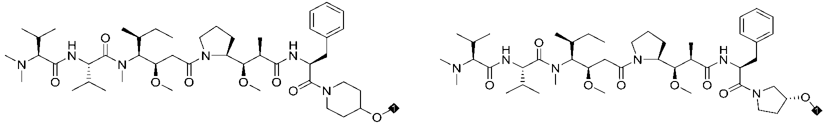

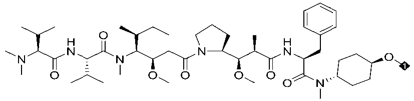

In some preferred embodiments, the MMAE/MMAF and the derivative thereof are

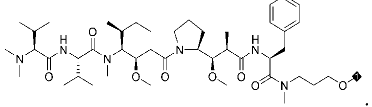

In some embodiments, D₃ is MMAE/MMAF and a derivative thereof.

In some embodiments, -L₁-D₁, -L₂-D₂, and -L₃-D₃ are selected from any combination of L₁, L₂, and L₃ with D₁, D₂, and D₃ according to any one of the embodiments of the present disclosure.

In some embodiments, -L₁-D₁ is selected from the following structural fragments:

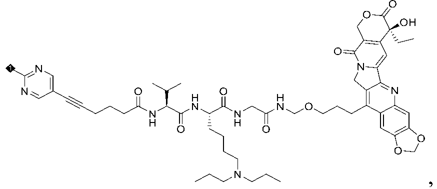

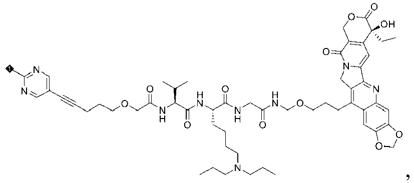

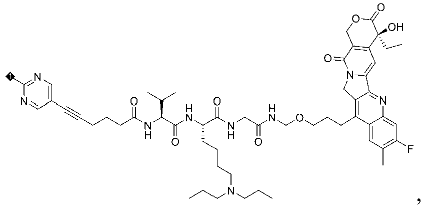

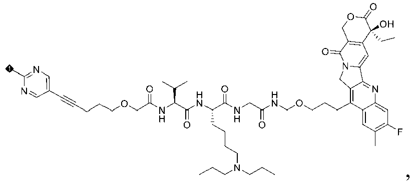

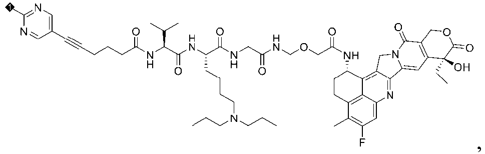

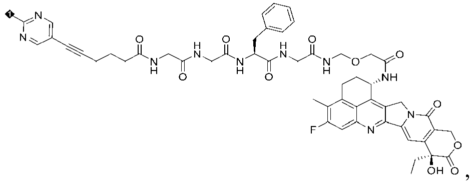

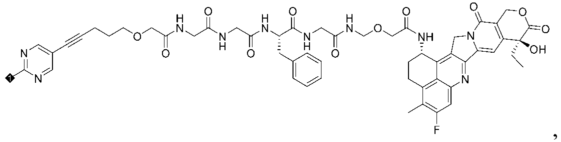

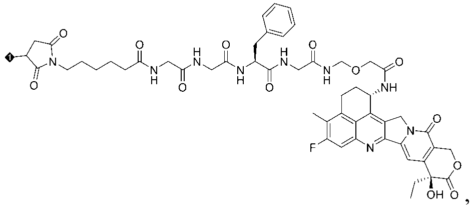

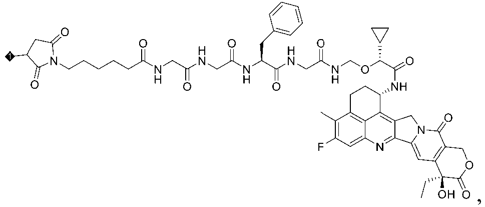

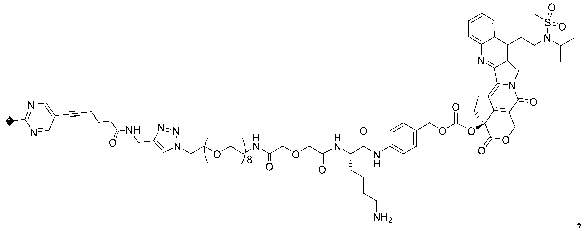

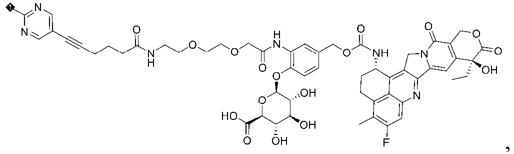

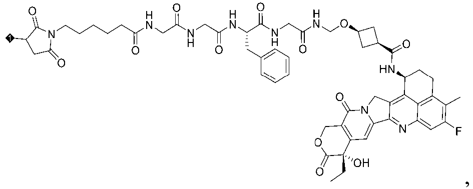

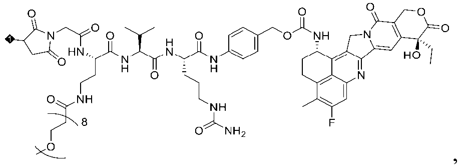

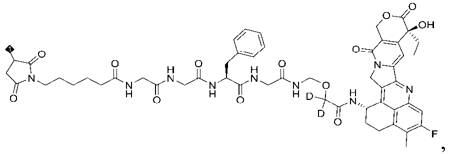

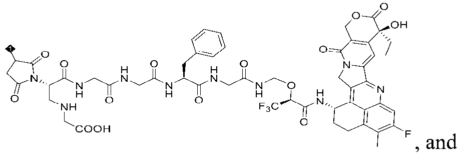

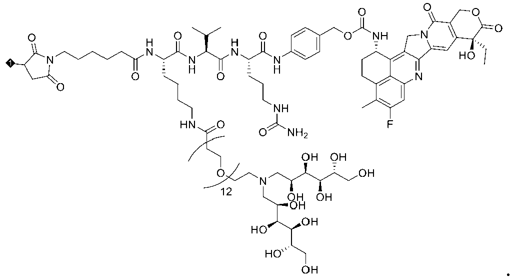

In some embodiments, -L₁-D₁ is selected from the following structural fragments: 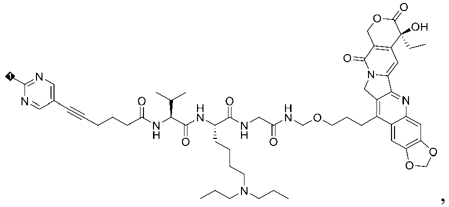 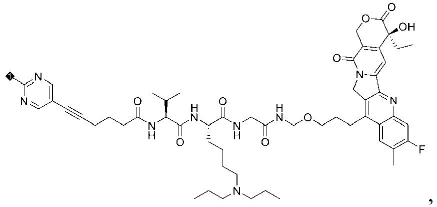 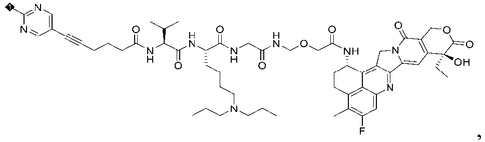 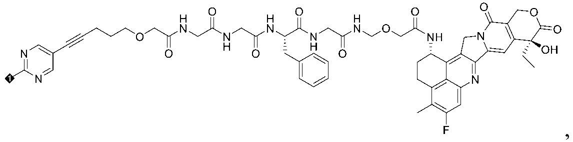 and

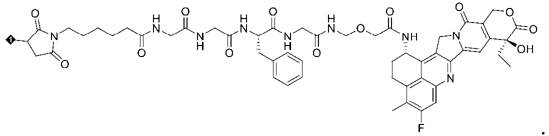

In some preferred embodiments, -L₁-D₁ is selected from the following structural fragments: 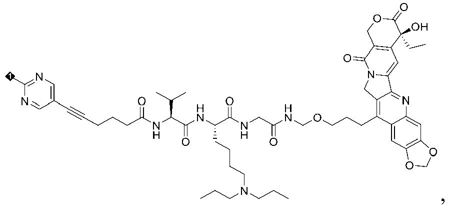 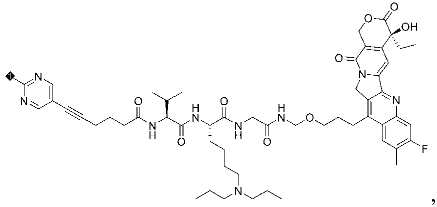 and

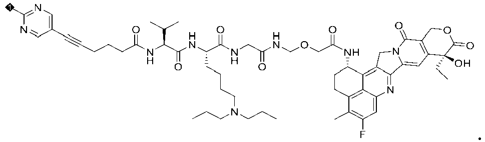

In some preferred embodiments, -L₁-D₁ is

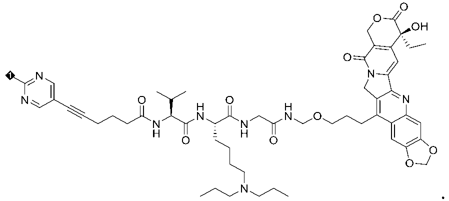

In some embodiments, -L₂-D₂ is selected from the following structural fragments: 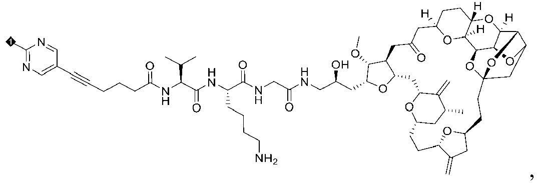 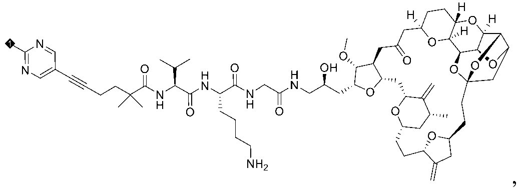 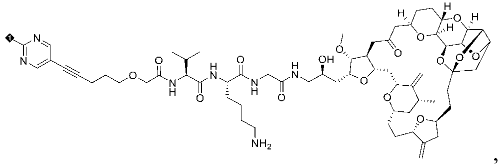 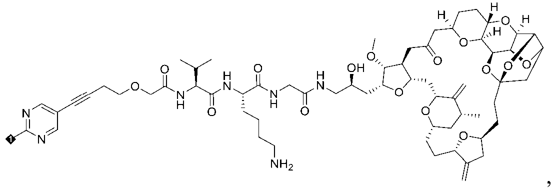 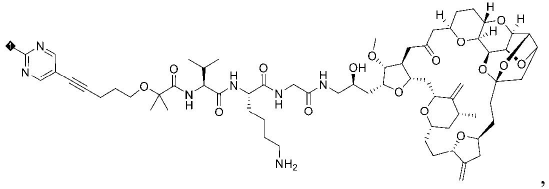 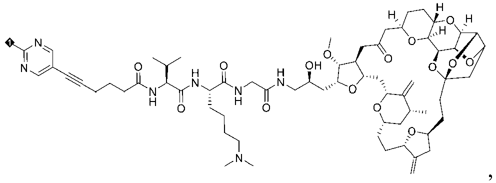 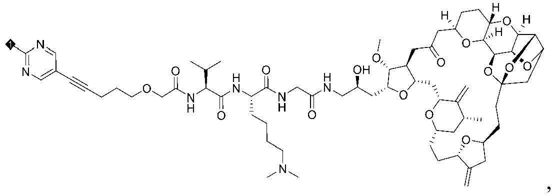 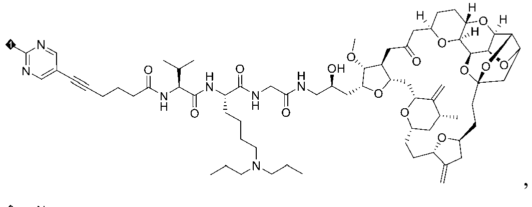 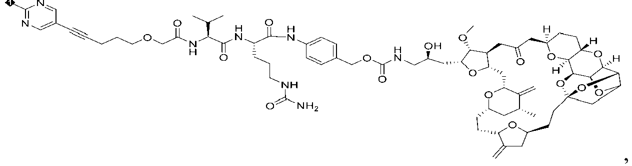 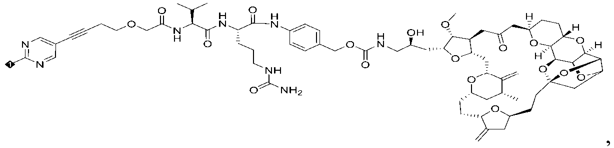 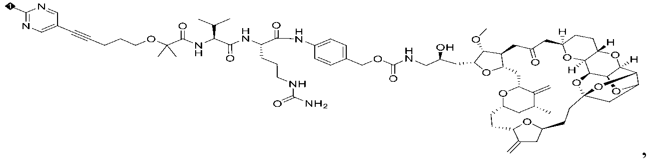 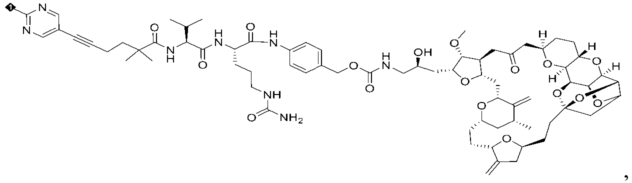 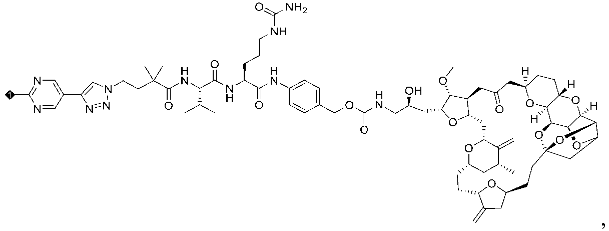 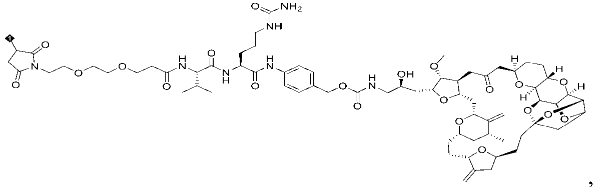 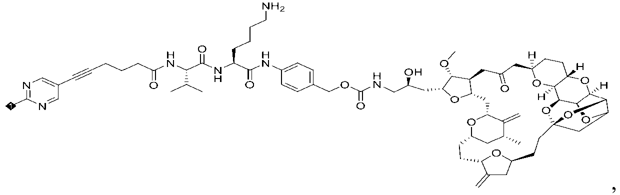 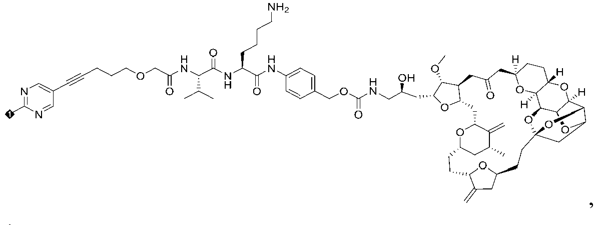 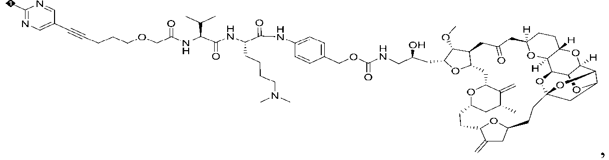 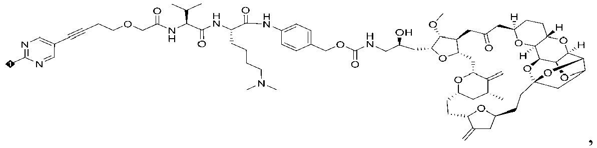 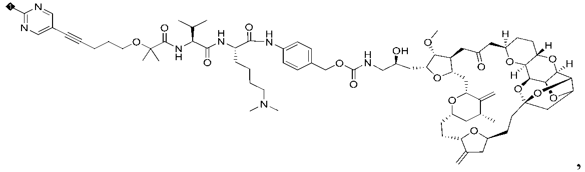 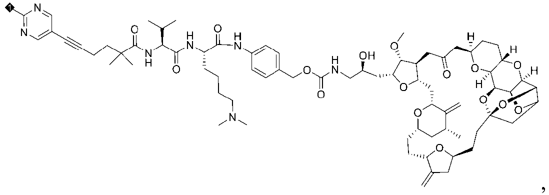 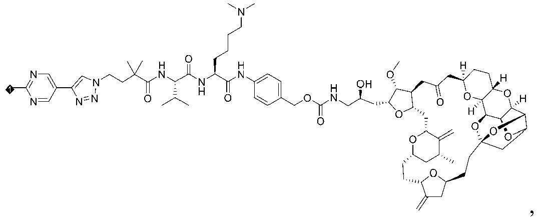 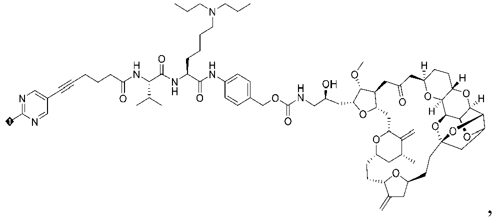 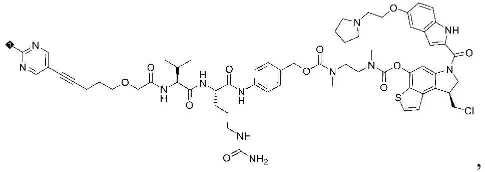 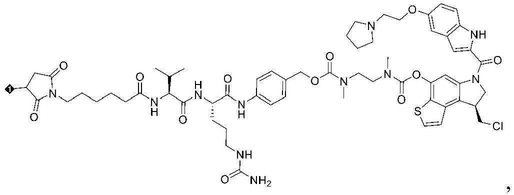 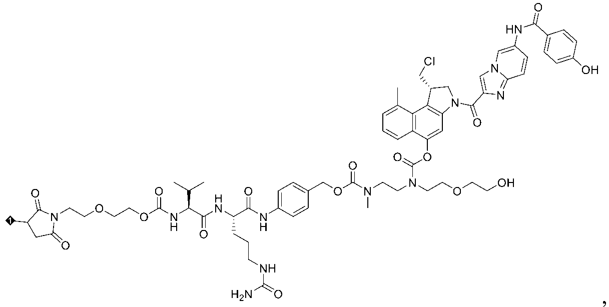 and

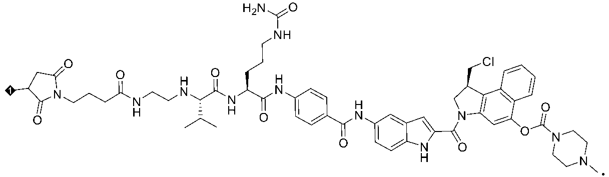

In some embodiments, -L₂-D₂ is selected from the following structural fragments: 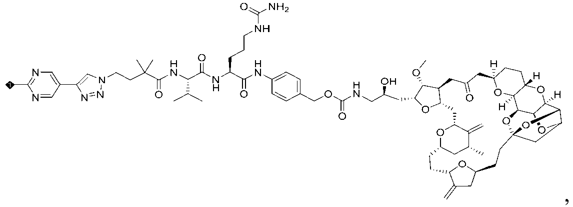 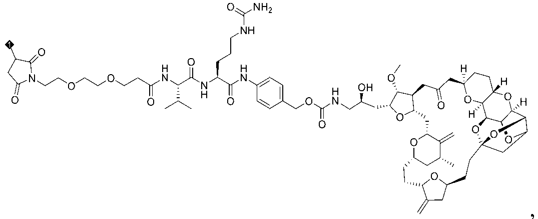 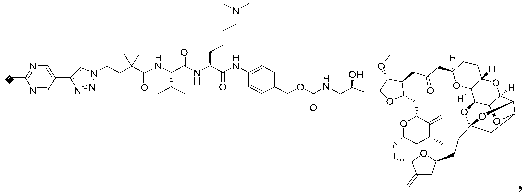 and

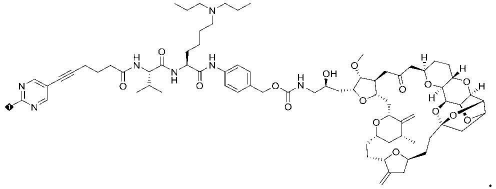

In some preferred embodiments, -L₂-D₂ is preferably

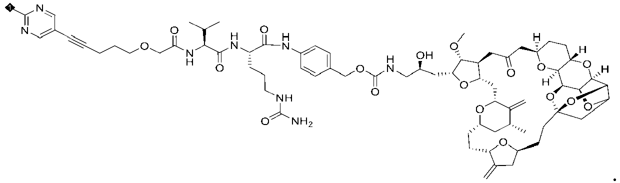

In some preferred embodiments, -L₂-D₂ is selected from the following structural fragments:

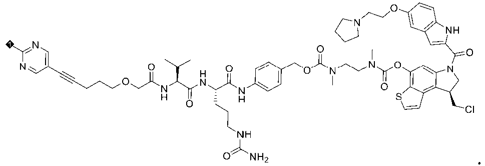

In some embodiments, -L₃-D₃ is selected from the following structural fragments: 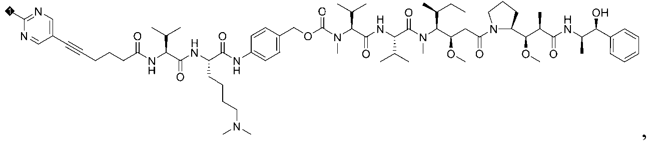 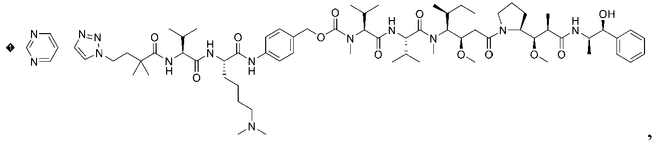 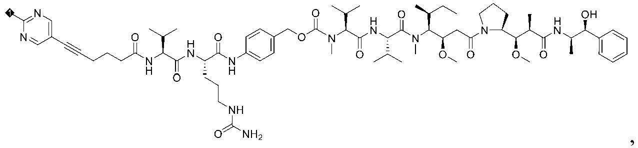 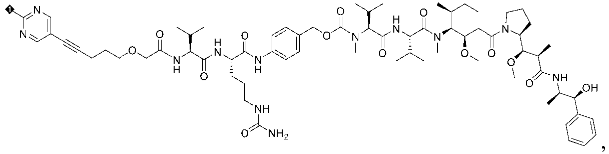 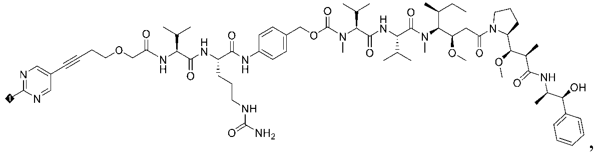 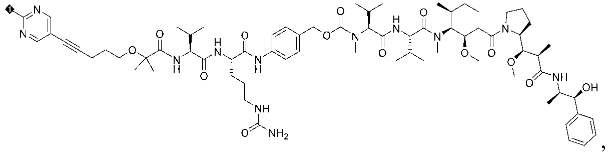 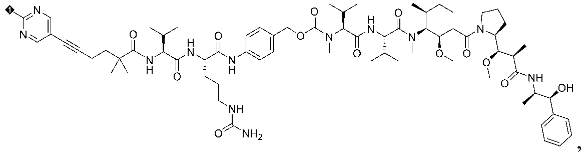 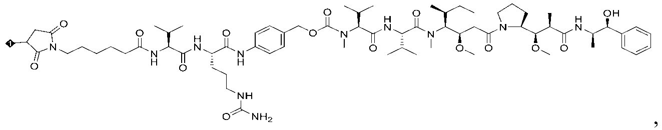 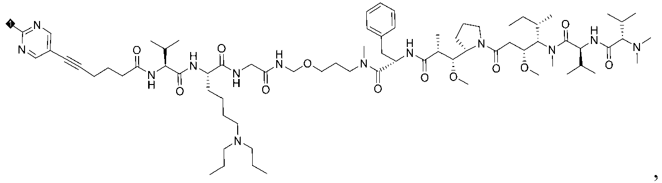 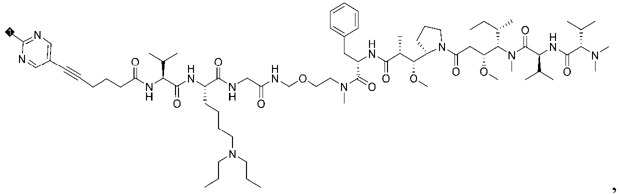 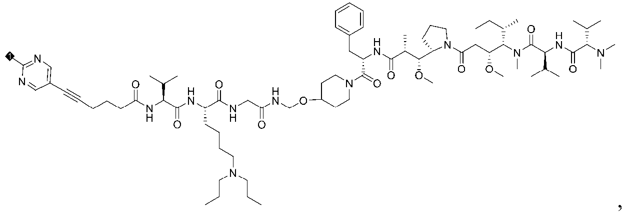 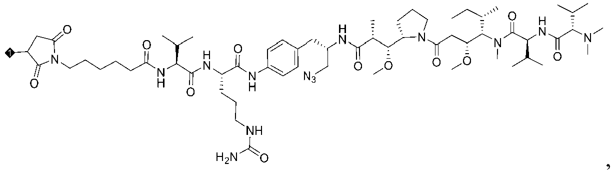 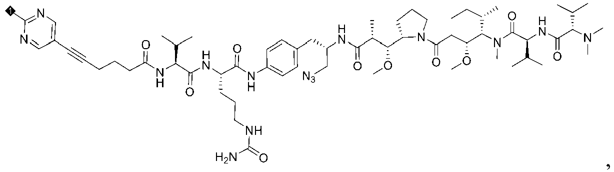 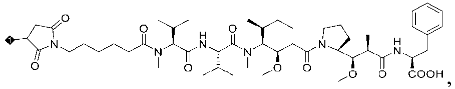 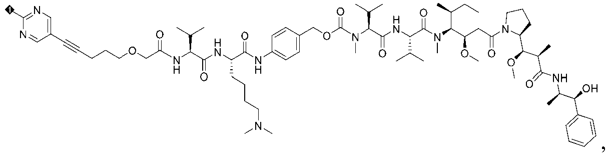 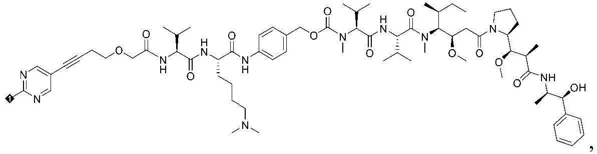 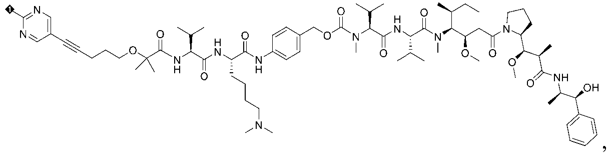 and

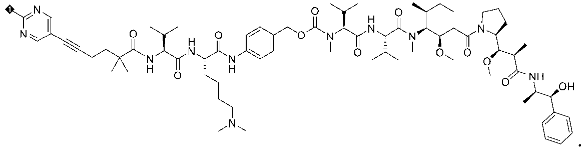

In some embodiments, -L₃-D₃ is selected from the following structural fragments: 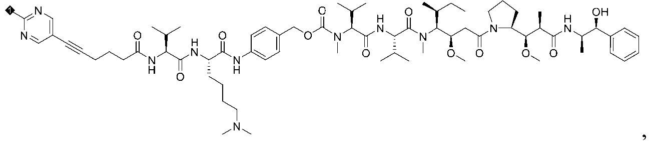 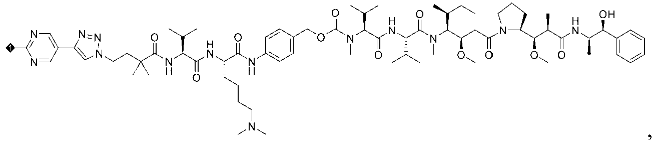 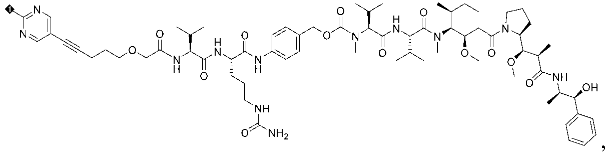 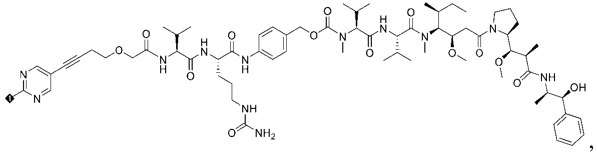 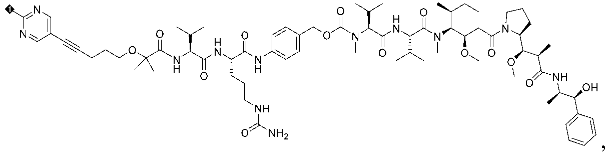 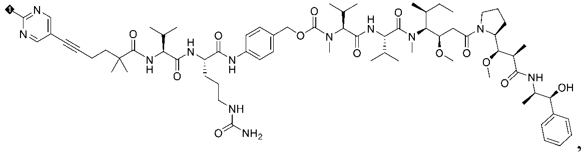 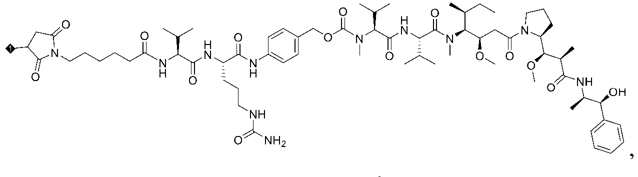 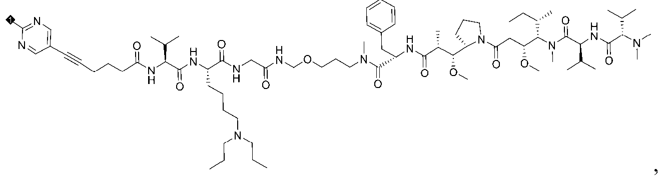 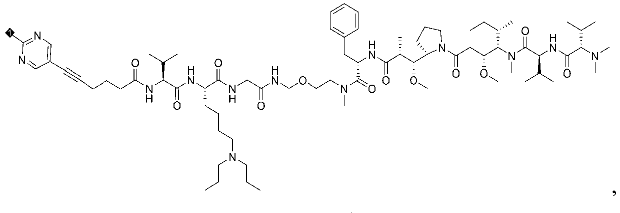 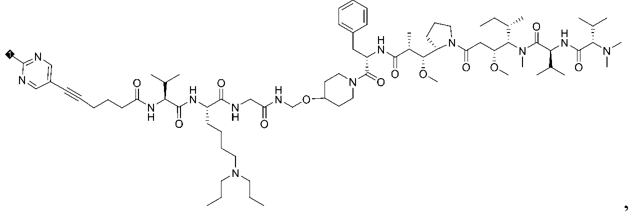 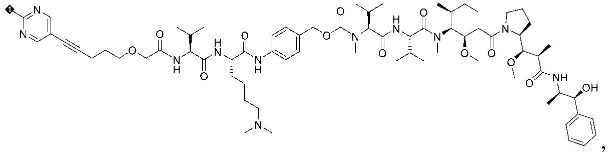 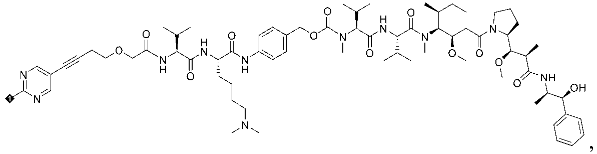 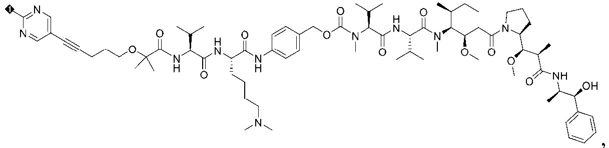 and

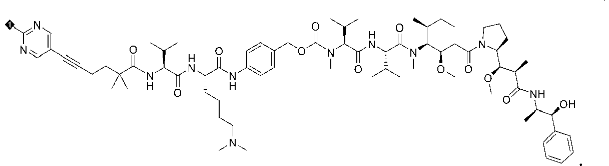

In some preferred embodiments, -L₃-D₃ is selected from the following structural fragments: 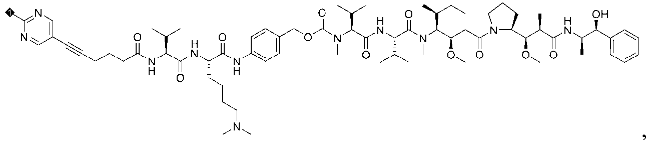 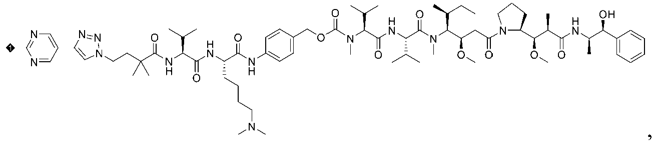 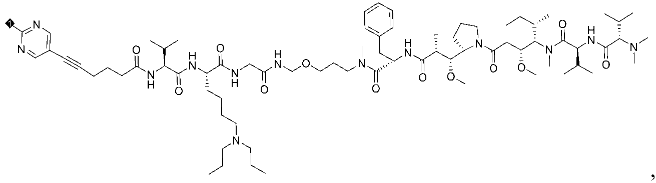 and

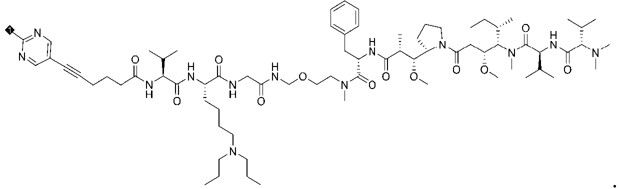

In some preferred embodiments, -L₃-D₃ is

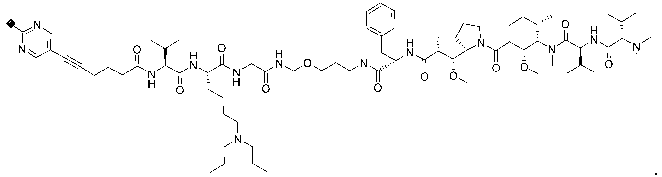

In some embodiments, T is selected from antibodies that bind to the following targets: epidermal growth factors, integrin α5β6, integrin α4β7, 0772P, 5T4, ACTA2, ADGRE1, AGS-16, AIF1, AKR1C1, AKR1C2, ANGPTL4, ApoE, ASLG659, BAFF-R, BMPR1B, BNIP3, Brevican, C1QA, C1QB, CA6, pCAD, P-cadherin, CADM1, CCL5, CCR5, CDl lb, CD1 lc, CD19, CD20, CD21, CD22, CD30, CD33, CD37, CD40, CD45 (PTPRC), CD49D (ITGA4), CD56, CD66e, CD70, CD72, CD74, CD79a, CD80, CD138, CD223, CDCP1, CDH11, Claudin18.2, COL6A3, COL7A1, CRIPTO, CSF1R, CTGF, CTSD, CTSS, CXCL10, CXCL11, CXCR5, DDIT4, DLL3, DLL4, DR5, E16, EFNA4, B7H3, EGFR, EGFRvIII, EGLN, EGLN3, EMR2, endothelin receptor, ENPP3, EpCAM, EphB2R, ETBR, FGF2, FGFR2, FcRH1, FcRH2, GEDA, GPNMB, GZMB, Her2, Her3, HLA-DOB, HMOX1, IFNG, IF16, IGF-1R, IGFBP3, IL10RA1, IL20Rα, IL-6, IRTA2, KISS1R, KRT33A, LOX, LRRC15, LUM, LY6E, LY64, LY86, LYPD3, MCPT8, MDP, mesothelin, c-Met, MFI2, MMP10, MMP14, MMP16, MPF, MSG783, MS4A7, MUC 1, MUC16, NaPi2b, NaPi3b, NCA, Nectin 4, NOG, P2X5, PD-L1, PDK1, PDGFRA, PFKFB3, PGF, PGK1, PIK3AP1, PIK3CD, PLOD2, PSCA, PSCA hlg, PSMA, RNF43, ROR1, Sema5b, SERPINE1, SLC39A6, SLITRK6, SLTRK6, STAT1, STC2, STEAP1, STEAP2, TCF4, TENB2, TGF, TGFBR1, TGFB2, tissue factor, TNFRSF21, TNFSF9, Trop-2, TrpM4, Tyro7, UPK1B, VEGFA, and WNT5A.

In some embodiments, T is an anti-Her2 antibody or an antigen-binding fragment thereof, an anti-B7H3 antibody or an antigen-binding fragment thereof, or an anti-Trop-2 antibody or an antigen-binding fragment thereof.

In some preferred embodiments, T is an anti-Her 2 antibody, for example, trastuzumab or pertuzumab, preferably trastuzumab.

In some preferred embodiments, T is an anti-B7H3 antibody, for example, 1D1, 1D1-01, 2E3, or 2E3-02 antibody, enoblituzumab, mirzotamab, or omburtamab.

In some embodiments, T is an anti-Trop-2 antibody, for example, sacituzumab.

In some preferred embodiments, provided is the double-warhead antibody conjugate of formula I or the stereoisomer of the double-warhead antibody-drug conjugate, the prodrug thereof, the pharmaceutically acceptable salt thereof, or the pharmaceutically acceptable solvate thereof,
wherein
n1 and n2 are each independently selected from any numerical value between 1 and 8, and n3 is 0;
D₁ is a camptothecin derivative;
D₂ is eribulin and a derivative thereof;
T, L₁, and L₂ are as defined in any one of the embodiments of the present disclosure.

Preferably, at least one of L₁ and L₂ is a linker that is cleavable in the tumor microenvironment.

In some preferred embodiments, provided is the double-warhead antibody conjugate of formula I or the stereoisomer of the double-warhead antibody-drug conjugate, the prodrug thereof, the pharmaceutically acceptable salt thereof, or the pharmaceutically acceptable solvate thereof,
wherein
n1 and n2 are each independently selected from any numerical value between 1 and 8, and n3 is 0;
D₁ is a camptothecin derivative;
D₂ is duocarmycin or calicheamicin and a derivative thereof;
T, L₁, and L₂ are as defined in any one of the embodiments of the present disclosure.

Preferably, at least one of L₁ and L₂ is a linker that is cleavable in the tumor microenvironment.

In some preferred embodiments, n1+n2 is approximately equal to 8, and n1/n2 is approximately equal to 1, 1.5, 2, 2.5, 3, 3.5, 4, 4.5, 5, 5.5, 6, 6.5, or 7.5.

In some preferred embodiments, n1+n2 is approximately equal to 7-8, for example, approximately equal to 8; n1/n2 is approximately equal to 1-8, for example, approximately equal to 1, 1.5, 2, 2.5, 3, 3.5, 4, 4.5, 5, 5.5, 6, 6.5, 7, or 7.5.

In some preferred embodiments, n1+n2 is approximately equal to 4, and n1/n2 is approximately equal to 1, 1.5, 2, 2.5, 3, 3.5, or 4.

In some preferred embodiments, provided is the double-warhead antibody-drug conjugate of formula I or the stereoisomer of the double-warhead antibody-drug conjugate, the prodrug thereof, the pharmaceutically acceptable salt thereof, or the pharmaceutically acceptable solvate thereof,
wherein
n1 and n3 are each independently selected from any numerical value between 1 and 8, and n2 is 0;
D₁ is a camptothecin derivative;
D₃ is MMAE/MMAF and a derivative thereof;
T, L₁, and L₃ are as defined in any one of the embodiments of the present disclosure.

Preferably, at least one of L₁ and L₃ is a linker that is cleavable in the tumor microenvironment.

In some preferred embodiments, n1+n3 is approximately equal to 8, and n1/n3 is approximately equal to 1, 1.5, 2, 2.5, 3, 3.5, 4, 4.5, 5, 5.5, 6, 6.5, or 7.5.

In some preferred embodiments, n1+n3 is approximately equal to 7-8, for example, approximately equal to 8; n1/n3 is approximately equal to 1-8, for example, approximately equal to 1, 1.5, 2, 2.5, 3, 3.5, 4, 4.5, 5, 5.5, 6, 6.5, 7, or 7.5; preferably, n1/n3 is approximately equal to 2.5-3.5, for example, approximately equal to 3.

In some preferred embodiments, n1+n3 is approximately equal to 4, and n1/n2 is approximately equal to 1, 1.5, 2, 2.5, 3, 3.5, or 4.

In some preferred embodiments, provided is the double-warhead antibody-drug conjugate of formula I or the stereoisomer of the double-warhead antibody-drug conjugate, the prodrug thereof, the pharmaceutically acceptable salt thereof, or the pharmaceutically acceptable solvate thereof,
wherein
n2 and n3 are each independently selected from any numerical value between 1 and 8, and n1 is 0;
D₂ is eribulin and a derivative thereof;
D₃ is MMAE/MMAF and a derivative thereof;
T, L₂, and L₃ are as defined in any one of the embodiments of the present disclosure.

In some preferred embodiments, n2+n3 is approximately equal to 8, and n3/n2 is approximately equal to 1, 1.5, 2, 2.5, 3, 3.5, 4, 4.5, 5, 5.5, 6, 6.5, or 7.5.

In some embodiments, the multi-warhead antibody-drug conjugate of formula I is selected from:

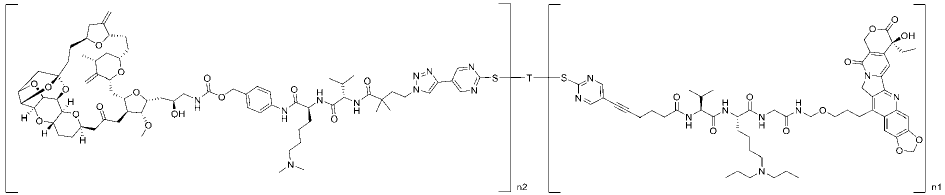

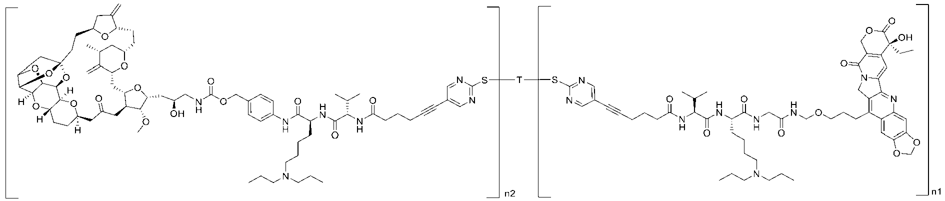

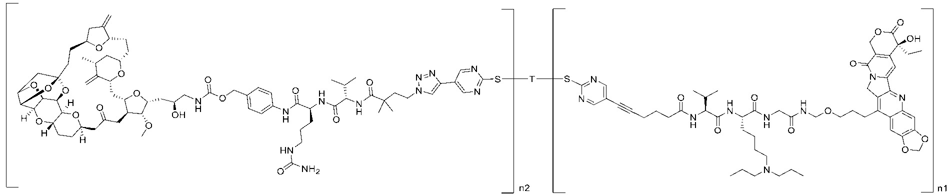

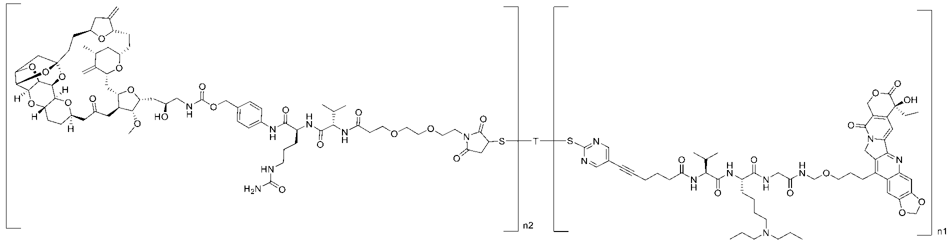

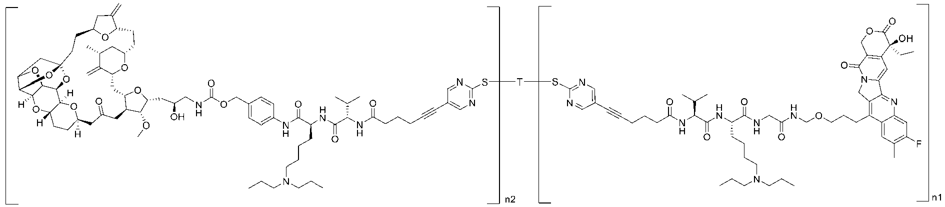

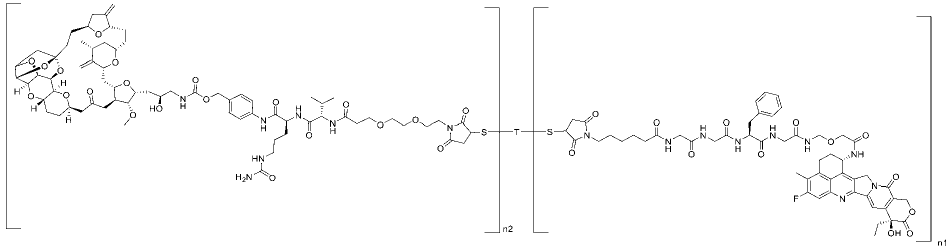

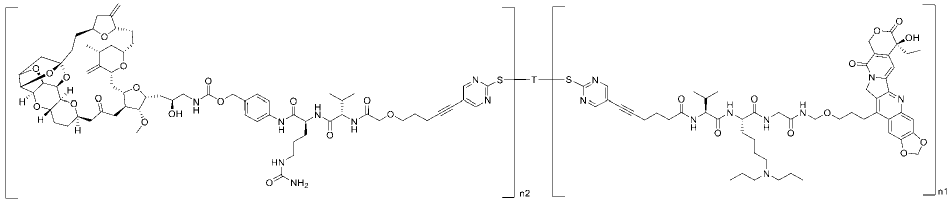

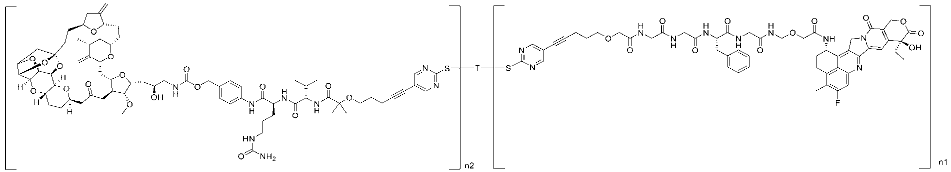

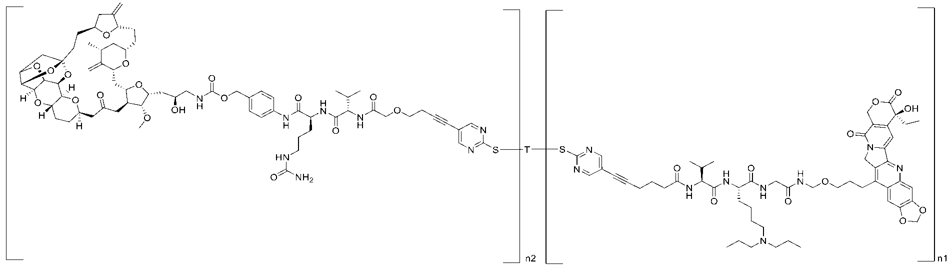

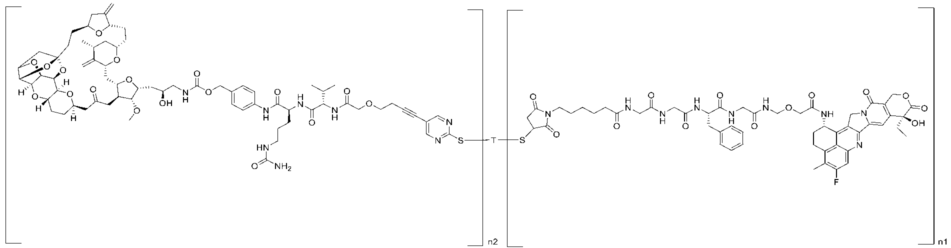

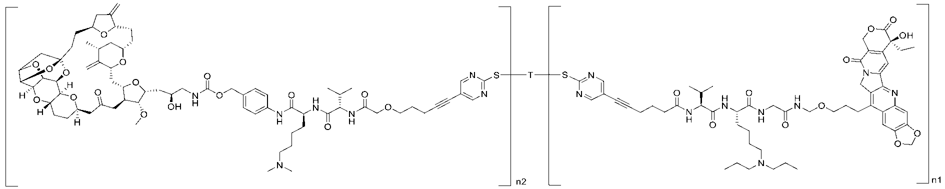

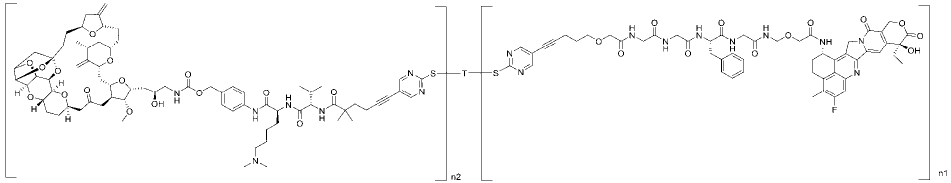

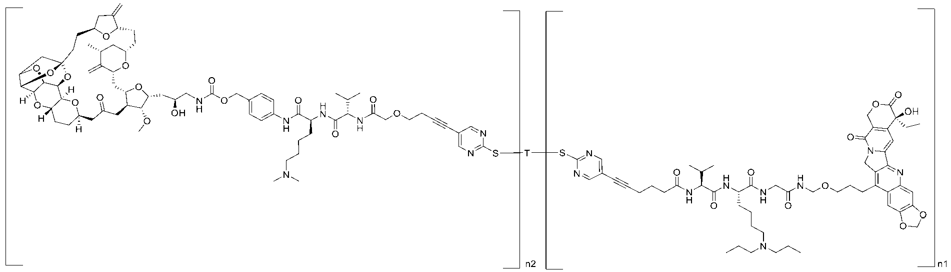

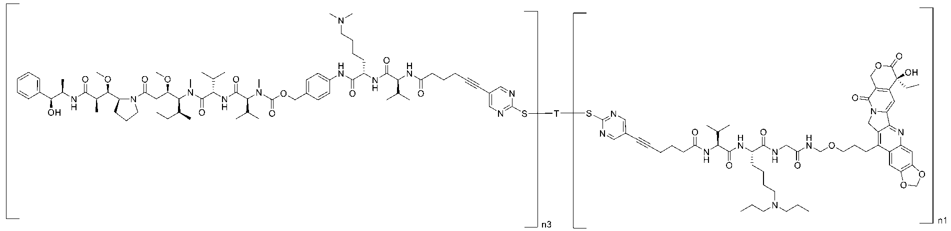

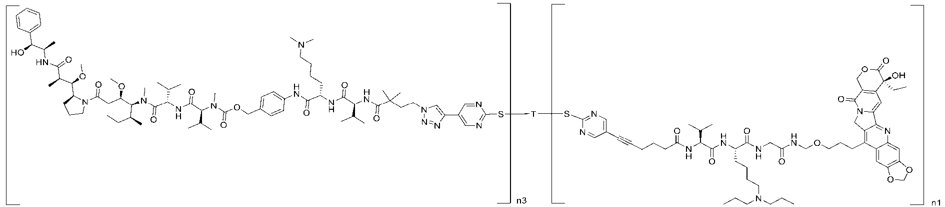

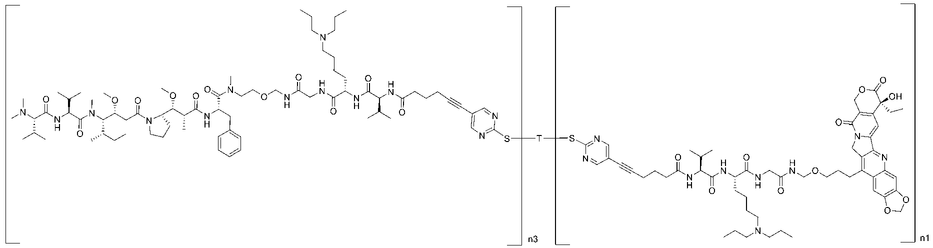

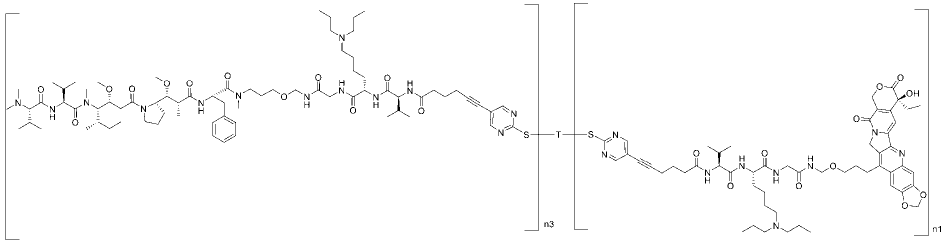

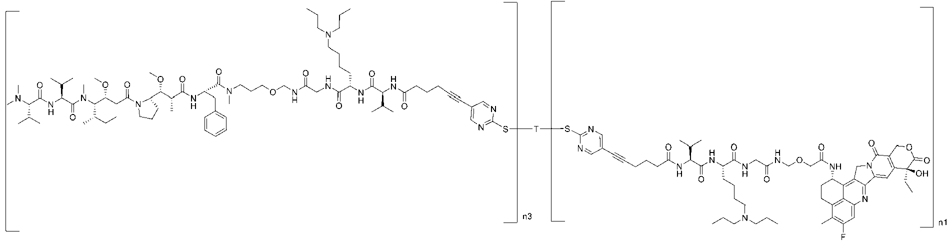

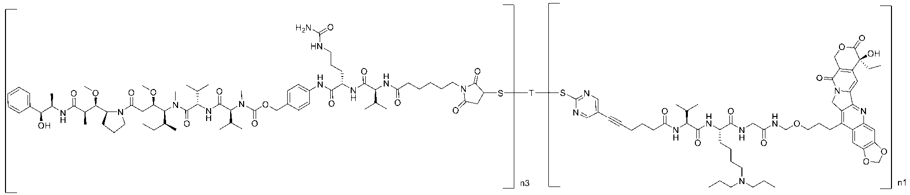

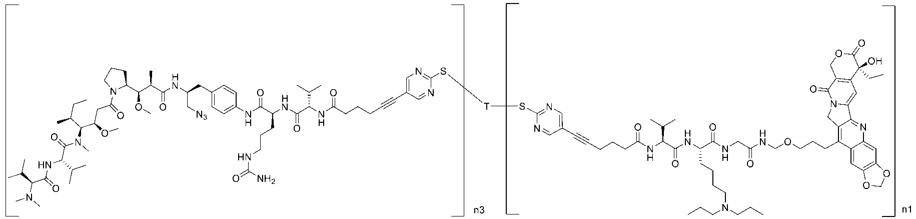

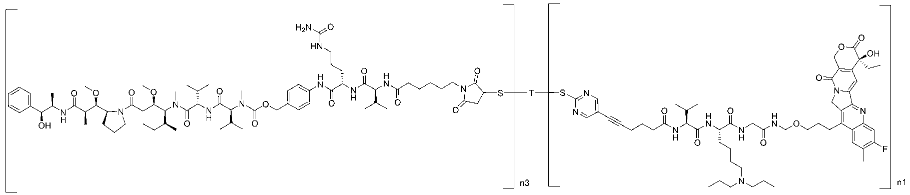

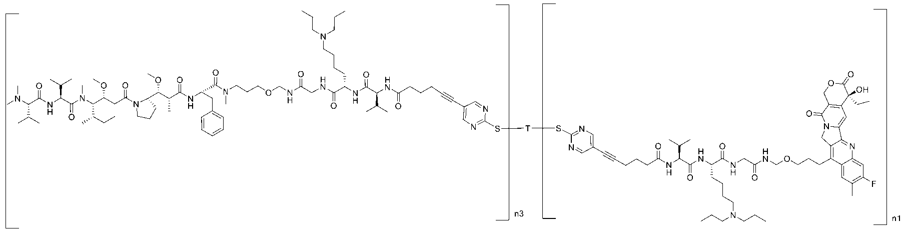

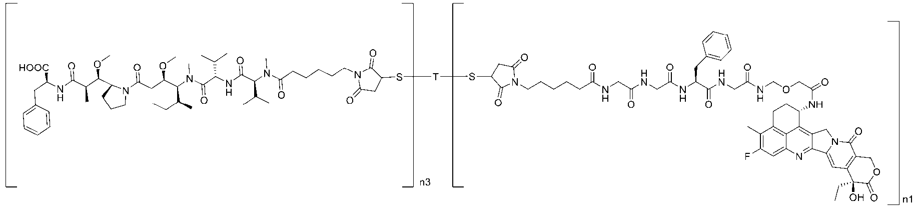

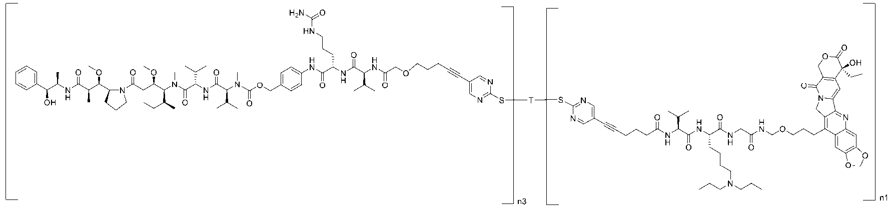

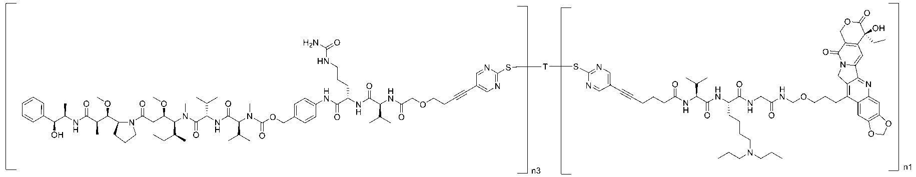

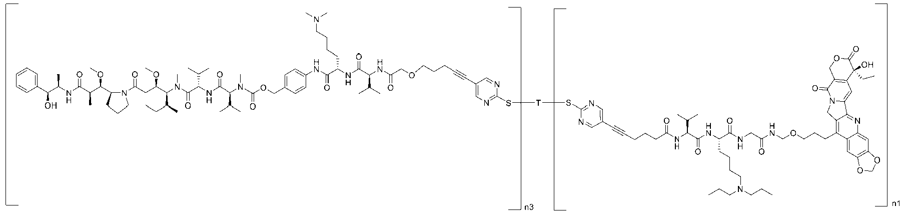

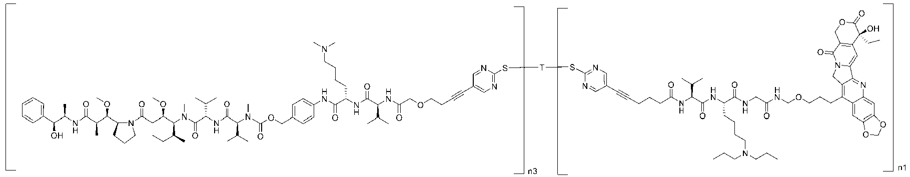

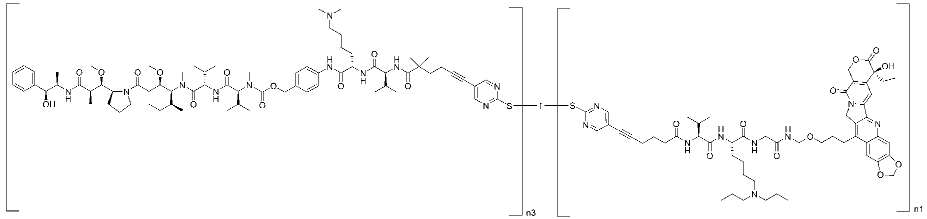

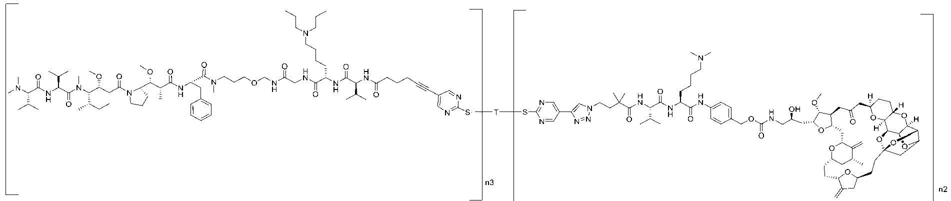

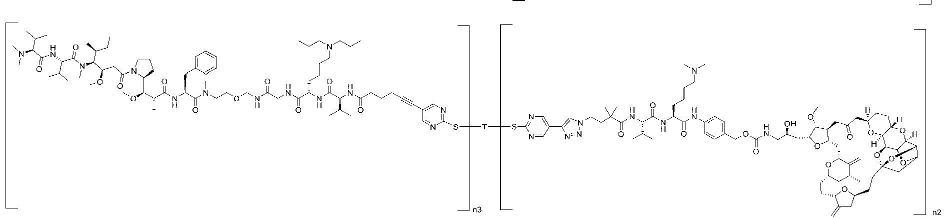

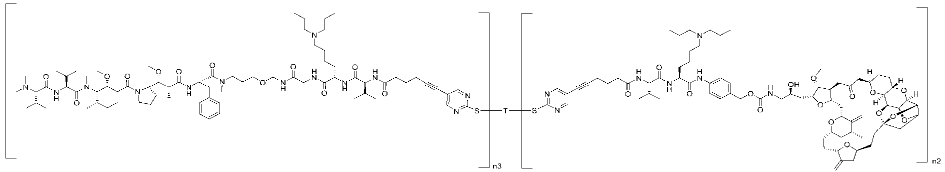

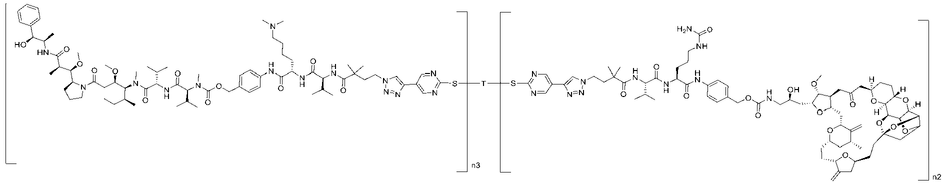

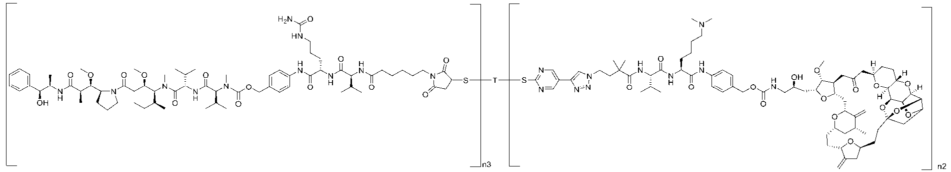

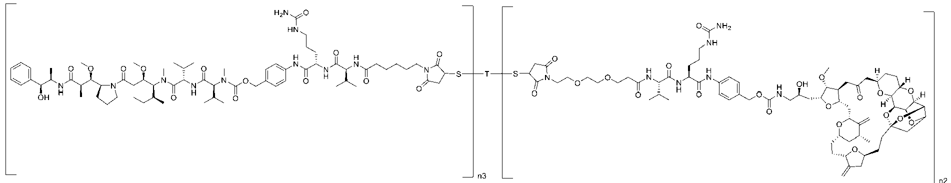

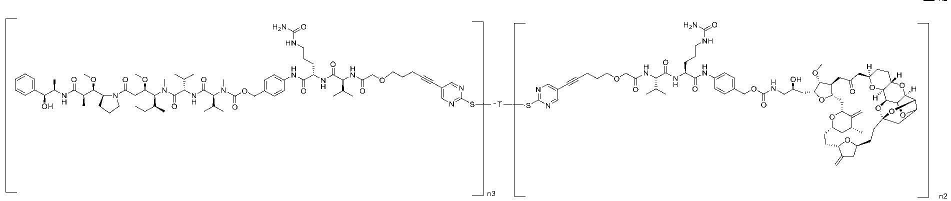

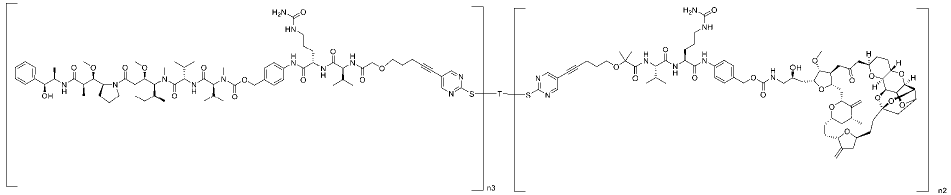

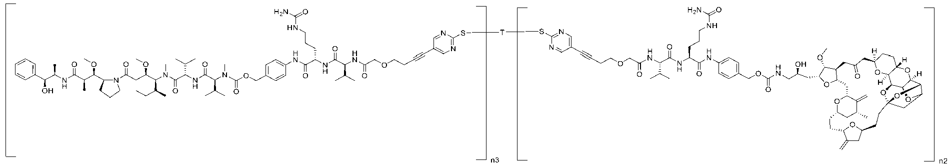

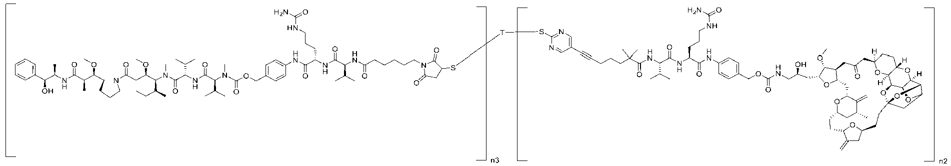

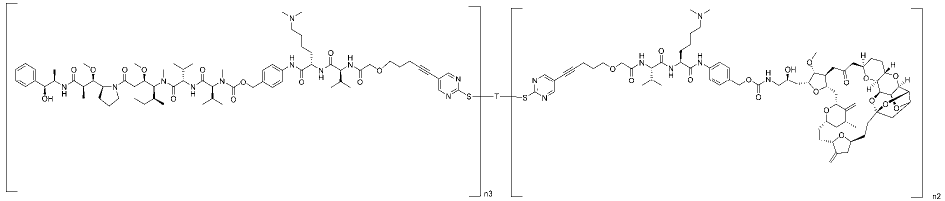

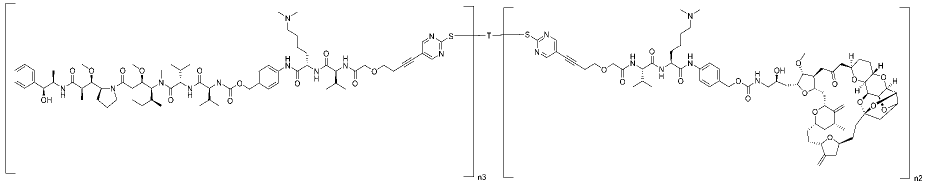

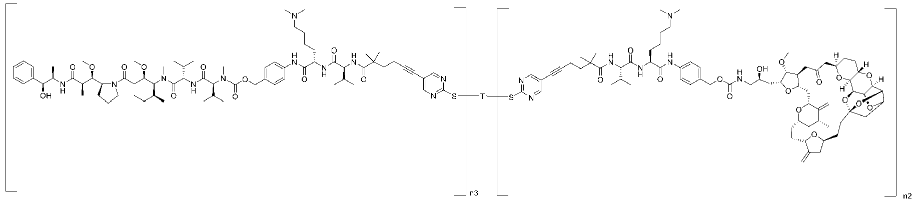

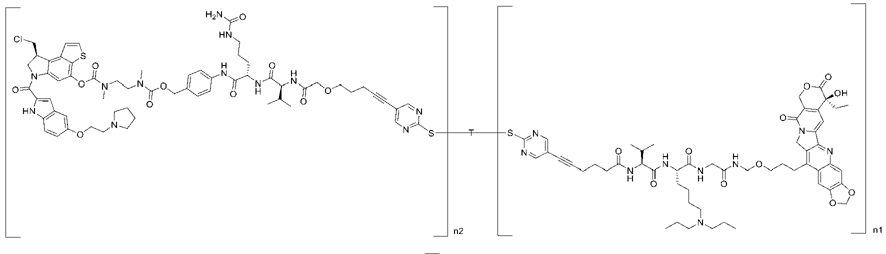

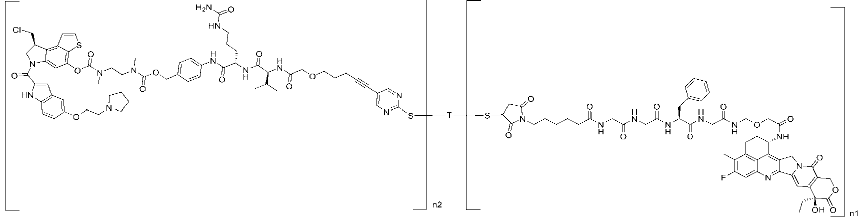

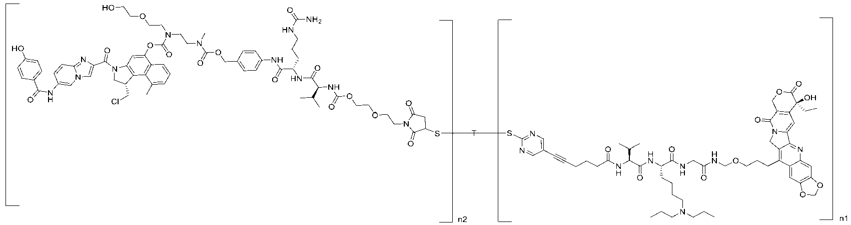

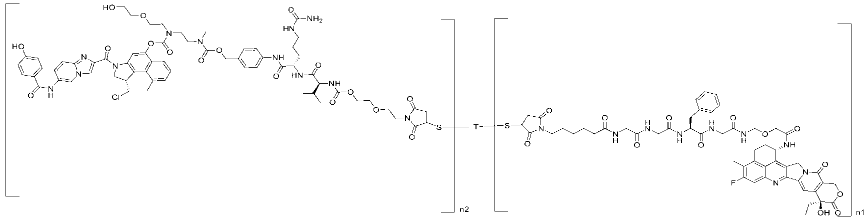

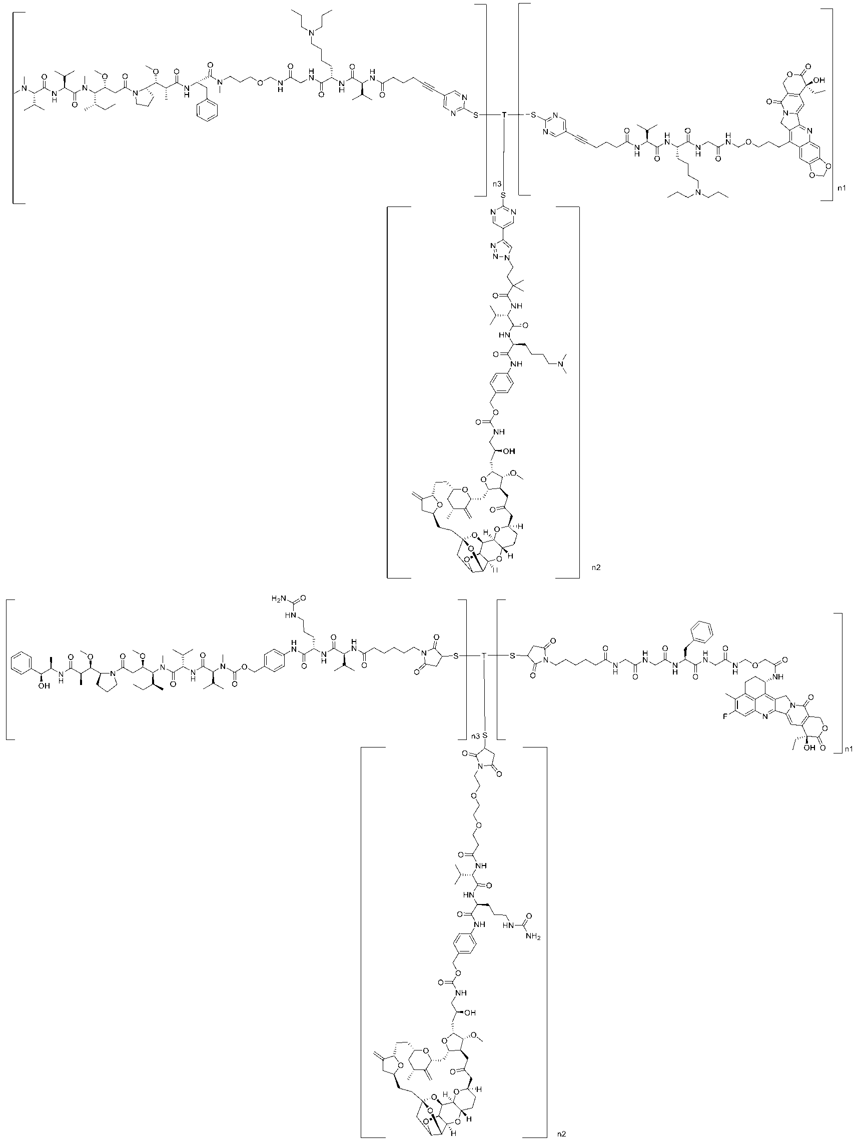

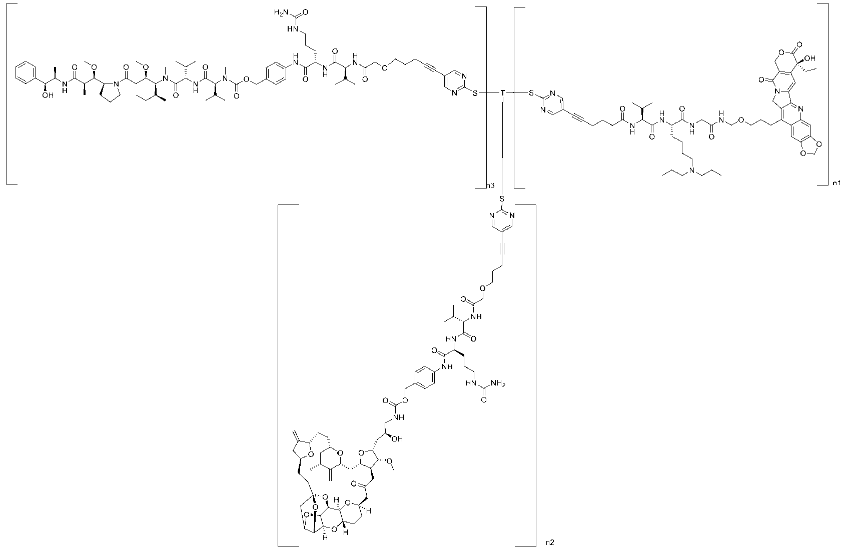

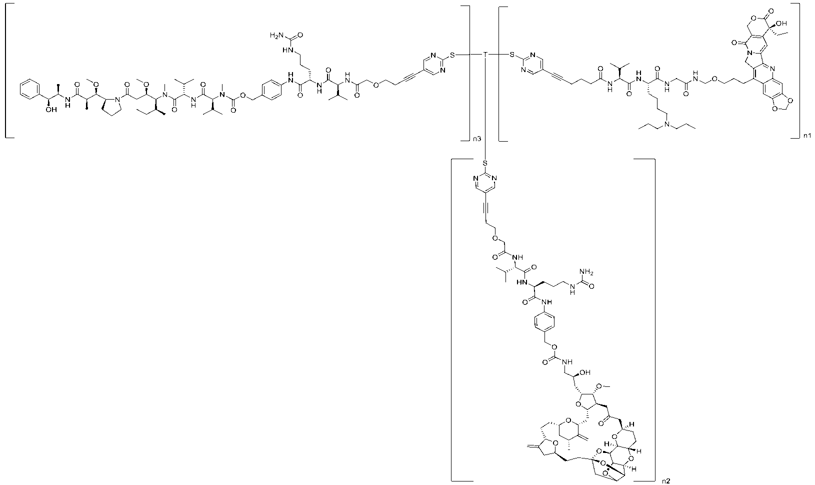

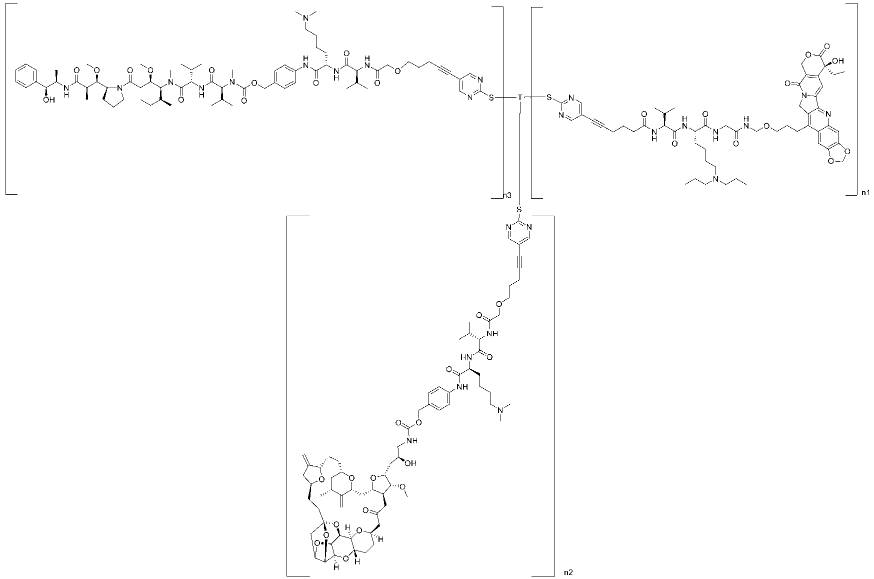

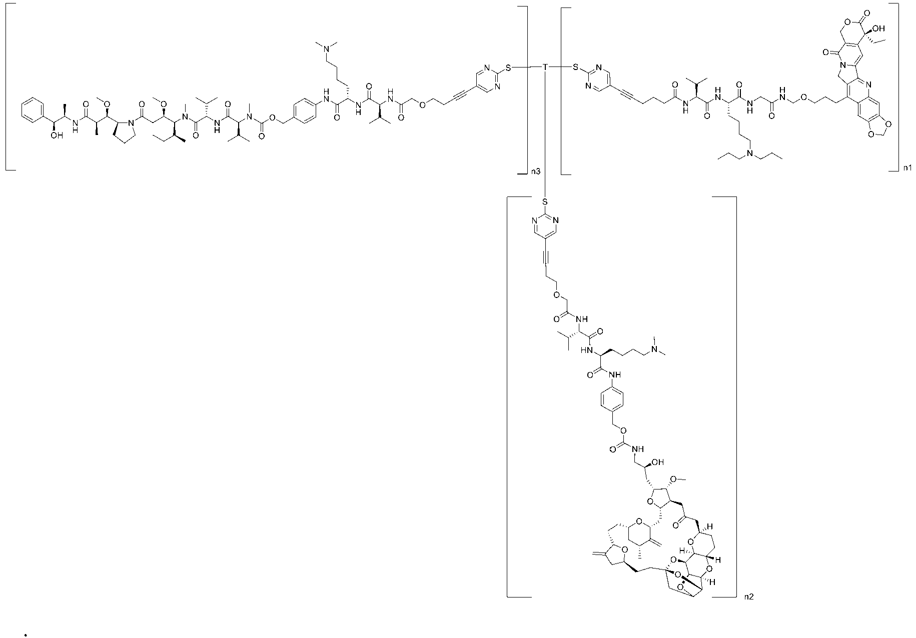

In some embodiments, the multi-warhead antibody-drug conjugate of formula I is selected from: 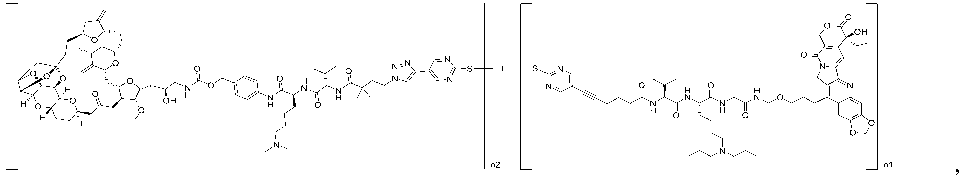 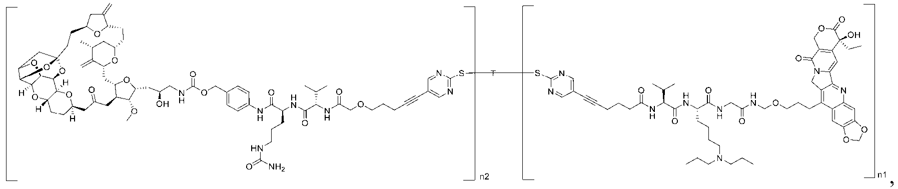 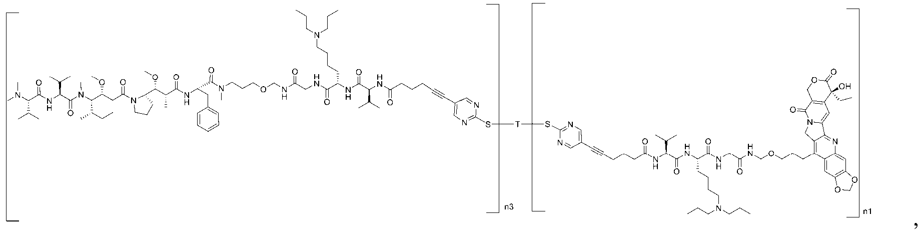 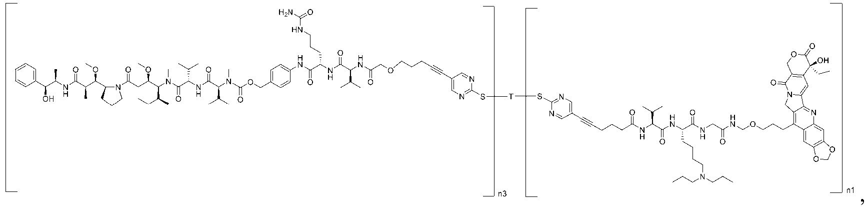 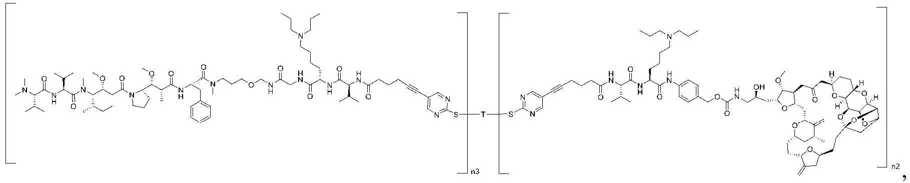 and 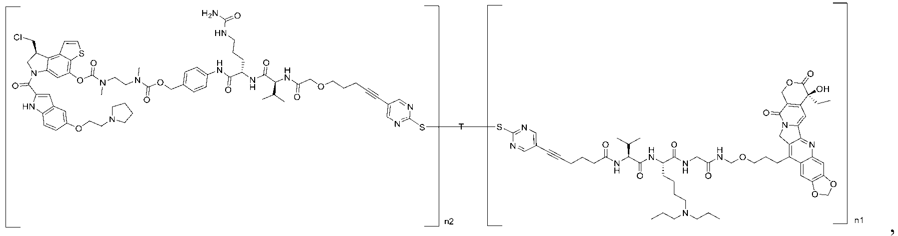 wherein T is an anti-Her 2 antibody, for example, trastuzumab or pertuzumab, preferably trastuzumab. In some embodiments, the multi-warhead antibody-drug conjugate of formula I is selected from: 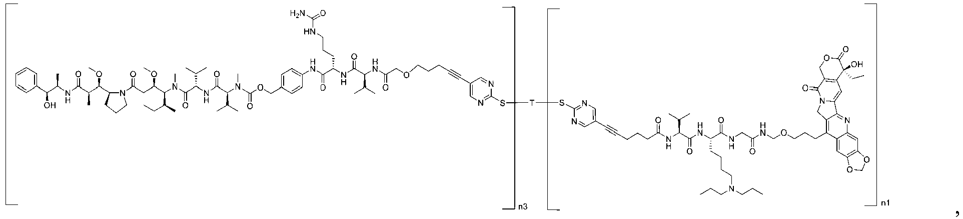 wherein T is an anti-B7H3 antibody, for example, 1D1, 1D1-01, 2E3, or 2E3-02 antibody, enoblituzumab, mirzotamab, or omburtamab.

The present disclosure further provides a preparation method for the multi-warhead antibody-drug conjugate of formula I or the stereoisomer of the multi-warhead antibody-drug conjugate, the prodrug thereof, the pharmaceutically acceptable salt thereof, or the pharmaceutically acceptable solvate thereof. It has been surprisingly found that the ratio of two or more toxins can be flexibly adjusted by the preparation method of the present disclosure.

The method comprises: subjecting a mixture of an antibody T(SH)n with an opened interchain disulfide bond and optionally Lg-L₁-D₁, Lg-L₂-D₂, and Lg-L₃-D₃ in a determined ratio to a conjugation reaction in a suitable solvent,
wherein:
when the linkers L₁, L₂, and L₃ comprise 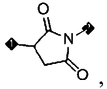 Lg-L₁, Lg-L₂, and Lg-L₃ comprise 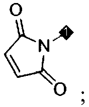
when the linkers L₁, L₂, and L₃ comprise 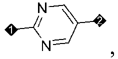 Lg is a leaving group when reacting with T(SH)n; preferably, Lg is selected from halogen, sulfonyl, tertiary amine salt groups (Me₃N⁺ and Et₃N⁺), diazonium salt groups, -OMs, MeSO₂-, and CF₃SO₃-; more preferably, Lg is selected from F and MeSO₂-;
L₁, D₁, L₂, D₂, L₃, D₃, n1, n2, and n3 are as defined in any one of the embodiments of the present disclosure. In some embodiments, the conjugation reaction is performed in water and/or an organic solvent.

In some embodiments, the organic solvent is selected from N,N-dimethylformamide, dimethyl sulfoxide, N-methylpyrrolidone, nitriles (for example, acetonitrile), alcohols (for example, methanol and ethanol), and any combination thereof.

In some embodiments, the method further comprises a step of purifying the conjugate product.

In some embodiments, the conjugate product is purified by chromatography.

In some embodiments, the chromatography comprises one or more of ion exchange chromatography, hydrophobic chromatography, reversed-phase chromatography, or affinity chromatography.

In some embodiments, the method comprises: subjecting a mixture of an antibody T(SH)n with an opened interchain disulfide bond and Lg-L₁-D₁ and Lg-L₂-D₂ in a determined ratio to a conjugation reaction in a suitable solvent, wherein L₁, D₁, L₂, D₂, Lg, and T are as defined in any one of the embodiments of the present disclosure.

In some embodiments, the determined ratio of Lg-L₁-D₁ to Lg-L₂-D₂ is 1, 1.5, 2, 2.5, 3, 3.5, 4, 4.5, 5, 5.5, 6, 6.5, or 7.5, preferably 1.5, 2, 2.5, 3, 3.5, 4, 4.5, 5, 5.5, 6, 6.5, or 7.5.

In some embodiments, the method comprises: adding Lg-L₁-D₁ and Lg-L₂-D₂ in a determined ratio, sequentially or simultaneously, to an antibody T(SH)n with an opened interchain disulfide bond in a suitable solvent, and performing a conjugation reaction.

In some embodiments, the method comprises: subjecting a mixture of an antibody T(SH)n with an opened interchain disulfide bond and Lg-L₁-D₁ and Lg-L₃-D₃ in a determined ratio to a conjugation reaction in a suitable solvent, wherein L₁, D₁, L₃, D₃, Lg, and T are as defined in any one of the embodiments of the present disclosure.

In some embodiments, the determined ratio of Lg-L₁-D₁ to Lg-L₃-D₃ is 1, 1.5, 2, 2.5, 3, 3.5, 4, 4.5, 5, 5.5, 6, 6.5, or 7.5, preferably 1.5, 2, 2.5, 3, 3.5, 4, 4.5, 5, 5.5, 6, 6.5, or 7.5.

In some embodiments, the method comprises: adding Lg-L₁-D₁ and Lg-L₃-D₃ in a determined ratio, sequentially or simultaneously, to an antibody T(SH)n with an opened interchain disulfide bond in a suitable solvent, and performing a conjugation reaction.

In some embodiments, Lg-L₁-D₁, Lg-L₂-D₂, and Lg-L₃-D₃ are selected from optional compounds shown below: 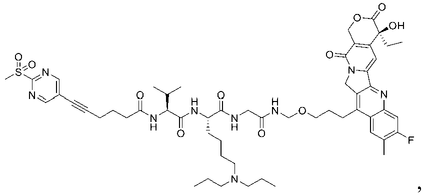 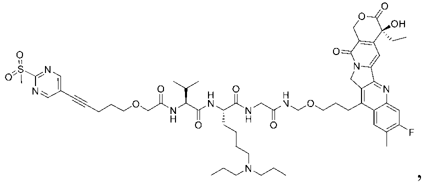 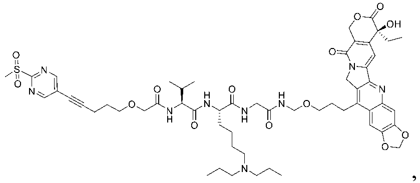 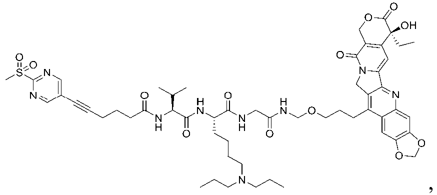 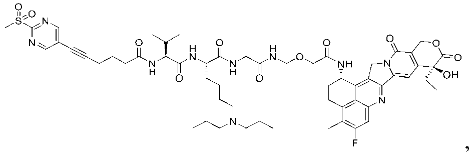 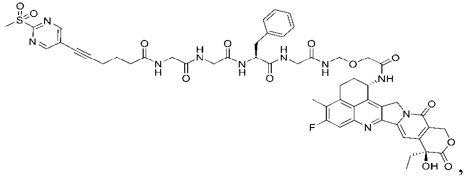 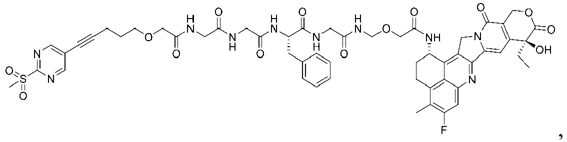 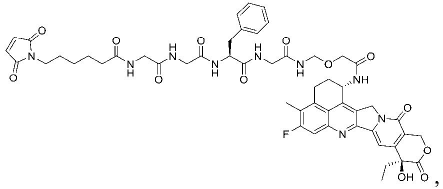 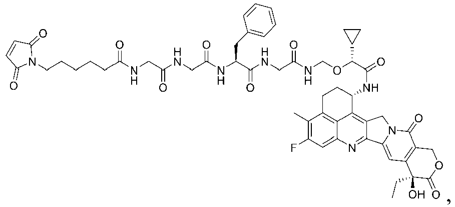 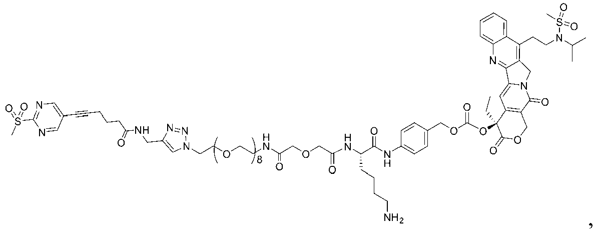 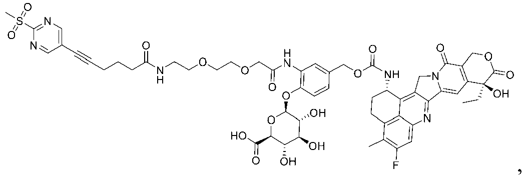 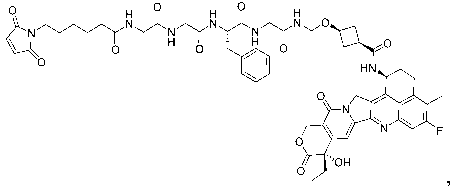 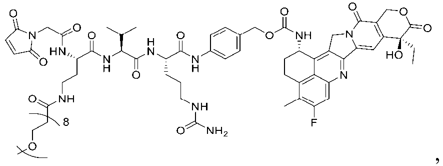 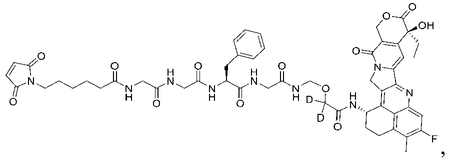 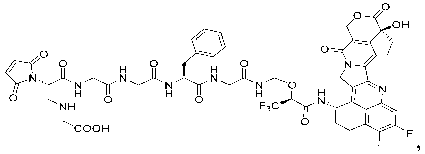 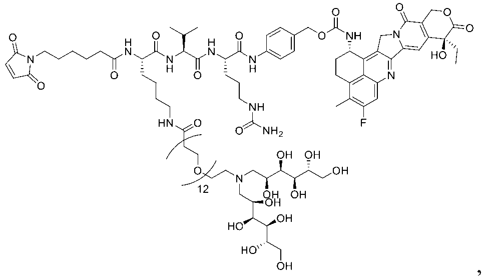 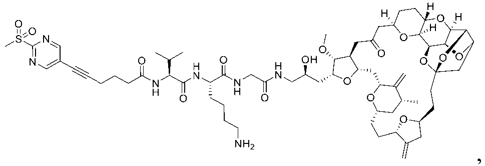 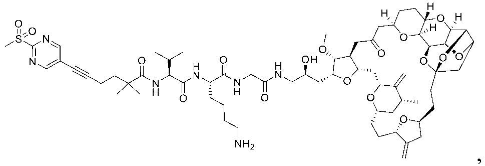 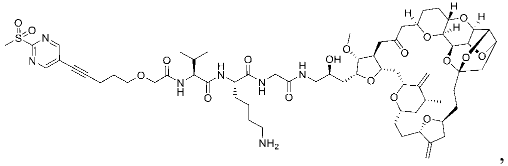 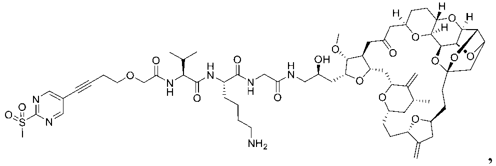 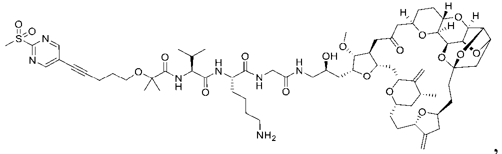 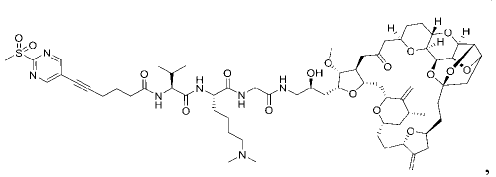 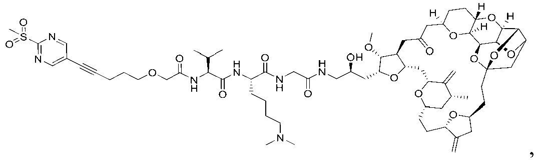 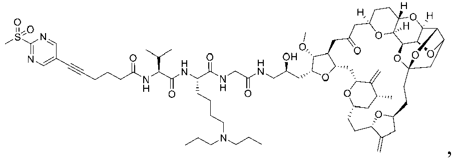 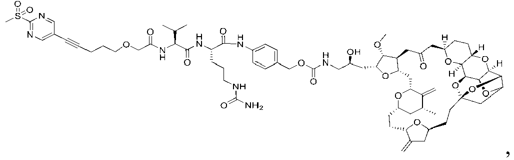 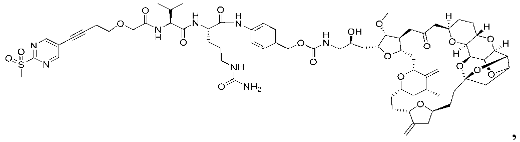 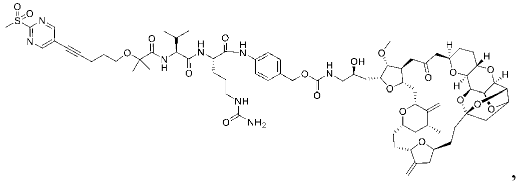 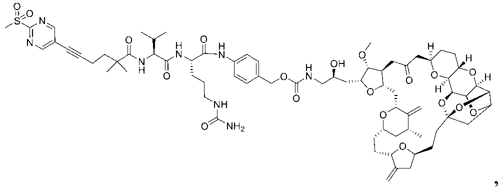 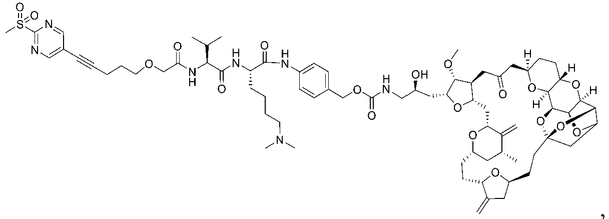 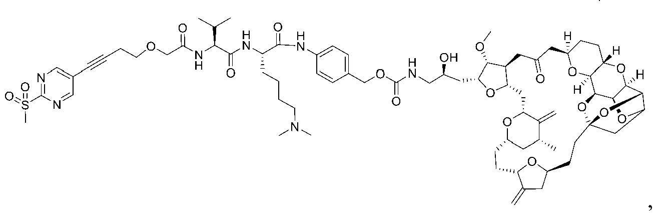 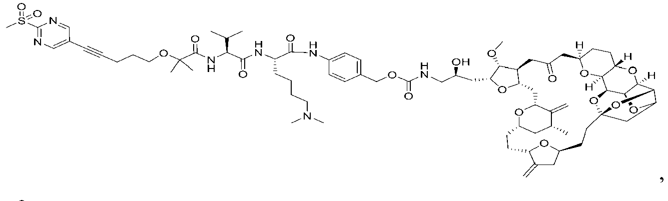 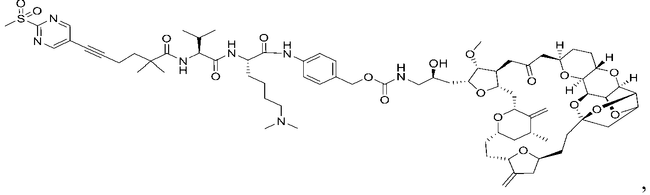 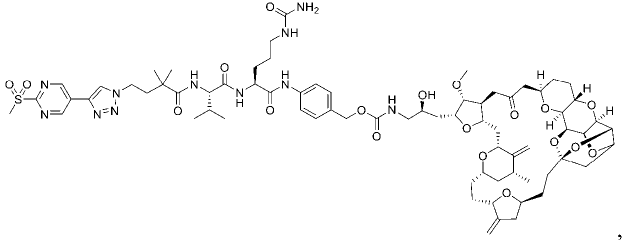 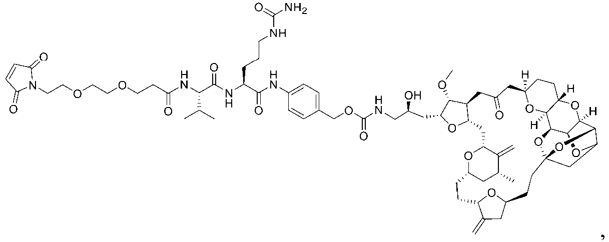 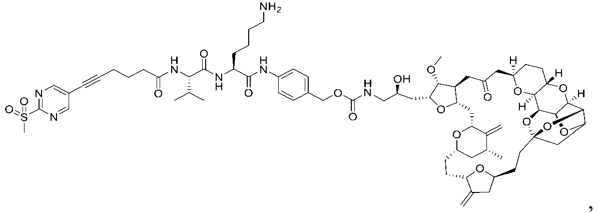 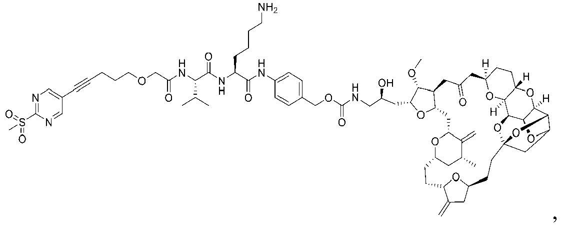 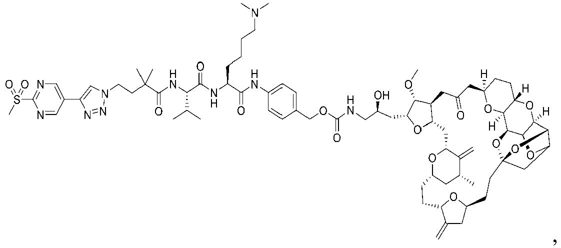 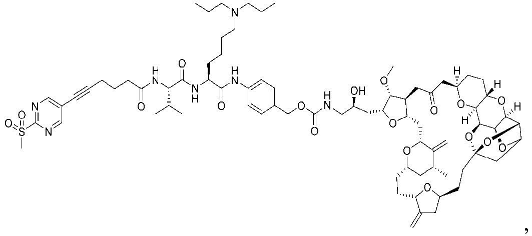 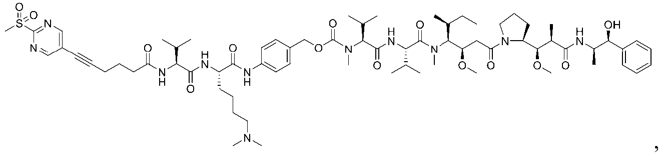 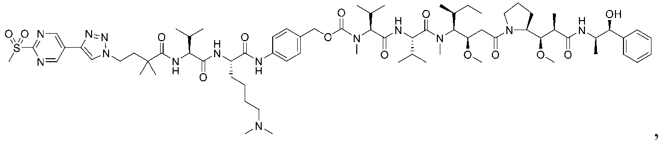 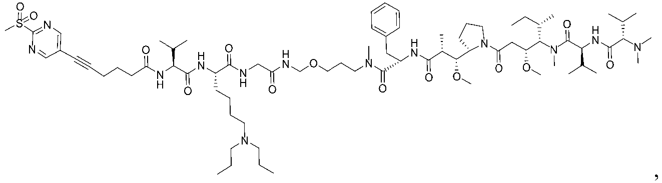 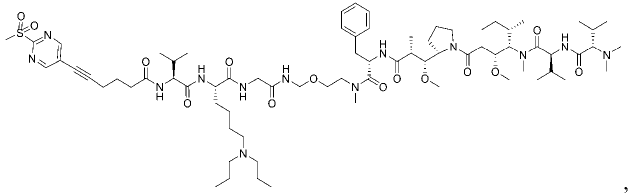 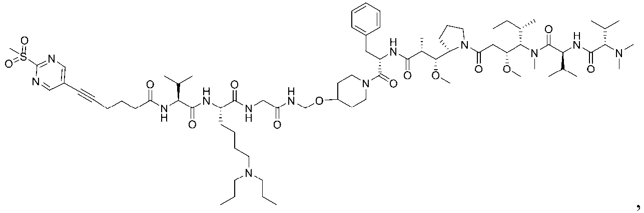 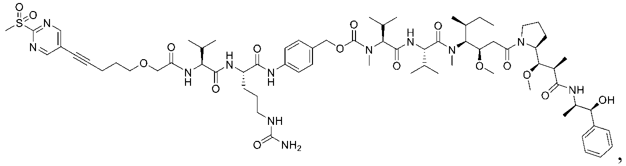 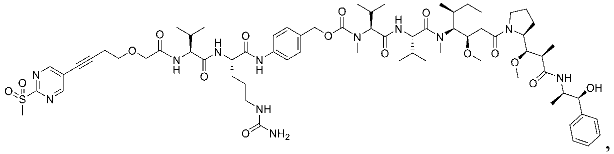 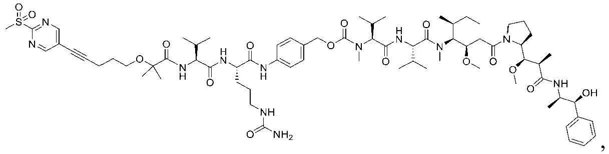 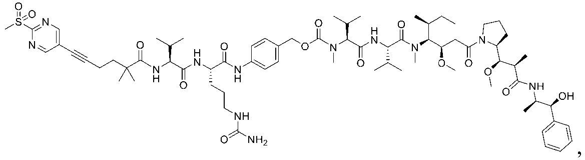 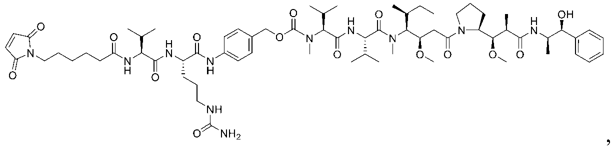 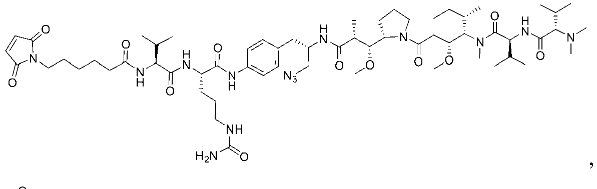 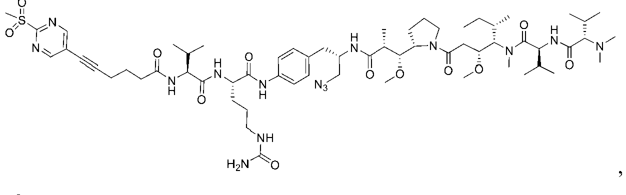 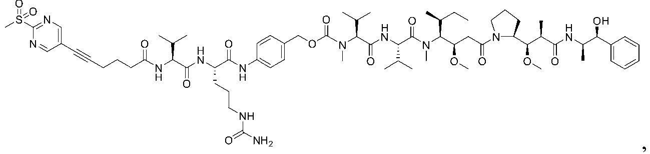 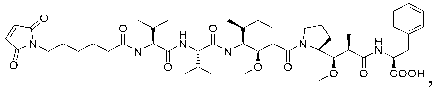 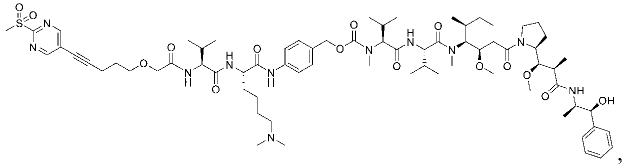 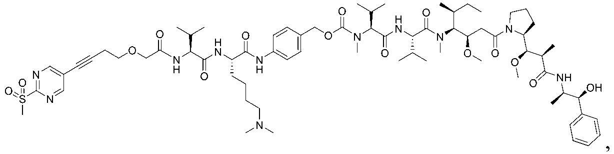 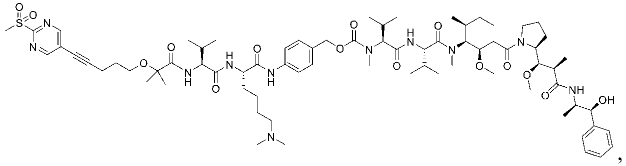 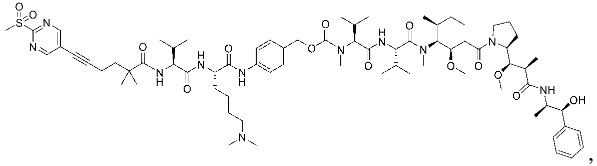 and

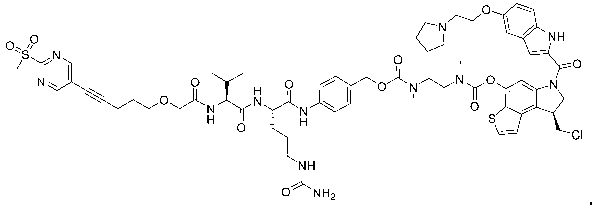

In some embodiments, Lg-L₁-D₁ is selected from optional compounds shown below: 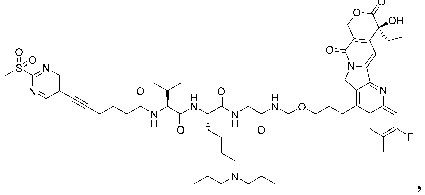 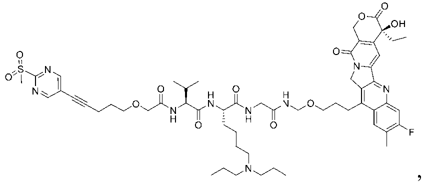 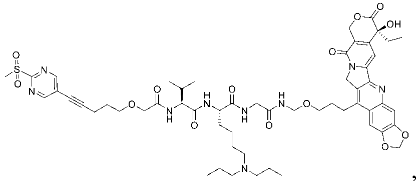 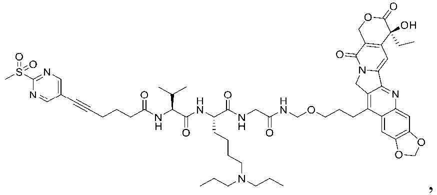 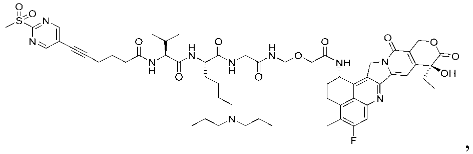 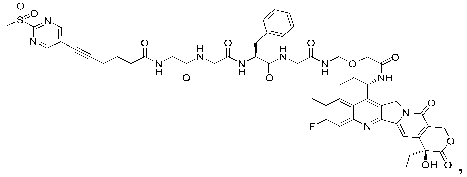 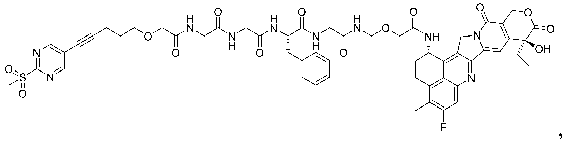 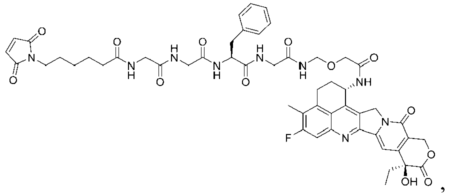 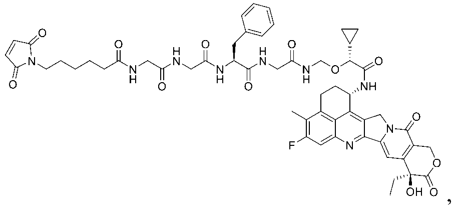 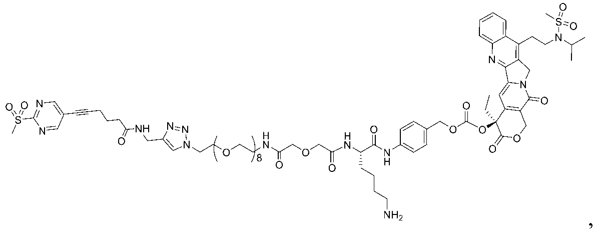 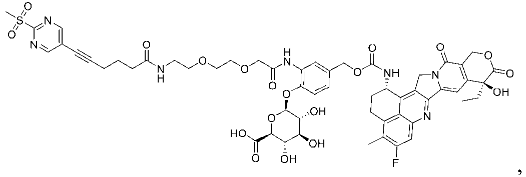 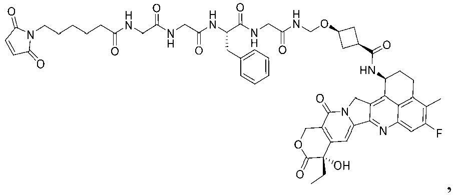 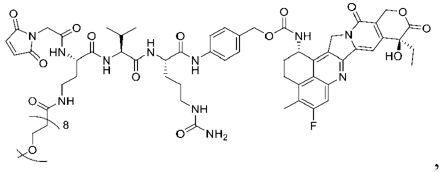 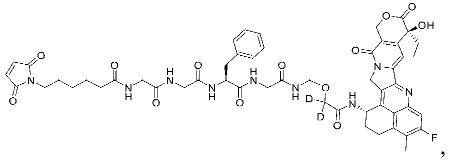 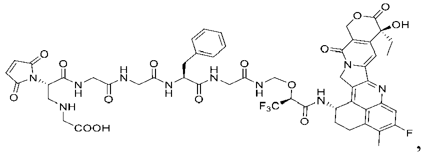 and

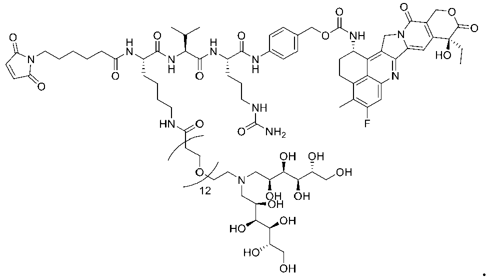

In some embodiments, Lg-L₁-D₁ is selected from optional compounds shown below: 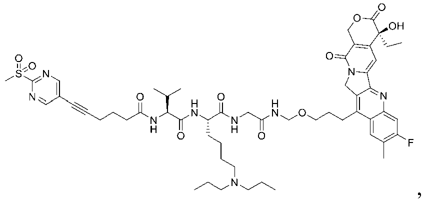 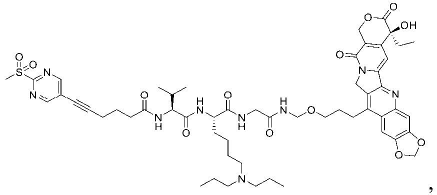 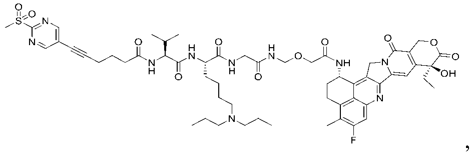 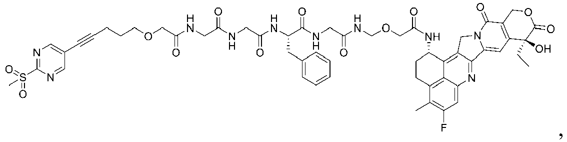 and

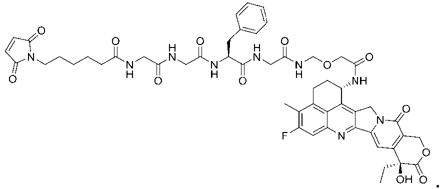

In some embodiments, Lg-L₁-D₁ is selected from optional compounds shown below: 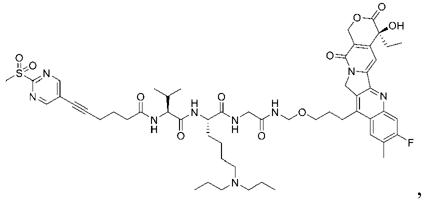 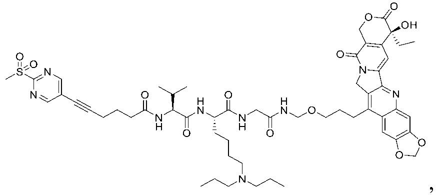 and

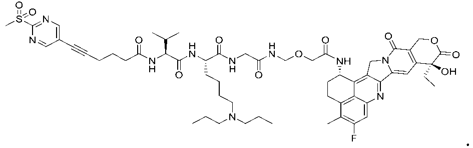

In some embodiments, Lg-L₁-D₁ is

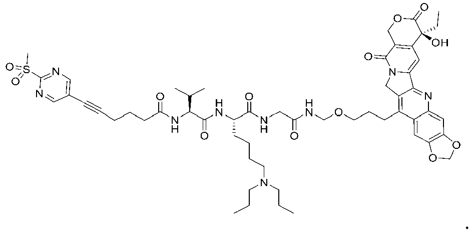

In some embodiments, Lg-L₂-D₂ is selected from optional compounds shown below: 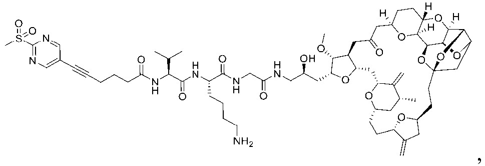 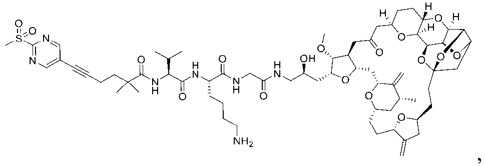 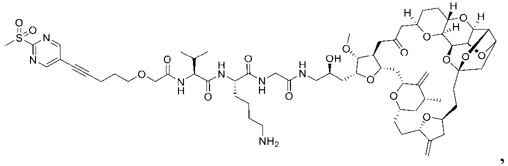 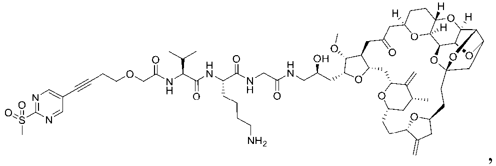 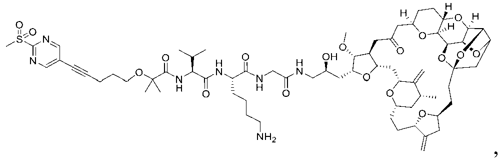 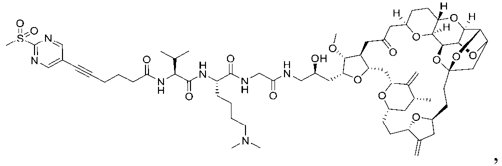 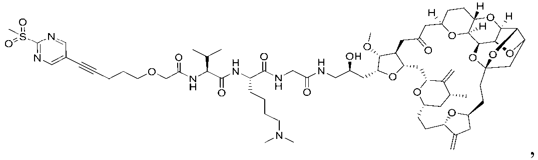 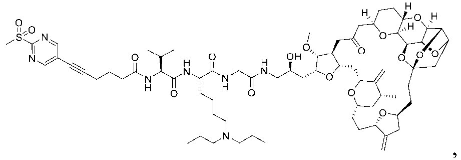 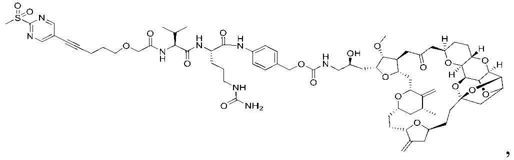 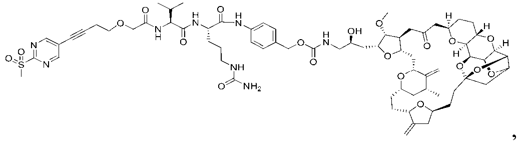 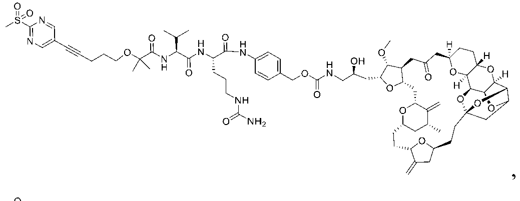 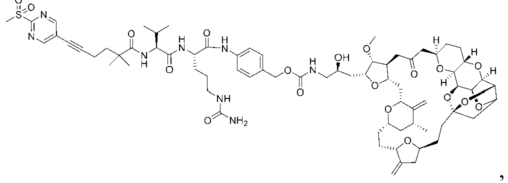 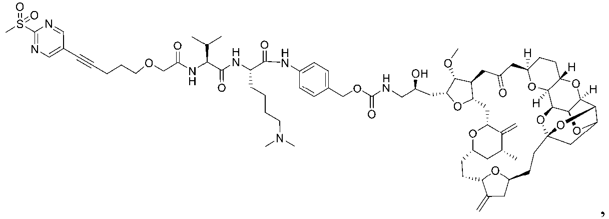 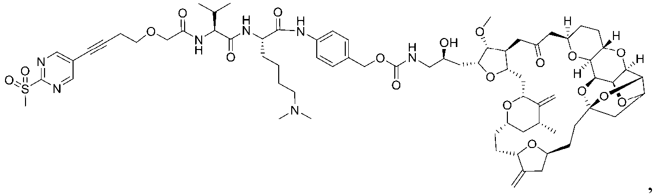 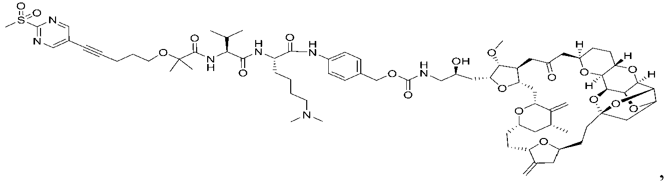 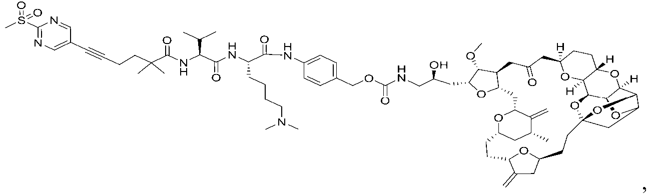 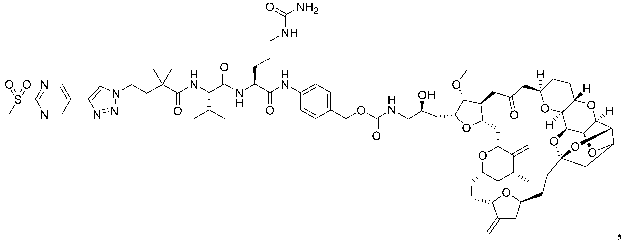 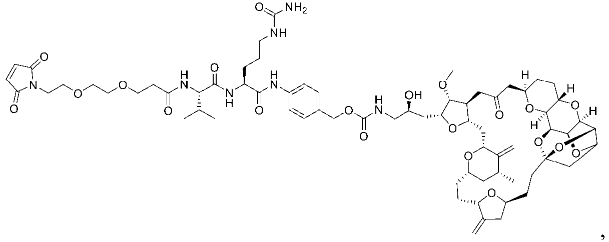 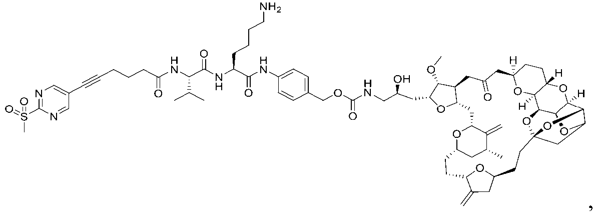 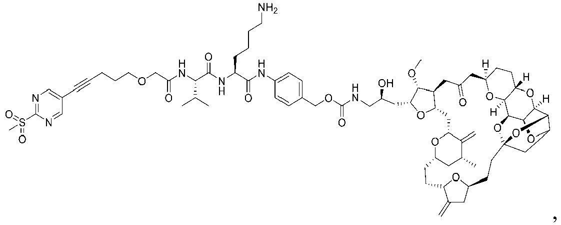 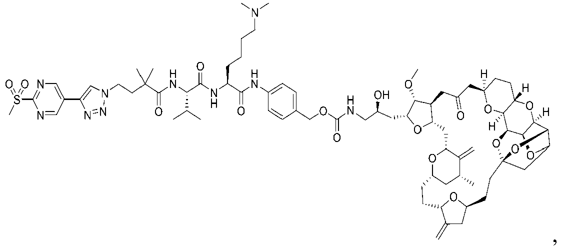 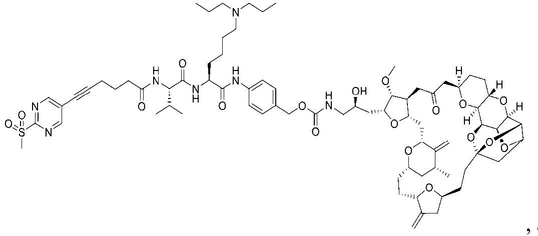 and

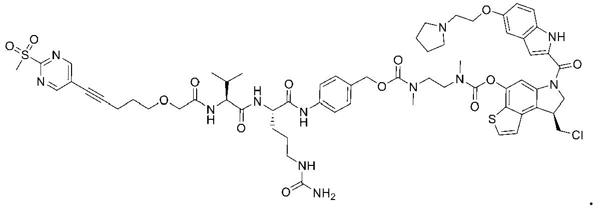

In some embodiments, Lg-L₂-D₂ is selected from optional compounds shown below:

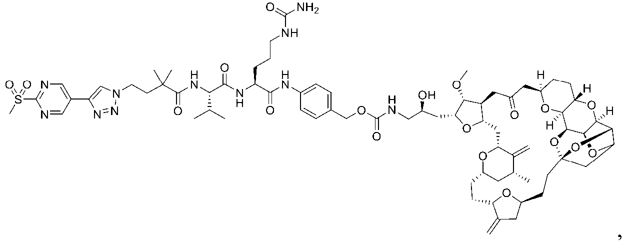

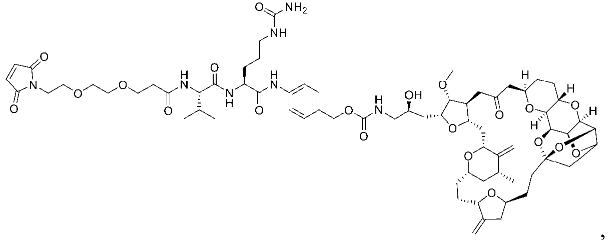

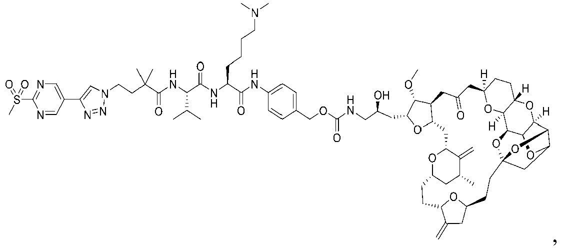

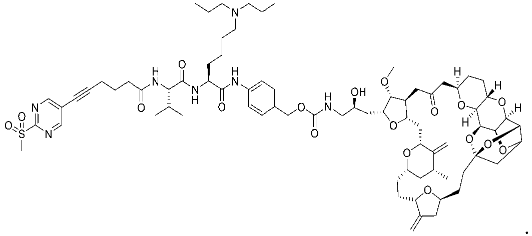

In some embodiments, Lg-L₂-D₂ is

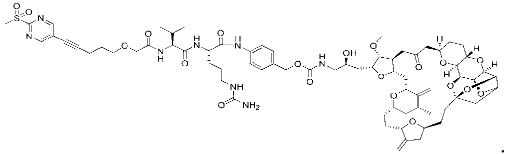

In some embodiments, Lg-L₂-D₂ is

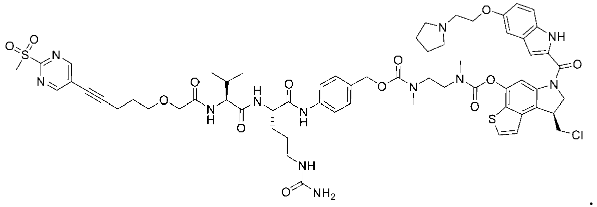

In some embodiments, Lg-L₃-D₃ is selected from optional compounds shown below: 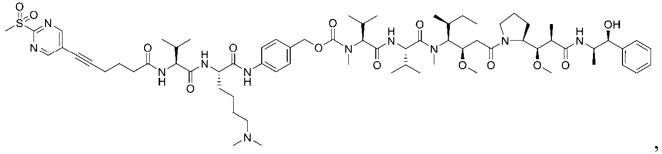 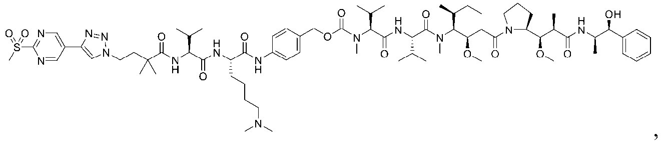 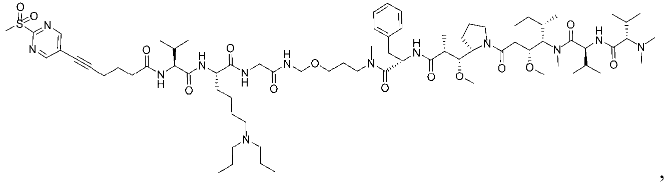 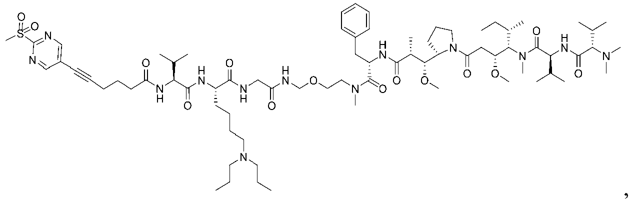 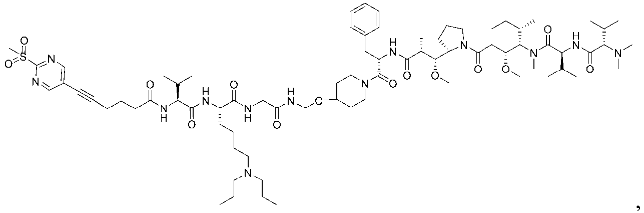 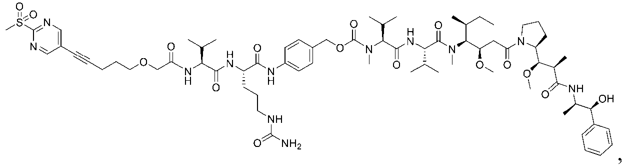 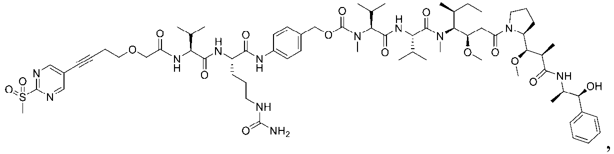 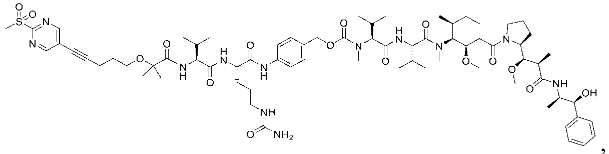 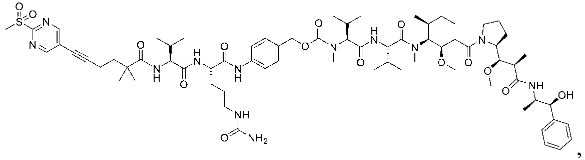 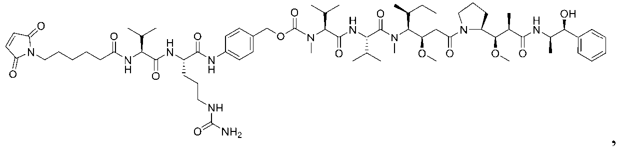 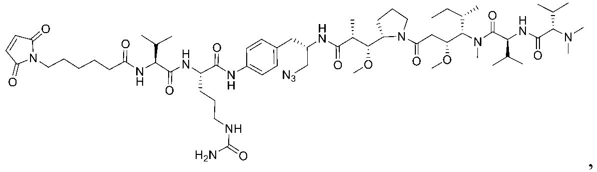 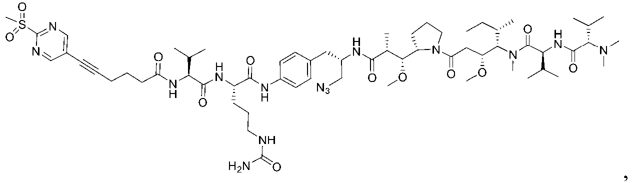 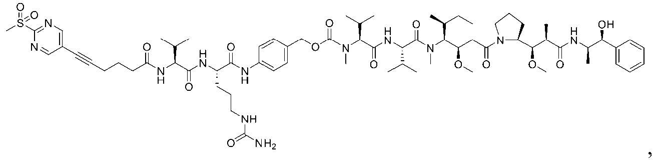 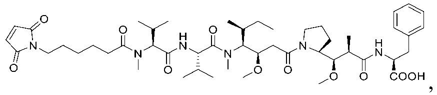 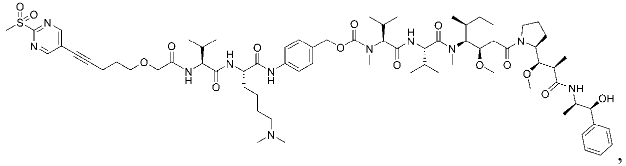 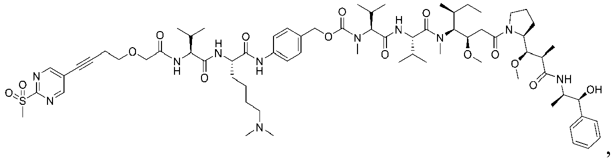 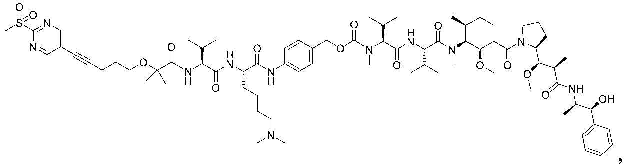 and

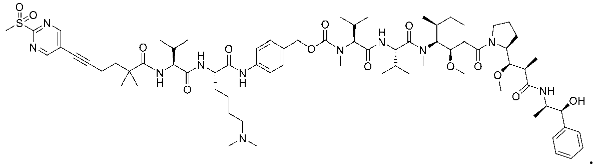

In some embodiments, Lg-L₃-D₃ is selected from optional compounds shown below: 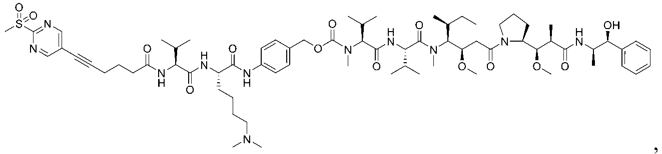 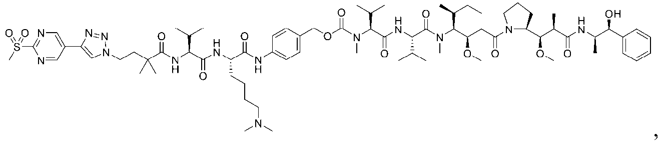 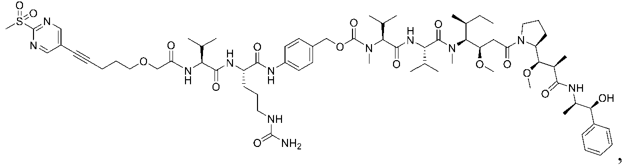 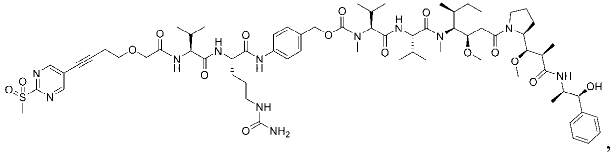 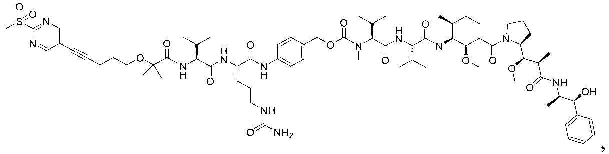 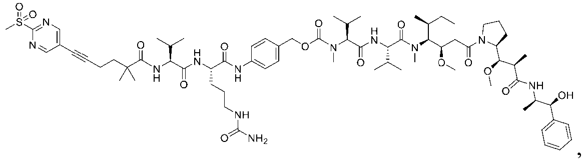 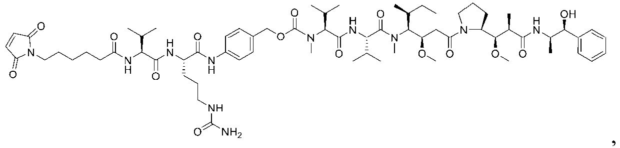 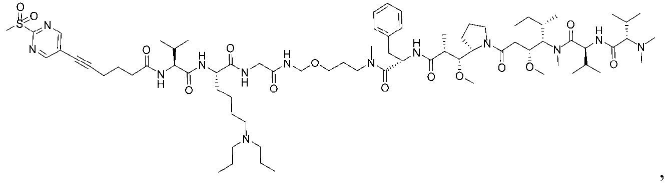 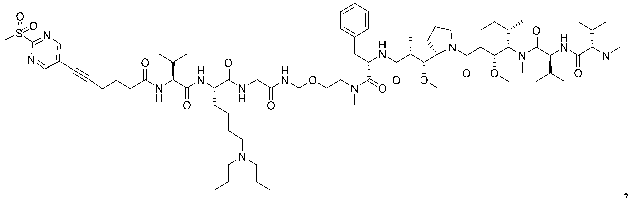 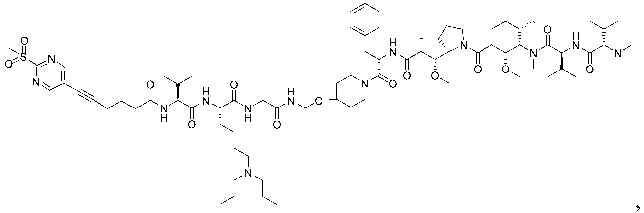 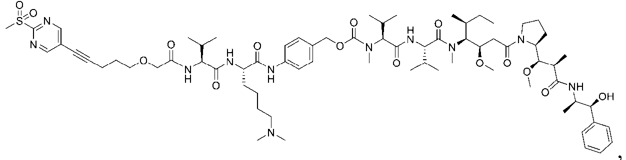 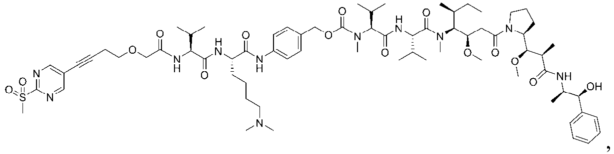 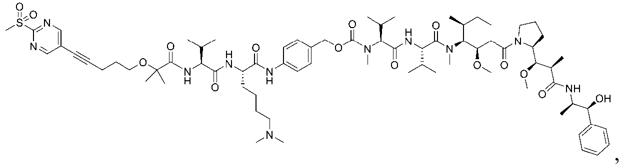 and

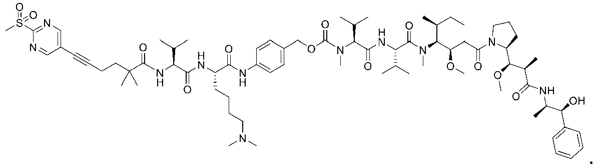

In some embodiments, Lg-L₃-D₃ is selected from optional compounds shown below:

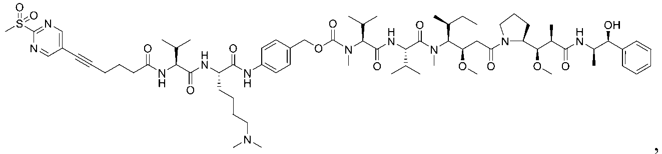

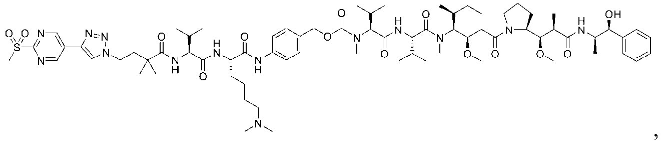

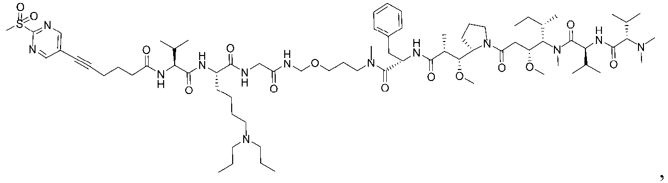

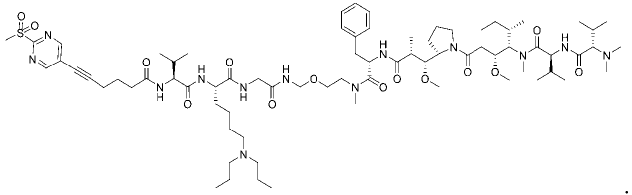

In some embodiments, Lg-L₃-D₃ is

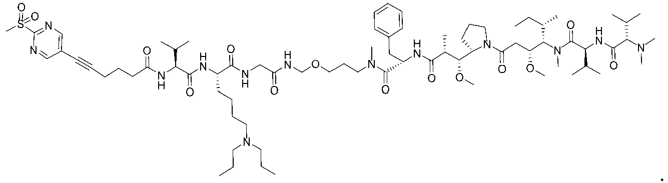

The present disclosure further provides a drug-linker conjugate or a stereoisomer of the drug-linker conjugate, a prodrug thereof, a pharmaceutically acceptable salt thereof, or a pharmaceutically acceptable solvate thereof, which has the following structures: 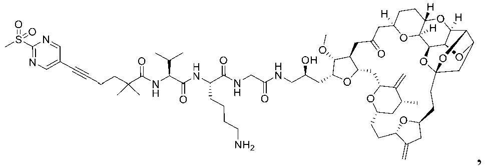 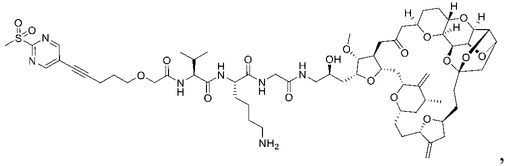 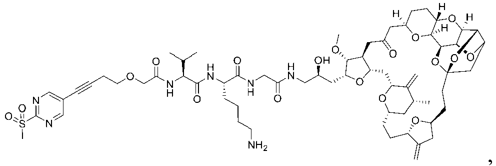 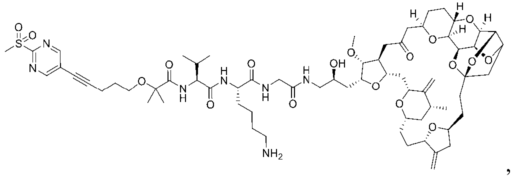 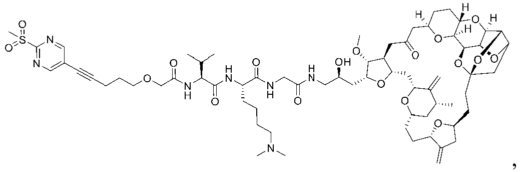 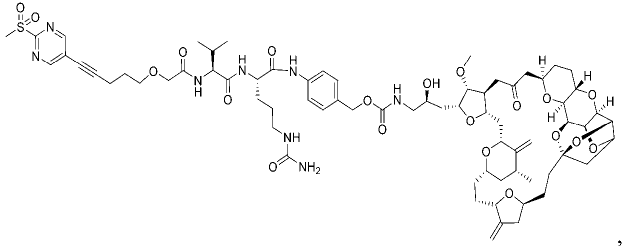 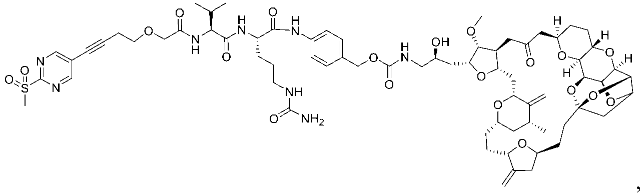 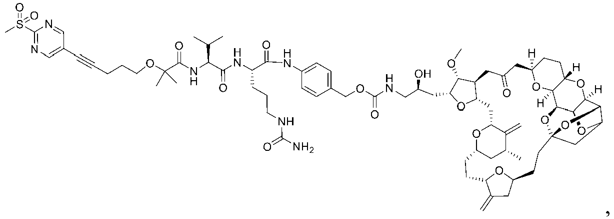 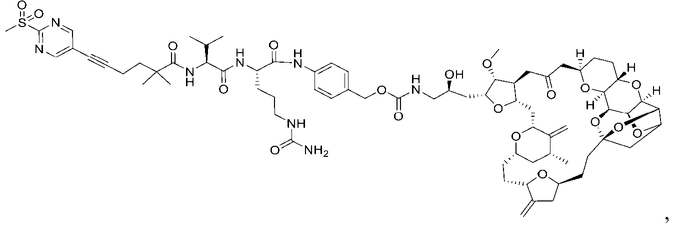 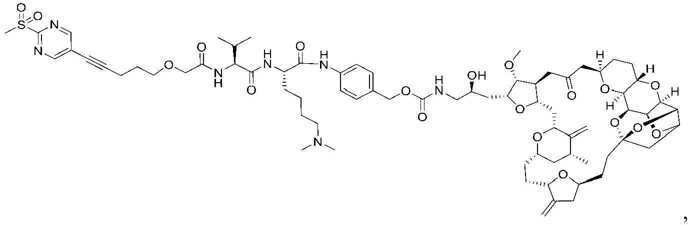 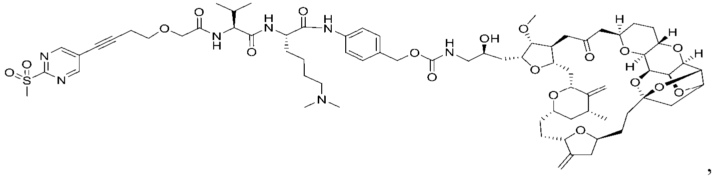 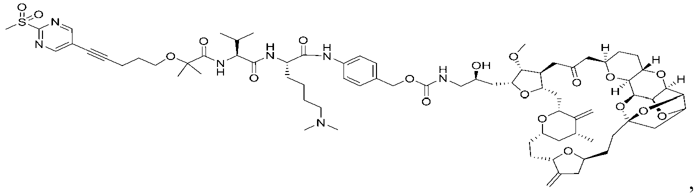 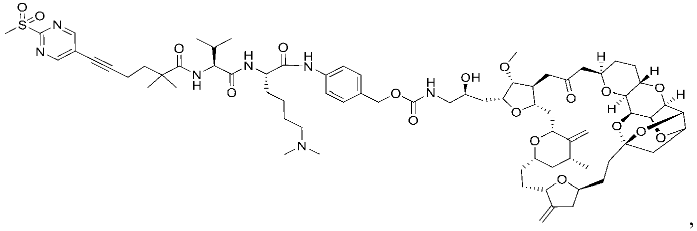 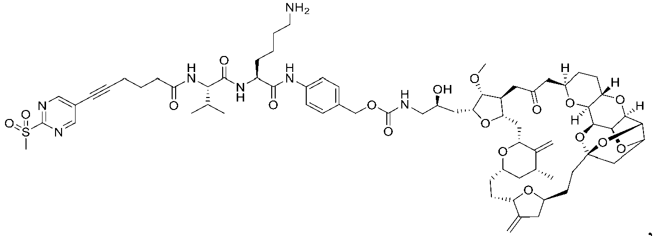 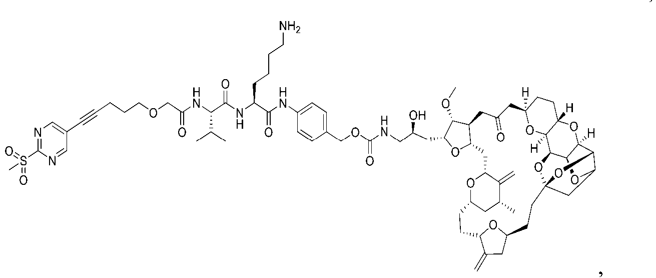 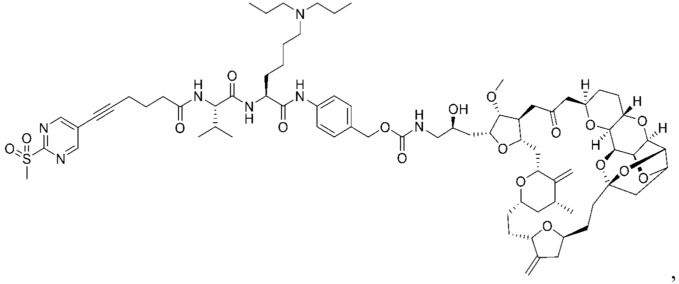 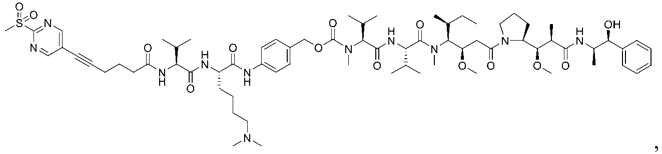 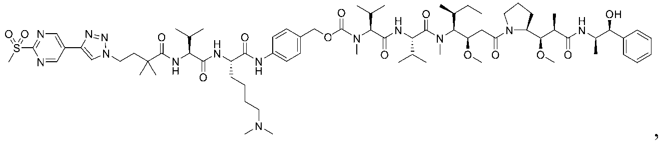 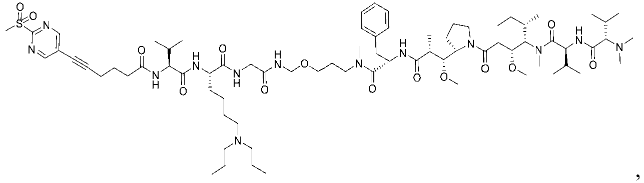 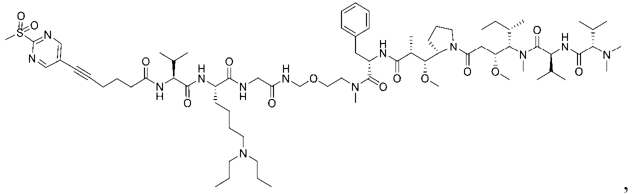 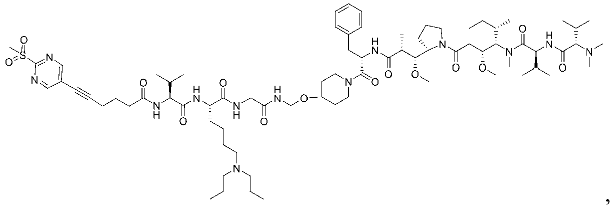 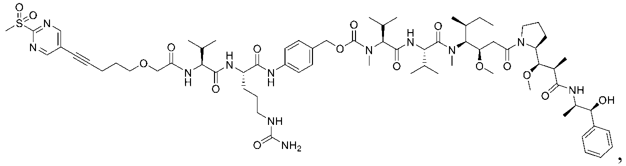 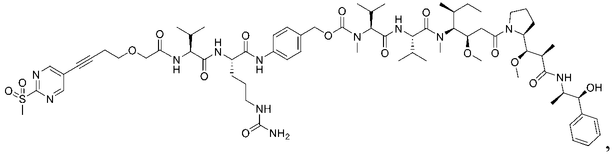 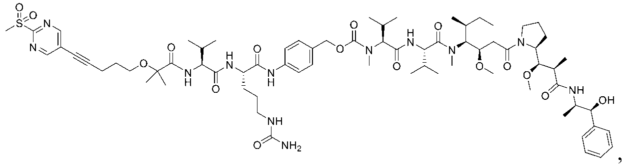 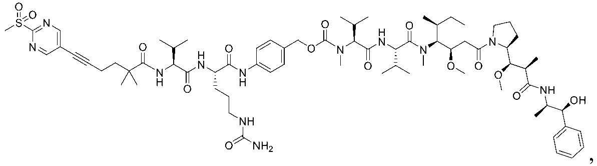 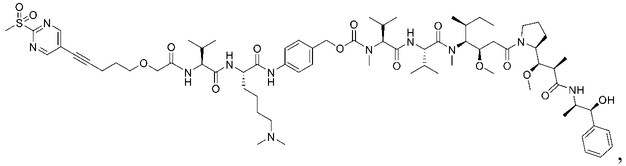 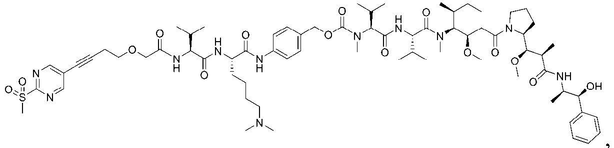 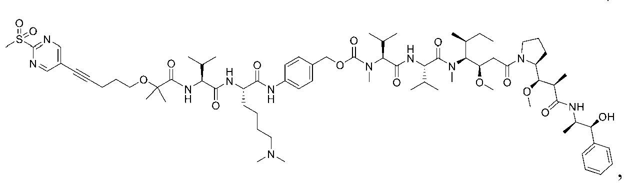 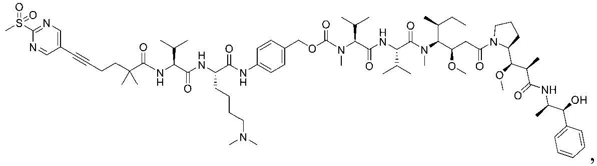 and

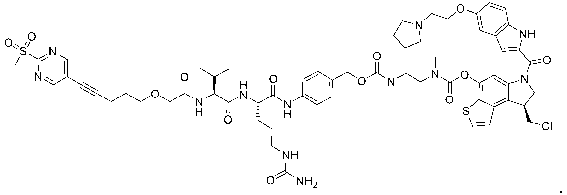

The present disclosure further provides a preparation method for drug-linker conjugates Lg-L₁-D₁, Lg-L₂-D₂, and Lg-L₃-D₃, which comprises: reacting Lg-L₁-Lg₁, Lg-L₂-Lg₂, and Lg-L₃-Lg₃ with corresponding D₁, D₂, and D₃,
for example,

Lg-L₁-Lg₁ + D₁→Lg-L₁-D₁;

Lg-L₂-Lg₂ + D₂→Lg-L₂-D₂;

Lg-L₃-Lg₃ + D₃→Lg-L₃-D₃,

wherein Lg, L₁, D₁, L₂, D₂, L₃, and D₃ are as defined in any one of the embodiments of the present disclosure, and Lg₁, Lg₂, and Lg₃ are each independently selected from hydroxy, halogen, and activated hydroxy, such as hydroxy, chlorine, bromine, 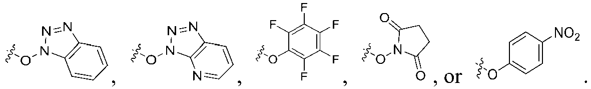 Specifically, the compound of general formula 12-9 and eribulin are subjected to a condensation reaction to obtain the target compound DL012. Drug-linker conjugates Lg-L₁-D₁, Lg-L₂-D₂, and Lg-L₃-D₃ of various structures may be obtained by subjecting analogs of 12-9 to a condensation reaction with different drug molecules.

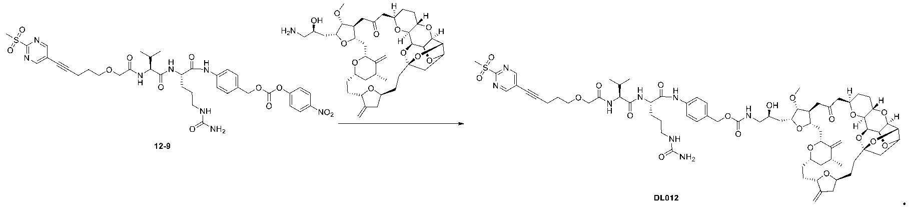

The present disclosure provides linkers Lg-L₁-Lg₁, Lg-L₂-Lg₂, and Lg-L₃-Lg₃. The present disclosure further provides a preparation method for Lg-L₁-Lg₁, Lg-L₂-Lg₂, and Lg-L₃-Lg₃, wherein Lg, L₁, L₂, L₃, Lg₁, Lg₂, and Lg₃ are as defined in any one of the embodiments of the present disclosure.

Specifically,
the compound of general formula 12-1 is subjected to substitution, conjugation, hydrolysis, and oxidation to obtain the compound of general formula 12-5, and then subjected to a condensation reaction and other reactions to obtain the compound of general formula 12-9. Analogs of 12-5 may be obtained by similar substitution, conjugation, hydrolysis, oxidation, and other reactions, and then Lg-L₁-Lg₁, Lg-L₂-Lg₂, and Lg-L₃-Lg₃ of various structures may be obtained by similar condensation reactions.

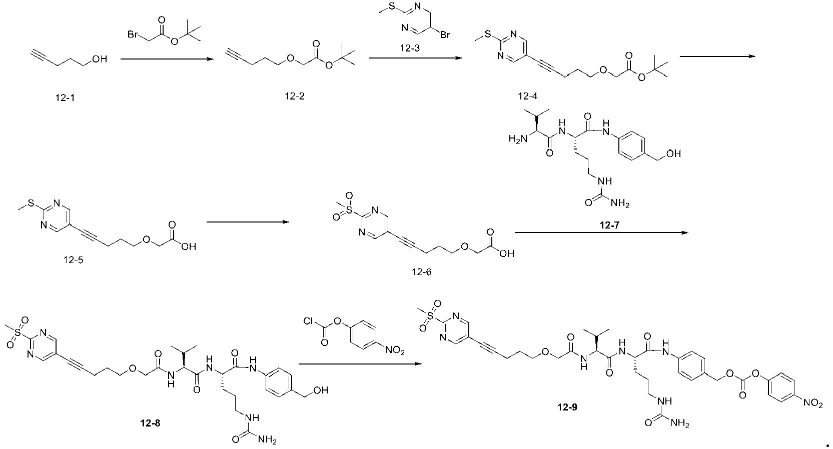

The present disclosure provides drugs D₁, D₂, and D₃. The present disclosure further provides a preparation method for drugs D₁, D₂, and D₃, wherein D₁, D₂, and D₃ are as defined in any one of the embodiments of the present disclosure, for example, MMAE/MMAF and a derivative thereof, eribulin and a derivative thereof, a maytansine derivative, a camptothecin derivative, etc., which may be produced from parent compounds in one or more chemical reaction steps.

Specifically,
the compound of general formula 6-13 is condensed with N-methylhydroxyethylamine to obtain the target compound 6-14. D₁, D₂, and D₃ of various structures may be obtained by subjecting MMAE/MMAF, eribulin, camptothecin, etc., to similar condensation reactions with different fragments.

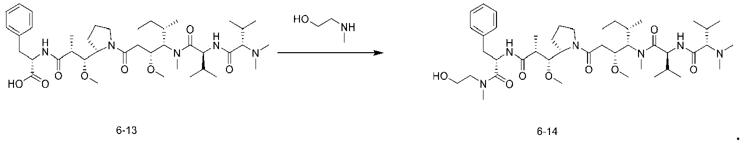

The present disclosure provides use of the aforementioned drug or the stereoisomer of the drug, the prodrug thereof, the pharmaceutically acceptable salt thereof, or the pharmaceutically acceptable solvate thereof in the preparation of an antibody-drug conjugate.

The present disclosure provides use of the aforementioned drug or the stereoisomer of the drug, the prodrug thereof, the pharmaceutically acceptable salt thereof, or the pharmaceutically acceptable solvate thereof in the preparation of a multi-warhead antibody-drug conjugate.

The present disclosure provides use of the aforementioned drug-linker conjugate or the stereoisomer of the drug-linker conjugate, the prodrug thereof, the pharmaceutically acceptable salt thereof, or the pharmaceutically acceptable solvate thereof in the preparation of an antibody-drug conjugate.

The present disclosure provides use of the aforementioned drug-linker conjugate or the stereoisomer of the drug-linker conjugate, the prodrug thereof, the pharmaceutically acceptable salt thereof, or the pharmaceutically acceptable solvate thereof in the preparation of a multi-warhead antibody-drug conjugate.

The present disclosure provides a pharmaceutical composition comprising the aforementioned multi-warhead antibody-drug conjugate or the stereoisomer of the multi-warhead antibody-drug conjugate, the prodrug thereof, the pharmaceutically acceptable salt thereof, or the pharmaceutically acceptable solvate thereof; and optionally one or more pharmaceutical adjuvants.

The present disclosure provides use of the aforementioned multi-warhead antibody-drug conjugate or the stereoisomer of the multi-warhead antibody-drug conjugate, the prodrug thereof, the pharmaceutically acceptable salt thereof, or the pharmaceutically acceptable solvate thereof, or the aforementioned pharmaceutical composition in the manufacture of a medicament for treating and/or preventing a disease associated with abnormal cell activity (for example, a cancer disease).

The present disclosure provides use of the aforementioned multi-warhead antibody-drug conjugate or the stereoisomer of the multi-warhead antibody-drug conjugate, the prodrug thereof, the pharmaceutically acceptable salt thereof, or the pharmaceutically acceptable solvate thereof, or the aforementioned pharmaceutical composition in the treatment and/or prevention of a disease associated with abnormal cell activity (for example, a cancer disease).

The present disclosure provides a method for preventing and/or treating a disease associated with abnormal cell activity (for example, a cancer disease), comprising: administering to an individual in need thereof a prophylactically and/or therapeutically effective amount of the aforementioned multi-warhead antibody-drug conjugate or the stereoisomer of the multi-warhead antibody-drug conjugate, the prodrug thereof, the pharmaceutically acceptable salt thereof, or the pharmaceutically acceptable solvate thereof, or the aforementioned pharmaceutical composition.

In some embodiments, the cancer disease is a solid tumor and/or a hematological tumor.

In some embodiments, the cancer disease is selected from esophageal cancer (for example, esophageal adenocarcinoma and esophageal squamous cell carcinoma), brain tumor, lung cancer (for example, small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, urothelial cancer, non-Hodgkin's lymphoma, central nervous system tumors (for example, neuroglioma, glioblastoma multiforme, glioma, or sarcoma), prostate cancer, and thyroid cancer; preferably selected from gastric cancer, non-small cell lung cancer, breast cancer, and breast adenocarcinoma.

In some specific embodiments, the cancer disease is selected from a tumor with high HER2 expression and a tumor with low HER2 expression; the tumor with high HER2 expression is preferably breast adenocarcinoma, and the tumor with low HER2 expression is preferably gastric cancer and/or breast cancer.

In some specific embodiments, the cancer disease is selected from tumors with drug resistance, and the drug resistance refers to including drug resistance to single-toxin ADCs and to a combination therapy with single-toxin ADCs; the tumor with drug resistance is selected from esophageal cancer (for example, esophageal adenocarcinoma and esophageal squamous cell carcinoma), brain tumor, lung cancer (for example, small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, urothelial cancer, non-Hodgkin's lymphoma, central nervous system tumors (for example, neuroglioma, glioblastoma multiforme, glioma, or sarcoma), prostate cancer, and thyroid cancer; preferably, an ABCB 1 or ABCG2 transporter is overexpressed in the tumor with drug resistance; the tumor with drug resistance is preferably Enhertu-resistant gastric cancer.

### Definitions:

In the present disclosure, unless otherwise indicated, scientific and technical terms used in the present disclosure have the meanings generally understood by those skilled in the art. In addition, the laboratory procedures of cell culture, molecular genetics, nucleic acid chemistry, and immunology used in the present disclosure are the conventional procedures widely used in the corresponding fields. Moreover, in order to better understand the present disclosure, the definitions and explanations of related terms are provided below. As used herein, examples of the term "pharmaceutically acceptable salt" are organic acid addition salts formed from organic acids that form pharmaceutically acceptable anions, including but not limited to formate, acetate, propionate, benzoate, maleate, fumarate, succinate, tartrate, citrate, ascorbate, α-ketoglutarate, α-glycerophosphate, alkylsulfonate or arylsulfonate; preferably, the alkylsulfonate is methylsulfonate or ethylsulfonate; the arylsulfonate is benzene sulfonate or p-toluenesulfonate. Suitable inorganic salts may also be formed, including but not limited to hydrochloride, hydrobromide, hydroiodide, nitrate, bicarbonate and carbonate, sulfate, or phosphate, etc.

Pharmaceutically acceptable salts may be obtained using standard procedures well known in the art, for example, by reacting a sufficient amount of an alkaline compound with a suitable acid that provides a pharmaceutically acceptable anion.

As used herein, the term "stereoisomer" refers to an isomer formed due to at least one asymmetric center. In compounds with one or more (for example, one, two, three, or four) asymmetric centers, racemic mixtures, single enantiomers, mixtures of diastereomers, and individual diastereomers may be produced. Certain individual molecules may also exist as geometric isomers (cis/trans). Similarly, the compounds of the present invention may exist as mixtures of two or more structurally different forms in rapid equilibrium (commonly referred to as tautomers). Representative examples of tautomers include keto-enol tautomers, phenol-keto tautomers, nitroso-oxime tautomers, imine-enamine tautomers, etc. It should be understood that the scope of the present disclosure encompasses all such isomers or mixtures thereof in any proportion (for example, 60%, 65%, 70%, 75%, 80%, 85%, 90%, 95%, 96%, 97%, 98%, or 99%).

A solid line (-), a solid wedge, or a dashed wedge may be used in the present disclosure to depict carbon-carbon bonds of the compounds of the present invention. Using the solid line to depict a bond that is bonded to an asymmetric carbon atom is intended to indicate that all possible stereoisomers (for example, particular enantiomers and racemic mixtures) at the carbon atom are included. Using the solid or dashed wedge to depict a bond that is bonded to an asymmetric carbon atom is intended to indicate that the stereoisomers shown are present. When present in a racemic mixture, the solid and dashed wedges are used to define relative stereochemistry rather than absolute stereochemistry. Unless otherwise indicated, the compounds of the present invention are intended to be present in the form of stereoisomers (including cis and trans isomers, optical isomers (for example, R and S enantiomers), diastereomers, geometric isomers, rotamers, conformers, atropisomers, and mixtures thereof). The compounds of the present invention may exhibit more than one type of isomerism and consist of mixtures thereof (for example, racemic mixtures and diastereomer pairs).

The compounds of the present disclosure may be present in the form of solvates (preferably hydrates), and the compounds of the present disclosure comprise a polar solvent as a structural element of the crystal lattices of the compounds, particularly, for example, water, methanol, or ethanol. The amount of the polar solvent, particularly water, may be present in a stoichiometric or non-stoichiometric ratio.

The present disclosure further comprises, within its scope, prodrugs of the compounds of the present invention. Generally, such prodrugs will be functional group derivatives of the compounds, which are readily converted into desired therapeutically active compounds *in vivo.* Thus, in these cases, the term "administering", "administered", or "administration" as used in the treatment method of the present disclosure shall include the treatment of various diseases or disorders with prodrug forms of one or more of the claimed compounds, but the prodrug forms are converted into the compounds described above *in vivo* upon administration to an individual. Conventional methods for selecting and preparing suitable prodrug derivatives are described in, for example, "Design of Prodrug", ed. H. Bundgaard, Elsevier, 1985.

In the present disclosure, the pharmaceutical adjuvants refer to excipients and additives used in the production of a pharmaceutical product and in the formulation of a pharmaceutical formula, and refer to substances, other than the active ingredient, which have been rationally evaluated for safety and are contained in a pharmaceutical formulation. In addition to shaping, serving as carriers, and enhancing stability, pharmaceutical adjuvants also have such important functions as solubilization, aiding dissolution, sustaining or controlling release, etc. They are important ingredients that possibly affect the quality, safety, and effectiveness of pharmaceutical products. According to their origins, pharmaceutical adjuvants may be classified into natural products, semi-synthetic products, and total synthetic products. According to their effects and uses, pharmaceutical adjuvants may be classified into solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adhesive

agents, antioxidants, chelating agents, permeation promoters, pH regulators, buffers, plasticizers, surfactants, foaming agents, defoaming agents, thickeners, inclusion agents, humectants, absorbents, diluents, flocculants, deflocculants, filter aids, release retardants, etc.; according to their routes of administration, pharmaceutical adjuvants may be classified into pharmaceutical adjuvants for oral administration, injection, mucosal administration, transdermal or local administration, nasal or oral inhalation administration, ocular administration, etc. The same pharmaceutical adjuvant may be used in pharmaceutical formulations for different routes of administration, and may have different effects and uses.

The pharmaceutical composition may be prepared into various suitable dosage forms according to the route of administration, for example, tablets, capsules, granules, oral solutions, oral suspensions, oral emulsions, powders, tinctures, syrups, injections, suppositories, ointments, creams, pastes, ophthalmic formulations, pills, implants, aerosols, powder aerosols, sprays, etc. The pharmaceutical composition or suitable dosage form may comprise 0.01 mg to 1000 mg of a compound of the present disclosure or a pharmaceutically acceptable salt thereof or a conjugate thereof, suitably 0.1 mg to 800 mg, preferably 0.5-500 mg, preferably 0.5 to 350 mg, and particularly preferably 1-250 mg.

The pharmaceutical composition may be administered in the form of an injection, including an injectable liquid, a sterile powder for injection, and a concentrated solution for injection. The carriers and solvents that may be used include water, Ringer's solution, and isotonic sodium chloride solution. In addition, sterilized non-volatile oils may also be used as solvents or suspending media, such as monoglycerides or diglycerides. The pharmaceutical composition may be administered in the form of an infusion.

The term "treat", "treated", "treating", or "treatment" as used herein usually refers to acquiring needed pharmacological effects and/or physiological effects. In terms of fully or partially preventing a disease or a symptom thereof, the effect may be prophylactic; and/or in terms of partially or fully stabilizing or curing the disease and/or a side effect caused by the disease, the effect may be therapeutic. As used herein, "treat", "treating", or "treatment" encompasses any treatment to a disease in a patient, including (a) preventing a disease or a symptom in a patient susceptible to the disease or the symptom that has not yet been diagnosed; (b) inhibiting a symptom of a disease, i.e., arresting its progression; or (c) alleviating a symptom of a disease, i.e., causing regression of the disease or the symptom.

In the present disclosure, the term "individual" includes a human or non-human animal. Exemplary human individuals include human individuals with a disease (for example, the disease described herein), who are referred to as patients, or normal individuals. In the present disclosure, the term "non-human animal" includes all vertebrates, such as non-mammals (for example, birds, amphibians, and reptiles) and mammals, such as non-human primates, livestock, and/or domesticated animals (for example, sheep, dogs, cats, cows, pigs, etc.). In the present disclosure, the term "effective dose" refers to the amount of a multi-target antibody-drug conjugate, a drug-linker conjugate, a compound, or a composition that can alleviate one or more symptoms of a disorder to be treated to some extent after administration.

In the present disclosure, the term "multi-warhead antibody-drug conjugate" refers to a drug molecule of an antibody-drug conjugate ADC having 2 or 3 different toxin molecules (payloads). Such a double- or triple-warhead antibody-drug conjugate can kill tumor cells through the mechanisms of action of different toxins (payloads), thereby increasing the anti-tumor effect of the double- or triple-warhead antibody-drug conjugate and reducing the rate of drug resistance.

In the present disclosure, the term "drug" or "medicament" refers to a substance that inhibits or prevents cell functions and/or causes cell death or cell destruction.

Unless otherwise indicated in the present disclosure, the "derivative" of a compound as used herein refers to a substance having a chemical structure similar to that of the compound but also containing at least one chemical group that is not present in the compound and/or lacking at least one chemical group that is present in the compound. The compound to which the derivative is compared is referred to as a "parent" compound. Generally, a "derivative" may be produced from a parent compound in one or more chemical reaction steps. In the present disclosure, the term "linker" refers to a fragment that attaches a bioactive compound fragment (drug molecule or drug unit) to a pharmacokinetic regulatory fragment moiety (antibody or antigen-binding fragment thereof).

In the present disclosure, the term "linker that is cleavable in a tumor microenvironment" means that the linker has the ability to be cleaved in the tumor microenvironment without the need for endocytosis, thereby releasing the toxin in the tumor microenvironment, for example, 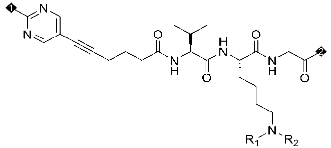 and

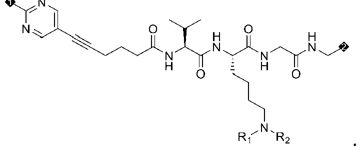

In the present disclosure, the term " pharmacokinetic regulatory fragment" is interpreted in its broadest sense and refers to a fragment that can regulate the pharmacokinetic properties of a molecule, for example, increasing tumor exposure and prolonging the duration of action of a drug. Such pharmacokinetic regulatory fragments include intact monoclonal antibodies, polyclonal antibodies, and multispecific antibodies (for example, bispecific antibodies) formed from at least two intact antibodies, ligands of tumor cell surface receptors, including small-molecule ligands, peptides of C3-40 amino acid residues (so long as they possess the desired bioactivity), polyethylene glycol, and long-chain aliphatic hydrocarbons.

In the present disclosure, the term "short peptide" refers to a peptide consisting of 2-10 amino acids and is also known as an oligopeptide.

In the present disclosure, unless otherwise expressly indicated, the description "each ... be independently selected from" and "... be each independently selected from" are used interchangeably throughout the present disclosure and should be understood in a broad sense, and it may mean that specific items expressed by the same or different symbols in different groups do not affect each other, or that specific items expressed by the same or different symbols in the same group do not affect each other.

In the present disclosure, "-S-" (S atom) in the general formula can be understood by those skilled in the art to mean that the linker is attached to a sulfhydryl group contained in T (for example, an antibody) itself after the disulfide bond is opened (for example, the disulfide bond may be opened by reducing the disulfide bond with the reducing agent TCEP, generating the sulfhydryl group -SH); that is, -S- between the linker and T is not an additional extraneous sulfur atom.

In the present disclosure, "attached to an S atom" can be understood by those skilled in the art to mean that the linker is attached to a sulfhydryl group contained in T (for example, an antibody) itself after the disulfide bond is opened (for example, the disulfide bond may be opened by reducing the disulfide bond with the reducing agent TCEP, generating the sulfhydryl group -SH); that is, -S- between the linker and T is not an additional extraneous sulfur atom. For example, -S- is not an additional extraneous sulfur atom, but is -S-formed by attaching the sulfhydryl group contained in T itself after the disulfide bond is opened to position 1 of the linker (for example,

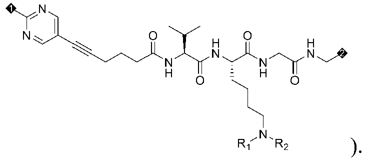

In each part of this specification, substituents for the compounds of the present disclosure are disclosed according to group types or ranges. Particularly, the present disclosure includes each independent subcombination of the members of these group types and ranges. For example, the term "C1-6 alkyl" refers particularly to independently disclosed methyl, ethyl, C3 alkyl, C4 alkyl, C5 alkyl, and C6 alkyl.

In the present disclosure, the term "include", "comprise", "have", "contain", or "involve" and other variant forms thereof herein are inclusive or open-ended and do not exclude other unenumerated elements or method steps.

In the present disclosure, the term "about" generally means varying by 0.5%-20% above or below the stated numerical value, for example, varying by 0.5%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, or 10.0% above or below the stated value.

In the present disclosure, the term "C1-6 alkyl" refers to linear or branched alkyl containing 1-6 carbon atoms, including, for example, "C1-3 alkyl" or "C1-4 alkyl", methyl, ethyl, etc., and specific examples include, but are not limited to, methyl, ethyl, n-propyl, isopropyl, n-butyl, isobutyl, sec-butyl, tert-butyl, pentyl, and hexyl. In the present disclosure, the term "C2-6 alkenyl" refers to linear, branched, or cyclic alkenyl containing at least one double bond and 2-6 carbon atoms, including, for example, "C2-4 alkenyl", etc. Examples thereof include, but are not limited to, ethenyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, cyclopentenyl, 1,3-cyclopentadienyl, cyclohexenyl, 1,4-cyclohexadienyl, etc.

In the present disclosure, the term "C2-6 alkynyl" refers to linear or branched alkynyl containing at least one triple bond and 2-6 carbon atoms, including, for example, "C2-4 alkynyl", etc. Examples thereof include, but are not limited to, ethynyl, propynyl, 2-butynyl, 2-pentynyl, 3-pentynyl, 4-methyl-2-pentynyl, 2-hexynyl, 3-hexynyl, 5-methyl-2-hexynyl, etc.

In the present disclosure, the term "halogen" includes fluorine, chlorine, bromine, and iodine.

In the present disclosure, the term "C3-6 cycloalkyl" refers to saturated cyclic alkyl containing 3-6 carbon atoms. Optionally, a carbon atom in the cyclic structure may be substituted with oxo, including cyclopropylalkyl (i.e., cyclopropyl), cyclobutylalkyl (i.e., cyclobutyl), cyclopentylalkyl (i.e., cyclopentyl), and cyclohexyl.

In the present disclosure, the term "C1-6 alkoxy" refers to alkyl as defined above which is attached to the parent molecular moiety via an oxygen atom, including, for example, "C1-3 alkoxy" or "C1-4 alkoxy". Specific examples include, but are not limited to, methoxy, ethoxy, propoxy, isopropoxy, n-propoxy, isopropoxy, n-butoxy, isobutoxy, tert-butoxy, pentoxy, hexoxy, etc.

In the present disclosure, the term "C1-6 haloalkyl" refers to alkyl as defined above which is attached to the parent molecular moiety via halogen, including, for example, "C1-3 haloalkyl" or "C1-4 haloalkyl". Specific examples include, but are not limited to, chloromethyl, fluoroethyl, bromopropyl, etc.

In the present disclosure, the term "4-10 membered heterocyclic ring" refers to a ring containing 4-10 ring atoms (at least one of which is a heteroatom, such as a nitrogen atom, an oxygen atom or a sulfur atom). The term "3-6 membered heterocyclic ring" refers to a ring containing 3-6 ring atoms (at least one of which is a heteroatom, such as a nitrogen atom, an oxygen atom or a sulfur atom). The term "3-7 membered heterocyclic ring" refers to a ring containing 3-7 ring atoms (at least one of which is a heteroatom, such as a nitrogen atom, an oxygen atom or a sulfur atom). The term "5-6 membered heterocyclic ring" refers to a ring containing 5-6 ring atoms (at least one of which is a heteroatom, such as a nitrogen atom, an oxygen atom or a sulfur atom). Optionally, a ring atom (for example, a carbon atom, a nitrogen atom, or a sulfur atom) in the cyclic structure may be substituted with oxo, including but not limited to, pyrrolidine, tetrahydrofuran, piperidine, piperazine, tetrahydropyran, pyrrolidone, and other rings.

In the present disclosure, the term "3-10 membered carbocyclic ring" refers to a ring having 3 to 10 ring atoms as carbon atoms. Optionally, a carbon atom in the cyclic structure may be substituted with oxo, for example, 3-6 membered carbocyclic rings, 3-7 membered carbocyclic rings, 5-8 membered carbocyclic rings, etc., including but not limited to, cyclopentane, cyclohexane, etc.

In the present disclosure, the term "4-10 membered heterocyclyl" refers to a cyclic group containing 4-10 ring atoms (at least one of which is a heteroatom, such as a carbon atom, a nitrogen atom, or a sulfur atom). The term "4-6 membered heterocyclyl" refers to a cyclic group containing 4-6 ring atoms (at least one of which is a heteroatom, such as a nitrogen atom, an oxygen atom, or a sulfur atom). The term "3-6 membered heterocyclyl" refers to a cyclic group containing 3-6 ring atoms (at least one of which is a heteroatom, such as a carbon atom, a nitrogen atom, or a sulfur atom). Optionally, a ring atom (for example, a carbon atom, a nitrogen atom, or a sulfur atom) in the cyclic structure may be substituted with oxo. "4-8 membered heterocyclyl" includes, for example, "4-8 membered nitrogen-containing heterocyclyl", "4-8 membered oxygen-containing heterocyclyl", "4-7 membered heterocyclyl", "4-7 membered oxygen-containing heterocyclyl", "4-7 membered heterocyclyl", "4-6 membered heterocyclyl", "5-7 membered heterocyclyl", "5-6 membered heterocyclyl", and "5-6 membered nitrogen-containing heterocyclyl", including but not limited to oxocyclobutanyl, pyrrolidinyl, tetrahydrofuranyl, piperidinyl, piperazinyl, tetrahydropyranyl, homopiperazinyl, etc.

In the present disclosure, the term "aryl" refers to a monocyclic or polycyclic hydrocarbon group which is aromatic, such as 6-10 membered aryl, 5-8 membered aryl, etc. Specific examples include, but are not limited to, phenyl, naphthyl, anthryl, phenanthryl, etc. The "6-10 membered aryl" refers to aryl containing 6-10 ring atoms. The "C6-10 aryl" refers to aryl containing 6-10 carbon atoms.

In the present disclosure, the term "heteroaryl" refers to an aromatic cyclic group, wherein at least one ring atom is a heteroatom, such as a nitrogen atom, an oxygen atom, or a sulfur atom. Optionally, a ring atom (for example, a carbon atom, a nitrogen atom, or a sulfur atom) in the cyclic structure may be substituted with oxo. Specific examples include, but are not limited to, 5-10 membered heteroaryl, 5-6 membered heteroaryl, 5-10 membered nitrogen-containing heteroaryl, 6-10 membered oxygen-containing heteroaryl, 6-8 membered nitrogen-containing heteroaryl, 5-8 membered oxygen-containing heteroaryl, etc., such as furanyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridinyl, 2-pyridonyl, 4-pyridonyl, pyrimidinyl, 1,4-dioxacyclohexadienyl, 2*H*-1,2-oxazinyl, 4*H*-1,2-oxazinyl, 6*H*-1,2-oxazinyl, 4*H*-1,3-oxazinyl, 6*H*-1,3-oxazinyl, 4*H*-1,4-oxazinyl, pyridazinyl, pyrazinyl, 1,2,3-triazinyl, 1,3,5-triazinyl, 1,2,4,5-tetrazinyl, azepinyl, 1,3-diazepinyl, azocinyl, etc. The bond in the structural formula represented by a wave line "~~" in the present disclosure is intended to represent that the structure represents a cis or trans isomer, or a mixture of cis and trans isomers in any proportion.

In the present disclosure, the term "linker unit" refers to a component of an antibody-drug conjugate or a drug-linker conjugate or a linker that functions to attach an antibody or an antigen-binding fragment thereof that binds to a target to the remainder of the antibody-drug conjugate. The linker unit is capable of attaching a T unit to Lb, and specific examples include, but are not limited to (wherein position 1 is attached to the antibody or the antigen-binding fragment thereof, and position 2 is attached to Lb or Lc):

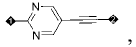

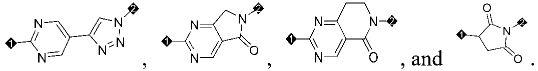

In the present disclosure, the term "connector unit" refers to a component of an antibody-drug conjugate or a drug-linker conjugate or a linker that functions to join a linker unit and an amino acid residue or a short peptide consisting of 2-10 amino acid residues. The connector unit, when present, is capable of attaching La to Lc. Specific examples include, but are not limited to (wherein position 1 is attached to the linker unit, and position 2 is attached to Lc): 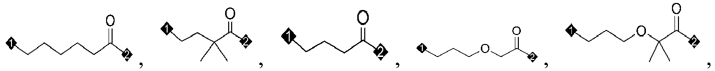 and

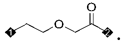

In the present disclosure, the term "spacer unit" refers to a component of an antibody-drug conjugate or a drug-linker conjugate or a linker that functions to join a bioactive compound fragment and an amino acid residue or a short peptide consisting of 2-10 amino acid residues, or may provide an additional structural component to further facilitate the release of the bioactive compound from the remainder of the antibody-drug conjugate. The spacer unit, when present, is capable of attaching the drug unit to Lc. Specific examples include, but are not limited to (wherein position 1 is attached to Lc, and position 2 is attached to D):

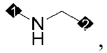

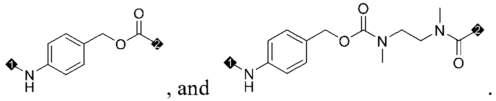

The preferred conditions described above may be combined arbitrarily to obtain preferred examples of the present invention without departing from the general knowledge in the art.

In the present disclosure, the term "antibody" is interpreted in its broadest sense and includes intact monoclonal antibodies, polyclonal antibodies, and multispecific antibodies (for example, bispecific antibodies) formed from at least two intact antibodies, so long as they possess the desired bioactivity. In the present disclosure, "antibody" and "immunoglobulin" are used interchangeably.

The term "non-engineered antibody" refers to an antibody that is not modified to introduce additional amino acids or peptides.

The term "antibody fragment" comprises a portion of an intact antibody. In a preferred embodiment, the antibody fragment is an antigen-binding fragment.

"Antigen-binding fragment" refers to a molecule different from an intact antibody, which comprises a portion of the intact antibody and binds to an antigen to which the intact antibody binds. Examples of the antibody fragments include, but are not limited to, an Fv, a Fab, a Fab', a Fab'-SH, a F(ab')2, a dAb (domain antibody), a linear antibody, a single-chain antibody (for example, scFv), a single-domain antibody (for example, VHH), a bivalent antibody or a fragment thereof, or a camelid antibody.

In the present disclosure, the term "monoclonal antibody" refers to an antibody from a population of substantially homogeneous antibodies, i.e., the individual antibodies constituting the population are identical except for a small number of natural mutations that may be present. Monoclonal antibodies have high specificity for one determinant (epitope) of an antigen, while polyclonal antibodies in contrast thereto comprise different antibodies against different determinants (epitopes). In addition to specificity, another advantage of monoclonal antibodies is that they can be synthesized without contamination by other antibodies. The modifier "monoclonal" used herein indicates that the antibody is characterized as being from a population of substantially homogeneous antibodies, and should not be construed as requiring a particular preparation method.

In some embodiments of the present disclosure, monoclonal antibodies also particularly include chimeric antibodies; that is, a portion of the heavy chain and/or light chain is identical or homologous to a type, a class, or a subclass of antibodies, and the remainder is identical or homologous to another type, another class, or another subclass of antibodies, so long as they possess the desired bioactivity (see, for example, US 4,816,567; and Morrison et al., 1984, PNAS, 81: 6851-6855). Chimeric antibodies that may be used in the present disclosure include primatized antibodies comprising variable region antigen-binding sequences from non-human primates (for example, ancient monkeys, chimpanzees, etc.) and human constant region sequences.

In the present disclosure, "humanized" forms of non-human (for example, murine) antibodies refer to chimeric antibodies that comprise minimal non-human immunoglobulin sequences. Most humanized antibodies are human recipient immunoglobulins with the hypervariable region residues replaced by non-human (for example, mouse, rat, rabbit, or non-human primate) hypervariable region residues having the desired specificity, affinity, and function (donor antibody). In some embodiments, framework region (FR) residues of the human immunoglobulin are also replaced by non-human residues. Furthermore, humanized antibodies may further comprise residues that are not present in the recipient antibody or the donor antibody. These modifications are made to further optimize the properties of the antibody. A humanized antibody generally comprises at least one, usually two, variable regions, in which all or nearly all of the hypervariable loops correspond to those of a non-human immunoglobulin, while the FRs are entirely or nearly entirely human immunoglobulin sequences. The humanized antibody may further comprise at least one portion of an immunoglobulin constant region (Fc), typically a human immunoglobulin Fc. For details see, for example, Jones et al., 1986, Nature, 321: 522-525; Riechmann et al., 1988, Nature, 332: 323-329; and Presta, 1992, Curr Op Struct Bwl 2: 593-596.

Intact antibodies may be divided into different "classes" according to the amino acid sequences of the heavy chain constant regions. The main five classes are IgA, IgD, IgE, IgG, and IgM, several of which may also be divided into different "subclasses" (isotypes), such as IgG1, IgG2, IgG3, IgG4, IgA1, and IgA2. Different classes of heavy chain constant regions of antibodies are referred to as α, β, ε, γ, and µ, respectively. Different classes of subunit structures and three-dimensional configurations of immunoglobulins are well known in the art.

Monoclonal antibodies used in the present disclosure may be produced by many methods. For example, monoclonal antibodies used in the present disclosure may be obtained by the hybridoma method using many species including mouse, hamster, rat, and human cells (see, for example, Kohler et al., 1975, Nature, 256: 495), or prepared by recombinant DNA techniques (see, for example, US 4,816,567), or isolated from phage antibody libraries (see, for example, Clackson et al., 1991, Nature, 352: 624-628; and Marks et al., 1991, Journal of Molecular Biology, 222: 581-597).

In the present disclosure, the term "multispecific antibody" includes, for example, a trispecific antibody and a tetraspecific antibody, the former being an antibody with three different kinds of antigen-binding specificity, and the latter being an antibody with four different kinds of antigen-binding specificity.

In the present disclosure, the term "intact antibody" refers to an antibody comprising antigen-binding variable regions, a light chain constant region (CL), and heavy chain constant regions (CH1, CH2, and CH3). The constant region may be a native sequence (for example, a human native constant region sequence) or a variant of an amino acid sequence thereof. The intact antibody is preferably an intact antibody with one or more effector functions.

In the present disclosure, the term "probody" is a modified antibody, comprising an antibody or an antibody fragment, capable of specifically binding to its target and capable of being coupled to a masking group, wherein the masking group means that the cleavage constant of the binding capacity of the antibody or the antibody fragment to its target is at least 100 times or 1000 times or 10000 times greater than that of the binding capacity of the antibody or the antibody fragment without the coupled masking group to its target.

Apparently, based on the content of the present invention described above, other various modifications, substitutions, or alterations may be made in accordance with general technical knowledge and conventional practice in the art without departing from the fundamental technical concept of the present disclosure described above. All techniques implemented based on the content of the present disclosure described above fall within the scope of the present disclosure.

### Beneficial Effects:

The antibody-drug conjugate having a variety of toxins provided in the present invention has a synergistic effect on anti-tumor activity due to the different mechanisms of action of the different toxins, and has a significant inhibitory effect on different tumor cells tested *in vivo* and *in vitro.*

The multi-warhead antibody-drug conjugate provided in the present invention can overcome the problem of high heterogeneity of tumors, and shows significant anti-tumor activity in a variety of tumors. Particularly, the multi-warhead antibody-drug conjugate shows good inhibitory effects in cell lines with high her2 expression (N87 and SK-BR-3) and cell lines with low her2 expression (H358 and MCF-7).

The multi-warhead antibody-drug conjugate provided in the present invention can form an antibody-drug conjugate with an antibody that has no endocytosis capacity by utilizing the extracellular cleavage capacity of the linker in the tumor microenvironment, thereby making up for the defect that traditional ADCs require endocytosis. Such an antibody-drug conjugate still has high anti-tumor activity.

The multi-warhead antibody-drug conjugate provided in the present invention can form an antibody-drug conjugate with an antibody that has no endocytosis capacity or extracellular tumor antigen binding capacity by utilizing the extracellular cleavage capacity of the linker in the tumor microenvironment and the enrichment capacity of the linker in the tumor microenvironment. Such an antibody-drug conjugate still has high anti-tumor activity.

The multi-warhead antibody-drug conjugate provided in the present invention has an anti-drug resistance effect, and in particular provides a solution to the problem of drug resistance caused by single-toxin ADCs or toxins produced during treatment. Particularly, the multi-warhead antibody-drug conjugate has a good inhibitory effect on cell lines with drug resistance to camptothecin ADCs.

In the preparation method for a multi-warhead antibody-drug conjugate of the present invention, the ratio of a variety of toxins can be flexibly adjusted, so that bioactive molecules with different mechanisms of action are capable of producing optimal efficacy at the same ADC dose while reducing toxicity and enabling the ADC to have an optimal safety window.

The preparation method for a multi-warhead antibody-drug conjugate of the present invention is extremely simple to operate, does not need to synthesize complex and multi-branched linkers, does not need to pre-treat the antibody for multiple times, and does not need to perform conjugation and purification on different sites for multiple times, so that thereby achieving high druggability, saving the cost, and providing a good basis for drug production.

### BRIEF DESCRIPTION OF THE DRAWINGS

FIG. 1 shows curves of changes in *in vitro* proliferation inhibition rates of Enhertu-resistant NCI-N87 tumor cells.
FIG. 2 shows curves of changes in tumor growth in an NCI-H358 xenograft tumor model.
FIG. 3 shows curves of changes in body weight of mice in an NCI-H358 xenograft tumor model.

### DETAILED DESCRIPTION

The present disclosure is further illustrated by the following specific examples, which, however, are not intended to limit the present disclosure solely to these examples. Based on the teachings of the present disclosure, those skilled in the art can make various modifications or improvements without departing from the basic spirit and scope of the present disclosure. Reagents or instruments without specified manufacturers used herein are commercially available conventional products.

The abbreviations in the present disclosure have the following meanings:

| | | | |
|---|---|---|---|
| DCM | Dichloromethane | FA | Formic acid |
| DMF | N,N-Dimethylformamide | ACN | Acetonitrile |
| MTBE | Methyl tert-butyl ether | DIEA/DIP EA | Diisopropylethylamine |
| TEA | Triethylamine | CDCl₃ | Deuterated chloroform |
| DMSO | Dimethyl sulfoxide | Oxone | Potassium hydrogen persulfate |
| HATU | O-(7-Azabenzotriazol-1-yl)-N,N,N',N'-tetramethyluronium hexafluorophosphate | PE | Petroleum ether |
| EA | Ethyl acetate | TCEP | Tris(2-carboxyethyl)phosphine |
| THF | Tetrahydrofuran | DEA | Diethylamine |
| EEDQ | 2-Ethoxy-1-ethoxycarbonyl-1,2-dihydroquinoline | DMTMM | 4-(4,6-dimethoxytriazin-2-yl)-4-methylmorpholine hydrochloride |
| TFA | Trifluoroacetic acid | DCC | N,N'-Dicyclohexylcarbodiimide |
| HOBT | 1-Hydroxybenzotriazole | | |

### Preparation Schemes

The structures of the compounds described in the following examples were determined by nuclear magnetic resonance (¹H NMR) or mass spectrometry (MS).

Nuclear magnetic resonance (¹H NMR) was determined using a Bruker 400 MHz nuclear magnetic resonance spectrometer, the solvents for the determination were deuterated methanol (CD₃OD), deuterated chloroform (CDCl₃), or hexadeuterodimethyl sulfoxide (DMSO-d₆); the internal standard substance was tetramethylsilane (TMS).

The abbreviations in the nuclear magnetic resonance (NMR) spectra used in the examples are shown as follows: s: singlet; d: doublet; t: triplet; q: quartet; dd: double doublet; qd: quartet doublet; ddd: double double doublet; ddt: double double triplet; dddd: double double double doublet; m: multiplet; br: broad; J: coupling constant; Hz: Hertz; DMSO-d₆: hexadeuterodimethyl sulfoxide. δ values were expressed in ppm.

Mass spectrometry (MS) was determined using an Agilent (ESI) mass spectrometer (model: Agilent 6120B).

### I. Preparation of Drug-Linker Conjugates

### Example 1.1: Preparation of Compound DL001

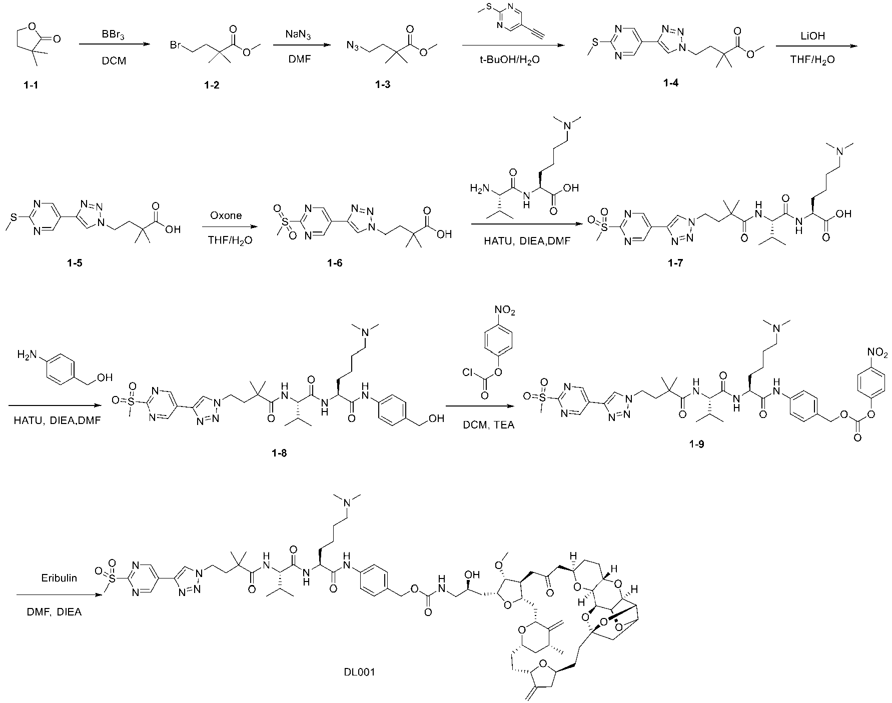

Step 1: Compound 1-1 (500 mg, 4.39 mmol) was dissolved in extra-dry DCM (5 mL), and boron tribromide (1.15 g, 4.6 mmol) was added dropwise at 0 °C. After the dropwise addition was completed, the reaction solution was heated to room temperature and stirred for 24 h. Then, the reaction solution was cooled to 0 °C, and methanol (5 mL) was added dropwise to the reaction solution to quench the reaction. The reaction solution was stirred at room temperature for another 24 h. A saturated aqueous sodium bicarbonate solution (30 mL) was added to the reaction solution, and then the resulting mixture was extracted with DCM (30 mL × 3). The organic phases were combined, dried over anhydrous sodium sulfate, and filtered, and the solvent was removed by evaporation to obtain 1-2 (830 mg, yield: 92.5%) as a brown oily liquid.

¹H NMR (400 MHz, CDCl₃) δ 3.69 (s, 3H), 3.40 - 3.27 (m, 2H), 2.20 - 2.09 (m, 2H), 1.21 (s, 6H).

Step 2: Compound 1-2 (650 mg, 3.13 mmol) was dissolved in DMF (10 mL), and sodium azide (1.02 g, 15.63 mmol) was added. The reaction solution was heated to 80 °C, stirred overnight, and then cooled to room temperature. Water (30 mL) was added to the reaction solution, and then the resulting mixture was extracted with MTBE (30 mL × 3). The organic phases were combined, dried over anhydrous sodium sulfate, filtered, and concentrated to dryness by rotary evaporation to obtain the target compound 1-3 (470 mg, yield: 88.9%) as a pale yellow oily liquid.

¹H NMR (400 MHz, CDCl₃) δ 3.69 (s, 3H), 3.31 - 3.23 (m, 2H), 1.89 - 1.81 (m, 2H), 1.22 (s, 6H).

Step 3: Compound 1-3 (365 mg, 2.13 mmol) and 5-ethynyl-2-(methylthio)pyrimidine (320 mg, 2.13 mmol) were dissolved in tert-butanol (5 mL) and water (5 mL), and sodium ascorbate (84 mg, 0.43 mmol) and anhydrous copper sulfate (34 mg, 0.21 mmol) were added. The reaction solution was stirred at room temperature for 1 h. Water (25 mL) was added to the reaction solution, and then the resulting mixture was extracted with EA (30 mL × 3). The organic phases were combined, dried over anhydrous sodium sulfate, filtered, and concentrated to dryness by rotary evaporation, and the residue was purified by silica gel column chromatography (PE:EA = 3:1) to obtain the target compound 1-4 (600 mg, yield: 88.2%) as a white solid. LCMS (ESI) [M+H]⁺= 322.1

¹H NMR (400 MHz, CDCl₃) δ 8.95 (s, 2H), 7.85 (s, 1H), 4.53 - 4.39 (m, 2H), 3.69 (s, 3H), 2.61 (s, 3H), 2.29 - 2.17 (m, 2H), 1.31 (s, 6H).

Step 4: Compound 1-4 (500 mg, 1.60 mmol) was dissolved in THF (5 mL) and water (5 mL), and lithium hydroxide (112 mg, 4.70 mmol) was added. The reaction solution was stirred at room temperature for 5 h. 1 N hydrochloric acid was added to the reaction solution to adjust the pH to 3-4. A large amount of white solid was precipitated. The mixture was filtered, and the filter cake was the target compound 1-5 (450 mg, yield: 93.5%) as a white solid.
LCMS (ESI) [M+H]⁺= 308.0
¹H NMR (400 MHz, DMSO-*d*₆) δ 12.43 (s, 1H), 9.06 (s, 2H), 8.76 (s, 1H), 4.55 - 4.31 (m, 2H), 2.56 (s, 3H), 2.15 - 2.04 (m, 2H), 1.20 (s, 6H).

Step 5: Compound 1-5 (1.5 g, 4.90 mmol) was dissolved in THF (10 mL) and water (10 mL), and Oxone (15.0 g, 24.5 mmol) was added. The reaction solution was stirred at room temperature overnight. The reaction solution was poured into water (200 mL), and the resulting mixture was filtered. The filter cake was the target compound 1-6 (1.6 g, yield: 93.5%) as a white solid.
LCMS (ESI) [M+H]⁺= 340.1
¹H NMR (400 MHz, DMSO-*d*₆) δ 9.48 (s, 2H), 8.98 (s, 1H), 4.54 - 4.48 (m, 2H), 3.44 (s, 3H), 2.16 - 2.09 (m, 2H), 1.21 (s, 6H).

Step 6: Compound 1-6 (1.9 g, 5.32 mmol) was dissolved in DMF (40 mL), and then HATU (2.13 g, 5.32 mmol) and DIEA (1.7 g, 13.3 mmol) were added. The reaction solution was stirred at room temperature for 30 min, and N2-(L-valyl)-N6,N6-dimethylamino-L-lysine (1.45 g, 5.32 mmol) was added to the reaction solution. The resulting mixture was stirred for another 1 h. The reaction solution was directly purified by reversed-phase chromatography (H₂O:ACN = 5%-100%) to obtain the target compound 1-7 (1.5 g, yield: 44.5%) as a white solid.
LCMS (ESI) [M+H]⁺= 595.5
¹H NMR (400 MHz, DMSO-*d*₆) δ 9.51 (s, 2H), 9.05 (s, 1H), 7.89 (d, J = 7.1 Hz, 1H), 7.48 (d, J = 8.6 Hz, 1H), 4.45 - 4.35 (m, 2H), 4.13 (t, 1H), 4.07-4.09 (m, 1H), 3.44 (s, 3H), 2.34 (t, J = 7.3 Hz, 2H), 2.23 (s, 6H), 2.19 - 2.06 (m, 4H), 1.73 - 1.57 (m, 2H), 1.45 - 1.36 (m, 2H), 1.31 - 1.27 (m, 1H), 1.22 (d, J = 9.6 Hz, 6H), 0.91 - 0.86 (m, 6H).

Step 7: Compound 1-7 (360 mg, 0.61 mmol) and 4-aminophenethyl alcohol (75 mg, 0.61 mmol) were dissolved in DMF (5 mL), and HATU (230 mg, 0.61 mmol) and DIEA (195 mg, 1.52 mmol) were added. The reaction solution was stirred at room temperature for 1 h. The reaction solution was directly purified by reversed-phase chromatography (H₂O:ACN = 5%-100%) to obtain the target compound 1-8 (255 mg, yield: 60.1%) as a white solid.
LCMS (ESI) [M+H]⁺= 700.4
¹H NMR (400 MHz, DMSO-*d*₆) δ 9.93 (d, J = 87.4 Hz, 1H), 9.47 (s, 1H), 9.39 (s, 1H), 8.89 (d, J = 57.3 Hz, 1H), 8.54 - 8.16 (m, 1H), 7.68 (d, J = 7.8 Hz, 0.5H), 7.56 - 7.48 (m, 2H), 7.47 (d, J = 8.3 Hz, 0.5H), 7.19 (d, J = 8.4 Hz, 1H), 7.13 (d, J = 8.5 Hz, 1H), 5.10 - 5.04 (m, 1H), 4.44 - 4.36 (m, 4H), 4.21 - 4.09 (m, 1H), 3.45 (s, 3H), 2.75 - 2.65 (m, 2H), 2.23 - 2.04 (m, 4H), 1.83 - 1.63 (m, 2H), 1.59 - 1.51 (m, 2H), 1.42 - 1.35 (m, 1H), 1.35 - 1.16 (m, 12H), 0.94 - 0.87 (m, 6H).

Step 8: Compound 1-8 (250 mg, 0.36 mmol) and 4-nitrophenyl chloroformate (288 mg, 1.43 mmol) were dissolved in DCM (10 mL), and TEA (72 mg, 0.72 mmol) was added. The reaction solution was stirred at room temperature for 3 h. The reaction solution was concentrated, and the residue was purified by reversed-phase chromatography (H₂O:ACN = 5%-100%) to obtain 1-9 (120 mg, yield: 38.9%) as a yellow solid.
LCMS (ESI) [M+H]⁺= 865.2;
¹H NMR (400 MHz, DMSO-*d*₆) δ 10.04 (d, J = 73.3 Hz, 1H), 9.46 (s, 1H), 9.36 (s, 1H), 8.86 (d, J = 56.6 Hz, 1H), 8.31 (d, J = 9.1 Hz, 2H), 8.08 (d, J = 9.2 Hz, 1H), 7.64 (d, J = 8.0 Hz, 1H), 7.59 - 7.55 (m, 3H), 7.42 (d, J = 8.3 Hz, 1H), 7.37 (d, J = 8.5 Hz, 1H), 7.27 (d, J = 8.5 Hz, 1H), 6.83 (d, J = 9.1 Hz, 1H), 5.19 (d, J = 14.8 Hz, 2H), 4.45 - 4.38 (m, 2H), 4.37 - 4.28 (m, 1H), 4.21 - 4.14 (m, 1H), 3.44 (s, 3H), 2.18 - 2.12 (m, 3H), 2.10 - 2.06 (m, 8H), 1.81 - 1.63 (m, 2H), 1.44 - 1.32 (m, 4H), 1.23 (d, J = 7.4 Hz, 7H), 0.94 - 0.88 (m, 6H).

Step 9: Compound 1-9 (36 mg, 0.041 mmol) and eribulin (20 mg, 0.027 mmol) were dissolved in DMF (2 mL). DIEA (7 mg, 0.054 mmol) was then added. After the addition was completed, the reaction solution was stirred at room temperature for 1 h. The crude product was purified by preparative chromatography (0.01% FA in water, ACN) to obtain the target compound DL001 (21.8 mg, yield: 52.6%) as a white solid.
LCMS (ESI) [M+H]⁺= 1455.6;
¹H NMR (400 MHz, DMSO-*d*₆) δ 10.01 (d, J = 69.6 Hz, 1H), 9.46 (s, 1H), 9.39 (s, 1H), 8.88 (d, J = 47.0 Hz, 1H), 8.50 (d, J = 7.3 Hz, 0.5H), 8.37 (s, 2H), 8.16 (d, J = 6.7 Hz, 0.5H), 7.67 - 7.46 (m, 3H), 7.23 (d, J = 8.5 Hz, 1H), 7.17 (d, J = 8.4 Hz, 1H), 7.07 (t, J = 5.4 Hz, 1H), 5.05 (s, 1H), 4.99 (s, 1H), 4.90 (d, J = 3.6 Hz, 1H), 4.87 (s, 1H), 4.83 (s, 1H), 4.75 (s, 1H), 4.63 (s, 1H), 4.55 (t, J = 4.0 Hz, 1H), 4.43 - 4.33 (m, 3H), 4.26 (d, J = 10.3 Hz, 1H), 4.21 - 4.14 (m, 2H), 4.11 (d, J = 10.6 Hz, 4H), 4.01 (d, J = 9.8 Hz, 1H), 3.84 - 3.73 (m, 4H), 3.72 - 3.65 (m, 3H), 3.44 (s, 3H), 3.27 - 3.22 (m, 5H), 2.96 (t, J = 5.6 Hz, 2H), 2.84 (d, J = 9.5 Hz, 1H), 2.80 - 2.64 (m, 3H), 2.60 - 2.55 (m, 1H), 2.36 - 2.29 (m, 2H), 2.27 - 2.23 (m, 2H), 2.21 - 2.11 (m, 6H), 2.08 (d, J = 4.8 Hz, 6H), 2.06 - 2.03 (m, 1H), 2.03 - 1.95 (m, 2H), 1.94 - 1.88 (m, 4H), 1.74 - 1.60 (m, 7H), 1.56 - 1.45 (m, 3H), 1.38 - 1.28 (m, 6H), 1.24 - 1.20 (m, 6H), 1.03 (d, J = 6.3 Hz, 3H), 0.97 (d, J = 12.0 Hz, 1H), 0.92 - 0.86 (m, 6H).

### Example 1.2: Preparation of Compound DL002

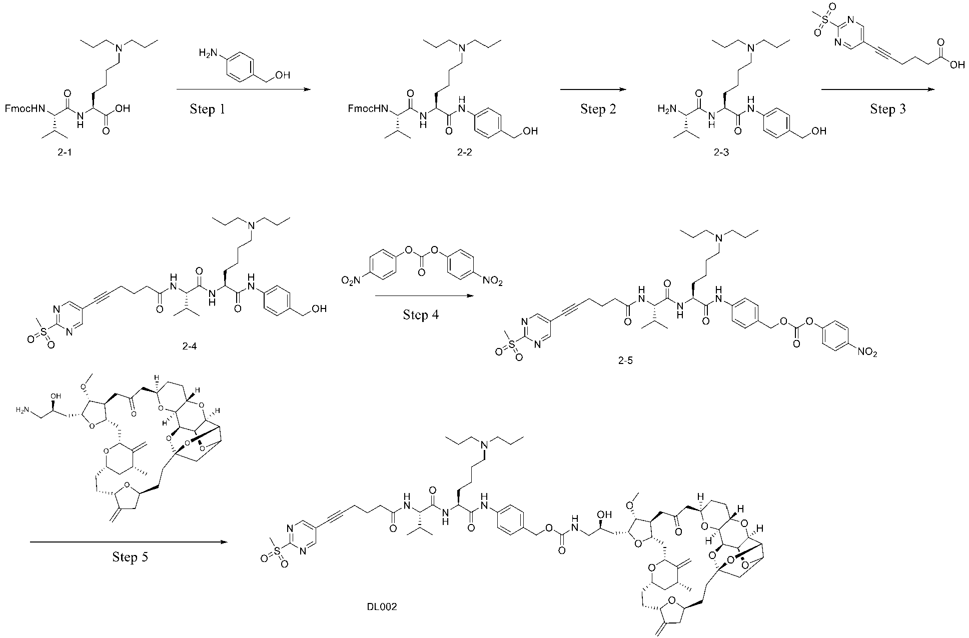

Step 1: Compound 2-1 (5.0 g, 9.07 mmol) was dissolved in DCM/anhydrous methanol (5/1) (50 mL), and 4-aminobenzyl alcohol (1.1 g, 9.07 mmol) and EEDQ (4.5 g, 18.2 mmol) were added. The reaction solution was stirred at room temperature for 2 h. The reaction solution was concentrated under reduced pressure and then purified by silica gel column chromatography (DCM:anhydrous methanol = 20:1) to obtain compound 2-2 (3.1 g, yield: 52.0%) as a yellow solid.
LCMS (ESI) [M+H]⁺= 657.5;
¹H NMR (400 MHz, DMSO-*d*₆) δ 10.05 (s, 1H), 8.15 (d, J = 7.5 Hz, 1H), 7.90 (d, J = 7.5 Hz, 2H), 7.74 (d, J = 7.5 Hz, 2H), 7.55 (d, J = 8.4 Hz, 2H), 7.50 - 7.39 (m, 3H), 7.32 (t, J = 7.4 Hz, 2H), 7.24 (d, J = 8.3 Hz, 2H), 5.11 (t, J = 5.6 Hz, 1H), 4.43 (d, J = 5.4 Hz, 3H), 4.34 - 4.16 (m, 3H), 3.92 (t, J = 8.0 Hz, 1H), 3.01 - 2.84 (m, 6H), 2.03 - 1.96 (m, 1H), 1.80 - 1.53 (m, 8H), 1.41 - 1.27 (m, 2H), 0.91 - 0.83 (m, 12H).

Step 2: Compound 2-2 (2.5 g, 3.81 mmol) was dissolved in DMF (35 mL), and DEA (557 mg, 7.62 mmol) was added. The reaction solution was left to react at room temperature for 1 h. The reaction solution was concentrated under reduced pressure to remove DEA, so as to obtain compound 2-3 (1.65 g, crude product). LCMS (ESI) [M+H]⁺= 435.4;

Step 3: Compound 2-3 (1.65 g, 3.81 mmol) was dissolved in DMF (35 mL), and 6-(2-(methylsulfonyl)pyrimidin-5-yl)hex-5-ynoic acid (1.02 g, 3.81 mmol), HATU (1.45 g, 3.81 mmol), and DIEA (1.23 g, 9.53 mmol) were added. The reaction solution was left to react at room temperature for 2 h. The reaction solution was purified by reversed-phase chromatography (0.05% TFA in water, ACN) to obtain compound 2-4 (1.1 g, yield: 42.3%) as a white solid.
LCMS (ESI) [M+H]⁺= 685.4;
¹H NMR (400 MHz, DMSO-*d*₆) δ 9.88 (s, 1H), 9.11 (s, 2H), 8.05 (d, J = 7.6 Hz, 1H), 7.94 (d, J = 8.5 Hz, 1H), 7.54 (d, J = 8.4 Hz, 2H), 7.22 (d, J = 8.3 Hz, 2H), 5.09 (t, J = 4.8 Hz, 1H), 4.42 (d, J = 3.4 Hz, 2H), 4.39 - 4.33 (m, 1H), 4.24 - 4.18 (m, 1H), 3.41 (s, 3H), 2.56 (t, J = 7.1 Hz, 2H), 2.45 - 2.29 (m, 7H), 2.03 - 1.96 (m, 2H), 1.87 - 1.78 (m, 2H), 1.76 - 1.68 (m, 1H), 1.67 - 1.58 (m, 1H), 1.45 - 1.26 (m, 8H), 0.90 - 0.78 (m, 12H).

Step 4: Under nitrogen atmosphere, compound 2-4 (510 mg, 0.746 mmol) was dissolved in extra-dry DCM (10 mL), and bis(4-nitrophenyl)carbonate (453 mg, 1.491 mmol) and DIEA (192 mg, 1.491 mmol) were added. The reaction solution was left to react for 2 h. The reaction solution was purified by reversed-phase chromatography (0.05% TFA in water, ACN) to obtain compound 2-5 (300 mg, yield: 44.7%) as a pale yellow solid.
LCMS (ESI) [M+H]⁺= 850.4;
¹H NMR (400 MHz, DMSO-*d*₆) δ 10.10 (s, 1H), 9.11 (s, 2H), 8.34 - 8.28 (m, 2H), 8.19 (d, J = 7.1 Hz, 1H), 7.94 (d, J = 8.2 Hz, 1H), 7.65 (d, J = 8.5 Hz, 2H), 7.59 - 7.53 (m, 2H), 7.42 (d, J = 8.6 Hz, 2H), 5.24 (s, 2H), 4.43 - 4.36 (m, 1H), 4.23 - 4.18 (m, 1H), 3.41 (s, 3H), 3.05 - 2.95 (m, 6H), 2.57 (d, J = 7.2 Hz, 2H), 2.44 - 2.36 (m, 2H), 2.36 - 2.27 (m, 2H), 2.03 - 1.94 (m, 1H), 1.88 - 1.75 (m, 3H), 1.67 - 1.54 (m, 7H), 0.92 - 0.84 (m, 12H).

Step 5: Eribulin (50 mg, 0.07 mmol) was dissolved in DMF (1.5 mL), and compound 2-5 (58 mg, 0.07 mmol) and DIEA (22 mg, 0.18 mmol) were sequentially added to the reaction solution. The resulting reaction solution was stirred at room temperature for 2 h. The reaction solution was directly purified by reversed-phase chromatography (0.05% TFA in water, ACN) to obtain the target compound DL002 (52 mg, yield: 53 %).
LCMS (ESI) [M+H]⁺= 1441.2;
¹H NMR (400 MHz, DMSO-*d*₆) δ 10.02 (s, 1H), 9.11 (s, 2H), 8.98 (s, 1H), 8.17 (d, J = 7.8 Hz, 1H), 7.94 (d, J = 8.6 Hz, 1H), 7.58 (d, J = 8.4 Hz, 2H), 7.28 (d, J = 8.5 Hz, 2H), 7.06 (t, J = 6.5 Hz, 1H), 5.05 (s, 1H), 5.00 (s, 1H), 4.93 (d, J = 3.3 Hz, 2H), 4.83 (s, 1H), 4.75 (s, 1H), 4.65 - 4.62 (m, 1H), 4.57 - 4.53 (m, 1H), 4.43 - 4.35 (m, 2H), 4.30 - 4.24 (m, 2H), 4.21 - 4.17 (m, 2H), 4.11 - 4.09 (m, 3H), 4.04 - 4.00 (m, 2H), 3.84 - 3.73 (m, 4H), 3.72 - 3.64 (m, 2H), 3.57 - 3.46 (m, 4H), 3.41 (s, 3H), 3.28 - 3.22 (m, 4H), 3.05 - 2.94 (m, 9H), 2.84 (d, J = 9.9 Hz, 1H), 2.79 - 2.63 (m, 3H), 2.60 - 2.53 (m, 4H), 2.44 - 2.36 (m, 2H), 2.35 - 2.29 (m, 3H), 2.28 - 2.23 (m, 2H), 2.21 - 2.11 (m, 2H), 2.02 - 1.97 (m, 2H), 1.94 - 1.91 (m, 3H), 1.85 - 1.79 (m, 2H), 1.69 - 1.57 (m, 11H), 1.36 - 1.27 (m, 5H), 1.03 (d, J = 6.5 Hz, 3H), 0.91 - 0.83 (m, 12H).

### Example 1.3: Preparation of Compound DL003

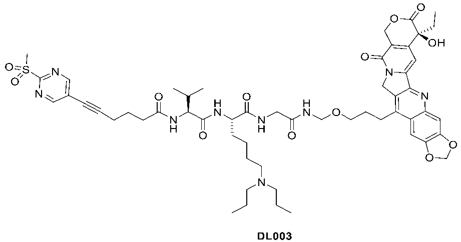

DL003 was prepared according to the method in WO2022170971.

### Example 1.4: Preparation of Compound DL004

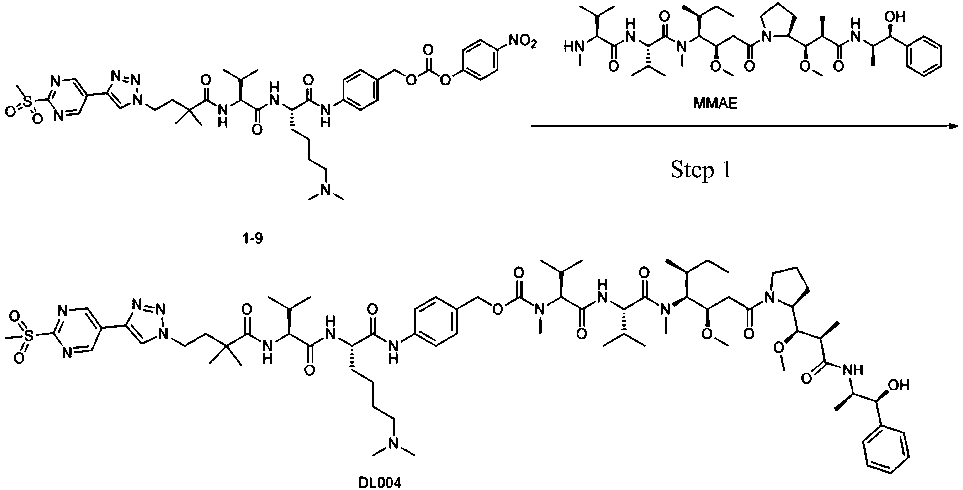

Step 1: The compound MMAE (108 mg, 0.15 mmol) was dissolved in DMF (2 mL), and compound 1-9 (130 mg, 0.15 mmol), 1-hydroxybenzotriazole (20.3 mg, 0.15 mmol), and DIEA (58.2 mg, 0.45 mmol) were sequentially added. The reaction solution was left to react at room temperature for 4 h. The reaction solution was directly purified by preparative high-performance liquid chromatography (0.1% FA in water, ACN) to obtain the target compound DL004 (55 mg, yield: 25.4%).
LCMS (ESI) [M/2+H]⁺ =722.52
¹H NMR (400 MHz, DMSO-*d*₆) δ 10.06 (s, 1H), 9.46 (s, 2H), 8.93 (s, 1H), 8.22 (s, 1H), 8.10 (d, J = 7.3 Hz, 1H), 8.02 (d, J = 8.5 Hz, 0.5H), 7.88 (d, J = 8.7 Hz, 0.5H), 7.61 (d, J = 8.5 Hz, 0.5H), 7.56-7.50 (m, 2H), 7.42 (d, J = 8.4 Hz, 1H), 7.35-7.08 (m, 7.5H), 5.09-4.94 (m, 2H), 4.76-4.38 (m, 6H), 4.29-4.16 (m, 2H), 4.08-3.91 (m, 3H), 3.81-3.75 (m, 1H), 3.24-2.82 (m, 15H), 2.45-1.27 (m, 31H), 1.25-1.19 (m, 6H), 1.05-0.75 (m, 30H).

### Example 1.5: Preparation of Compound DL005

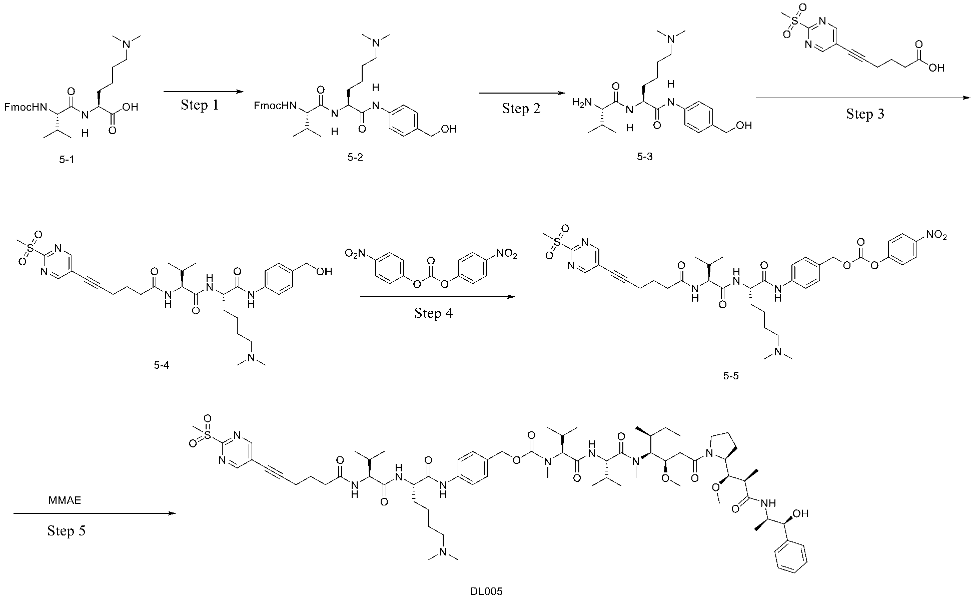

Step 1: Compound 5-1 (10 g, 20.2 mmol) was dissolved in DCM/anhydrous methanol (5/1) (36 mL), and 4-aminobenzyl alcohol (2.5 g, 20.2 mmol) and EEDQ (10 g, 40.4 mmol) were added. The reaction solution was stirred at room temperature for 2 h. The reaction solution was concentrated and then purified by column chromatography (DCM:methanol = 15:1) to obtain the target compound 5-2 (7 g, yield: 57.9%) as a white solid.
LCMS (ESI) [M+H]⁺= 601.4;
¹H NMR (400 MHz, DMSO-*d*₆) δ 10.09 (s, 1H), 8.18 (d, J = 7.7 Hz, 1H), 7.90 (d, J = 7.5 Hz, 2H), 7.79 - 7.72 (m, 2H), 7.56 (d, J = 8.4 Hz, 2H), 7.48 - 7.39 (m, 3H), 7.33 (t, J = 7.4 Hz, 2H), 7.23 (d, J = 8.4 Hz, 2H), 4.47 - 4.40 (m, 3H), 4.34 - 4.28 (m, 1H), 4.25 - 4.20 (m, 2H), 3.96 - 3.90 (m, 1H), 3.02 - 2.92 (m, 2H), 2.69(s,3H), 2.68 (s, 3H), 2.07 - 1.96 (m, 1H), 1.83 - 1.73 (m, 1H), 1.71 - 1.60 (m, 3H), 1.42 - 1.29 (m, 2H), 0.90 - 0.85 (m, 6H).

Step 2: Compound 5-2 (3.5 g, 5.8 mmol) was dissolved in DMF (15 mL), and DEA (852 mg, 11.7 mmol) was added. The reaction solution was stirred at room temperature for 1 h. The reaction solution was concentrated under reduced pressure to obtain compound 5-3 (2.2 g, crude), which was directly used in the next step.
LCMS (ESI) [M+H]⁺= 379.3;

Step 3: 6-(2-(Methylsulfonyl)pyrimidin-5-yl)hex-5-ynoic acid (1.34 g, 5.02 mmol) was dissolved in DMF (10 mL), and HATU (1.9 g, 5.02 mmol) and DIEA (1.61 g, 12.56 mmol) were sequentially added. The reaction solution was stirred at 25 °C for 0.5 h, and compound 5-3 (1.9 g, 5.02 mmol) was added. The resulting reaction solution was stirred at 25 °C for 1 h. The reaction solution was purified by reversed-phase column chromatography (0.1% TFA in water, ACN) to obtain the target compound 5-4 (2 g, yield: 55.6%).
LCMS (ESI) [M+H]⁺= 629.6;
¹H NMR (400 MHz, DMSO-*d*₆) δ 9.93 (s, 1H), 9.12 (s, 2H), 8.16 (d, J = 7.6 Hz, 1H), 7.95 (d, J = 8.4 Hz, 1H), 7.54 (d, J = 8.4 Hz, 2H), 7.23 (d, J = 8.4 Hz, 2H), 4.42 (s, 2H), 4.40 - 4.35 (m, 1H), 4.23 - 4.18 (m, 1H), 3.41 (s, 3H), 3.41 - 3.40 (m, 1H), 3.06 - 2.98 (m, 2H), 2.75(s,3H), 2.74 (s, 3H), 2.56 (t, J = 7.0 Hz, 2H), 2.41 - 2.33 (m, 2H), 2.01 - 1.95 (m, 1H), 1.87 - 1.80 (m, 2H), 1.68 - 1.59 (m, 3H), 1.38 - 1.27 (m, 2H), 0.89 - 0.82 (m, 6H).

Step 4: Compound 5-4 (1.8 g, 2.87 mmol) was dissolved in DMF (10 mL), and the compounds bis(4-nitrophenyl)carbonate (1.74 g, 5.74 mmol) and DIEA (740 mg, 5.74 mmol) were added. The reaction solution was stirred at 25 °C for 2 h. After the reaction was substantially completed as detected by LCMS, the reaction solution was slurried with MTBE (80 mL) and anhydrous diethyl ether (100 mL) to obtain the target compound 5-5 (1.8 g, 79.1% yield) as a pale yellow solid.
LCMS (ESI) [M+H]⁺= 794.6;
¹H NMR (400 MHz, DMSO-*d*₆) δ 10.08 (s, 1H), 9.11 (s, 2H), 8.31 (d, J = 9.1 Hz, 2H), 8.17 (d, J = 7.5 Hz, 1H), 7.93 (d, J = 8.5 Hz, 1H), 7.64 (d, J = 8.5 Hz, 2H), 7.56 (d, J = 9.1 Hz, 2H), 7.41 (d, J = 8.5 Hz, 2H), 5.24 (s, 2H), 4.44 - 4.36 (m, 1H), 4.25 - 4.17 (m, 1H), 3.41 (s, 3H), 2.90 - 2.79 (m, 2H), 2.68 - 2.57 (m, 6H), 2.58 - 2.54 (m, 2H), 2.42 - 2.31 (m, 2H), 2.05 - 1.92 (m, 1H), 1.88 - 1.78 (m, 2H), 1.77 - 1.62 (m, 2H), 1.61 - 1.53 (m, 2H), 1.41 - 1.26 (m, 2H), 0.89 - 0.82 (m, 6H).

Step 5: The compound MMAE (108 mg, 0.15 mmol) was dissolved in DMF (2 mL), and compound 5-5 (119 mg, 0.15 mmol), 1-hydroxybenzotriazole (20.3 mg, 0.15 mmol), and DIEA (58.2 mg, 0.45 mmol) were added. The reaction solution was left to react at 22 °C for 4 h. The reaction solution was directly purified by preparative high-performance liquid chromatography (0.1% FA in water, ACN) to obtain the target compound DL005 (75 mg, yield: 36.4%).
LCMS (ESI) [M/2+H]⁺=686.98
¹H NMR (400 MHz, DMSO-*d*₆) δ 10.07-9.86 (m, 1H), 9.11 (s, 2H), 8.22 (s, 1H), 8.14-7.98 (m, 2H), 7.96-7.85 (m, 1.5H), 7.66-7.50 (m, 2.5H), 7.36-7.23 (m, 6H), 7.21-7.09 (m, 1H), 5.60-5.19 (m, 1H), 5.13-4.90 (m, 2H), 4.79-4.55 (m, 1H), 4.54-4.14 (m, 6H), 4.08-3.87 (m, 3H), 3.83-3.74 (m, 1H), 3.20-2.82 (m, 14H), 2.55 (t, J = 7.1 Hz, 2H), 2.44-2.21 (m, 7H), 2.15-1.28 (m, 26H), 1.06-0.97 (m, 6H), 0.88-0.74 (m, 24H).

### Example 1.6: Preparation of Compound DL006

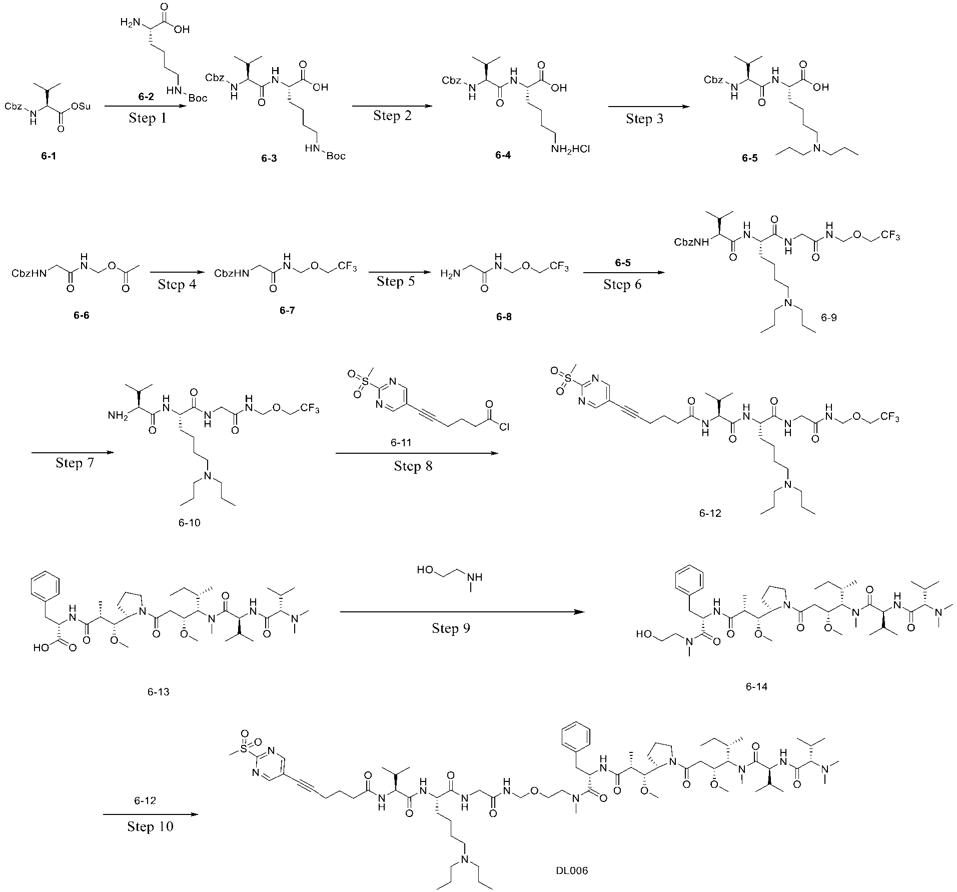

Step 1: Compound 6-2 (35.35 g, 143.53 mmol) was weighed and added to a solution of NaHCO₃ (24.12 g, 287.06 mmol) in water (250 mL), and a solution of 6-1 (50.0 g, 143.53 mmol) in THF (250 mL) was added dropwise in an ice-water bath. After the dropwise addition was completed, the reaction system was stirred at room temperature for 4 h, and the reaction solution was concentrated to remove the organic solvent. Hydrochloric acid (4 mol/L) was added to adjust the pH to 5. The reaction solution was stirred for 1 h and then filtered under vacuum, and the filter cake was slurried with DCM (700 mL) for 30 min, then filtered under vacuum, and dried to obtain the target product 6-3 (65.0 g, yield: 94.5%).

¹H NMR (400 MHz, DMSO-*d*₆) δ 8.07 (d, J = 8.0 Hz, 1H), 7.36 - 7.30 (m, 5H), 7.23 (d, J = 8.0 Hz, 1H), 6.74 (t, J = 8 Hz, 1H), 5.04 (s, 2H), 4.18-4.12 (m, 1H), 3.94-3.90 (m, 1H), 2.91-2.87 (m, 2H), 1.97-1.85 (m, 1H), 1.74 - 1.64 (m, 1H), 1.62 - 1.52 (m, 1H), 1.42 - 1.23 (m, 13H), 0.90 - 0.83 (m, 6H).

Step 2: Compound 6-3 (50.0 g, 104.26 mmol) was weighed and added to THF (20 mL), and then concentrated hydrochloric acid (17.2 mL, 206.40 mmol) was added. The reaction solution was heated to 60 °C, left to react for 1 h, and cooled to room temperature, which was directly used in the next step.

¹H NMR (400 MHz, DMSO-*d*₆) δ 12.59 (s, 1H), 8.19 (d, J = 8.0 Hz, 1H), 7.96 (s, 3H), 7.40 - 7.34 (m, 5H), 8.27 (d, J = 8.0 Hz, 1H), 5.06 (s, 2H), 4.22 - 4.17 (m, 1H), 3.96 - 3.92 (m, 1H), 2.80 - 2.77 (m, 2H), 2.04-2.00 (m, 1H), 1.63 - 1.57 (m, 4H), 1.41 - 1.39 (m, 2H), 0.93 - 0.87 (m, 6H).

Step 3: THF (300 mL) was added to the solution obtained in step 2, sodium cyanoborohydride (19.7 g, 312.78 mmol) was added in an ice-water bath, and n-propionaldehyde (24.3 g, 417.04 mmol) was added. The reaction solution was heated to room temperature and stirred for 1 h. Hydrochloric acid was added to adjust the pH to 4-5, followed by stirring for 30 min. The reaction system was concentrated to remove THF, water (200 mL) and DCM (200 mL) were added, and the organic phase was collected. 60.0 g of anhydrous sodium sulfate was added to the aqueous phase, followed by re-extraction with DCM (100 mL). The organic phases were combined, dried over anhydrous sodium sulfate, and filtered to remove the insoluble substances. The residue was concentrated to obtain the target compound 6-5 (38.0 g, two-step yield: 78.6%).

¹H NMR (400 MHz, DMSO-*d*₆) δ 7.93 (d, J = 8.0 Hz, 1H), 7.35 - 7.26 (m, 6H), 5.03 (s, 2H), 4.15 - 4.10 (m, 1H), 3.91 - 3.87 (m, 1H), 2.76 - 2.58 (m, 6H), 2.00 - 1.96 (m, 1H), 1.82 - 1.19 (m, 10H), 0.89 - 0.84(m, 12H). Step 4: Compound 6-6 (5 g, 17.84 mmol) was added to trifluoroethanol (30 mL) under argon atmosphere, and the mixture was stirred at 40 °C for dissolution. Then, a solution of HCl in DMF (2.1 mL, 0.4 mol/L) was added, and the reaction solution was stirred at 40 °C for 2.5 h. The reaction solution was cooled to room temperature and then added to 120 mL of an aqueous sodium bicarbonate solution (1%, W/V). The resulting reaction solution was stirred and filtered under vacuum, and the filter cake was dried to obtain the target compound 6-7 (5.3 g, yield: 92.8%).

¹H NMR (400 MHz, DMSO-*d*₆) δ 8.88 (t, J = 6.5 Hz, 1H), 7.54 (t, J = 6.0 Hz, 1H), 7.40-7.32 (m, 5H), 5.07 (s, 2H), 4.70 (d, J = 6.8 Hz, 2H), 4.03 (q, J = 9.4 Hz, 2H), 3.68 (d, J = 6.1 Hz, 2H).

Step 5: Compound 6-7 (1.00 g, 3.29 mmol) was dissolved in THF (5 mL), and 5% Pd/C (50 mg) was added. The reaction solution was purged with hydrogen and then left to react at room temperature for 3 h. DMF (5 mL) was added to the reaction solution. The resulting reaction solution was filtered and concentrated to remove THF to obtain a solution of the target compound 6-8 in DMF (quantitative yield), which was directly used in the next step.

The nuclear magnetic hydrogen spectrum of the HOBt salt of compound 6-8 was as follows: ¹H NMR (400 MHz, DMSO-*d*₆) δ 9.18 (br, 1H), 7.79 - 7.71 (m, 1H), 7.49 - 7.41 (m, 1H), 7.25-7.18 (m, 2H), 4.74 (d, J = 4.9 Hz, 2H), 4.06 (q, J = 9.4 Hz, 2H), 3.47 (s, 2H).

Step 6: Compound 6-5 (1.52 g, 3.29 mmol) was added to the solution of compound 6-8 in DMF obtained in step 5. The temperature was controlled at -10 °C, DMTMM (1.16 g, 3.98 mmol) was added. The reaction system was left to react for 1.5 h. EA (50 mL) was added to the reaction system, and the reaction system was washed successively with a saturated aqueous NaHCO₃ solution (15 mL × 3) and saturated brine (15 mL). The organic phase was dried over anhydrous sodium sulfate and filtered under vacuum, and the filtrate was concentrated to dryness. The resulting residue was dissolved in EA (3 mL) and added to n-heptane (30 mL). The mixture was stirred to precipitate a solid and filtered under vacuum, and the filter cake was the target compound 6-9 (1.55 g, yield: 74.9%).

LCMS (ESI) [M+H]⁺= 632.3.

¹H NMR (400 MHz, DMSO-*d*₆) δ 8.85 - 8.73 (m, 1H), 8.37 - 8.26 (m, 1H), 8.03 (d, J = 7.0 Hz, 1H), 7.40-7.32 (m, 6H), 5.10 - 5.01 (m, 2H), 4.73-4.66 (m, 2H), 4.29 - 4.20 (m, 1H), 4.02 (q, J = 9.4 Hz, 2H), 3.93 (dd, J = 13.8, 6.6 Hz, 1H), 3.85 - 3.67 (m, 2H), 2.38-2.32 (m, 6H), 2.02-1.96 (m, 1H), 1.75 - 1.51 (m, 2H), 1.46 - 1.23 (m, 8H), 0.90-0.82(m, 12H).

Step 7: Compound 6-9 (4 g, 6.3 mmol) was dissolved in 100 mL of methanol, and a program was set as follows by using a fully automatic hydrogenation microreactor and using palladium hydroxide loaded by aluminum oxide as a catalyst: temperature: 50 °C; pressure: 2.5 Mpa; flow rate of the starting material solution: 0.3 mL/min; and flow rate of hydrogen: 30 mL/min. The effluent solution was concentrated under reduced pressure to obtain the target compound 6-10 (3.0 g, yield: 95.1%).

LCMS (ESI) [M+H]⁺= 498.3.

¹H NMR (400 MHz, DMSO-*d*₆) δ 8.89 - 8.77 (m, 1H), 8.42 - 8.30 (m, 1H), 8.14 (s, 1H), 4.70 (p, J = 10.3 Hz, 2H), 4.28 (s, 1H), 4.02 (q, J = 9.4 Hz, 2H), 3.80 (dd, J = 15.7, 4.9 Hz, 1H), 3.75 - 3.67 (m, 1H), 3.20 (s, 1H), 3.11 (d, J = 4.8 Hz, 1H), 2.35 (dd, J = 14.4, 7.3 Hz, 6H), 2.04 - 1.90 (m, 1H), 1.76 - 1.51 (m, 2H), 1.45 -1.20 (m, 8H), 0.95 - 0.77 (m, 12H).

Step 8: Compound 6-10 (50.0 g, 100 mmol) was dissolved in 350 mL of ACN. The reaction solution was cooled to 10 °C, and DIPEA (3.25 g, 25 mmol) was added. A solution of compound 6-11 (prepared from 6-(2-(methylsulfonyl)pyrimidin-5-yl)hex-5-ynoic acid and thionyl chloride, prepared for immediate use) (34.4 g, 120 mmol) in ACN (150 mL) was added dropwise. The reaction was continued for 1 h, and then 2-methyltetrahydrofuran (2.1 L) was added to the reaction solution to precipitate a solid. The mixture was filtered under vacuum, and the filter cake was dried to obtain a hydrochloride of the target compound 6-12 (71.8 g, yield: 91.6%).
LCMS (ESI) [M+H]⁺= 748.4
¹H NMR (400 MHz, DMSO-*d*₆) δ 9.15 (s, 2H), 8.78 (t, J = 6.8 Hz, 1H), 8.24 (t, J = 5.8 Hz, 1H), 8.05 (d, J = 7.3 Hz, 1H), 7.95 (d, J = 8.6 Hz, 1H), 4.75-4.66 (m, 2H), 4.24-4.19 (m, 2H), 4.03 (q, J = 9.4 Hz, 2H), 3.78-3.76 (m, 2H), 3.44 (s, 3H), 2.58 (t, J = 7.1 Hz, 2H), 2.46-2.29 (m, 8H), 2.03-1.95 (m, 1H), 1.89-1.81 (m, 2H), 1.73-1.66 (m, 1H), 1.62-1.56 (m, 1H), 1.41-1.36 (m, 6H), 1.31-1.25 (m, 2H), 0.89-0.83 (m, 12H).

Step 9: Compound 6-13 (100.00 mg, 0.13 mmol) was dissolved in DMF (1 mL), and the compound N-methylhydroxyethylamine (9.76 mg, 0.13 mmol) was added to the solution. The reaction solution was cooled to -5 °C with stirring, and then DMTMM (53.96 mg, 0.20 mmol) was added. The reaction solution was stirred for 2 h. The reaction solution was directly purified by preparative high-performance liquid chromatography (0.05% FA in water, ACN) to obtain the target compound 6-14 (57 mg, yield: 54.6%).
LCMS (ESI) [M/2+H]⁺=402.56;
¹H NMR (400 MHz, DMSO-*d*₆) δ 8.55-8.44 (m, 0.5H), 8.32-8.17 (m, 0.5H), 8.13-7.98 (m, 1H), 7.32-7.08 (m, 5H), 5.25-4.80 (m, 2H), 4.78-4.40 (m, 3H), 3.99-3.49 (m, 6H), 3.25-3.14 (m, 8H), 3.09-2.62 (m, 10H), 2.38-1.29 (m, 17H), 1.01-0.69 (m, 21H).

Step 10: Compound 6-14 (47.00 mg, 0.057 mmol) was dissolved in DMF (0.5 mL), and the hydrochloride of 6-12 (138.25 mg, 0.17 mmol) and HCl-DMF (4 M, 0.5 mL) were added. The reaction solution was stirred at 25 °C for 5 h. The reaction was monitored by LCMS. The reaction solution was directly purified by preparative high-performance liquid chromatography (0.1% TFA in water, ACN) to obtain the target compound DL006 (8 mg, yield: 8.4%).
LCMS (ESI) [M/3+H]⁺ =484.85;
¹H NMR (400 MHz, DMSO-*d*₆) δ 9.65-9.49 (m, 1H), 9.20-9.05 (m, 3H), 8.99-8.89 (m, 1H), 8.72-8.61 (m, 1H), 8.58-8.46 (m, 0.5H), 8.37-8.25 (m, 0.5H), 8.24-8.18 (m, 1H), 8.14-8.07 (m, 1H), 7.99-7.90 (m, 1H), 7.29-7.16 (m, 5H), 5.09-4.54 (m, 6H), 4.29-4.16 (m, 2H), 4.03-3.97 (m, 1H), 3.79-3.72 (m, 3H), 3.33-3.18 (m, 10H), 3.10-2.98 (m, 12H), 2.91-2.68 (m, 11H), 2.46-2.20 (m, 7H), 2.16-1.95 (m, 3H), 1.89-1.58 (m, 14H), 1.55-1.26 (m, 6H), 1.05-0.78 (m, 35H).

### Example 1.7: Preparation of Compound DL007

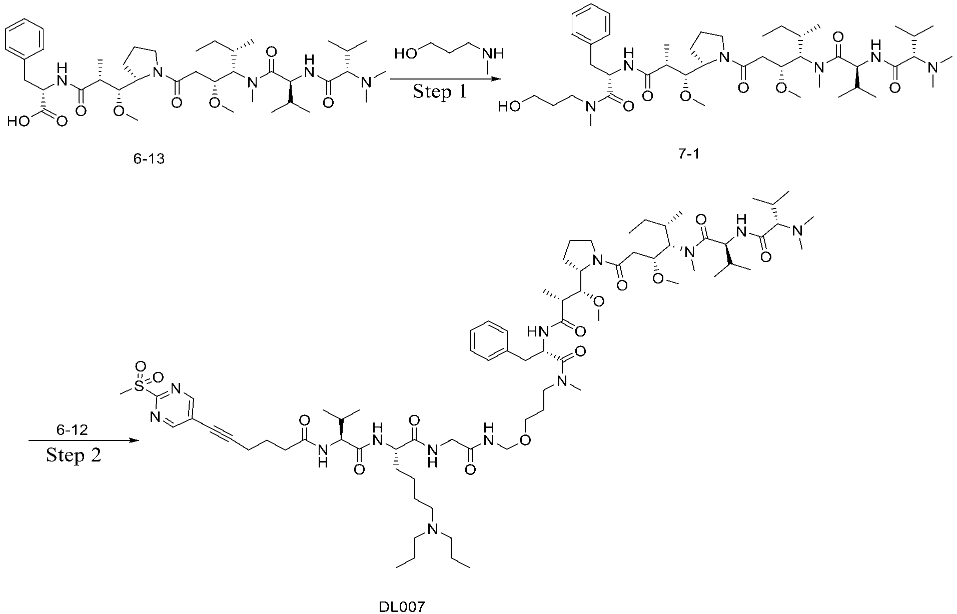

Step 1: Compound 6-13 (30 mg, 0.040 mmol) was dissolved in DMF (0.5 mL), and N-methylhydroxypropylamine (10.7 mg, 0.12 mmol) was added. The reaction solution was cooled to -5 °C, and DMTMM monohydrate (15.3 mg, 0.052 mmol) was added. The reaction solution was left to react for 1 h. The reaction solution was directly purified by preparative high-performance liquid chromatography (0.1% TFA in water/ACN) to obtain the target compound 7-1 (17 mg, yield: 45.6%).
LCMS (ESI) [M/2+H]⁺ =409.57
¹H NMR (400 MHz, DMSO-*d*₆) δ 9.61 (s, 1H), 9.05-8.84 (m, 1H), 8.56 (d, J = 8.7 Hz, 0.5H), 8.36-8.22 (m, 0.5H), 7.33-7.16 (m, 5H), 5.16-4.83 (m, 1H), 4.79-4.56 (m, 2H), 4.05-3.92 (m, 1H), 3.84-3.69 (m, 2H), 3.37-3.21 (m, 8H), 3.11-2.99 (m, 5H), 2.87-2.74 (m, 9H), 2.50-1.97 (m, 6H), 1.93-1.20 (m, 9H), 1.09-0.73 (m, 25H).

Step 2: Compound 7-1 (30 mg, 0.035 mmol) was dissolved in DMF (0.5 mL), and the hydrochloride of 6-12 (87 mg, 0.11 mmol) and HCl-DMF (4 M, 0.5 mL) were added. The reaction solution was left to react at room temperature for 5 h. The reaction solution was directly purified by preparative high-performance liquid chromatography (0.1 % TFA in water/ACN) to obtain a trifluoroacetate of the target compound DL007 (13 mg, yield: 23.8%).
LCMS (ESI) [M/3+H]⁺ =489.29
¹H NMR (400 MHz, DMSO-*d*₆) δ 9.57 (s, 1H), 9.19-9.02 (m, 3H), 8.99-8.83 (m, 1H), 8.67-8.53 (m, 1H), 8.52-8.45 (m, 0.5H), 8.33-8.21 (m, 0.5H), 8.17 (t, 1H), 8.08 (t, J = 6.9 Hz, 1H), 7.96-7.84 (m, 1H), 7.25-7.13 (m, 5H), 5.03-4.40 (m, 6H), 4.28-4.13 (m, 2H), 4.00-3.93 (m, 1H), 3.76-3.70 (m, 3H), 3.33-3.14 (m, 12H), 3.07-2.97 (m, 9H), 2.83-2.73 (m, 9H), 2.55 (t, J = 7.1 Hz, 2H), 2.46-1.90 (m, 9H), 1.88-1.18 (m, 22H), 1.03-0.73 (m, 37H).

### Example 1.8: Preparation of Compound DL008

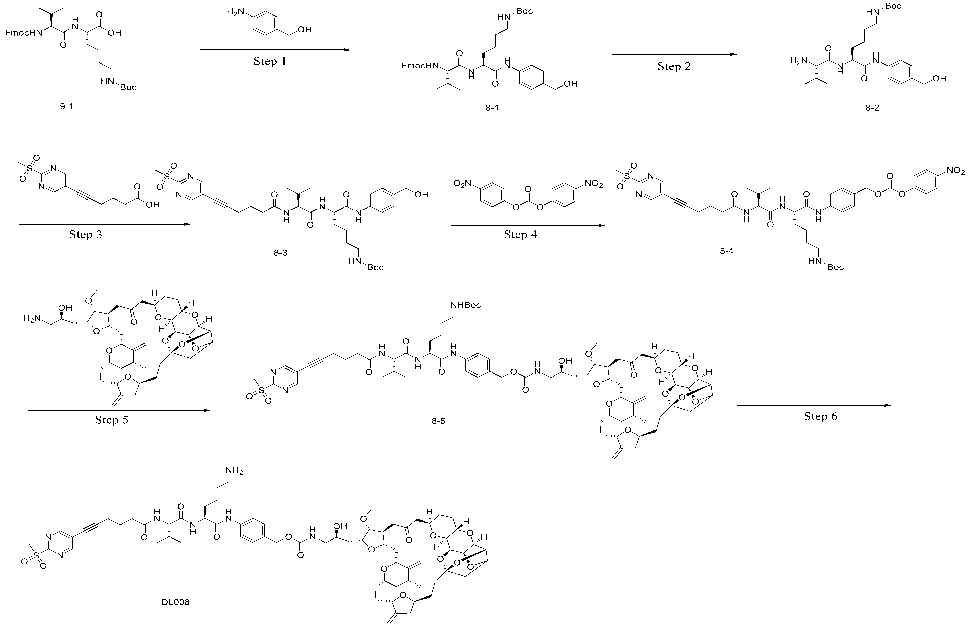

Step 1: Compound 9-1 (5 g, 8.8 mmol) was dissolved in a mixed solution of DCM (30 mL) and methanol (6 mL), and p-aminobenzyl alcohol (1.1 g, 8.8 mmol) and EEDQ (4.3 g, 17.6 mmol) were added. The reaction solution was stirred at room temperature for 2 h. The reaction solution was concentrated and then purified by column chromatography (DCM:methanol = 20:1) to obtain the target compound 8-1 (2.5 g, yield: 42.4%).
LCMS (ESI) [M+H]⁺= 673.5;
¹H NMR (400 MHz, DMSO-*d₆*)δ 9.94 (s, 1H), 8.04 (d, *J* = 7.6 Hz, 1H), 7.89 (d, *J* = 7.5 Hz, 2H), 7.74 (t, *J* = 8.0 Hz, 2H), 7.53 (d, *J* = 8.4 Hz, 2H), 7.47 - 7.38 (m, 3H), 7.32 (t, *J* = 7.4 Hz, 2H), 7.23 (d, *J* = 8.4 Hz, 2H), 6.72 (t, *J* = 5.2 Hz, 1H), 5.09 (t, *J* = 5.7 Hz, 1H), 4.48 - 4.34 (m, 3H), 4.32 - 4.17 (m, 3H), 3.96 - 3.87 (m, 1H), 2.95 - 2.77 (m, 2H), 2.06 - 1.92 (m, 1H), 1.65 (d, *J* = 30.4 Hz, 2H), 1.41 - 1.35 (m, 2H), 1.33 (s, 9H), 1.24 (s, 2H), 0.92 - 0.82 (m, 6H).

Step 2: Compound 8-1 (3 g, 4.46 mmol) was dissolved in DMF (15 mL), and DEA (391 mg, 5.36 mmol) was added. The reaction solution was stirred at 25 °C for 1 h. The reaction solution was concentrated by rotary evaporation to obtain the target compound 8-2 (2.0 g, yield: 95.8%) as a colorless oil.
LCMS (ESI) [M+H]⁺= 451.1;

Step 3: 6-(2-(Methylsulfonyl)pyrimidin-5-yl)hex-5-ynoic acid (1.2 g, 4.46 mmol) was dissolved in DMF (15 mL), and HATU (1.7 g, 4.46 mmol) and DIPEA (1.4 g, 11.15 mmol) were sequentially added. The reaction solution was stirred at 25 °C for 0.5 h, and 8-2 (2 g, 4.46 mmol) was added. The resulting reaction solution was stirred at 25 °C for 1 h. The reaction solution was purified by reversed-phase column chromatography (ACN/H₂O containing 0.1% TFA = 5%-60%) to obtain the target compound 8-3 (1 g, yield: 37.0%).
LCMS (ESI) [M+H]⁺= 701.5;
¹H NMR (400 MHz, DMSO-*d*₆) δ 9.87 (s, 1H), 9.11 (s, 2H), 8.05 (d, *J* = 7.8 Hz, 1H), 7.93 (d, *J* = 8.5 Hz, 1H), 7.53 (d, *J =* 8.5 Hz, 2H), 7.22 (d, *J =* 8.3 Hz, 2H), 6.75 (t, *J =* 4.4 Hz, 1H), 5.08 (t, *J* = 5.6 Hz, 1H), 4.42 (d, *J* = 5.6 Hz, 2H), 4.38 - 4.30 (m, 1H), 4.21 (t, *J* = 7.5 Hz, 1H), 3.41 (s, 3H), 2.92 - 2.84 (m, 2H), 2.55 (t, *J =* 7.3 Hz, 3H), 2.43 - 2.38 (m, 1H), 2.37 - 2.32 (m, 1H), 2.03 - 1.96 (m, 2H), 1.88 - 1.79 (m, 2H), 1.73 - 1.54 (m, 2H), 1.52 - 1.38 (m, 2H), 1.35 (s, 9H), 0.88 - 0.83 (m, 6H).

Step 4: Compound 8-3 (1.0 g, 1.43 mmol) was dissolved in DMF (10 mL), and bis(4-nitrophenyl)carbonate (869 mg, 2.86 mmol) and DIPEA (369 mg, 2.86 mmol) were added. The reaction solution was stirred at 25 °C for 2 h. The reaction solution was slurried with MTBE (80 mL) for 1 h and filtered under vacuum. The filter cake was slurried with anhydrous diethyl ether (80 mL) for 1 h, filtered under vacuum, and dried to obtain the target compound 8-4 (1.1 g, yield: 89.4%).
LCMS (ESI) [M+H-Boc]⁺= 766.4;
¹H NMR (400 MHz, DMSO-*d*₆) δ 10.06 (s, 1H), 9.12 (s, 2H), 8.35 - 8.28 (m, 2H), 8.00 - 7.89 (m, 2H), 7.64 (d, *J* = 8.5 Hz, 2H), 7.59 - 7.54 (m, 2H), 7.41 (d, *J* = 8.6 Hz, 2H), 6.78 (t, *J* = 5.3 Hz, 1H), 5.23 (s, 2H), 4.39 - 4.30 (m, 1H), 4.27 - 4.18 (m, 1H), 3.41 (s, 3H), 2.92 - 2.90 (m, 1H), 2.89 - 2.85 (m, 2H), 2.55 (t, *J* = 7.1 Hz, 2H), 2.43 - 2.31 (m, 2H), 2.03 - 1.95 (m, 1H), 1.88 - 1.77 (m, 2H), 1.74 - 1.56 (m, 2H), 1.42 - 1.35 (m, 3H), 1.35 (s, 9H), 0.89 - 0.82 (m, 6H).

Step 5: Compound 8-4 (18 mg, 0.02 mmol) was dissolved in DMF (1.5 mL), and eribulin (15 mg, 0.02 mmol) and DIPEA (7 mg, 0.05 mmol) were sequentially added to the reaction solution. The resulting reaction solution was stirred at room temperature for 2 h. The reaction solution was directly purified by reversed-phase chromatography (ACN/H₂O containing 0.05% TFA = 5%-50%) to obtain the target compound 8-5 (20 mg, yield: 67%).
LCMS (ESI) [M+Na]⁺ = 1479.0;

Step 6: Compound 8-5 (20 mg, 0.02 mmol) was dissolved in DCM (8 mL), and TFA (0.4 mL) was added to the reaction solution. The resulting reaction solution was stirred at room temperature for 2 h. The reaction was monitored by LCMS. The reaction solution was directly purified by reversed-phase chromatography (ACN/H₂O containing 0.05% TFA = 5%-50%) to obtain the target compound DL008 (12.5 mg, yield: 67%). 61.9%
LCMS (ESI) [M+Na]⁺= 1379.0;
¹H NMR (400 MHz, DMSO-*d*₆) δ 9.99 (s, 1H), 9.11 (s, 2H), 8.15 (d, *J* = 7.6 Hz, 1H), 7.95 (d, *J* = 8.4 Hz, 1H), 7.61 (s, 2H), 7.58 - 7.52 (m, 2H), 7.28 (d, *J =* 8.5 Hz, 2H), 7.07 (t, *J* = 5.3 Hz, 1H), 4.99 - 4.90 (m, 3H), 4.89 - 4.84 (m, 2H), 4.81 (s, 1H), 4.75 (s, 1H), 4.63 - 4.57 (m, 2H), 4.40 - 4.32 (m, 2H), 4.29 (d, *J* = 9.7 Hz, 1H), 4.20 (t, *J =* 7.6 Hz, 1H), 4.09 - 4.02 (m, 1H), 4.03 - 3.98 (m, 1H), 3.88 - 3.72 (m, 2H), 3.72 - 3.68 (m, 1H), 3.62 - 3.58 (m, 1H), 3.55 - 3.51 (m, 1H), 3.41 (s, 3H), 3.19 (s, 3H), 3.02 - 2.94 (m, 4H), 2.80 - 2.73 (m, 4H), 2.55 (t, *J =* 7.4 Hz, 5H), 2.42 - 2.35 (m, 3H), 2.04 - 1.90 (m, 7H), 1.89 - 1.78 (m, 4H), 1.77 - 1.67 (m, 6H), 1.66 - 1.59 (m, 3H), 1.58 - 1.49 (m, 4H), 1.48 - 1.39 (m, 5H), 1.36 - 1.28 (m, 5H), 1.04 (d, *J* = 6.3 Hz, 3H), 0.88 - 0.84 (m, 6H).

### Example 1.9: Preparation of Compound DL009

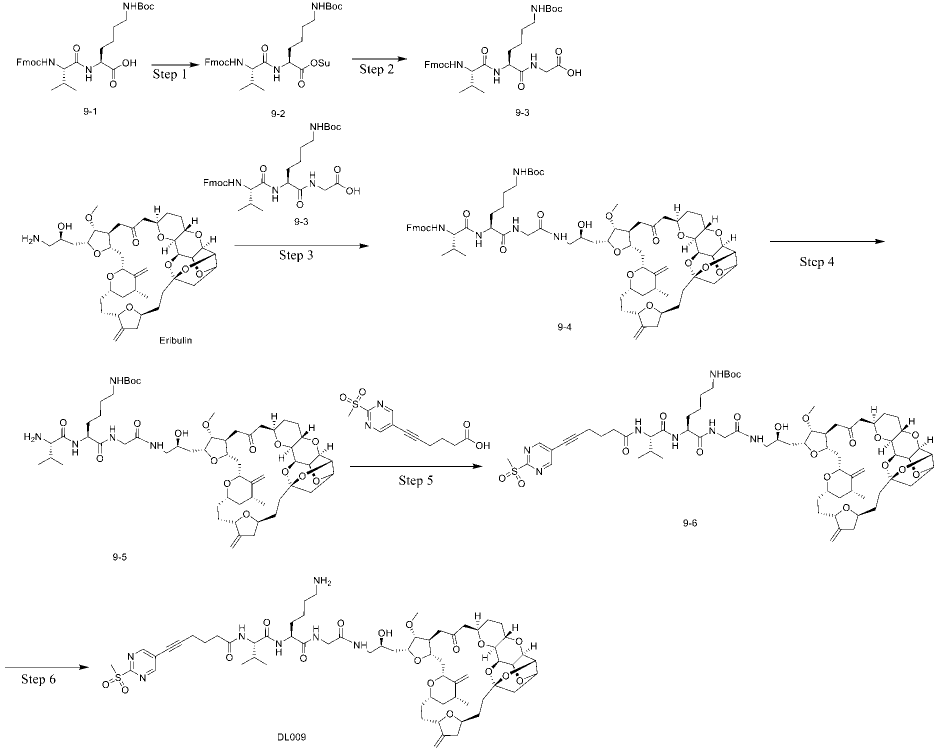

Step 1: Compound 9-1 (1.0 g, 1.76 mmol), N-hydroxysuccinimide (0.24 g, 2.11 mmol), and DCC (0.44 g, 2.11 mmol) were dissolved in DMF (10 mL). The reaction solution was stirred at 25 °C for 16 h under nitrogen atmosphere. The reaction solution was added dropwise to methyl tert-butyl ether (50 mL), and a solid was precipitated. The mixture was filtered to obtain the target compound 9-2 (1.1 g, yield: 93.9%).

LCMS (ESI) [M-Boc+H]⁺ =565.36.

Step 2: TEA (70.83 mg, 0.70 mmol) was added to a solution of compound 9-2 (200 mg, 0.35 mmol) and glycine (52.55 mg, 0.70 mmol) in DCM (2 mL). The reaction solution was stirred at room temperature for 2 h. The reaction solution was purified by column chromatography (MeOH:DCM = 0%-10%) to obtain the target compound 9-3 (90.0 mg, yield: 47.9%).

LCMS (ESI) [M+H]⁺ =525.26.

¹H NMR (400 MHz, DMSO-*d*₆) δ 7.95-7.97 (m, 1H), 7.88-7.90 (m, 2H), 7.72-7.75 (m, 2H), 7.57-7.61 (m, 1H), 7.39-7.43 (m, 2H), 7.30-7.34 (m, 2H), 6.69-6.71 (m, 1H), 4.22-4.30 (m, 4H), 3.96 - 3.83 (m, 1H), 3.47 (s, 2H), 3.35 (s, 2H), 2.84-2.86 (m, 2H), 1.98-2.03 (m, 1H), 1.62-1.66 (m, 1H), 1.49-1.50 (m, 1H), 1.36 - 1.23 (m, 13H), 0.83-0.87 (m, 6H).

Step 3: Eribulin (25.0 mg, 0.034 mmol), 9-3 (25.49 mg, 0.041 mmol), and HATU (15.51 mg, 0.041 mmol) were dissolved in DMF (1 mL), and then DIEA (5.27 mg, 0.041 mmol) was added. The reaction solution was stirred at 25 °C for 2 h under nitrogen atmosphere. The reaction solution was concentrated and purified by column chromatography (MeOH:DCM = 0%-10%) to obtain the target compound 9-4 (33.0 mg, yield: 72.6%). LCMS (ESI) [(M-Boc)/2+H]⁺ =619.03.

Step 4: Compound 9-4 (33 mg, 0.025 mmol) was dissolved in DMF (1 mL) and DEA (0.05 mL). The reaction solution was stirred at room temperature for 1 h. THF (10 mL) was added to the reaction solution. The resulting mixture was concentrated for half an hour to obtain a solution of the target compound 9-5 (27.51 mg) in DMF. LCMS (ESI) [(M-56)/2+H]⁺=529.91.

Step 5: 6-(2-(Methylsulfonyl)pyrimidin-5-yl)hex-5-ynoic acid (8.05 mg, 0.030 mmol), HATU (11.41 mg, 0.030 mmol), and DIEA (9.69 mg, 0.075 mmol) were added to the solution of compound 9-5 (27.51 mg, 0.025 mmol) in DMF (1 mL). The reaction solution was stirred at 25 °C for 2 h under nitrogen atmosphere to obtain a solution of the target compound 9-6 (33.69 mg) in DMF.

LCMS (ESI) [(M-Boc)/2+H]⁺ =633.04.

Step 6: 4 M HCl-DMF (1 mL) was added to the solution of compound 9-6 (33.69 mg, 0.025 mmol) in DMF (1 mL). The reaction solution was stirred at room temperature for 1 h. The reaction solution was purified by preparative high-performance liquid chromatography (ACN:0.1% FA in H₂O = 10%-50%) to obtain the target compound DL009 (10.0 mg, three-step yield: 31.6%).

LCMS (ESI) [M/2+H]⁺=633.10.

¹H NMR (400 MHz, DMSO-*d*₆) δ 9.10 (s, 2H), 8.10 - 8.00 (m, 2H), 7.89 (d, J = 12.0 Hz, 1H), 7.69 (t, J = 8.0 Hz, 1H), 6.73-6.71 (m, 1H), 6.30 (d, J = 4.0 Hz, 1H), 5.97 (d, J = 4.0 Hz, 1H), 5.02-4.98 (m, 2H), 4.90-4.88 (m, 1H), 4.78 - 4.72 (m, 2H), 4.71-4.68 (m, 2H), 4.40-4.36 (m, 1H), 4.17-4.12 (m, 2H), 3.88-3.81 (m, 4H), 3.70 - 3.60 (m, 6H), 3.39 (s, 3H), 3.21 (s, 3H), 3.12 - 2.98 (m, 3H), 2.89-2.84 (m, 2H), 2.76 - 2.61 (m, 4H), 2.55-2.54 (m, 3H), 2.39 - 2.29 (m, 4H), 2.22-2.20 (m, 2H), 2.00 - 1.94 (m, 1H), 1.86-1.89 (m, 6H), 1.69 - 1.45 (m, 8H), 1.38-1.33 (m, 13H), 1.23-1.19 (m, 3H), 0.99 (d, J = 4.0 Hz, 3H), 0.85-0.81 (m, 6H).

### Example 1.10: Preparation of Compound DL010

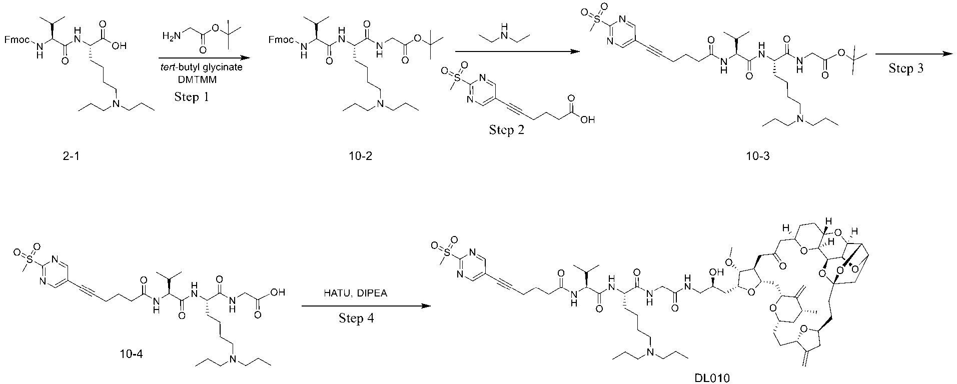

Step 1: Compound 2-1 (1.0 g, 1.81 mmol) and tert-butyl glycinate (0.26 g, 1.99 mmol) were dissolved in DMF (10 mL). The temperature was controlled at 0 °C, and the reaction solution was stirred for 5 min. DMTMM (0.6 g, 2.17 mmol) was then added. The reaction solution was left to react for another 1 h. The reaction solution was diluted with 50 mL of DCM and washed twice with 50 mL of 2% aqueous NaHCO₃ solution. The organic phase was washed with saturated brine, dried over anhydrous sodium sulfate, concentrated under reduced pressure, and purified by column chromatography (MeOH:DCM = 0-10%) to obtain compound 10-2 (1.0 g, yield: 82.98%).
LCMS (ESI) [M+H]⁺=665.54;

Step 2: Compound 10-2 (800.0 mg, 1.2 mmol) was dissolved in DMF (4 mL), and DEA (0.2 mL, 1.94 mmol) was added. The reaction solution was left to react at room temperature for 1 h. The reaction solution was concentrated under reduced pressure to obtain a crude product, which was directly used in the next step. The above crude product (400.0 mg, 0.9 mmol) and 6-(2-(methylsulfonyl)pyrimidin-5-yl)hex-5-ynoic acid (265 mg, 0.99 mmol) were dissolved in DMF (5 mL). The temperature was controlled at 0 °C, and the reaction solution was stirred for 5 min. DMTMM (274 mg, 0.99 mmol) was then added. The reaction solution was left to react for another 1 h. The reaction solution was diluted with 30 mL of DCM and washed twice with 30 mL of 2% aqueous NaHCO₃ solution. The organic phase was washed with saturated brine, dried over anhydrous sodium sulfate, concentrated under reduced pressure, and purified by column chromatography (MeOH:DCM = 0-10%) to obtain compound 10-3 (0.3 g, yield: 36.1%).
LCMS (ESI) [M+H]⁺ =693.56;

Step 3: Compound 10-3 (300 mg, 0.43 mmol) was dissolved in HCl/dioxane (4 N, 10 mL). The reaction solution was left to react at room temperature for 1 h. The reaction solution was concentrated under reduced pressure to obtain the target compound 10-4 (250 mg, yield: 85.8%).
LCMS (ESI) [M+H]⁺ =637.41;

Step 4: Compound 10-4 (30.0 mg, 0.045 mmol), eribulin (33 mg, 0.045 mmol), and HATU (18.8 mg, 0.050 mmol) were dissolved in DMF (2 mL), and then DIPEA (17 mg, 0.14 mmol) was added. The reaction solution was left to react at room temperature for 1 h. The reaction solution was purified by preparative high-performance liquid chromatography (ACN:H₂O containing 0.05% FA = 5%-50%) to obtain the target compound DL010 (18 mg, yield: 28.67%).
LCMS (ESI) [M/2+H]⁺ =675.47;
¹H NMR (400 MHz, DMSO-*d*₆) δ 9.14 (s, 2H), 8.30 (s, 1H), 8.08 (t, *J=* 5.6 Hz, 1H), 8.01 (t, *J* = 8.0 Hz, 1H), 7.91 (t, *J=* 8.5 Hz, 1H), 7.73 - 7.67 (m, 1H), 5.08 (s, 1H), 5.02 (s, 1H), 4.86 (s, 1H), 4.78 (s, 1H), 4.66 (s, 1H), 4.58 (t, *J=* 4.1 Hz, 1H), 4.31 - 4.17 (m, 4H), 4.13 (s, 3H), 4.05 (s, 1H), 3.82 - 3.78 (m, 2H), 3.73 - 3.68 (m, 3H), 3.59 - 3.50 (m, 4H), 3.43 (s, 3H), 3.28 (s, 3H), 3.16 - 3.01 (m, 3H), 2.89 - 2.85 (m, 1H), 2.82 - 2.69 (m, 2H), 2.62 - 2.56 (m, 3H), 2.42 - 2.27 (m, 13H), 2.17 - 2.13 (m, 1H), 2.05 - 1.91 (m, 7H), 1.88 - 1.79 (m, 2H), 1.77 - 1.61 (m, 7H), 1.59 - 1.45 (m, 3H), 1.41 - 1.35 (m, 9H), 1.31 - 1.19 (m, 3H), 1.05 - 1.01 (m, 4H), 0.89 - 0.82 (m, 12H).

### Example 1.11: Preparation of Compound DL011

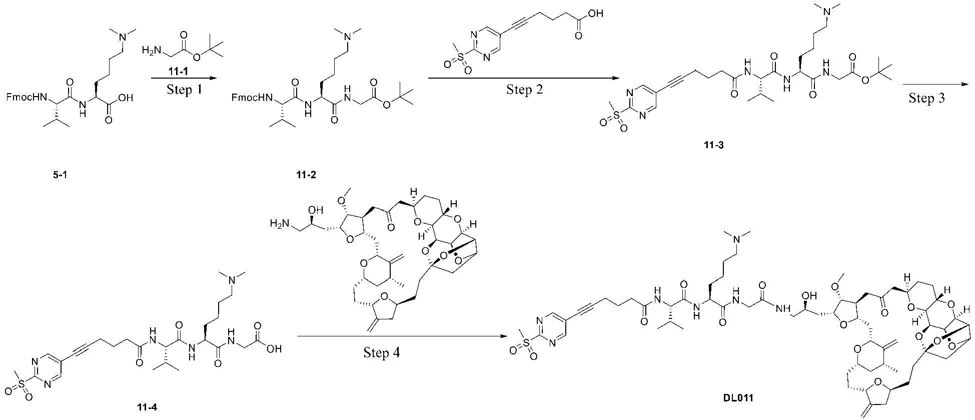

Step 1: Compounds 5-1 (500 mg, 1.01 mmol) and 11-1 (265 mg, 2.02 mmol) were dissolved in DMF, and DMTMM (500 mg, 2.02 mmol) was added at 0 °C. The reaction solution was stirred at room temperature for 2 h. The reaction solution was purified by preparative high-performance liquid chromatography (ACN:H₂O containing 0.05% FA = 10%-90%) to obtain the target compound 11-2 (62.2 mg, yield: 100%).
LCMS (ESI) [M+H]+ = 609.44

Step 2: Compound 11-2 (200 mg, 0.33 mmol) was dissolved in DMF (4 mL), and DEA (0.4 mL) was added. The reaction solution was stirred at room temperature for 1 h and concentrated under vacuum for 10 min to remove a large amount of DEA. The compounds 6-(2-(methylsulfonyl)pyrimidin-5-yl)hex-5-ynoic acid (106 mg, 0.40 mmol) and DMTMM (117 mg, 0.40 mmol) were then added. The reaction solution was stirred at room temperature for 1 h. The reaction solution was purified by liquid chromatography (ACN:H₂O containing 0.1% FA = 10%-90%) to obtain 11-3 (120 mg, yield: 57.1%).

LCMS (ESI) [M/3+1]⁺= 637.41.

Step 3: Compound 11-3 (50 mg, 0.079 mmol) was dissolved in 4 M HCl/DMF (2 mL). The reaction solution was stirred at room temperature for 1 h. The reaction solution was concentrated under vacuum and evaporated with DCM (5 × 3 mL) to obtain 11-4 (45 mg, yield: 92.3%).

LCMS (ESI) [M+H]⁺= 581.34.

Step 4: 11-4 (32.24 mg, 0.052 mmol), eribulin (29.20 mg, 0.040 mmol), and DMTMM (0.89 mg, 0.0032 mmol) were dissolved in DMF (1 mL). The reaction solution was stirred at room temperature for 1 h. The reaction solution was purified by preparative high-performance liquid chromatography (ACN:H₂O containing 0.1% FA = 10%-90%) to obtain DL011 (11.2 mg, yield: 21.7%).
LCMS (ESI) [M/3+1]⁺ =647.44
¹H NMR (400 MHz, DMSO-*d*₆) δ 9.15 (s, 2H), 8.28 (s, 1H), 8.11 (t, *J=* 5.7 Hz, 1H), 8.06 (d, *J=* 7.5 Hz, 1H), 7.94 (d, *J* = 8.6 Hz, 1H), 7.72 (t, *J* = 5.7 Hz, 1H), 5.08 (s, 1H), 5.03 (s, 1H), 4.86 (s, 1H), 4.78 (s, 1H), 4.69-4.65 (m, 1H), 4.59 (t, *J=* 4.2 Hz, 1H), 4.33-4.27 (m, 1H), 4.26-4.17 (m, 3H), 4.15-4.11 (m, 3H), 4.09-4.01 (m, 1H), 3.88- 3.75 (m, 3H), 3.75-3.67 (m, 4H), 3.63-3.51 (m, 7H), 3.44 (s, 3H), 3.29 (s, 3H), 3.17-3.10 (m, 2H), 3.09-3.02 (m, 1H), 2.87 (d, *J=* 10.0 Hz, 1H), 2.84-2.67 (m, 3H), 2.6 -2.57 (m, 2H), 2.44-2.36 (m, 2H), 2.35-2.27 (m, 3H), 2.26-2.21 (m, 3H), 2.15 (s, 6H), 2.06-1.92 (m, 7H), 1.90-1.79 (m, 2H), 1.75-1.64 (m, 6H), 1.60-1.50 (m, 2H), 1.43- 1.21 (m, 8H), 1.07 (d, *J* = 6.4 Hz, 3H), 0.87 (dd, *J* = 8.9 6.7 Hz 6H).

### Example 1.12: Preparation of Compound DL012

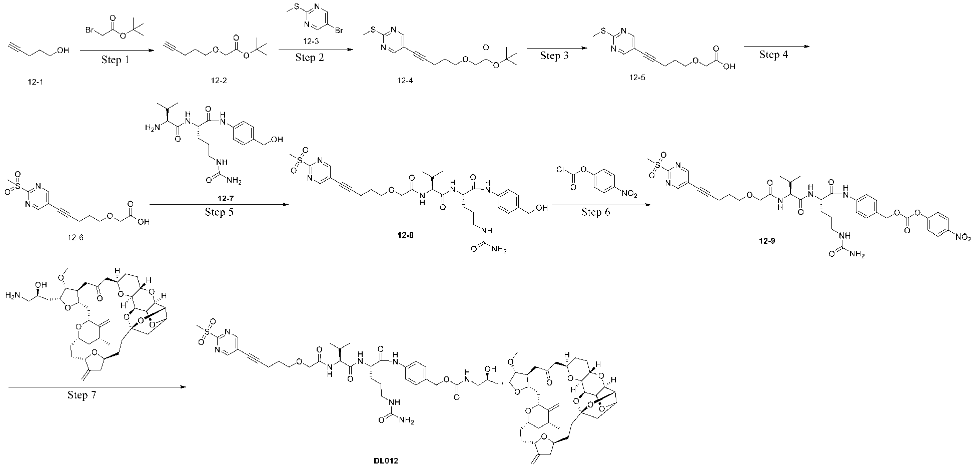

Step 1: Compound 12-1 (5.0 g, 50.9 mmol) was dissolved in toluene (170 mL), and tetrabutylammonium bromide (6.3 g, 19.6 mmol) was added. The mixture was cooled to 0 °C, sodium hydroxide (61.2 g, 535.5 mmol) was added, and tert-butyl bromoacetate (34.78 g, 178.32 mmol) was then added. The reaction solution was left to react at room temperature overnight. Water (80 mL) was added to the reaction solution, and then the resulting mixture was extracted three times with EA (80 mL × 3). The organic phases were combined, dried over anhydrous sodium sulfate, filtered, and concentrated to dryness by rotary evaporation to obtain compound 12-2. (10.2 g, yield: 87%)

¹H NMR (400 MHz, DMSO-*d*₆) δ 8.67 (s, 2H), 3.98 (s, 2H), 3.57 (t, *J=* 6.2 Hz, 2H), 2.57 - 2.54 (m, 2H), 2.52 (s, 3H), 1.83 - 1.76 (m, 2H), 1.42 (s, 9H).

Step 2: Compound 12-2 (10 g, 50.5 mmol) was dissolved in THF (100 mL), and 12-3 (10.35 g, 50.5 mmol), bis(triphenylphosphine)palladium(II) dichloride (3.5 g, 5.05 mmol), cuprous iodide (1.92 g, 10.1 mmol), and TEA (15.3 g, 151.5 mmol) were added. The reaction solution was stirred at 70 °C overnight under nitrogen atmosphere. The reaction solution was cooled and filtered, and the filtrate was concentrated under reduced pressure and then purified by column chromatography (PE:EA = 5:1) to obtain compound 12-4 (5.2 g, yield: 31.9%).
LCMS (ESI) [M+H]⁺= 323.0;
¹H NMR (400 MHz, DMSO-*d*₆) δ 8.67 (s, 2H), 3.98 (s, 2H), 3.57 (t, *J=* 6.2 Hz, 2H), 2.57 - 2.54 (m, 2H), 2.52 (s, 3H), 1.83 - 1.76 (m, 2H), 1.42 (s, 9H).

Step 3: Compound 12-4 (1.0 g, 3.1 mmol) was dissolved in DCM (50 mL), and TFA (3.54 g, 31 mmol) was added. The reaction solution was stirred at room temperature overnight. The reaction solution was directly concentrated under reduced pressure to obtain compound 12-5 (1.5 g, crude product).
LCMS (ESI) [M+H]⁺= 267.0;
¹H NMR (400 MHz, DMSO-*d*₆) δ 8.67 (s, 2H), 4.03 (s, 2H), 3.59 (t, *J=* 6.2 Hz, 2H), 2.58 - 2.53 (m, 2H), 2.53 (s, 3H), 1.84 - 1.77 (m, 2H).

Step 4: Compound 12-5 (1.5 g, 5.6 mmol) was dissolved in THF (20 mL) and water (20 mL), and Oxone (10.3 g, 16.8 mmol) was added. The reaction solution was stirred at room temperature for 2 h. The reaction solution was concentrated under reduced pressure to remove THF, and the aqueous phase was extracted three times with EA (50 mL). The organic phases were combined, dried over anhydrous sodium sulfate, concentrated under reduced pressure, and then purified by reversed-phase chromatography (ACN/H₂O containing 0.05% HCl = 5%-50%) to obtain compound 12-6 (0.4 g, two-step yield: 43.2%).
LCMS (ESI) [M+H]⁺= 299.1;
¹H NMR (400 MHz, DMSO-*d*₆) δ 12.60 (s, 1H), 9.11 (s, 2H), 4.03 (s, 2H), 3.60 (t, *J* = 6.2 Hz, 2H), 3.41 (s, 3H), 2.63 (t, *J =* 7.1 Hz, 2H), 1.87 - 1.80 (m, 2H).

Step 5: Compound 12-7 (200.0 mg, 0.53 mmol), 12-6 (158.10 g, 0.53 mmol), and DMTMM (161.33 mg, 0.58 mmol) were dissolved in DMF (2 mL). The reaction solution was stirred at 25 °C for 2 h under nitrogen atmosphere. DCM (10 mL) was added to the reaction solution, and the organic phase was washed twice with a saturated aqueous sodium bicarbonate solution (5 mL × 2), dried, and concentrated to obtain the target compound 12-8 (310 mg, yield: 88.7%).

LCMS (ESI) [M+H]⁺ =660.45.

Step 6: DIPEA (182.23 mg, 1.41 mmol) was added to a solution of compound 12-8 (310 mg, 0.47 mmol) and p-nitrophenyl chloroformate (189.47 mg, 0.94 mmol) in DMF (3 mL). The reaction solution was stirred at room temperature for 2 h. The reaction solution was purified by reversed-phase column chromatography (ACN:0.1% FA H₂O = 10%-50%) to obtain the target compound 12-9 (120.0 mg, yield: 31.0%).

LCMS (ESI) [M+H]⁺=825.46.

Step 7: Compound 12-9 (80.0 mg, 0.11 mmol) and eribulin (99.81 mg, 0.12 mmol) were dissolved in DMF (1 mL), and DIPEA (42.65 mg, 0.33 mmol) was then added at 0 °C. The reaction solution was stirred at 0 °C for 2 h under nitrogen atmosphere. The reaction solution was concentrated and then purified by preparative high-performance liquid chromatography (ACN:0.1% TFA H₂O = 10%-60%) to obtain the target compound DL012 (53.0 mg, yield: 77.1%).

LCMS (ESI) [M/2+H]⁺ =709.03.

¹H NMR (400 MHz, DMSO-*d*₆) δ 10.03 (s, 1H), 9.10 (s, 2H), 8.33 (d, J = 8.0 Hz, 1H), 7.57 (d, J = 8.0 Hz, 2H), 7.48 (d, J = 12.0 Hz, 1H), 7.27 (d, J = 8.0 Hz, 2H), 7.08-7.06 (m, 1H), 5.05 - 4.93 (m, 4H), 4.83-4.75 (m, 2H), 4.63-4.54 (m, 2H), 4.40 - 4.25 (m, 3H), 4.20 - 3.96 (m, 8H), 3.83 - 3.73 (m, 6H), 3.50-3.46 (m, 4H), 3.40 (s, 3H), 3.26-3.22 (m, 4H), 3.04-2.97 (m, 4H), 2.85 - 2.62 (m, 6H), 2.34 - 2.12 (m, 6H), 2.07 - 1.86 (m, 9H), 1.75 - 1.16 (m, 18H), 1.04-0.96 (m, 4H), 0.87-0.79 (m, 6H).

### Example 1.13: Preparation of Compound DL013

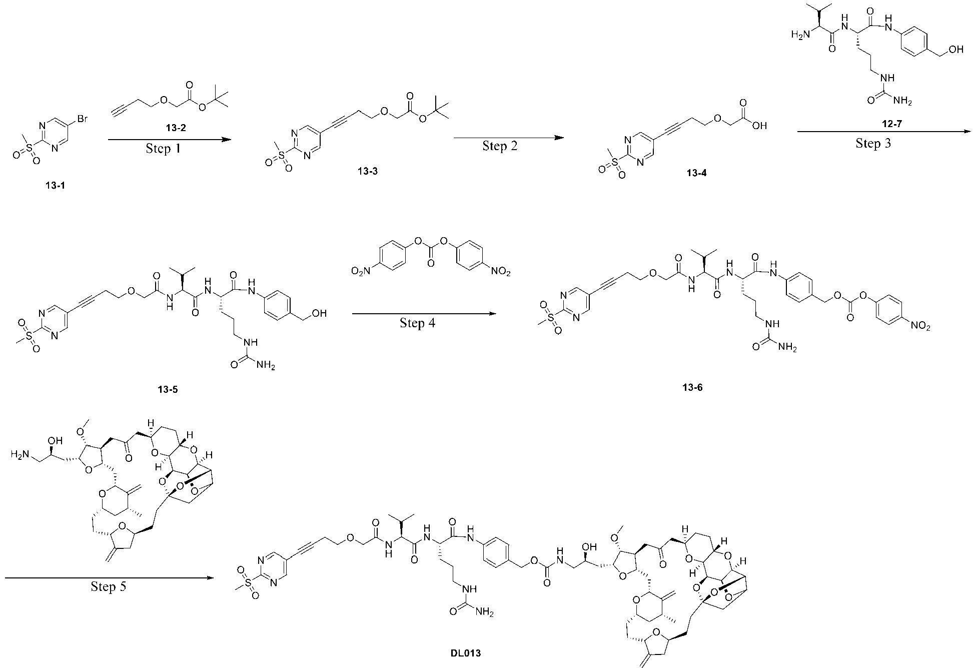

Step 1: Compound 13-1 (500.0 mg, 2.11 mmol), compound 13-2 (388.55 g, 2.11 mmol), and bis(triphenylphosphine)palladium(II) dichloride (148.04 mg, 0.21 mmol) were dissolved in DMF (2 mL), and then TEA (640.53, 6.33 mmol) was added. The reaction solution was stirred at 65 °C for 3 h under nitrogen atmosphere, concentrated, and directly purified by column chromatography (0-50% EA in PE) to obtain the target compound 13-3 (580 mg, yield: 80.8%).

LCMS (ESI) [M+H]⁺ =341.22.

Step 2: Compound 13-3 (580.0 mg, 1.70 mmol) was dissolved in DCM (6 mL) and TFA (1 mL). The reaction solution was stirred at room temperature for 2 h, concentrated, and directly purified by column chromatography (0-10% MeOH in DCM) to obtain the target compound 13-4 (250 mg, yield: 51.7%).

LCMS (ESI) [M+H]⁺ =285.13.

¹H NMR (400 MHz, DMSO-*d*₆) δ 9.11 (s, 2H), 4.10 (s, 2H), 3.71 (t, J = 8.0 Hz, 2H), 3.41 (s, 3H), 2.84 (t, J = 8.0 Hz, 2H).

Step 3: Compound 13-4 (284 mg, 1.0 mmol), DMF (1 mL), and DMTMM-H₂O (295 mg, 1.0 mmol) were added to a reaction flask. The reaction solution was stirred at room temperature for 10 min. Compound 12-7 (380 mg, 1.0 mmol) was added, and the reaction solution was left to react for 30 min. The reaction solution was purified by preparative high-performance liquid chromatography (ACN:H₂O containing 0.05% HCl = 5%-50%). After lyophilization, the solid was dissolved in 40 mL of DCM. The mixture was washed once with an aqueous sodium bicarbonate solution (30 mL, wt = 2%) and washed once with 40 mL of water, filtered, and subjected to liquid separation. The filtered solid was mixed with DCM, and the mixture was concentrated to dryness under reduced pressure in a water bath at 35 °C to obtain the target compound 13-5 (500 mg, yield: 77.4%).
LCMS (ESI) [M+H]⁺ =646.37;

Step 4: Compound 13-5 (100 mg, 0.15 mmol), DCM (10 mL), bis(4-nitrophenyl)carbonate (137 mg, 0.45 mmol), and TEA (46 mg, 0.45 mmol) were added to a reaction flask. The reaction solution was stirred at 27 °C for 16 h. 6 mL of DMF was added. The mixture was concentrated under reduced pressure in a water bath at room temperature to remove DCM, and the residue was purified by high-performance liquid chromatography (ACN:H₂O containing 0.1% FA = 5%-50%) to obtain the target compound 13-6 (40 mg, yield: 31.9%).
LCMS (ESI) [M+H]⁺ =811.59;

Step 5: DMF (1 mL), compound 13-6 (23.4 mg, 0.029 mmol), eribulin mesylate (20 mg, 0.024 mmol), and DIPEA (9.3 mg, 0.072 mmol) were added to a reaction flask. The reaction solution was left to react at room temperature for 1 h. The reaction solution was purified by high-performance liquid chromatography (ACN:H₂O containing 0.1% FA = 5%-50%) to obtain the target compound DL013 (16 mg, yield: 46.8%).
LCMS (ESI) [M/2+H]⁺ =701.48;
¹H NMR (400 MHz, DMSO-*d*₆) δ 10.02 (s, 1H), 9.11 (s, 2H), 8.34 (d, *J* = 7.5 Hz, 1H), 7.63-7.47 (m, 3H), 7.34-7.21 (m, 2H), 7.07 (t, *J=* 5.8 Hz, 1H), 5.97 (t, *J* = 5.9 Hz, 1H), 5.40 (s, 2H), 5.08-4.88 (m, 4H), 4.83 (s, 1H), 4.75 (s, 1H), 4.66-4.61 (m, 1H), 4.58-4.50 (m, 2H), 4.45-4.36 (m, 1H), 4.36-4.30 (m, 1H), 4.29-4.22 (m, 1H), 4.21-4.13 (m, 1H), 4.13-4.07 (m, 3H), 4.06-3.97 (m, 3H), 3.84-3.64 (m, 5H), 3.57-3.45 (m, 3H), 3.38 (s, 3H), 3.31-3.29 (m, 1H), 3.27-3.21 (m, 4H), 3.07-2.92 (m, 4H), 2.90-2.81 (m, 3H), 2.80-2.64 (m, 2H), 2.61-2.52 (m, 1H), 2.35-2.11 (m, 6H), 2.05-1.84 (m, 7H), 1.77-1.12 (m, 17H), 1.08-0.92 (m, 4H), 0.86 (d, *J* = 6.8 Hz, 3H), 0.79 (d, *J* = 6.8 Hz, 3H).

### Example 1.14: Preparation of Compound DL014

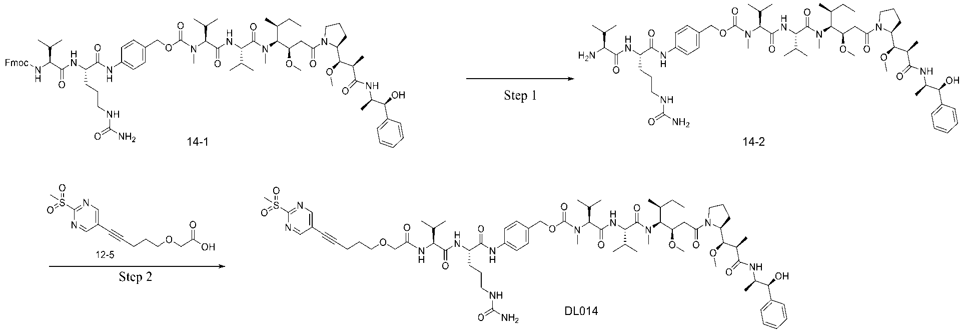

Step 1: 14-1 (120 mg, 0.089 mmol) was dissolved in DMF (2 mL), and DEA (0.2 mL) was added. The reaction solution was stirred at room temperature for 2 h. The reaction solution was diluted with THF and concentrated by rotary evaporation three times to remove DEA to obtain a solution of 14-2 in DMF, which was directly used in the next step.

LCMS (ESI) [M/2+H]⁺ = 562.42.

Step 2: 12-5 (31.86 mg, 0.11 mmol) was added to the solution of 14-2 in DMF (0.089 mmol, 2 mL). The reaction solution was cooled to -10 °C, and then DMTMM (29.55 mg, 0.11 mmol) was added. The reaction solution was left to react at -10 °C for 2 h. The reaction solution was warmed to room temperature and then filtered through a microfiltration membrane to remove the insoluble substances. The clear solution was purified by preparative high-performance liquid chromatography (ACN:H₂O containing 0.05% FA = 5%-50%) to obtain the target compound DL014 (26.7 mg, two-step yield: 21.3%)

LCMS (ESI) [M/2+H]⁺ = 702.63.

¹H NMR (400 MHz, DMSO-*d*₆) δ 10.03 (s, 1H), 9.10 (s, 2H), 8.33 (d, *J* = 7.4 Hz, 1H), 8.28-7.94 (m, 1H), 7.89-7.54 (m, 3H), 7.47 (d, *J* = 9.0 Hz, 1H), 7.38-7.11 (m, 8H), 5.97 (s, 1H), 5.39 (s, 3H), 5.33 (d, *J* = 4.9 Hz, 1H), 5.15-4.90 (m, 3H), 4.55-4.21 (m, 6H), 4.05-3.90 (m, 6H), 3.61 (t, *J =* 6.2 Hz, 3H), 3.39 (s, 3H), 3.23 (d, *J* = 6.3 Hz, 3H), 3.19 (d, *J* = 9.3 Hz, 3H), 3.11 (s, 2H), 3.08-2.91 (m, 4H), 2.86 (d, *J =* 14.1 Hz, 3H), 2.64 (t, *J =* 7.1 Hz, 2H), 2.40 (d, *J=* 15.8 Hz, 1H), 2.31-2.21 (m, 1H), 2.15-1.97 (m, 4H), 1.87 (t, *J =* 6.6 Hz, 2H), 1.79-1.67 (m, 2H), 1.62-1.40 (m, 2H), 1.07-0.97 (m, 6H), 0.94-0.72 (m, 28H).

### Example 1.15: Preparation of Compound DL015

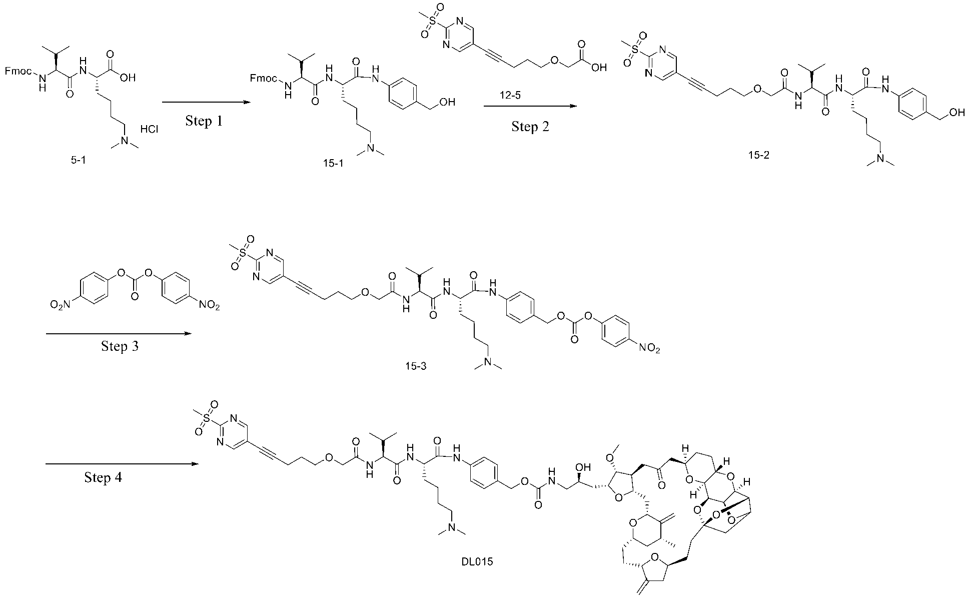

Step 1: Compound 5-1 (300 mg, 0.61 mmol), p-aminobenzyl alcohol (150 mg, 0.22 mmol), DCM (1.5 mL), MeOH (0.5 mL), and EEDQ (302 mg, 1.22 mmol) were added to a reaction flask. The reaction solution was stirred at 27 °C for 16 h. The reaction was monitored by LCMS, and the reaction solution was purified by preparative high-performance liquid chromatography (ACN:H₂O containing 0.05% HCl = 5%-50%). The resulting solution was adjusted to pH 7-8 with an aqueous sodium bicarbonate solution (30 mL, wt = 2%), and extracted 3 times with DCM (30 mL). The organic phases were combined, dried over anhydrous sodium sulfate for 0.5 h, and concentrated to dryness under reduced pressure in a water bath at 30 °C to obtain the target compound 15-1 (106 mg, yield: 29.2%).
LCMS (ESI) [M+H]⁺ =601.41

Step 2: Compound 15-1 (100 mg, 0.17 mmol), THF (4 mL), and DEA(0.8 mL) were added to a reaction flask. The reaction solution was stirred at 27 °C for 2 h. The reaction was monitored by LCMS. The reaction solution was concentrated to dryness under reduced pressure in a water bath at 40 °C. The residue was dissolved in DCM (4 mL), and the resulting solution was further concentrated to dryness. The procedures were repeated once to obtain a concentrated reaction mixture. 12-5 (61 mg, 0.20 mmol) was dissolved in DMF (2 mL), and DMTMM-H₂O (60 mg, 0.20 mmol) was added. The reaction solution was stirred at 27 °C for 10 min, and the above concentrated reaction mixture was added. The resulting solution was left to react for 30 min. The reaction was monitored by LCMS. The reaction solution was purified by preparative high-performance liquid chromatography (ACN:H₂O containing 0.05% HCl = 5%-50%). The resulting solution was adjusted to pH 7-8 with an aqueous sodium bicarbonate solution (30 mL, wt = 2%), and extracted 4 times with DCM (20 mL). The organic phases were combined, dried over anhydrous sodium sulfate for 0.5 h, and concentrated to dryness under reduced pressure in a water bath at 30 °C to obtain the target compound 15-2 (73 mg, yield: 66.6 %).
LCMS (ESI) [M+H]⁺ =659.44

Step 3: Compound 15-2 (73 mg, 0.11 mmol), DMF (4 mL), bis(4-nitrophenyl)carbonate (134 mg, 0.44 mmol), and DIPEA (71 mg, 0.55 mmol) were added to a reaction flask. The reaction solution was stirred at 27 °C for 16 h. The reaction was monitored by LCMS. The reaction solution was purified by high-performance liquid chromatography (ACN:H₂O containing 0.1% FA = 5%-50%) to obtain a formate of the target compound 15-3 (50 mg, yield: 51.9%).
LCMS (ESI) [M+H]⁺ =824.44

Step 4: DMF (1 mL), compound 15-3 (25 mg, 0.029 mmol), eribulin mesylate (20 mg, 0.024 mmol), and DIPEA (9.3 mg, 0.072 mmol) were added to a reaction flask. The reaction solution was left to react at 26 °C for 1 h. The reaction was monitored by LCMS. The reaction solution was purified by high-performance liquid chromatography (ACN:H₂O containing 0.1% TFA = 5%-50%) to obtain a trifluoroacetate of the target compound DL015 (8 mg, yield: 21.6%).
LCMS (ESI) [M/2+H]⁺ =708.18
¹H NMR (400 MHz, DMSO-*d*₆) δ 10.08 (s, 1H), 9.30 (s, 1H), 9.13 (s, 2H), 8.38 (d, *J =* 7.5 Hz, 1H), 7.60 (d, *J= 8.3* Hz, 2H), 7.52 (d, *J* = 8.8 Hz, 1H), 7.31 (d, *J =* 8.3 Hz, 2H), 7.08 (t, *J =* 6.0 Hz, 1H), 5.13-5.05 (m, 1H), 5.04-4.92 (m, 3H), 4.86 (s, 1H), 4.78 (s, 1H), 4.70-4.64 (m, 1H), 4.60-4.56 (m, 1H), 4.47-4.39 (m, 1H), 4.36-4.26 (m, 2H), 4.24-4.17 (m, 1H), 4.16-4.10 (m, 3H), 4.09-4.01 (m, 1H), 3.99 (s, 2H), 3.88-3.75 (m, 2H), 3.75-3.68 (m, 1H), 3.65 (t, *J* = 6.2 Hz, 2H), 3.57-3.51 (m, 3H), 3.30-3.25 (m, 4H), 3.07-2.97 (m, 4H), 2.90-2.84 (m, 1H), 2.81-2.74 (m, 7H), 2.70-2.64 (m, 2H), 2.63-2.54 (m, 3H), 2.36-2.14 (m, 6H), 2.08-1.86 (m, 10H), 1.82-1.61 (m, 9H), 1.58-1.46 (m, 3H), 1.39-1.19 (m, 8H), 1.11-0.94 (m, 4H), 0.89 (d, *J =* 6.8 Hz, 3H), 0.83 (d, *J* = 6.8 Hz, 3H).

### Example 1.16: Preparation of Compound DL016

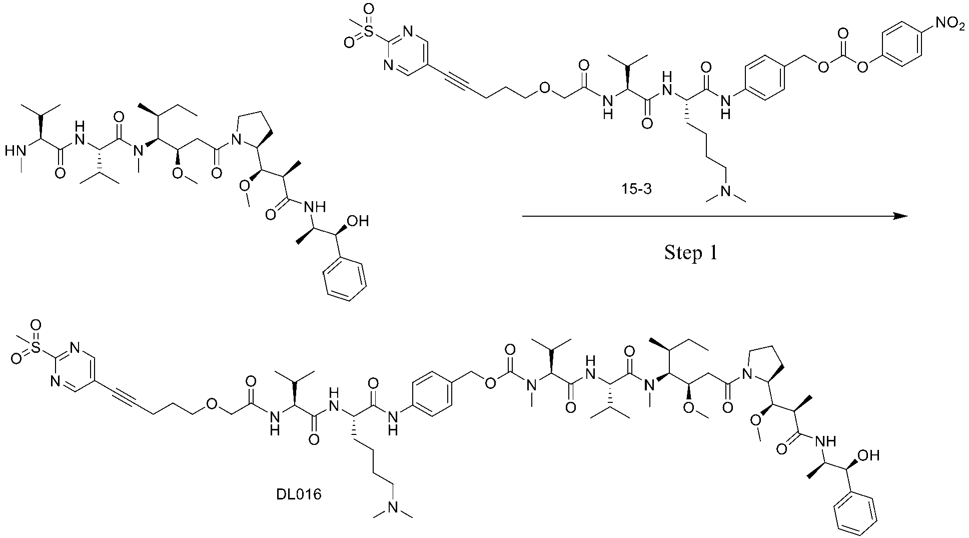

Step 1: DMF (1 mL), the compound MMAE (20 mg, 0.028 mmol), compound 15-3 (34.02 mg, 0.042 mmol), and DIPEA (6.64 mg, 0.084 mmol) were added to a reaction flask, and HOBT (5.68 mg, 0.042 mmol) was added with stirring. The reaction solution was stirred at room temperature for 4 h. The reaction solution was purified by high-performance liquid chromatography (ACN:H₂O containing 0.1% TFA = 10%-60%) to obtain a trifluoroacetate of the target compound DL016 (3.5 mg).
LCMS (ESI) [M/2+H]⁺ =702.09
¹H NMR (400 MHz, DMSO-*d*₆) δ 10.07 (s, 1H), 9.32 (s, 1H), 9.10 (s, 2H), 8.36 (d, J = 8.0 Hz, 1H), 8.27-8.25 (m, 1H), 8.04 (d, J = 8.0 Hz, 1H), 7.88 (d, J = 8.0 Hz, 1H), 7.62-7.56 (m, 2H), 7.49 (d, J = 8.0 Hz, 1H), 7.41 - 7.17 (m, 7H), 7.19-6.98 (m, 1H), 5.06-4.96 (m, 2H), 4.75-6.50 (m, 1H), 4.56 - 4.35 (m, 3H), 4.31-4.26 (m, 2H), 4.06-3.92 (m, 4H), 3.78-3.76 (m, 3H), 3.40 (s, 3H), 3.25-3.15 (m, 7H), 3.16 - 2.93 (m, 6H), 2.88-2.83 (m, 3H), 2.75-2.73 (m, 6H), 2.66-2.62 (m, 2H), 2.43-2.38 (m, 1H), 2.35-2.19 (m, 1H), 2.18-1.91 (m, 4H), 1.91-1.84 (m, 2H), 1.82-1.57 (m, 7H), 1.57-1.43 (m, 2H), 1.40-1.18 (m, 4H), 1.11-0.93 (m, 7H), 0.92-0.72 (m, 25H).

### Example 1.17: Preparation of Compound DL017

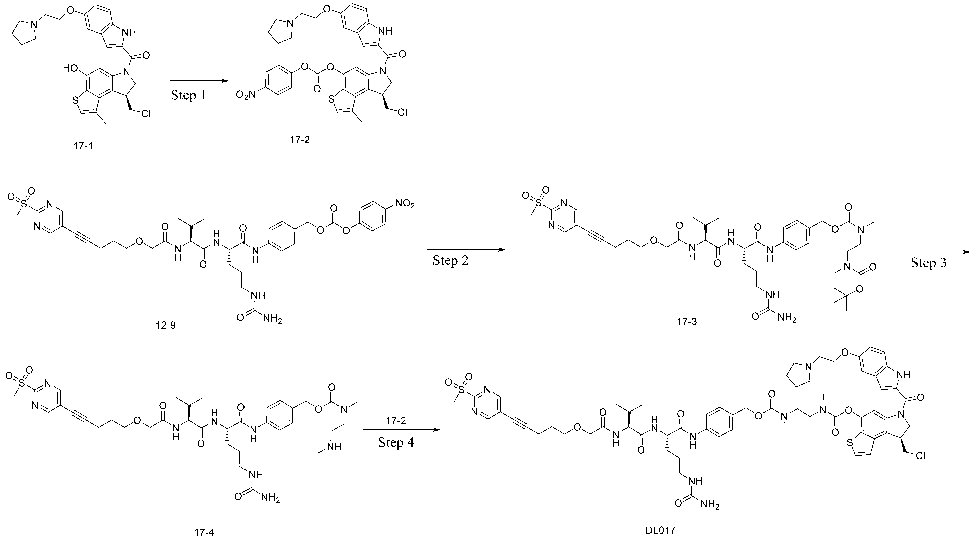

Step 1: 17-1 (50.0 mg, 0.098 mmol) was dissolved in THF (3 mL) and DCM (3 mL). The reaction solution was stirred at 0 °C for 15 min, and p-nitrophenyl chloroformate (59.26 mg, 0.29 mmol) and TEA (49.58 mg, 0.49 mmol) were added. The reaction solution was stirred at 20 °C for 3 h, and p-nitrophenyl chloroformate (39.51 mg, 0.20 mmol) and TEA (19.83 mg, 0.20 mmol) were further added. The reaction solution was concentrated under reduced pressure, and the crude product was purified by reversed-phase column chromatography (ACN:H₂O containing 0.05% formic acid = 10%-90%) to obtain the target compound 17-2 (66.0 mg).

LCMS (ESI) [M+H]+ = 675.33.

Step 2: Compound 12-9 (200 mg, 0.24 mmol) was dissolved in DMF (5 mL), and N-Boc-N,N'-dimethylethylenediamine (50 mg, 0.26 mmol) and DIPEA (37 mg, 0.29 mmol) were added. The reaction solution was left to react at 18 °C for 2 h. The reaction solution was purified by preparative high-performance liquid chromatography (ACN:H₂O containing 0.05% FA = 5%-50%) to obtain the target compound 17-3 (170 mg).

LCMS (ESI) [M+H]+ =874.57.

Step 3: TFA (400 µL) was dissolved in DCM (4 mL). The reaction solution was cooled to 0 °C, and 17-3 (165 mg) was added. The temperature was maintained at 0 °C, and the reaction solution was left to react for 2 h. The reaction solution was purified by preparative high-performance liquid chromatography (ACN:H₂O containing 0.05% FA = 5%-50%) to obtain a formate of 17-4 (87 mg).

LCMS (ESI) [M/2+H]+ =387.91.

1H NMR (400 MHz, DMSO-d6) δ 10.14 (s, 1H), 9.13 (s, 2H), 8.42 (d, J = 7.4 Hz, 1H), 8.33 (s, 1H), 7.67-7.58 (m, 2H), 7.52 (d, J = 8.9 Hz, 1H), 7.38-7.22 (m, 2H), 6.08 (t, J = 5.8 Hz, 1H), 5.47 (s, 2H), 5.01 (s, 2H), 4.46-4.37 (m, 1H), 4.37-4.30 (m, 1H), 3.98 (s, 2H), 3.64 (t, J = 6.2 Hz, 2H), 3.47-3.36 (m, 5H), 3.10-2.94 (m, 2H), 2.93-2.79 (m, 5H), 2.66 (t, J = 7.1 Hz, 2H), 2.52-2.37 (m, 5H), 2.08-1.98 (m, 1H), 1.95-1.85 (m, 2H), 1.78-1.55 (m, 2H), 1.53-1.29 (m, 2H), 0.95-0.75 (m, 6H).

### Step 4:

17-4 (61.0 mg, 0.074 mmol) was dissolved in DCM (5 mL) and DMF (0.5 mL), and 17-2 (50.0 mg, 0.074 mmol) and TEA (22.0 mg, 0.22 mmol) were added. The reaction solution was stirred at 20 °C for 2 h and concentrated under reduced pressure, and the crude product was purified by preparative high-performance liquid chromatography (ACN:H₂O containing 0.1% formic acid = 10%-90%) to obtain the target compound DL017 (46.9 mg).

LCMS (ESI) [M+H]+ = 1309.74.

1H NMR (400 MHz, DMSO-d6) δ 11.59 (d, J = 11.8 Hz, 1H), 10.03 (d, J = 12.2 Hz, 1H), 9.10 (s, 2H), 8.36 (d, J = 7.2 Hz, 1H), 8.29 (s, 1H), 8.08 (dt, J = 9.1, 4.7 Hz, 1H), 7.50 (qd, J = 17.8, 16.3, 9.3 Hz, 4H), 7.39 (d, J = 8.9 Hz, 1H), 7.28 (dd, J = 40.9, 8.1 Hz, 2H), 7.17 (d, J = 2.4 Hz, 1H), 7.07 (d, J = 7.3 Hz, 1H), 6.91 (dd, J = 8.9, 2.3 Hz, 1H), 6.00 (t, J = 5.8 Hz, 1H), 5.42 (s, 2H), 5.03 (s, 1H), 4.98 (d, J = 7.6 Hz, 1H), 4.72 (q, J = 8.3, 6.5 Hz, 1H), 4.67 - 4.57 (m, 1H), 4.38 (s, 1H), 4.31 (d, J = 7.1 Hz, 2H), 4.08 (t, J = 6.0 Hz, 2H), 3.96 (s, 3H), 3.69 (d, J = 8.8 Hz, 2H), 3.60 (d, J = 6.2 Hz, 2H), 3.39 (m, 6H), 3.12 (s, 2H), 3.02 (d, J = 10.0 Hz, 2H), 2.99 - 2.89 (m, 4H), 2.87 (d, J = 3.9 Hz, 2H), 2.82 (t, J = 5.9 Hz, 2H), 2.63 (t, J = 7.1 Hz, 2H), 2.57 (s, 3H), 2.56 (s, 2H), 2.00 (dt, J = 13.2, 6.5 Hz, 1H), 1.87 (p, J = 6.9 Hz, 2H), 1.77 - 1.19 (m, 8H), 0.83 (dd, J = 24.0, 6.6 Hz, 6H).

### II. Preparation of Multi-Warhead Antibody-Drug Conjugates

### Example 2.1: Preparation of HER2-ADC-001

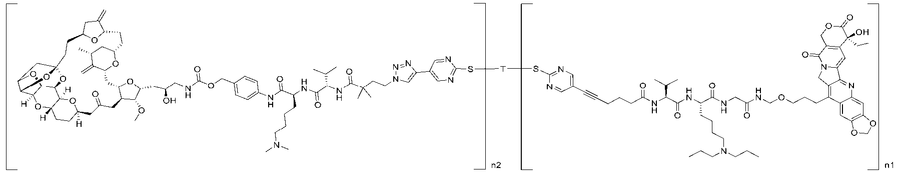

Trastuzumab (12.55 mg) was added to a sodium edetate solution with a final concentration of 1 mM and well mixed, and the pH of the sample was adjusted to 7.5 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. 6 molar equivalents (relative to the antibody) of a 10 mmol/L solution of DL003 in DMSO and 3 molar equivalents (relative to the antibody) of a 10 mmol/L solution of DL001 in DMSO were well mixed, and the resulting mixture was added to the above antibody and well mixed. The mixture was then left to stand at 25 °C for 2 h to obtain the conjugated sample. After the reaction was completed, the sample was exchanged into a 20 mM histidine buffer at a pH of 6.0 using a 30 KDa ultrafiltration tube to remove low-molecular-weight substances, and finally, the sample was concentrated to obtain HER2-ADC-001.

The DAR value of the conjugated sample was determined by RP-LC/MS

### LC/MS model:

| Name | Manufacturer | Model |
|---|---|---|
| UPLC | AB SCIEX | ExionLC |
| High resolution mass spectrometer | AB SCIEX | X500B |

### Liquid phase parameters

| Chromatographic column | Xbridge Protein BEH SEC (2.5 µm,4.6*150 mm) | | | | |
|---|---|---|---|---|---|
| Flow rate | 0.3 ml/min | | | | |
| Column temperature | 30 °C | | | | |
| Injection volume | 1 µl | | | | |
| Sample chamber temperature | 8 °C | | | | |
| Mobile phase | Mobile phase A: 0.1% FA in H₂O | | | | |
| | Mobile phase B: 0.1% FA in ACN | | | | |
| Time/min | 0 | 5 | 7 | 7.1 | 10 |
| A% | 90 | 20 | 20 | 90 | 90 |
| B% | 10 | 80 | 80 | 10 | 10 |

The mass spectrometry results show that in the HER2-ADC-001 sample, the light chain was conjugated to 0-1 toxin molecule (the proportions of LC, DAR1-DL003, and DAR1-DL001 were 18.2%, 55.3%, and 26.4%, respectively), and the heavy chain was conjugated to 0-3 toxin molecules (the proportions of HC, DAR1, DAR2, DAR3-2*DL003-DL001, DAR3-DL003-2*DL001, DAR3-3*DL003, and DAR3-3*DL001 were 0%, 0%, 0%, 16.2%, 2.5%, 70.0%, and 11.2%, respectively). Therefore, the conjugation ratio (DAR value) of the HER2-ADC-001 sample was calculated to be 7.6, and each mole of the antibody was conjugated to 6.00 moles of DL003 and 1.62 moles of DL001. That is, n1 = 6.00, and n2 = 1.62.

### Example 2.2: Preparation of HER2-ADC-002

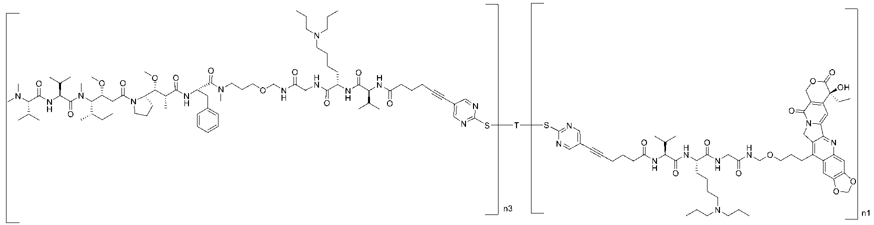

Method 1: Trastuzumab (6.28 mg) was added to a sodium edetate solution with a final concentration of 1 mM and well mixed, and the pH of the sample was adjusted to 7.45 with a 0.5 M disodium hydrogen phosphate solution. 5.5 molar equivalents (relative to the antibody) of a 20 mmol/L TCEP solution was added and well mixed, and the mixture was left to stand at 30 °C for 90 min. 5.5 molar equivalents (relative to the antibody) of a 10 mmol/L solution of DL003 in DMSO and 3.5 molar equivalents (relative to the antibody) of a 10 mmol/L solution of DL007 in DMSO were well mixed, and the resulting mixture was added to the above antibody and well mixed. The mixture was then left to stand at 30 °C for 3 h to obtain the conjugated sample HER2-ADC-002.

The mass spectrometry detection method in Example 2.1 was used, and the results show that in the HER2-ADC-002 sample, the light chain was conjugated to 0-1 toxin molecule (the proportions of LC, DAR1-DL003, and DAR1-DL007 were 13.3%, 47.6%, and 39.1%, respectively), and the heavy chain was conjugated to 3 toxin molecules (the proportions of DAR3-2*DL003-DL007, DAR3-DL003-2*DL007, DAR3-3*DL003, and DAR3-3*DL007 were 22.2%, 1.2%, 54.2%, and 22.5%, respectively). Therefore, the conjugation ratio (DAR value) of the HER2-ADC-002 sample was calculated to be 7.7, and each mole of the antibody was conjugated to 5.1 moles of DL003 and 2.6 moles of DL007. That is, n1 = 5.1, and n3 = 2.6.

Method II: Trastuzumab (6.28 mg) was added to a sodium edetate solution with a final concentration of 1 mM and well mixed, and the pH of the sample was adjusted to 7.45 with a 0.5 M disodium hydrogen phosphate solution. 5.5 molar equivalents (relative to the antibody) of a 20 mmol/L TCEP solution was added and well mixed, and the mixture was left to stand at 30 °C for 90 min. 5.5 molar equivalents (relative to the antibody) of a 10 mmol/L solution of DL003 in DMSO was first added. After 1 h of reaction, 3.5 molar equivalents (relative to the antibody) of a 10 mmol/L solution of DL007 in DMSO was added. After 2 h of reaction, the conjugated sample HER2-ADC-002 was obtained.

The mass spectrometry detection method in Example 2.1 was used, and the results show that in the HER2-ADC-002 sample, the light chain was conjugated to 0-1 toxin molecule (the proportions of LC, DAR1-DL003, and DAR1-DL007 were 12%, 50.2%, and 37.8%, respectively), and the heavy chain was conjugated to 3 toxin molecules (the proportions of DAR3-2*DL003-DL007, DAR3-DL003-2*DL007, DAR3-3*DL003, and DAR3-3*DL007 were 5%, 0%, 66%, and 29%, respectively). Therefore, the conjugation ratio (DAR value) of the HER2-ADC-002 sample was calculated to be 7.8, and each mole of the antibody was conjugated to 5.2 moles of DL003 and 2.6 moles of DL007. That is, n1 = 5.2, and n3 = 2.6.

### Example 2.3: Preparation of HER2-ADC-003

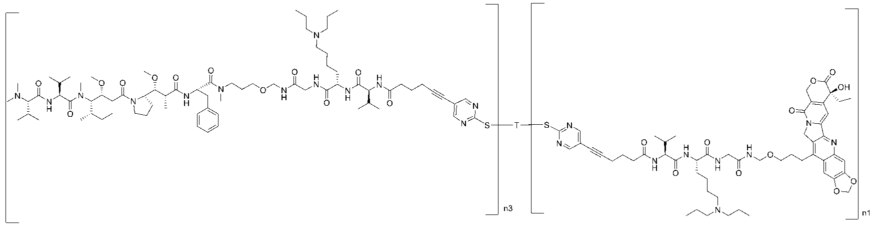

Trastuzumab (25.1 mg) was added to a sodium edetate solution with a final concentration of 1 mM and well mixed, and the pH of the sample was adjusted to 7.47 with a 0.5 M disodium hydrogen phosphate solution. 5.5 molar equivalents (relative to the antibody) of a 20 mmol/L TCEP solution was added and well mixed, and the mixture was left to stand at 30 °C for 90 min. 6.2 molar equivalents (relative to the antibody) of a 10 mmol/L solution of DL003 in DMSO was first added. After 1 h of reaction, 3.0 molar equivalents (relative to the antibody) of a 10 mmol/L solution of DL007 in DMSO was added. After 2 h of reaction, cysteine was added in excess, and the mixture was designated as HER2-ADC-003. The buffer was exchanged for a 20 mM His-HCl buffer solution (pH 5.9) using a centrifugal ultrafiltration tube (Merck, Amicon Ultra-15) to obtain the HER2-ADC-003 sample. The sample was taken and detected for DAR value.

The results show that in the HER2-ADC-003 sample, the light chain was conjugated to 0-1 toxin molecule (the proportions of LC, DL003, and DAR1-DL007 were 7.6%, 56.9%, and 35.5%, respectively), and the heavy chain was conjugated to 3 toxin molecules (the proportions of DAR3-2*DL003-DL007, DAR3-DL003-2*DL007, DAR3-3*DL003, and DAR3-3*DL007 were 14.9%, 8.2%, 67.4%, and 9.5%, respectively). Therefore, the conjugation ratio (DAR value) of the HER2-ADC-003 sample was calculated to be 7.8, and each mole of the antibody was conjugated to 5.9 moles of DL003 and 1.9 moles of DL007. That is, n1 = 5.9, and n3 = 1.9.

### Example 2.4: Preparation of HER2-ADC-004

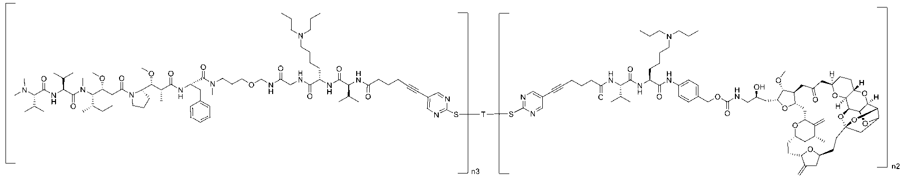

Trastuzumab (14.0 mg) was added to a sodium edetate solution with a final concentration of 1 mM and well mixed, and the pH of the sample was adjusted to 7.47 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. 4.2 molar equivalents (relative to the antibody) of a 10 mmol/L solution of DL007 in DMSO and 4.5 molar equivalents (relative to the antibody) of a 10 mmol/L solution of DL002 in DMSO were well mixed, and the resulting mixture was added to the above antibody and well mixed. The mixture was then left to stand at 25 °C for 2 h. After that, the buffer was exchanged for a 20 mM His-HCl buffer solution (pH 5.9) using a centrifugal ultrafiltration tube (Merck, Amicon Ultra-15) to obtain the conjugated sample Her2-ADC-004.

The mass spectrometry results show that in the Her2-ADC-004 sample, the light chain was conjugated to 0-1 toxin molecule (the proportions of LC, DAR1-DL002, and DAR1-DL007 were 8.4%, 47.7%, and 43.9%, respectively), and the heavy chain was conjugated to 3 toxin molecules (the proportions of DAR3-2*DL002-DL007, DAR3-DL002-2*DL007, DAR3-3*DL002, DAR3-3*DL007, DAR2-2*DL002, DAR2-2*DL007, and DAR2-DL002-DL007 were 34.2%, 28.5%, 22.0%, 13.6%, 0.5%, 0.5%, and 0.6%, respectively). Therefore, the conjugation ratio (DAR value) of the Her2-ADC-004 sample was calculated to be 7.8, and each mole of the antibody was conjugated to 4.3 moles of DL002 and 3.5 moles of DL007. n2 = 4.3, and n3 = 3.5.

### Example 2.5: Preparation of HER2-ADC-005

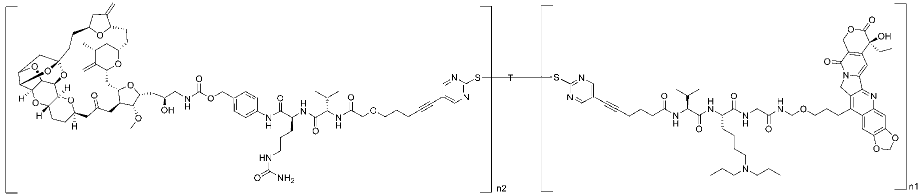

Trastuzumab (0.5 mL, 12.35 mg) was added to a sodium edetate solution with a final concentration of 1 mM and well mixed, and the pH of the sample was adjusted to 7.56 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. A mixed solution of 6.0 molar equivalents (relative to the antibody) of a 10 mmol/L solution of DL003 in DMSO and 5 molar equivalents (relative to the antibody) of a 10 mmol/L solution of DL012 in DMSO was added. After 2 h of reaction at 25 °C, cysteine was added in excess. The buffer was exchanged for a 20 mM His-HCl buffer solution (pH 5.9) using a centrifugal ultrafiltration tube (Merck, Amicon Ultra-15) to obtain the HER2-ADC-005 sample. The sample was taken and detected for DAR value.

The results show that in the HER2-ADC-005 sample, the light chain was conjugated to 0-1 toxin molecule (the proportions of LC, DAR1-DL003, and DAR1-DL012 were 3.8%, 65.7%, and 30.6%, respectively), and the heavy chain was conjugated to 3 toxin molecules (the proportions of DAR3-2*DL003-DL012, DAR3-DL003-2*DL012, DAR3-3*DL003, and DAR3-3*DL012 were 24.5%, 4.8%, 66.5%, and 4.2%, respectively). Therefore, the conjugation ratio (DAR value) of the HER2-ADC-005 sample was calculated to be 7.9, and each mole of the antibody was conjugated to 6.38 moles of DL003 and 1.54 moles of DL012. n1 = 6.38, and n2 = 1.54.

### Example 2.6: Preparation of HER2-ADC-006

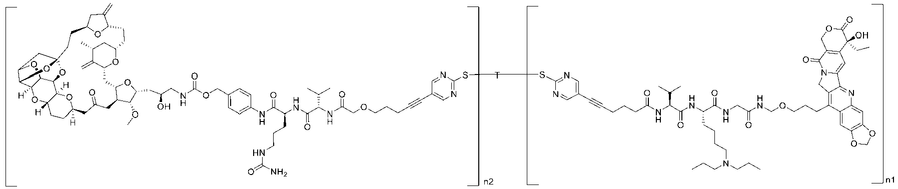

Trastuzumab (0.5 mL, 12.35 mg) was added to a sodium edetate solution with a final concentration of 1 mM and well mixed, and the pH of the sample was adjusted to 7.56 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. A mixed solution of 7.0 molar equivalents (relative to the antibody) of a 10 mmol/L solution of DL003 in DMSO and 4 molar equivalents (relative to the antibody) of a 10 mmol/L solution of DL012 in DMSO was added. After 2 h of reaction at 25 °C, cysteine was added in excess. The buffer was exchanged for a 20 mM His-HCl buffer solution (pH 5.9) using a centrifugal ultrafiltration tube (Merck, Amicon Ultra-15) to obtain the HER2-ADC-006 sample. The sample was taken and detected for DAR value.

The results show that in the HER2-ADC-006 sample, the light chain was conjugated to 0-1 toxin molecule (the proportions of LC, DAR1-DL003, and DAR1-DL012 were 2.7%, 77.7%, and 19.5%, respectively), and the heavy chain was conjugated to 3 toxin molecules (the proportions of DAR3-2*DL003-DL012, DAR3-DL003-2*DL012, DAR3-3*DL003, and DAR3-3*DL012 were 23.2%, 2.6%, 74.3%, and 0.0%, respectively). Therefore, the conjugation ratio (DAR value) of the HER2-ADC-006 sample was calculated to be 7.9, and each mole of the antibody was conjugated to 6.99 moles of DL003 and 0.96 moles of DL012. n1 = 6.99, and n2 = 0.96.

### Example 2.7: Preparation of HER2-ADC-007

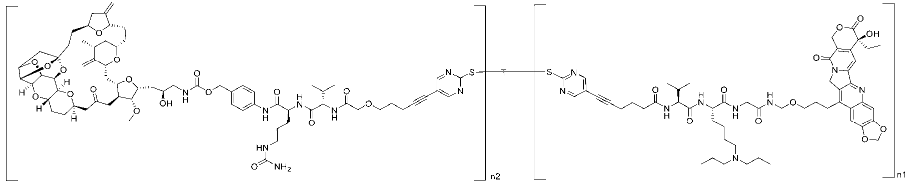

Trastuzumab (0.6 mL, 14.82 mg) was added to a sodium edetate solution with a final concentration of 1 mM and well mixed, and the pH of the sample was adjusted to 7.44 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. A mixed solution of 5.6 molar equivalents (relative to the antibody) of a 10 mmol/L solution of DL003 in DMSO and 2 molar equivalents (relative to the antibody) of a 10 mmol/L solution of DL012 in DMSO was added. After 2 h of reaction at 25 °C, cysteine was added in excess. The buffer was exchanged for a 20 mM His-HCl buffer solution (pH 5.9) using a centrifugal ultrafiltration tube (Merck, Amicon Ultra-15) to obtain the HER2-ADC-007 sample. The sample was taken and detected for DAR value.

The results show that in the HER2-ADC-007 sample, the light chain was conjugated to 0-1 toxin molecule (the proportions of LC, DAR1-DL003, and DAR1-DL012 were 11.3%, 59.3%, and 29.4%, respectively), and the heavy chain was conjugated to 3 toxin molecules (the proportions of DAR3-2*DL003-DL012, DAR3-DL003-2*DL012, DAR3-3*DL003, and DAR3-3*DL012 were 16.8%, 2.0%, 65.8%, and 15.4%, respectively). Therefore, the conjugation ratio (DAR value) of the HER2-ADC-007 sample was calculated to be 7.8, and each mole of the antibody was conjugated to 5.85 moles of DL003 and 1.93 moles of DL012. n1 = 5.85, and n2 = 1.93.

### Example 2.8: Preparation of HER2-ADC-008

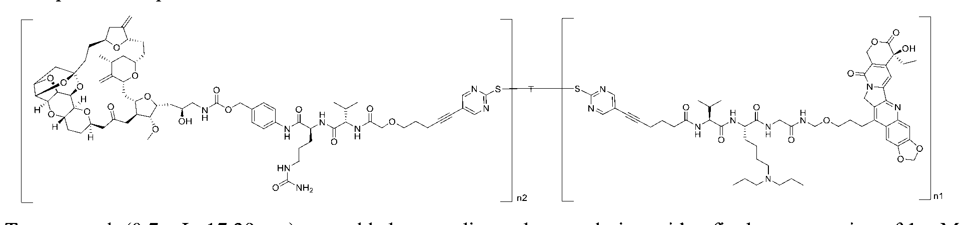

Trastuzumab (0.7 mL, 17.29 mg) was added to a sodium edetate solution with a final concentration of 1 mM and well mixed, and the pH of the sample was adjusted to 7.37 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. A mixed solution of 4.7 molar equivalents (relative to the antibody) of a 10 mmol/L solution of DL003 (0.073 mg) in DMSO and 4.5 molar equivalents (relative to the antibody) of a 10 mmol/L solution of DL012 (0.087 mg) in DMSO was added. After 2 h of reaction at 25 °C, cysteine was added in excess. The buffer was exchanged for a 20 mM His-HCl buffer solution (pH 5.9) using a centrifugal ultrafiltration tube (Merck, Amicon Ultra-15) to obtain the HER2-ADC-008 sample. The sample was taken and detected for DAR value.

The results show that in the HER2-ADC-008 sample, the light chain was conjugated to 0-1 toxin molecule (the proportions of LC, DAR1-DL003, and DAR1-DL012 were 0.0%, 36.0%, and 64.0%, respectively), and the heavy chain was conjugated to 3 toxin molecules (the proportions of DAR3-2*DL003-DL012, DAR3-DL003-2*DL012, DAR3-3*DL003, and DAR3-3*DL012 were 23.9%, 7.4%, 48.8%, and 19.9%, respectively). Therefore, the conjugation ratio (DAR value) of the HER2-ADC-008 sample was calculated to be 8.0, and each mole of the antibody was conjugated to 4.75 moles of DL003 and 3.25 moles of DL012. n1 = 4.75, and n2 = 3.25.

### Example 2.9: Preparation of HER2-ADC-009

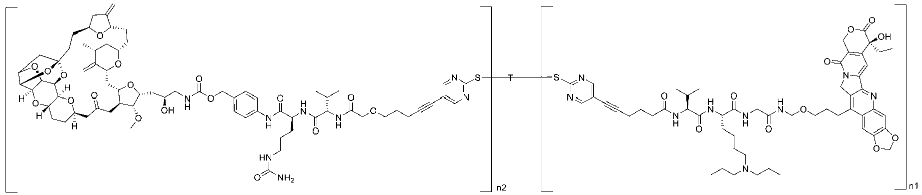

Trastuzumab (0.7 mL, 17.29 mg) was added to a sodium edetate solution with a final concentration of 1 mM and well mixed, and the pH of the sample was adjusted to 7.37 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. A mixed solution of 5.4 molar equivalents (relative to the antibody) of a 10 mmol/L solution of DL003 (0.084 mg) in DMSO and 4.0 molar equivalents (relative to the antibody) of a 10 mmol/L solution of DL012 (0.077 mg) in DMSO was added. After 2 h of reaction at 25 °C, cysteine was added in excess. The buffer was exchanged for a 20 mM His-HCl buffer solution (pH 5.9) using a centrifugal ultrafiltration tube (Merck, Amicon Ultra-15) to obtain the HER2-ADC-009 sample. The sample was taken and detected for DAR value.

The results show that in the HER2-ADC-009 sample, the light chain was conjugated to 0-1 toxin molecule (the proportions of LC, DAR1-DL003, and DAR1-DL012 were 2.5%, 48.4%, and 49.1%, respectively), and the heavy chain was conjugated to 3 toxin molecules (the proportions of DAR3-2*DL003-DL012, DAR3-DL003-2*DL012, DAR3-3*DL003, and DAR3-3*DL012 were 21.5%, 7.8%, 57.1%, and 13.6%, respectively). Therefore, the conjugation ratio (DAR value) of the HER2-ADC-009 sample was calculated to be 7.9, and each mole of the antibody was conjugated to 5.41 moles of DL003 and 2.54 moles of DL012. n1 = 5.41, and n2 = 2.54.

### Example 2.10: Preparation of HER2-ADC-010

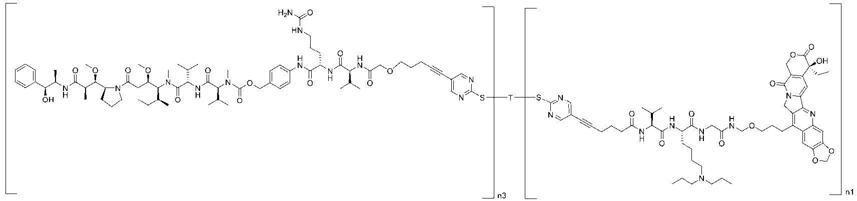

Trastuzumab (0.8 mL, 19.76 mg) was added to a sodium edetate solution with a final concentration of 1 mM and well mixed, and the pH of the sample was adjusted to 7.43 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. A mixed solution of 6.0 molar equivalents (relative to the antibody) of a 10 mmol/L solution of DL003 in DMSO and 4.0 molar equivalents (relative to the antibody) of a 10 mmol/L solution of DL014 in DMSO was added. After 2 h of reaction at 25 °C, cysteine was added in excess. The buffer was exchanged for a 20 mM His-HCl buffer solution (pH 5.9) using a centrifugal ultrafiltration tube (Merck, Amicon Ultra-15) to obtain the HER2-ADC-010 sample. The sample was taken and detected for DAR value.

The results show that in the HER2-ADC-010 sample, the light chain was conjugated to 0-1 toxin molecule (the proportions of LC, DAR1-DL003, and DAR1-DL014 were 1.0%, 53.9%, and 45.1%, respectively), and the heavy chain was conjugated to 3 toxin molecules (the proportions of DAR3-2*DL003-DL014, DAR3-DL003-2*DL014, DAR3-3*DL003, and DAR3-3*DL014 were 11.9%, 2.6%, 70.0%, and 15.6%, respectively). Therefore, the conjugation ratio (DAR value) of the HER2-ADC-010 sample was calculated to be 8.0, and each mole of the antibody was conjugated to 5.8 moles of DL003 and 2.2 moles of DL014. n1 = 5.8, and n3 = 2.2.

### Example 2.11: Preparation of HER2-ADC-011

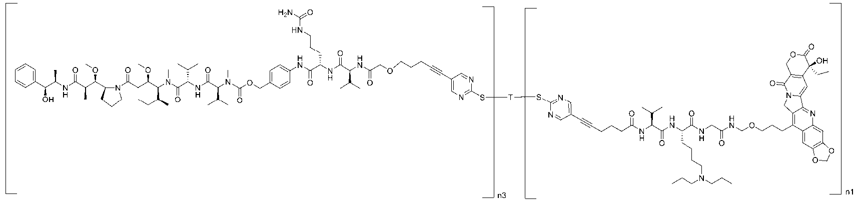

Trastuzumab (0.5 mL, 12.35 mg) was added to a sodium edetate solution with a final concentration of 1 mM and well mixed, and the pH of the sample was adjusted to 7.5 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. A mixed solution of 3.0 molar equivalents (relative to the antibody) of a 10 mmol/L solution of DL003 in DMSO and 1.6 molar equivalents (relative to the antibody) of a 10 mmol/L solution of DL014 in DMSO was added. After 2 h of reaction at 25 °C, cysteine was added in excess. The buffer was exchanged for a 20 mM His-HCl buffer solution (pH 5.9) using a centrifugal ultrafiltration tube (Merck, Amicon Ultra-15) to obtain the HER2-ADC-011 sample. The sample was taken and detected for DAR value, showing that each mole of the antibody was conjugated to 3.0 moles of DL003 and 0.9 moles of DL014. n1 = 3.0, and n3 = 0.9.

### Example 2.12: Preparation of HER2-ADC-012

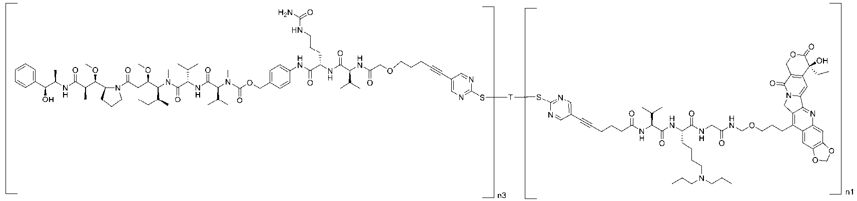

Trastuzumab (0.5 mL, 12.35 mg) was added to a sodium edetate solution with a final concentration of 1 mM and well mixed, and the pH of the sample was adjusted to 7.5 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. A mixed solution of 4.0 molar equivalents (relative to the antibody) of a 10 mmol/L solution of DL003 in DMSO and 1.6 molar equivalents (relative to the antibody) of a 10 mmol/L solution of DL014 in DMSO was added. After 2 h of reaction at 25 °C, cysteine was added in excess. The buffer was exchanged for a 20 mM His-HCl buffer solution (pH 5.9) using a centrifugal ultrafiltration tube (Merck, Amicon Ultra-15) to obtain the HER2-ADC-012 sample. The sample was taken and detected for DAR value, showing that each mole of the antibody was conjugated to 4.1 moles of DL003 and 1.3 moles of DL014. n1 = 4.1, and n3 = 1.3.

### Example 2.13: Preparation of HER2-ADC-013

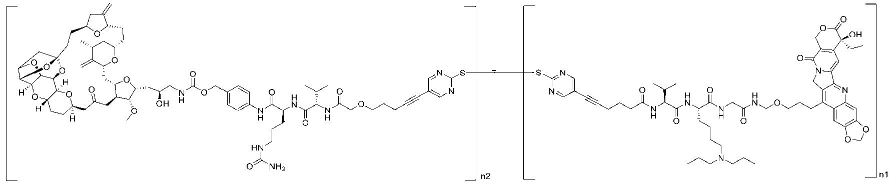

Trastuzumab (0.5 mL, 12.35 mg) was added to a sodium edetate solution with a final concentration of 1 mM and well mixed, and the pH of the sample was adjusted to 7.5 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. A mixed solution of 6.1 molar equivalents (relative to the antibody) of a 10 mmol/L solution of DL003 in DMSO and 2.5 molar equivalents (relative to the antibody) of a 10 mmol/L solution of DL012 in DMSO was added. After 2 h of reaction at 25 °C, cysteine was added in excess. The buffer was exchanged for a 20 mM His-HCl buffer solution (pH 5.9) using a centrifugal ultrafiltration tube (Merck, Amicon Ultra-15) to obtain the HER2-ADC-013 sample. The sample was taken and detected for DAR value, showing that each mole of the antibody was conjugated to 4.7 moles of DL003 and 0.9 moles of DL012. n1 = 4.7, and n2 = 0.9.

### Example 2.14: Preparation of HER2-ADC-014

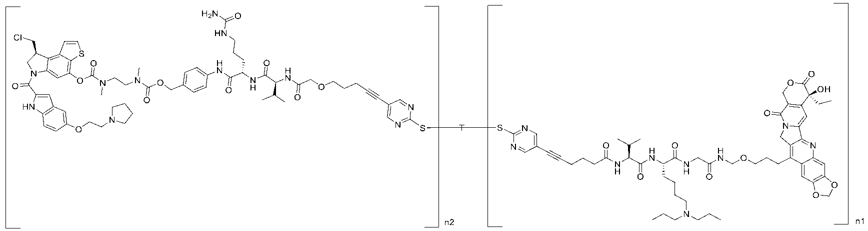

Trastuzumab (0.4 mL, 11.52 mg) was added to a sodium edetate solution with a final concentration of 1 mM and well mixed, and the pH of the sample was adjusted to 7.45 with a 0.5 M disodium hydrogen phosphate solution. 5.75 molar equivalents (relative to the antibody) of a 20 mmol/L TCEP solution was added and well mixed, and the mixture was left to stand at 25 °C for 90 min. 6.8 molar equivalents (relative to the antibody) of a 10 mmol/L solution of DL003 in DMSO was first added. After 2 h of reaction at 25 °C, 3 molar equivalents (relative to the antibody) of a 10 mmol/L solution of DL017 in DMSO was then added. After 4 h of reaction at 25 °C, cysteine was added in excess. The buffer was exchanged for a 20 mM His-HCl buffer solution (pH 5.98) using a centrifugal ultrafiltration tube (Merck, Amicon Ultra-15) to obtain the HER2-ADC-014 sample. The sample was taken and detected for DAR value, showing that each mole of the antibody was conjugated to 7.2 moles of DL003 and 0.8 moles of DL017. n1 = 7.2, and n2 = 0.8.

### Example 2.15: Preparation of HER2-ADC-015

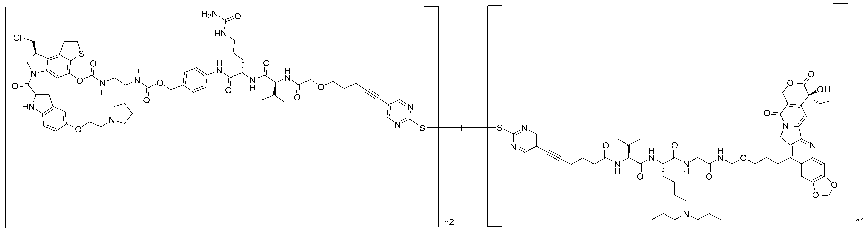

Trastuzumab (0.4 mL, 11.52 mg) was added to a sodium edetate solution with a final concentration of 1 mM and well mixed, and the pH of the sample was adjusted to 7.45 with a 0.5 M disodium hydrogen phosphate solution. 5.75 molar equivalents (relative to the antibody) of a 20 mmol/L TCEP solution was added and well mixed, and the mixture was left to stand at 25 °C for 90 min. 6.2 molar equivalents (relative to the antibody) of a 10 mmol/L solution of DL003 in DMSO was first added. After 2 h of reaction at 25 °C, 3 molar equivalents (relative to the antibody) of a 10 mmol/L solution of DL017 in DMSO was then added. After 4 h of reaction at 25 °C, cysteine was added in excess. The buffer was exchanged for a 20 mM His-HCl buffer solution (pH 5.98) using a centrifugal ultrafiltration tube (Merck, Amicon Ultra-15) to obtain the HER2-ADC-015 sample. The sample was taken and detected for DAR value, showing that each mole of the antibody was conjugated to 7.0 moles of DL003 and 1.0 moles of DL017. n1 = 7.0, and n2 = 1.0.

### Example 2.16: Preparation of B7H3-ADC-001

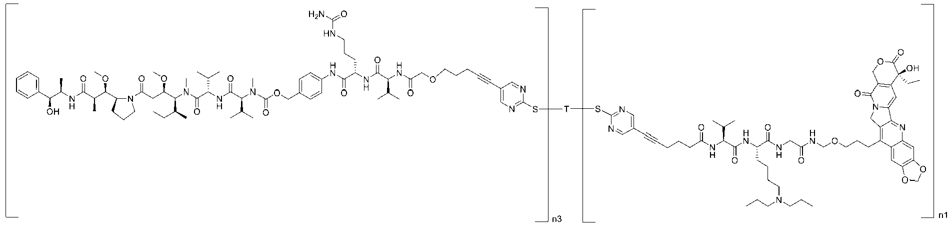

A B7H3 antibody (2E3-02, 342.71 mg) with a concentration of 17.2 mg/mL was added to a sodium edetate solution with a final concentration of 1 mM and well mixed, and the pH of the sample was adjusted to 7.5 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. A mixture (0.680 g) of 6.0 molar equivalents (relative to the antibody) of a solution of DL003 in DMSO and 2.05 molar equivalents (relative to the antibody) of a solution of DL014 in DMSO was added. After 4 h of reaction at 25 °C, cysteine was added in excess. The buffer was exchanged for a 20 mM His-HCl buffer solution (pH 5.98) using a centrifugal ultrafiltration tube (Merck, Amicon Ultra-15) to obtain the B7H3-ADC-001 sample. The sample was taken and detected for DAR value, showing that each mole of the antibody was conjugated to 5.96 moles of DL003 and 1.82 moles of DL014. n1 = 5.96, and n3 = 1.82.

### Example 2.17: Preparation of B7H3-ADC-002

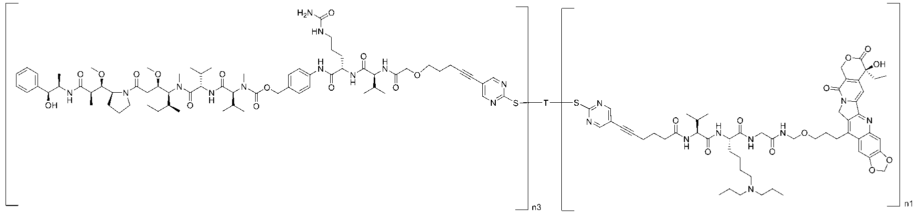

A B7H3 antibody (2E3-02, 345.01 mg) with a concentration of 17.2 mg/mL was added to a sodium edetate solution with a final concentration of 1 mM and well mixed, and the pH of the sample was adjusted to 7.5 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. A mixture (0.714 g) of 6.25 molar equivalents (relative to the antibody) of a solution of DL003 in DMSO and 2.14 molar equivalents (relative to the antibody) of a solution of DL014 in DMSO was added. After 4 h of reaction at 25 °C, cysteine was added in excess. The buffer was exchanged for a 20 mM His-HCl buffer solution (pH 5.98) using a centrifugal ultrafiltration tube (Merck, Amicon Ultra-15) to obtain the B7H3-ADC-002 sample. The sample was taken and detected for DAR value, showing that each mole of the antibody was conjugated to 6.21 moles of DL003 and 1.77 moles of DL014. n1 = 6.21, and n3 = 1.77.

### Example 2.18: Preparation of B7H3-ADC-003

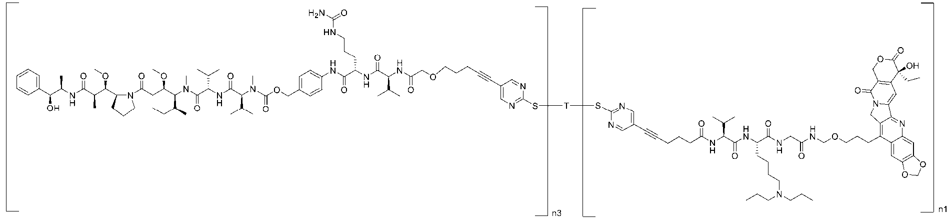

A B7H3 antibody (2E3-02, 339.44 mg) with a concentration of 17.2 mg/mL was added to a sodium edetate solution with a final concentration of 1 mM and well mixed, and the pH of the sample was adjusted to 7.5 with a 0.5 M disodium hydrogen phosphate solution. 5.5 molar equivalents (relative to the antibody) of a 20 mmol/L TCEP solution was added and well mixed, and the mixture was left to stand at 25 °C for 120 min. 6.03 molar equivalents (relative to the antibody) of a 10 mmol/L solution of DL003 in DMSO was first added. After 1 h of reaction at 25 °C, 3.0 molar equivalents (relative to the antibody) of a 10 mmol/L solution of DL014 in DMSO was added. The mixture was left to stand at 25 °C for 180 min, and then cysteine was added in excess to obtain the B7H3-ADC-003 sample. The sample was taken and detected for DAR value, showing that each mole of the antibody was conjugated to 6.0 moles of DL003 and 1.99 moles of DL014. n1 = 6.01, and n3 = 1.99.

### III. Preparation of Single-Toxin Antibody-Drug Conjugates

### Example 3.1: Preparation of HER2-DL012-DAR2

Trastuzumab (0.4 mL, 9.88 mg) was added to a sodium edetate solution with a final concentration of 1 mM and well mixed, and the pH of the sample was adjusted to 7.45 with a 0.5 M disodium hydrogen phosphate solution. 5.5 molar equivalents (relative to the antibody) of a 20 mmol/L TCEP solution was added and well mixed, and the mixture was left to stand at 25 °C for 90 min. 5.0 molar equivalents (relative to the antibody) of a 10 mmol/L solution of DL012 (0.055 mg) in DMSO was added. After 2 h of reaction at 25 °C, cysteine was added in excess. The buffer was exchanged for a 20 mM His-HCl buffer solution (pH 5.9) using a centrifugal ultrafiltration tube (Merck, Amicon Ultra-15) to obtain the HER2-DL012-DAR2 sample. The DAR value was 1.8.

### Example 3.2: Preparation of HER2-DL003-DAR8

5.0 mL of trastuzumab (16.1 mg/mL) was taken and diluted with 0.10 mL of a 20 mM PB + 100 mM disodium edetate solution (pH 7.6), and the pH was adjusted to 7.46 with a 0.5 M Na₂HPO₄ solution. 1.0 mL of the above solution was taken, and a 20 mM TCEP (0.0278 mL, 0.556 µmol) solution was added and well mixed. The mixture was left to stand at room temperature for 90 min. Then, a solution of DL003 (1.332 mg, 10 times the amount of substance (moles) of the antibody) in DMSO (0.1213 mL) was added and well mixed, and the mixture was left to stand at room temperature for 2 h. After that, the buffer was exchanged for a 20 mM His-HCl buffer solution (pH 5.9) using a centrifugal ultrafiltration tube (Merck, Amicon Ultra-15). The HER2-DL003-DAR8 was obtained, and the DAR value was 8.0.

### IV. In Vitro Cell Activity Assay of Antibody-Drug Conjugates (ADCs)

### Example 4.1: Assay for Inhibitory Activity of ADCs Against Proliferation of N87 Tumor Cells

### Method

N87 (1640 + 10% FBS) cells were thawed and cultured, plated onto a 96-well flat-bottom plate at a density of 5000 cells/well and 100 µL/well, and cultured overnight in an incubator at 37 °C. The next day, the test ADC molecules were diluted in a complete culture medium to a maximum concentration of 1000 nM, and the solutions were 4-fold diluted to obtain 10 concentration points. 100 µL of the diluted ADC-003 was added to the cell culture wells (2 replicates per well). After 4 days of culture at 37 °C, 50 µL of Cell titer glo substrate was added to each well. After 5 min of incubation, the fluorescence values were measured. The key material sources are shown in Table 1 below:

**Table 1. Material sources**

| Name | Manufacturer or source | Catalog No. |
|---|---|---|
| N87 cells | Procell | CL-0211 |
| 1640 culture medium | ADamas life | C8016 |
| Fetal bovine serum | Cornning | 35-081-CV |
| Cell titer glo substrate | ADamas life | RA-GL11-A |

### Experimental results:

**Table 2. The inhibitory activity of ADCs against the in vitro proliferation of N87 tumor cells**

| No. | IC₅₀ value |
|---|---|
| HER2-ADC-003 | 2.45 nM |
| HER2-ADC-005 | 0.05 nM |
| HER2-ADC-006 | 0.05 nM |
| HER2-ADC-007 | 0.04 nM |
| HER2-ADC-008 | 0.01 nM |
| HER2-ADC-009 | 0.02 nM |
| HER2-ADC-014 | 2.21 nM |
| HER2-ADC-015 | 3.23 nM |
| B7H3-ADC-003 | 0.80 nM |

As shown in Table 2, the multi-warhead ADC molecules of the present disclosure all exhibited significant inhibitory activity against the proliferation of the N87 tumor cell line.

### Example 4.2: Assay for Inhibitory Activity of Multi-Warhead ADCs Against In Vitro Proliferation of a Variety of Human Tumor Cell Lines

### Method

The inhibitory activity of the multi-warhead ADCs against cell growth was determined using an SKBR3 cell line with high HER2 expression and H358 and MCF7 cell lines with low HER2 expression. H358 (RPMI1640 + 10% FBS), SKBR3 (RPMI1640 + 10% FBS), and MCF7 (DMEM + 10% FBS + 5 µg/mL insulin) tumor cells were cultured in a specified cell culture medium in a 5% CO₂ incubator at 37 °C according to a conventional method. The H358, SKBR3, and MCF7 tumor cells were digested with trypsin by a conventional method, collected, and counted. The cells were resuspended in corresponding complete culture media and added to 96-well plates at 5000 cells/well and 100 µL/well. The test drugs were diluted in a complete culture medium. The multi-warhead ADCs were 4-fold diluted from a concentration of 2000 nM to obtain 10 concentration points. A total of 100 µL of the diluted ADCs was added to 96-well plates containing 100 µL of culture medium and cells, and the plates were incubated in a 5% CO₂ incubator at 37 °C for 4 days. After the incubation was completed, 50 µL of cell titer glow substrate was added to each cell well. After 5 min of incubation at room temperature, the full-wavelength fluorescence values were measured. GraphPad software was used to plot dose-dependent cell proliferation inhibition and analyze their half maximal inhibitory concentrations (IC₅₀, nM). The key material sources are shown in Table 3 below:

**Table 3. Material sources**

| Name | Manufacturer or source | Catalog No. |
|---|---|---|
| SKBR3 cells | Procell | CL-0211 |
| H358 cells | Cell Bank of the Chinese Academy of Sciences | TCHu151 |
| MCF7 cells | ATCC | HTB-22 |
| RPMI1640 | ADamas life | C8016 |
| DMEM | ADamas life | C8013 |
| Insulin | YEASEN | 40112ES60 |

### 2. Experimental results

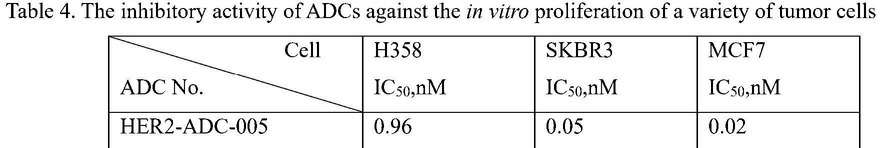

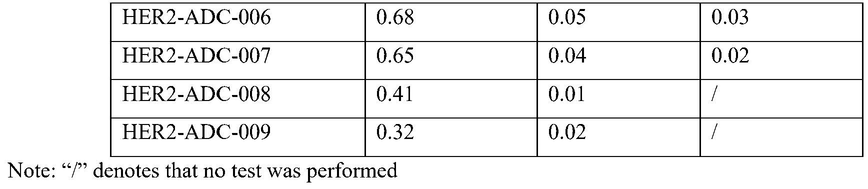

As shown in Table 4, the multi-warhead ADC molecules of the present disclosure exhibited significant inhibitory activity against the proliferation of tumor cell lines with high HER2 expression or low HER2 expression.

### Example 4.3: Assay for Inhibitory Activity of Multi-Warhead ADC Against In Vitro Proliferation of N87 Cells Overexpressing ABCB 1/ABCG2 Transporters

### 1. Method

The inhibitory activity of a multi-warhead ADC and single-toxin ADCs against proliferation was assessed using N87 cells overexpressing human ABCB1 or human ABCG2 (ATCC, engineered by WuXi AppTec). N87 tumor cells overexpressing human ABCB1 and human ABCG2 were cultured in a specified cell culture medium (RPMI1640 + 10% FBS) in a 5% CO₂ incubator at 37 °C according to a conventional method. The tumor cells were digested with trypsin by a conventional method, collected, and counted. The cells were resuspended in corresponding complete culture media and added to 96-well plates at 3000 cells/well and 100 µL/well. Preparation of ADC sample concentration gradients: The test drugs were diluted in a complete culture medium. For N87 cells overexpressing human ABCB1, the ADCs were 5-fold diluted from a concentration of 2000 nM to obtain 10 concentration points. For N87 cells overexpressing human ABCG2, HER2-DL003-DAR8 was 5-fold diluted from 2000 nM to obtain 10 concentration points, and the other ADC drugs were diluted from 200 nM to obtain 10 concentration points. A total of 100 µL of the diluted ADCs was added to a 96-well plate containing 100 µL of culture medium and cells, and the plate was incubated in a 5% CO₂ incubator at 37 °C for 6-7 days (7 days for the toxin and 6 days for the ADC). After the incubation was completed, 75 µL of cell titer glow substrate was added to each cell well. After 10 min of incubation at room temperature, the full-wavelength fluorescence values were measured. GraphPad software was used to plot dose-dependent cell proliferation inhibition and analyze their half maximal inhibitory concentrations (IC₅₀, nM).

### 2. Experimental results

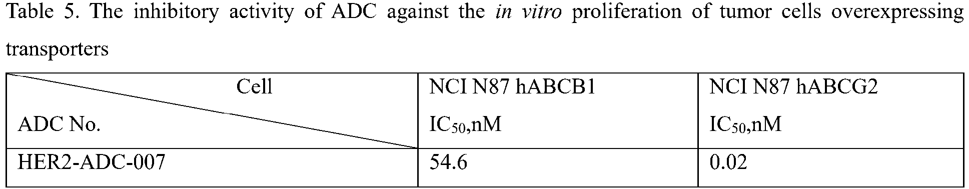

HER2-DL012-DAR2 (single-toxin ADC) lost activity in N87 tumor cells overexpressing human ABCB1, and HER2-DL003-DAR8 (single-toxin ADC) lost activity in N87 cells overexpressing human ABCG2. However, the multi-warhead ADC molecule retained good activity in the cells overexpressing the above two transporters (as shown in Table 5). It can be seen that the multi-warhead ADC of the present disclosure can overcome the problem of drug resistance to the single-toxin ADCs.

### Example 4.4: Assay for Inhibition of Multi-Warhead ADC Against In Vitro Activity of Enhertu-Resistant NCI-N87 Tumor Cells

### 1. Method:

Enhertu-resistant NCI-N87 tumor cells (RPMI 1640 + 10% FBS + 1 µg/mL puromycin + 100 ng/mL Enhertu; engineered by WuXi AppTec) were thawed and cultured, plated onto a 96-well flat-bottom plate at a density of 2E4 cells/well and 135 µL/well, and cultured overnight in an incubator at 37 °C. The next day, the test ADC molecule was diluted in a serum-free culture medium to a maximum final concentration of 300 nM, and the solution was 4-fold diluted to obtain 9 concentration points in total. The diluted ADC samples (15 µL) (10*) were added to cell culture wells. After 6 days of culture at 37 °C, 75 µL of CellTiter-Glo substrate was added to each well, and the cell plate was covered by aluminum foil to keep it out of the light. The culture plate was shaken on an orbital shaker for 2 min to induce cell lysis. The culture plate was left to stand at room temperature for 10 min to stabilize luminescence signals, and then luminescence signals were measured on a 2104 EnVision plate reader.

### 2. Experimental results:

**Table 6. The inhibitory activity of ADC on the in vitro proliferation of Enhertu-resistant NCI-N87 tumor cells**

| ADC No. | IC₅₀ value |
|---|---|
| HER2-ADC-007 | 1.85 nM |

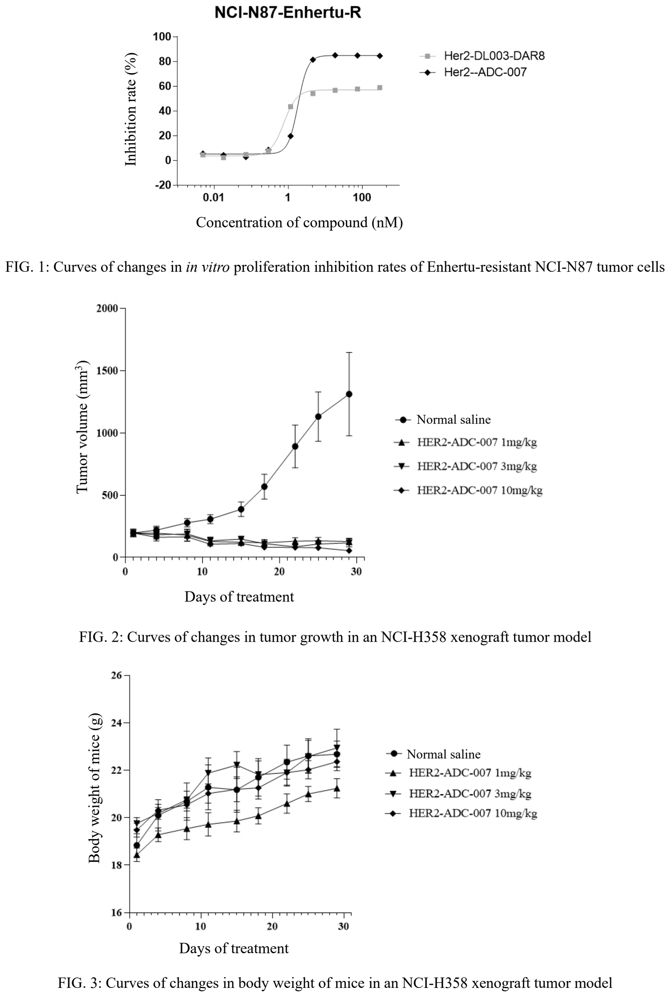

As shown in Table 6, the double-toxin ADC molecule of the present disclosure exhibited significant inhibitory activity against the proliferation of Enhertu-resistant NCI-N87 tumor cells. As shown in FIG. 1, the inhibition rate of the double-toxin ADC molecule of the present disclosure against Enhertu-resistant NCI-N87 tumor cells was significantly higher than that of the single-toxin ADC molecule.

### V. In Vivo Tumor Activity Assay of Antibody-Drug Conjugate (ADC)

### Example 5.1: Efficacy Assay of Multi-Warhead ADC on NCI-H358 Xenograft Tumor

### 1. Experimental materials

Test drug: HER2-ADC-007;
Negative control: normal saline;
Experimental cells: NCI-H358 human non-small cell lung cancer cells, purchased from ATCC;
Experimental animals: Balb/c nu nude mice, female, 5-6 weeks old, purchased from Chengdu GemPharmatech Co., Ltd.

### 2. Method

### 2.1. Cell treatment

NCI-H358 cells were cultured in a 1640 culture medium containing 10% FBS in a culture dish with a diameter of 15 cm and digested with trypsin-EDTA when the confluence reached about 80%-90%. The cells were washed twice with PBS, centrifuged, resuspended in pre-cooled PBS, and counted on a cell counter. The cells were diluted with PBS to bring the cell concentration to 5 × 10⁷ cells/mL.

### 2.2. Tumor cell transplantation

Balb/c nu mice were acclimated to the laboratory environment for 3-5 days, and subcutaneously inoculated with NCI-H358 cells in the right flank in an inoculation amount of 5 × 10⁶ cells/mouse with an inoculation volume of 0.2 mL (containing 50% Matrigel). When the tumors grew to about 200 mm³, the mice were used for the experiment. The mice were divided into 4 groups of 5 according to the tumor volume in S-type grouping.

### 2.3. Animal administration and detection

The enrolled tumor-bearing nude mice were administered through the tail vein according to the following regimens:

**Table 7. Administration regimens for NCI-H358 xenograft tumor model**

| No. | Group | Number of animals | Administrati on dose | Route and cycle of administration |
|---|---|---|---|---|
| 1 | Normal saline | 5 | / | i.v., 10 mL/kg QW×3 |
| 2 | HER2-ADC-007 | 5 | 1 mg/kg | i.v., 10 mL/kg QW×3 |
| 3 | HER2-ADC-007 | 5 | 3 mg/kg | i.v., 10 mL/kg QW×3 |
| 4 | HER2-ADC-007 | 5 | 10 mg/kg | i.v., 10 mL/kg QW×3 |

The tumor volume, photographs, and weight were recorded at the end of the experiment.

### 2.4. Measurement of tumor volume and body weight:

The tumor volume and body weight were measured on days 1, 4, 8, 11, 15, 18, 22, 25, and 29 of the experiment, and TGI% was calculated.

Tumor volume (V) was calculated using the formula: V = 1/2 × L_{long} × Lₛₕₒᵣₜ².

TGI was calculated using the formula: TGI (%) = (1 - T/C) × 100% (T and C are the relative tumor volume of the treatment group and control group, respectively, at a particular time point).

### Experimental results

**Table 8. Effect of multi-warhead ADC on tumor size in NCI-H358 xenograft tumor model (tumor volume: mm³)**

| Group | Mean tumor volume (mm³) D1 | Mean tumor volume (mm³) D29 | TGI(%) D29 |
|---|---|---|---|
| Normal saline | 197.83 | 1312.02 | / |
| HER2-ADC-007 1mg/kg | 196.86 | 128.20 | 90.18 |
| HER2-ADC-007 3mg/kg | 196.01 | 117.17 | 90.99 |
| HER2-ADC-007 10mg/kg | 196.36 | 54.74 | 95.80 |

As shown in Table 8, FIG. 2, and FIG. 3, the multi-warhead ADC of the present disclosure had a very strong *in vivo* tumor-inhibiting effect, with TGI greater than 90% and up to 95.80%. During the administration, the body weight of the mice did not significantly decrease, and there was no other significant toxicity.

### VI. Assay for Conjugation Efficiency of Antibody-Drug Conjugate (ADC)

During the preparation of B7H3-ADC-001, 0.2 mL of samples were taken after 10 min, 30 min, 1 h, 2 h, 3 h, and 4 h, respectively, and cysteine was added in excess. The samples were taken and detected for DAR value. The results (see Table 9) show that the DAR of the conjugation reaction of DL003 could reach 6.0 in about 10 min, showing a relatively high reaction rate and a utilization rate close to 100%; the conjugation reaction of DL014 ended in more than 2 h, and the DAR reached 1.82, showing a utilization rate of about 91%.

**Table 9. Conjugation efficiency of multi-toxin antibody-drug conjugate**

| Conjugation time | DAR(B7H3-ADC-001) | DAR(DL003) | DAR(DL014) |
|---|---|---|---|
| 10 min | 6.59 | 6.06 | 0.53 |
| 30 min | 7.15 | 6.02 | 1.13 |
| 1 h | 7.53 | 6.05 | 1.48 |
| 2 h | 7.70 | 5.95 | 1.76 |
| 3 h | 7.77 | 6.02 | 1.75 |
| 4 h | 7.78 | 5.96 | 1.82 |

As can be seen from the experimental results of the conjugation efficiency of the antibody-drug conjugate described above, the conjugation of DL003 was substantially completed after 10 min and tended to be stable after 30 min, and the utilization rate was close to 100%. The other toxin linker tended to be stable after 2 h of conjugation. Therefore, in the present invention, the ratio of a variety of toxins can be precisely controlled by controlling the feeding amount of the toxin-linker to achieve an appropriate ratio of toxin-linker.

### VII. Assay for Stability of Antibody-Drug Conjugates (ADCs)

### Objective of study:

The ADCs were simulated to be cleaved in the tumor tissue/tumor microenvironment, the stability of the ADCs in nude mouse tumor tissue homogenates, mouse muscle tissue homogenates, homogenate matrix normal saline, human plasma, monkey plasma, rat plasma, and mouse plasma was investigated *in vitro,* and the toxin release was determined in an incubation system. The tumor tissue homogenate results were compared with the muscle tissue and plasma stability results to evaluate the release efficiency of toxin molecules in the tumor tissue/tumor microenvironment.

### Procedures:

The tissue homogenate was diluted with normal saline for later use. The protein concentration in each homogenate was measured and adjusted to the same level by dilution. The prepared ADC dilutions were added to the homogenates or blood and incubated at 37 °C, and the toxin release was determined at 0 h, 4 h, 24 h, and 48 h. See Example 6.11 of WO2022170971 for details.

### Results and discussion:

From the release of the toxin molecules, the ADCs of the present invention could release a large amount of toxin molecules in tumor tissue homogenates, while there was no significant release in normal tissue homogenates and plasma. It is suggested that the toxin-linkers of the ADCs of the present invention have the characteristic of specifically releasing toxin molecules from tumor tissues.

Although specific embodiments of the present invention have been described above, it will be appreciated by those skilled in the art that these embodiments are merely illustrative and that many changes or modifications can be made to these embodiments without departing from the principles and spirit of the present invention. The protection scope of the present invention is therefore defined by the appended claims.

## Claims

1. A multi-warhead antibody-drug conjugate of formula I, 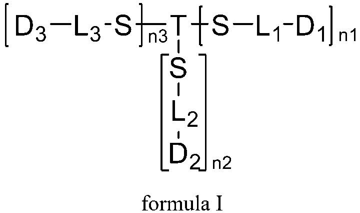
or a stereoisomer, a prodrug, a pharmaceutically acceptable salt, or a solvate thereof,
wherein
T is a non-engineered antibody;
L₁, L₂, and L₃ are linkers that covalently bind to T and corresponding D₁, D₂, and D₃, and L₁, L₂, and L₃ may be identical or different;
D₁, D₂, and D₃ are drug units, and D₁, D₂, and D₃ may be identical or different; and when D₁, D₂, and D₃ are identical, L₁, L₂, and L₃ are different;
n1, n2, and n3 are each independently selected from any numerical value between 0 and 8, and when one of n1, n2, and n3 is 0, the other two are not 0;
an S atom attached to T is not an additional extraneous sulfur atom, but is -S- formed by attaching a sulfhydryl group contained in T itself after a disulfide bond is opened to the linker.

2. The drug conjugate or the stereoisomer, the prodrug, the pharmaceutically acceptable salt, or the solvate thereof according to claim 1, wherein one or more of the following conditions are met:
(1) L₁, L₂, and L₃ are selected from cleavable linkers and non-cleavable linkers;
preferably, L₁, L₂, and L₃ are selected from linkers that are cleavable in a tumor microenvironment;
wherein the linker preferably comprises an amino acid residue or a short peptide consisting of 2-10 amino acid residues, and more preferably comprises a dipeptide, a tripeptide, or a tetrapeptide;
(2) D₁, D₂, and D₃ are each independently selected from a tubulin inhibitor, such as MMAE/MMAF and a derivative thereof, eribulin and a derivative thereof, or a maytansine derivative; a topoisomerase inhibitor, such as a camptothecin derivative; and a drug that interferes with DNA synthesis, such as duocarmycin or calicheamicin and a derivative thereof;
preferably, D₁, D₂, and D₃ are each independently selected from MMAE/MMAF and a derivative thereof, eribulin and a derivative thereof, a camptothecin derivative, and duocarmycin or calicheamicin and a derivative thereof;
more preferably, D₁ is a camptothecin derivative;
more preferably, D₂ is eribulin and a derivative thereof, or duocarmycin or calicheamicin and a derivative thereof;
more preferably, D₃ is MMAE/MMAF and a derivative thereof;
(3) T is an anti-Her 2 antibody or an antigen-binding fragment thereof, an anti-H7H3 antibody or an antigen-binding fragment thereof, or an anti-Trop-2 antibody or an antigen-binding fragment thereof, preferably an anti-Her 2 antibody or an antigen-binding fragment thereof;
the anti-Her 2 antibody is, for example, trastuzumab or pertuzumab, preferably trastuzumab;
the anti-B7H3 antibody is, for example, 1D1, 1D1-01, 2E3, or 2E3-02 antibody, enoblituzumab, mirzotamab, or omburtamab;
the anti-Trop-2 antibody is, for example, sacituzumab;
(4) the S atoms attached to T in formula I are all derived from disulfide bonds in the antibody;
(5) n1 is selected from integers or decimals from 0 to 1, from 1 to 2, from 2 to 3, from 3 to 4, from 4 to 5, from 5 to 6, from 6 to 7, and from 7 to 8;
(6) n2 is selected from integers or decimals from 0 to 1, from 1 to 2, from 2 to 3, from 3 to 4, from 4 to 5, from 5 to 6, from 6 to 7, and from 7 to 8;
(7) n3 is selected from integers or decimals from 0 to 1, from 1 to 2, from 2 to 3, from 3 to 4, from 4 to 5, from 5 to 6, from 6 to 7, and from 7 to 8.

3. The drug conjugate or the stereoisomer, the prodrug, the pharmaceutically acceptable salt, or the solvate thereof according to claim 2, wherein one or more of the following conditions are met:
(1) the camptothecin derivative is selected from the following structures: 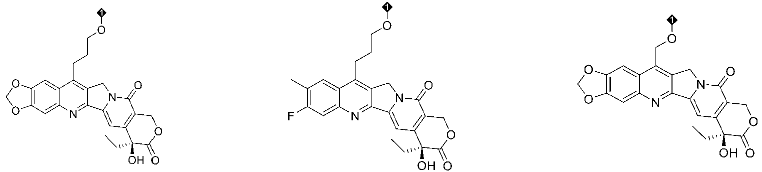 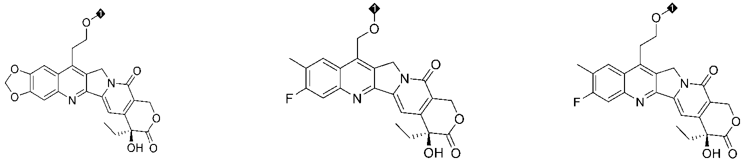 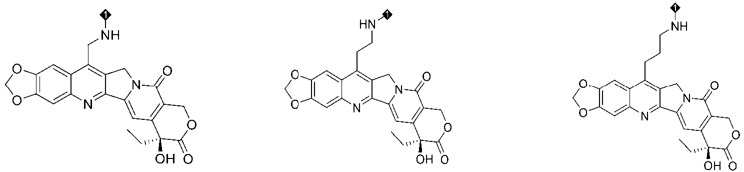 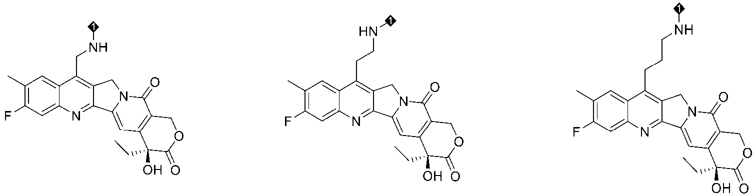 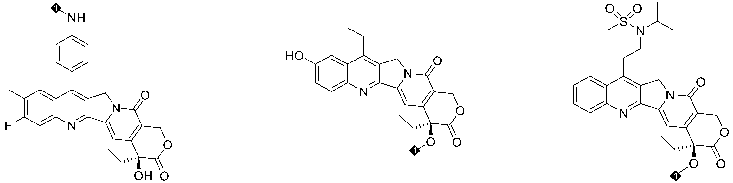 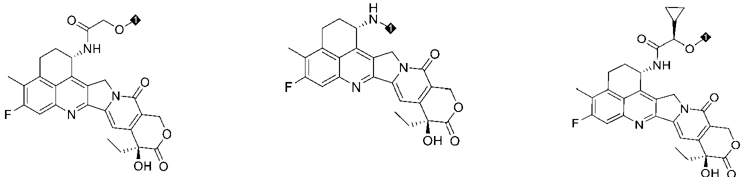 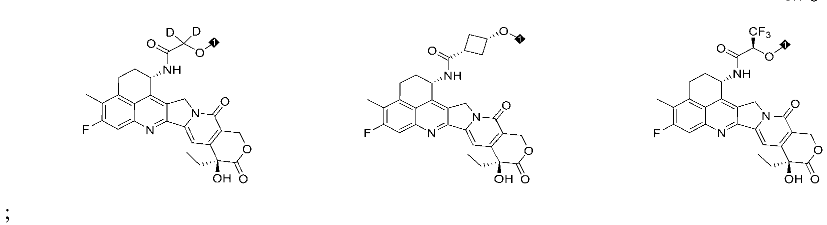 preferably selected from the following structures: 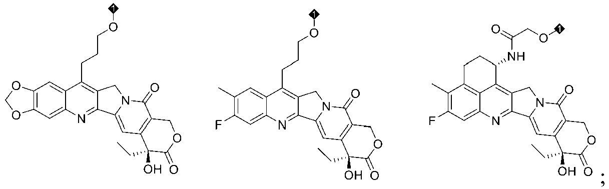
(2) the eribulin derivative is selected from the following structures: 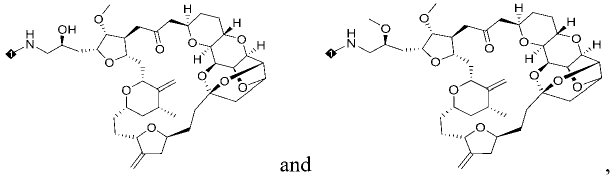 preferably 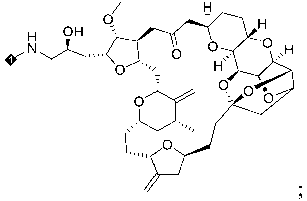
(3) the MMAE/MMAF and the derivative thereof are selected from the following structures: 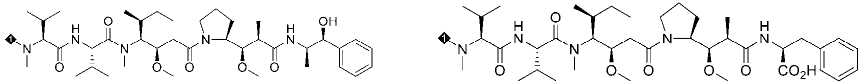 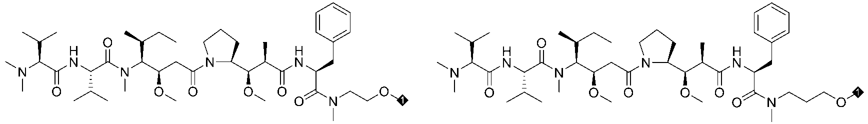 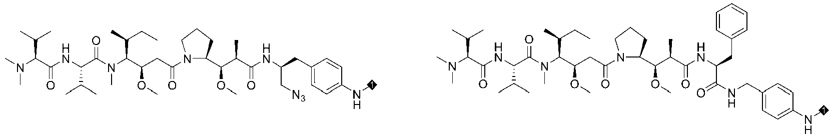 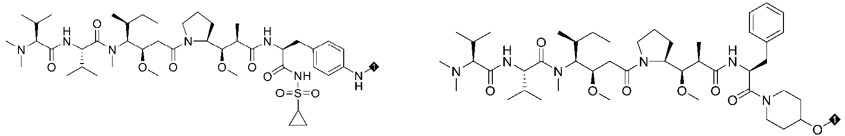 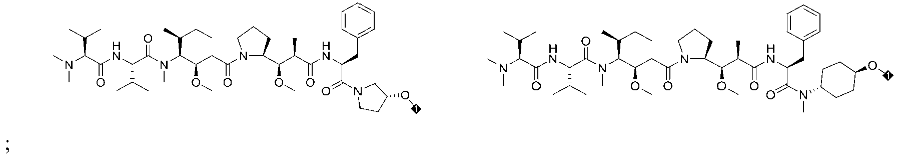 preferably selected from the following structures: 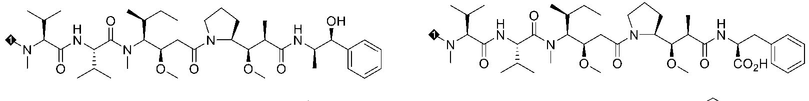 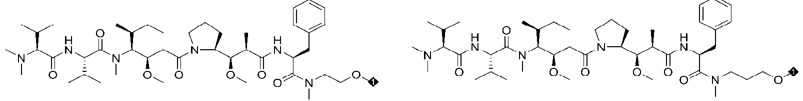 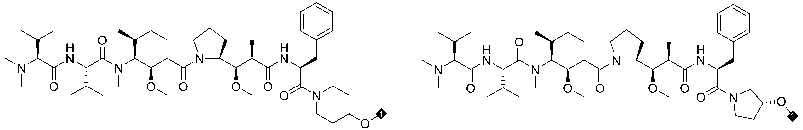 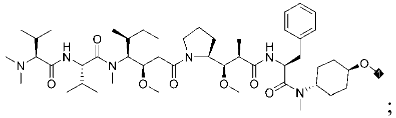
(4) the duocarmycin or calicheamicin and the derivative thereof are selected from the following structures: 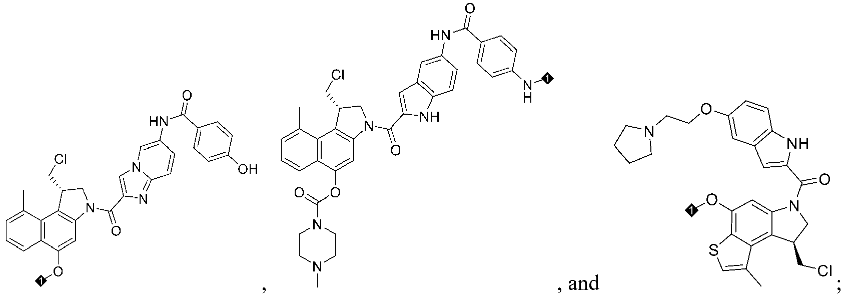
(5) D₁ and D₂ are different, n1 and n2 are not 0, and n3 is 0; or D₁ and D₃ are different, n1 and n3 are not 0, and n2 is 0.

4. The drug conjugate or the stereoisomer, the prodrug, the pharmaceutically acceptable salt, or the solvate thereof according to any one of claims 1-3, wherein L₁, L₂, and L₃ are each independently selected from the following structures: 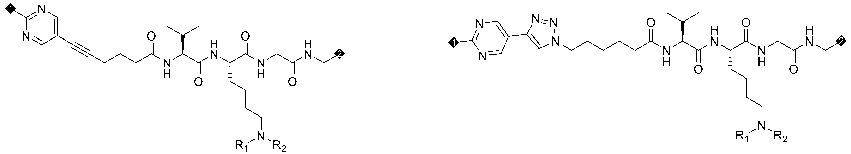 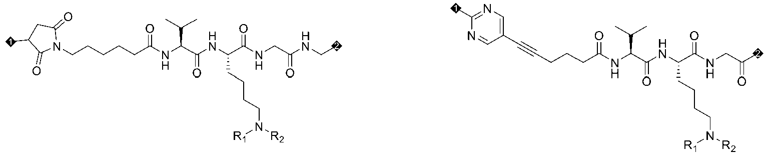 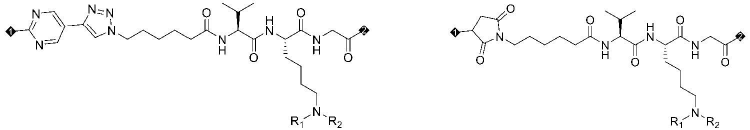 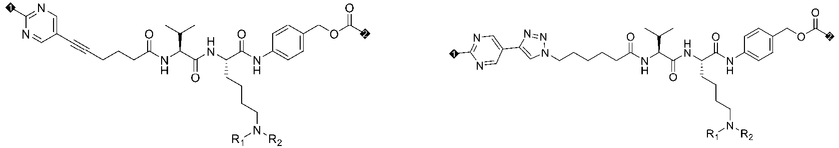 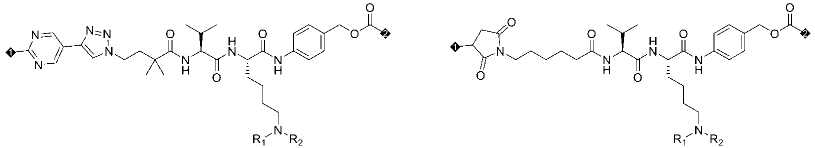 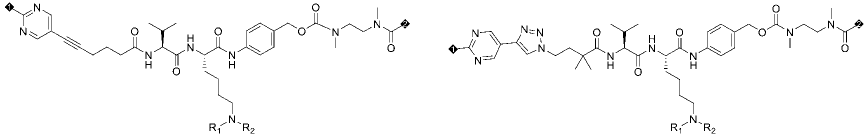 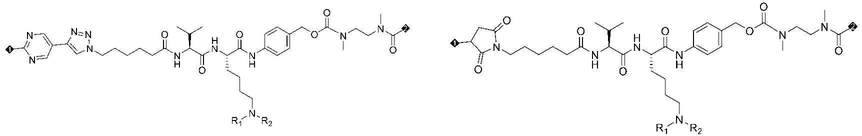 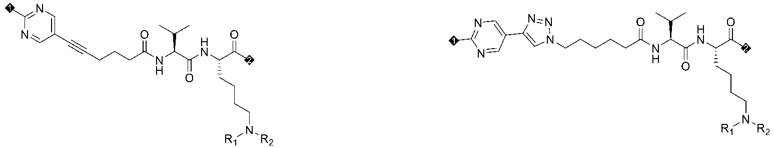 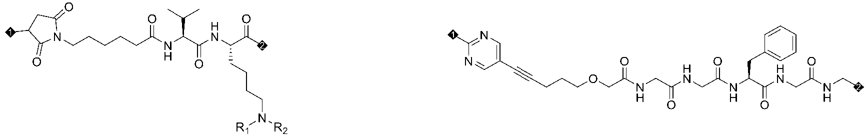 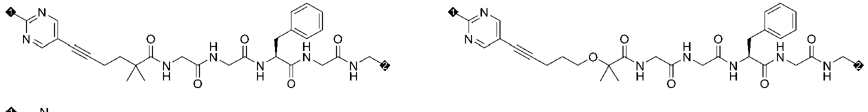 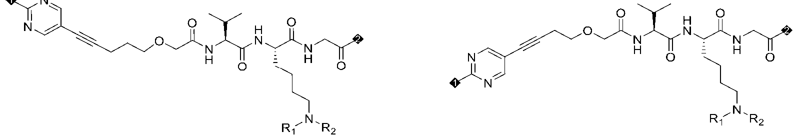 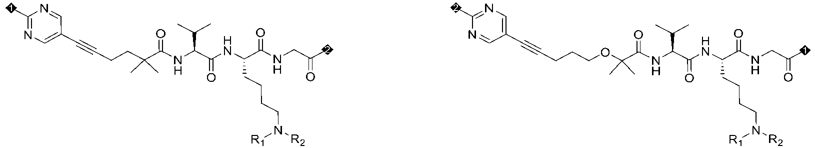 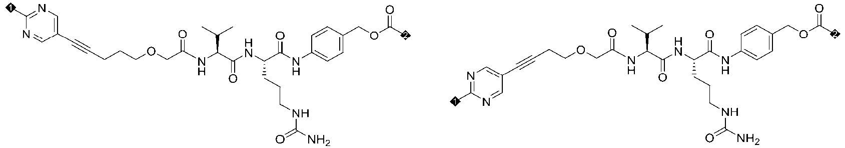 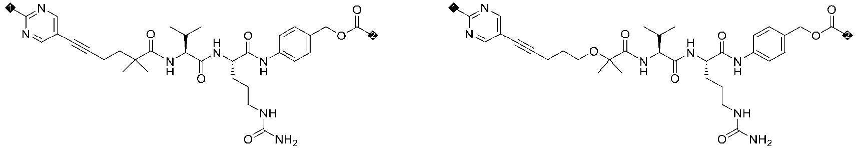 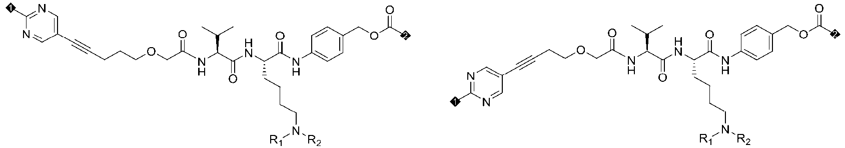 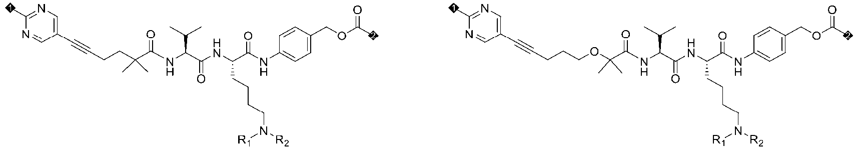 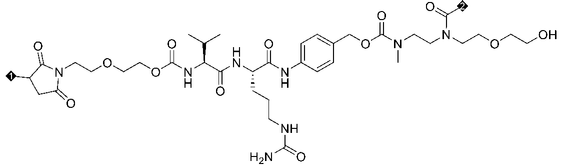 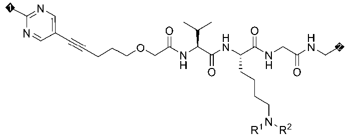
wherein position 1 is attached to an S atom, and position 2 is attached to D₁, D₂, or D₃; R₁ and R₂ are each independently selected from H, C1-6 alkyl (for example, methyl, ethyl, or propyl), and C3-6 cycloalkyl (for example, cyclopropyl); preferably, R₁ and R₂ are both methyl, ethyl, or propyl;
preferably, L₁, L₂, and L₃ are each independently selected from the following structures: 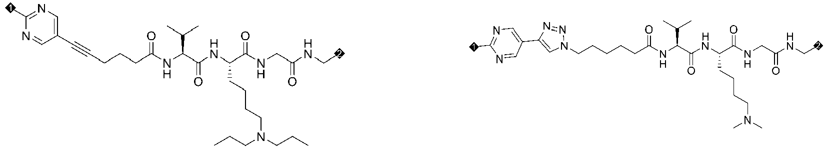 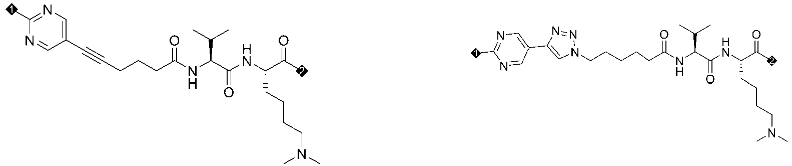 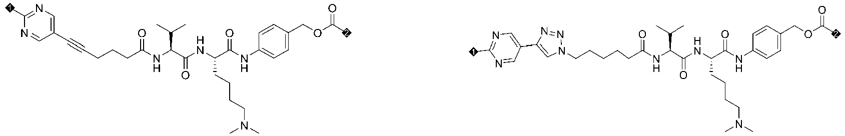 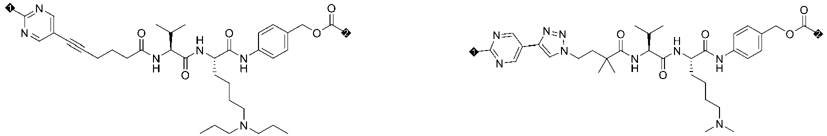 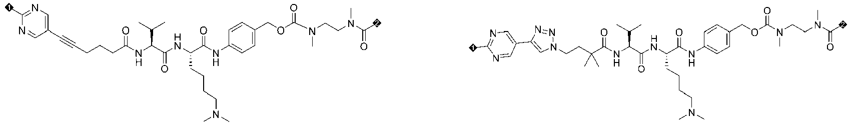 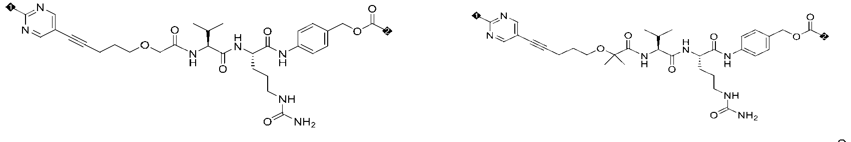 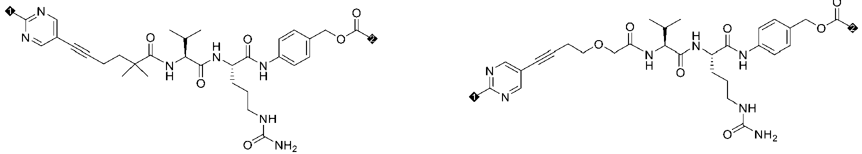 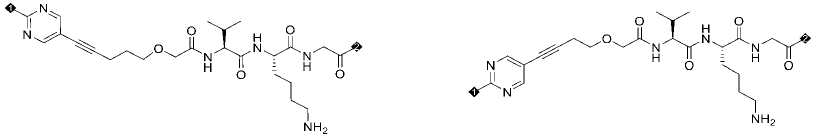 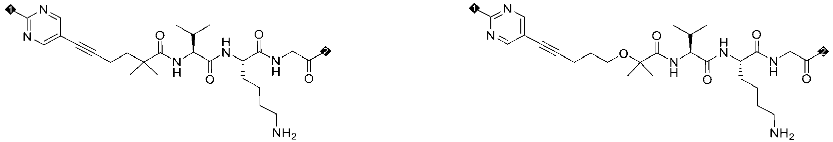 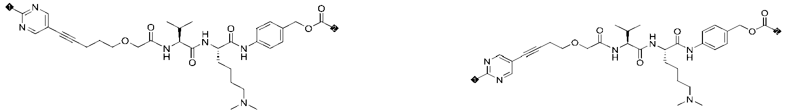 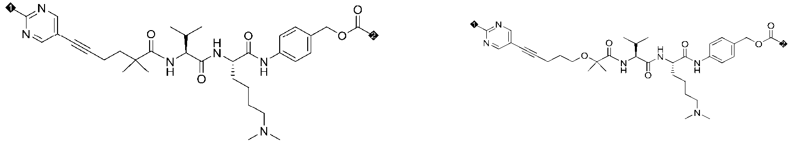 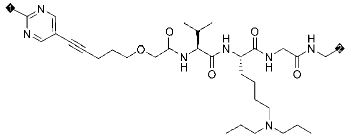
wherein position 1 is attached to an S atom, and position 2 is attached to D₁, D₂, or D₃.

5. The drug conjugate or the stereoisomer, the prodrug, the pharmaceutically acceptable salt, or the solvate thereof according to any one of claims 1-3, wherein one or more of the following conditions are met:
(1) L₁ is 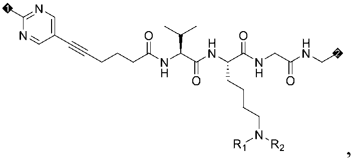 preferably 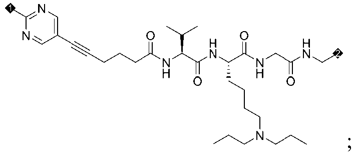
(2) L₂ is 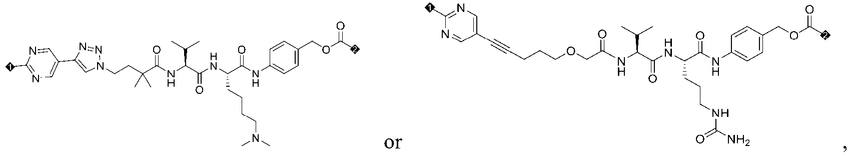 preferably 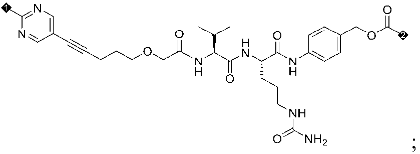
(3) L₃ is 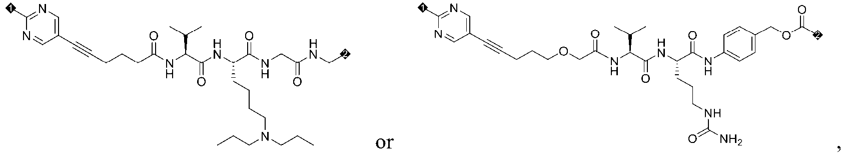 preferably

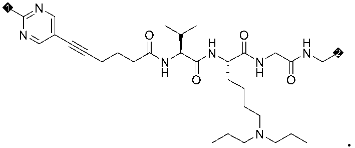

6. The drug conjugate or the stereoisomer, the prodrug, the pharmaceutically acceptable salt, or the solvate thereof according to any one of claims 1-3, wherein one or more of the following conditions are met:
(1) -L₁-D₁ is selected from the following structural fragments: 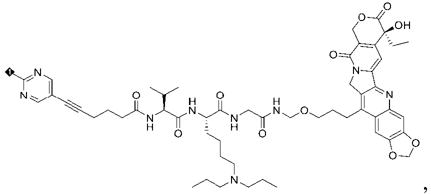 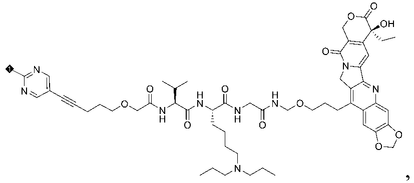 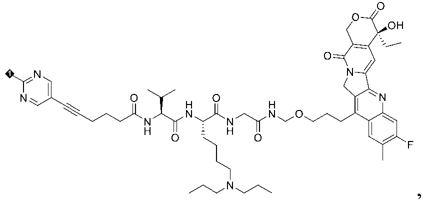 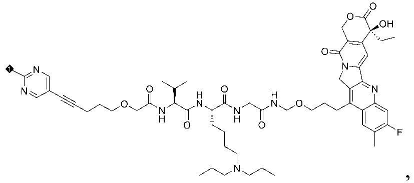 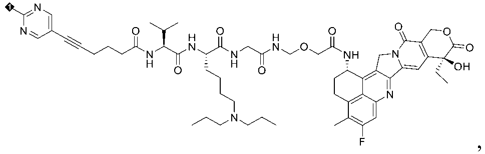 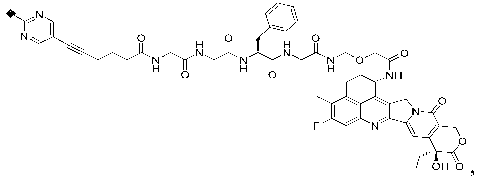 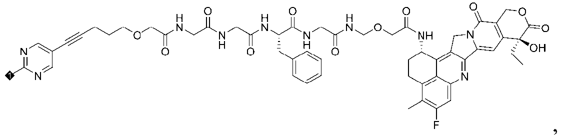 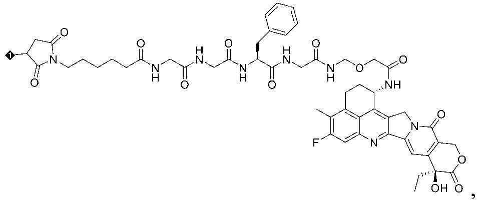 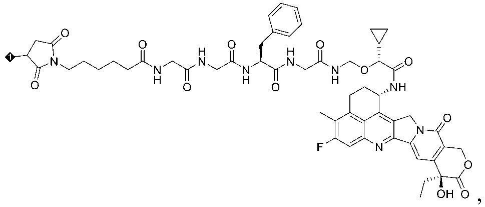 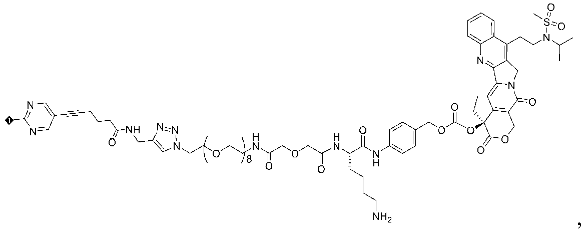 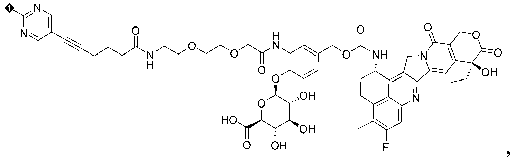 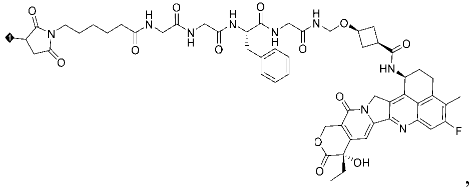 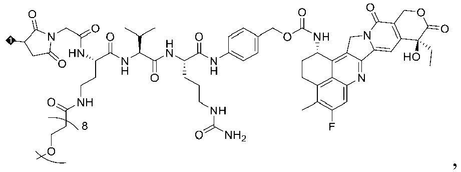 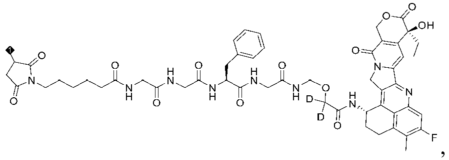 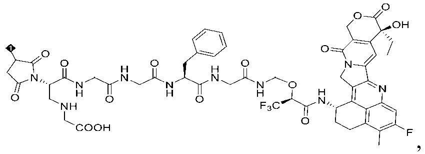 and 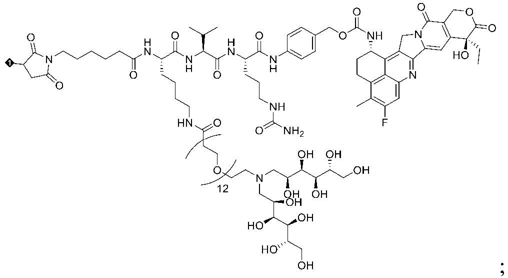 preferably selected from the following structural fragments: 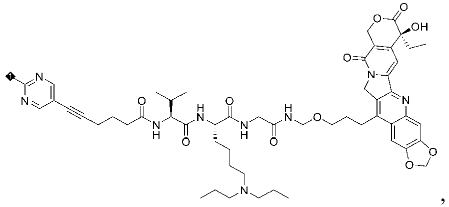 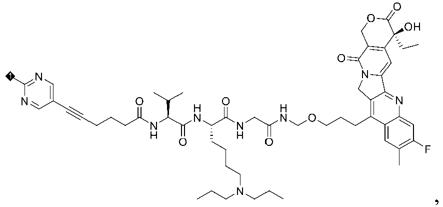 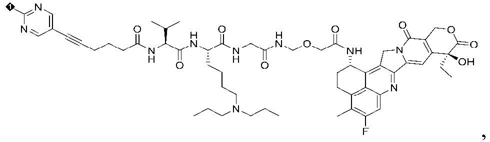 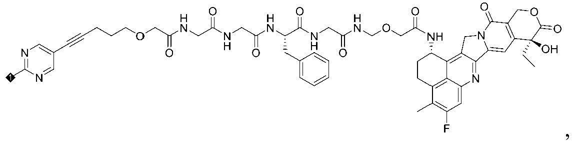 and 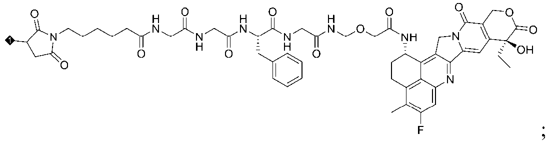
(2) -L₂-D₂ is selected from the following structural fragments: 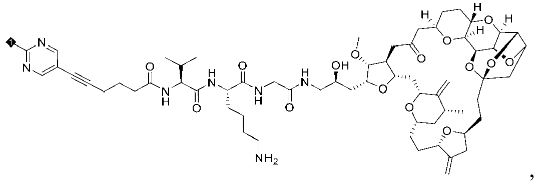 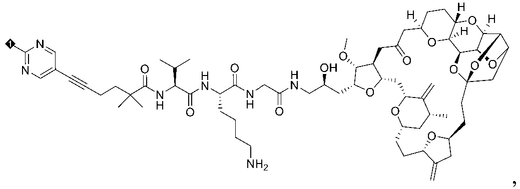 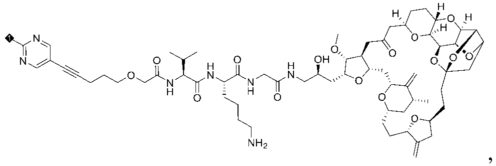 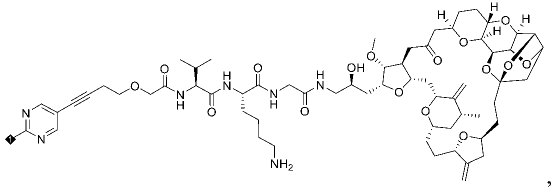 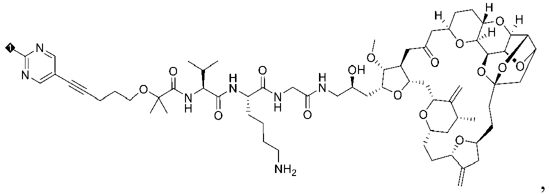 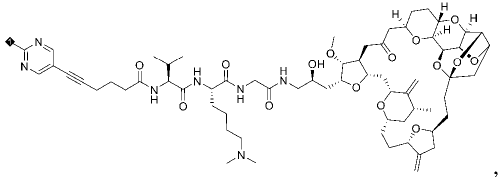 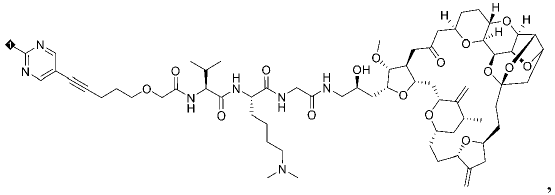 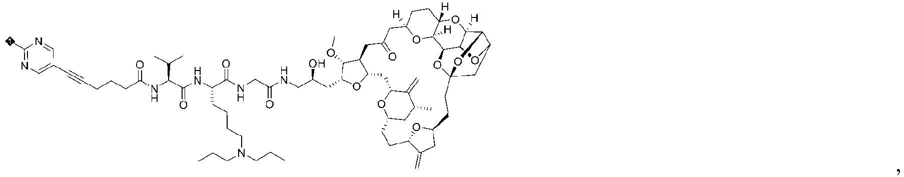 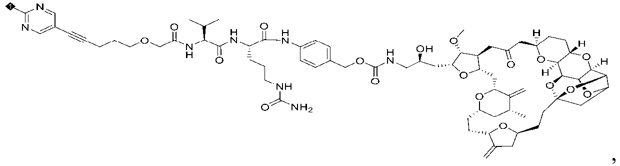 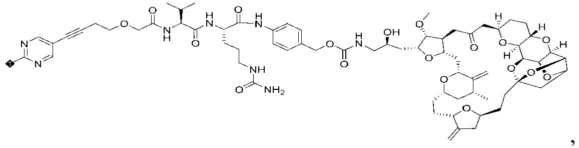 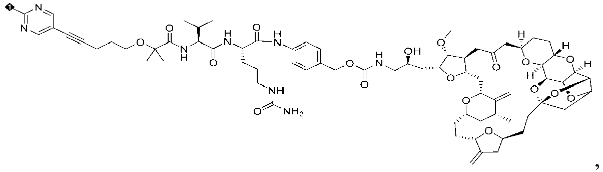 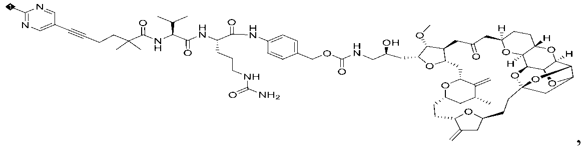 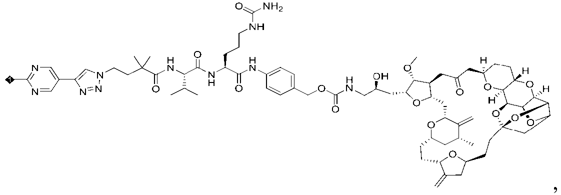 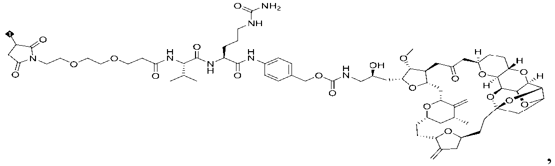 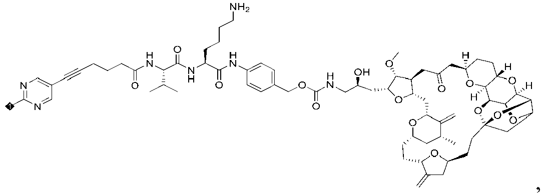 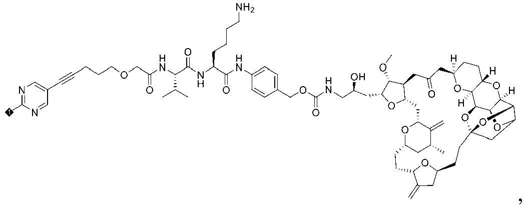 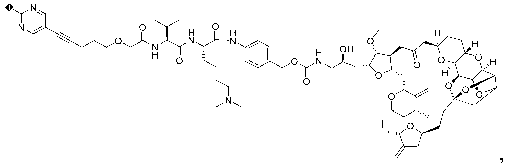 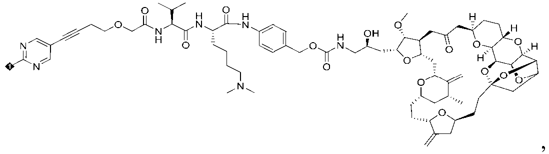 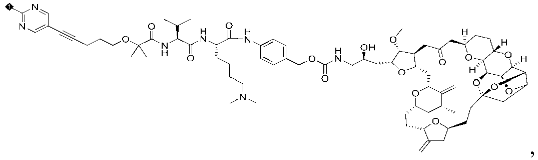 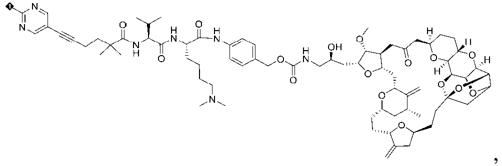 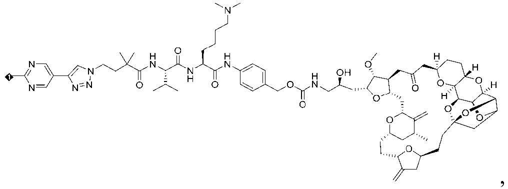 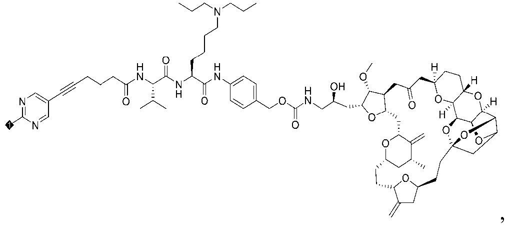 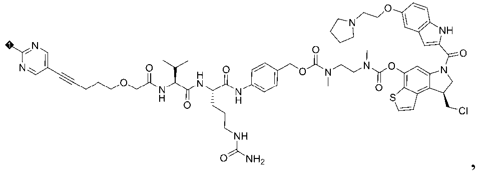 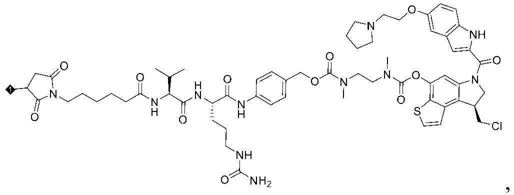 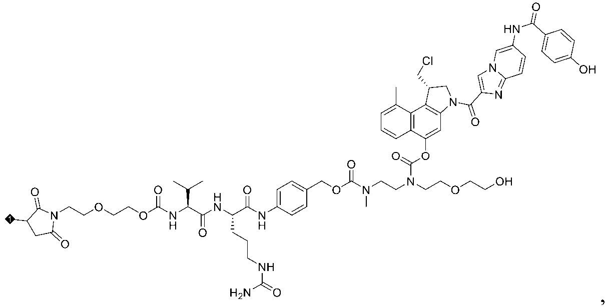 and 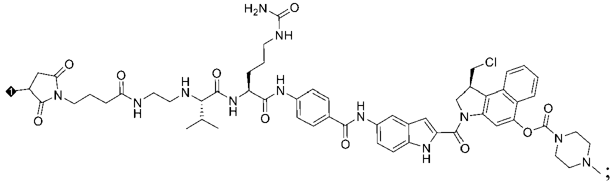
(3) -L₃-D₃ is selected from the following structural fragments: 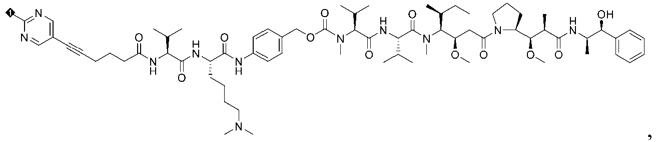 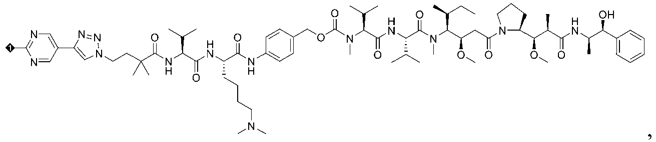 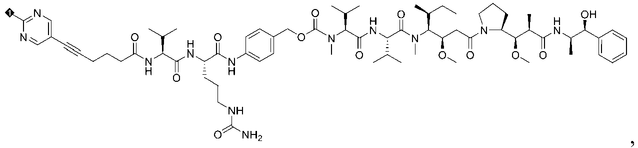 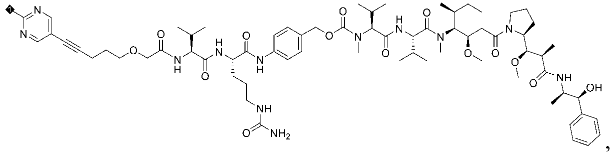 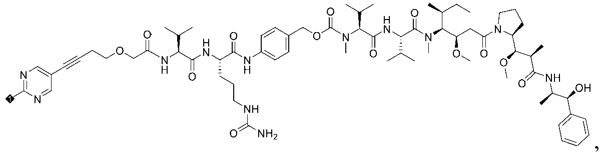 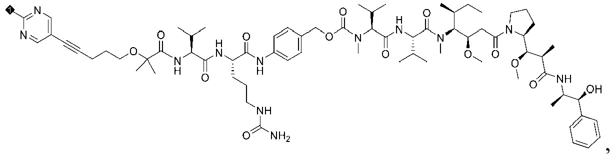 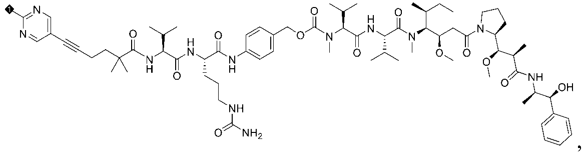 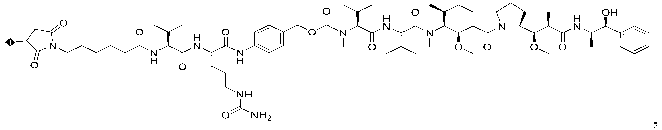 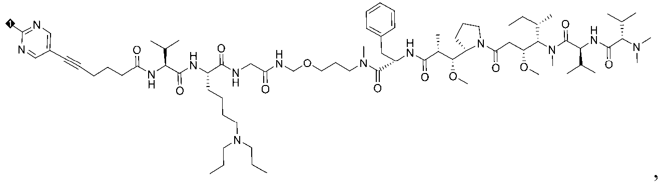 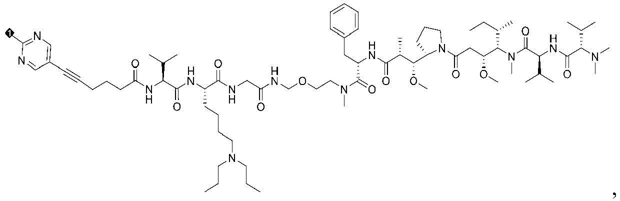 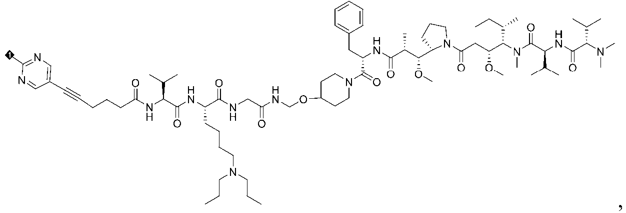 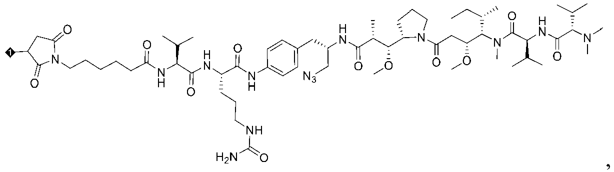 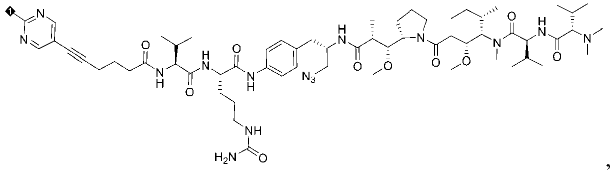 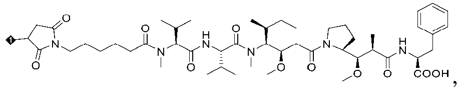 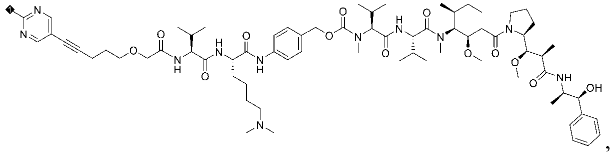 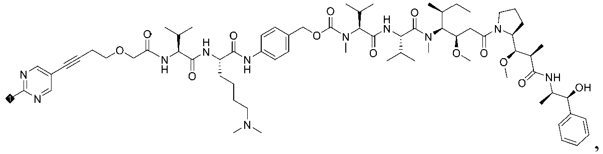 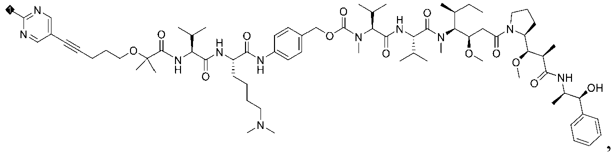 and 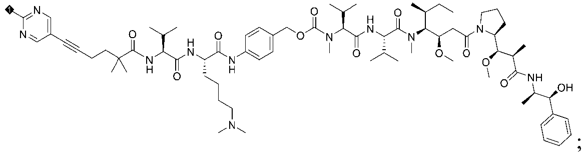 preferably selected from the following structural fragments: 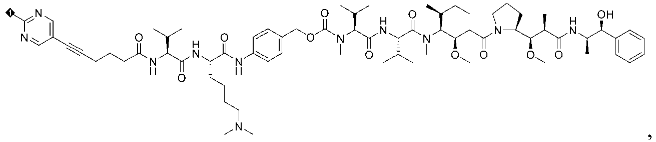 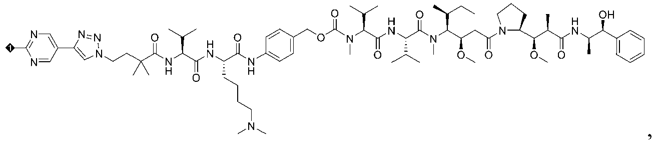 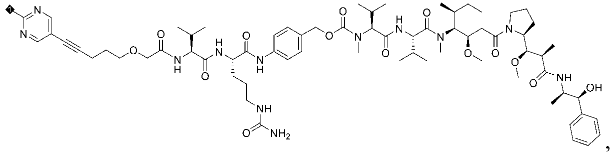 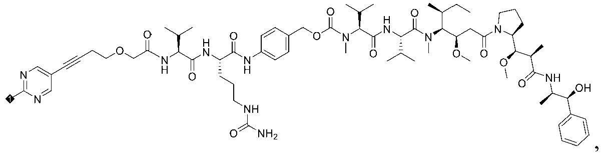 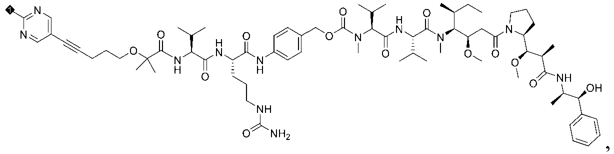 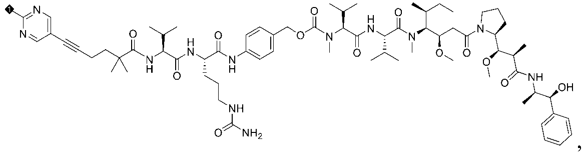 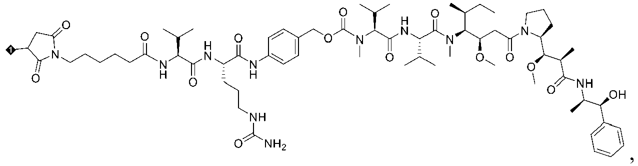 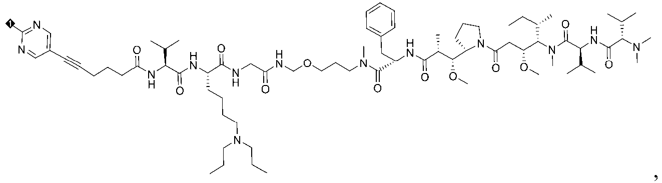 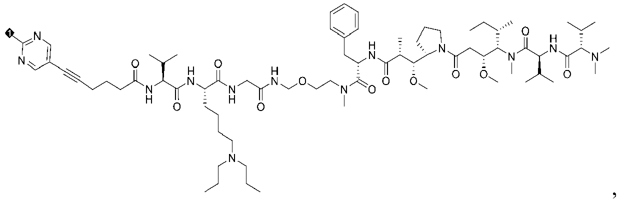 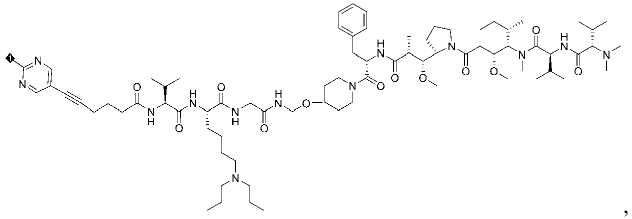 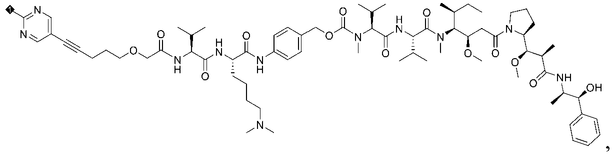 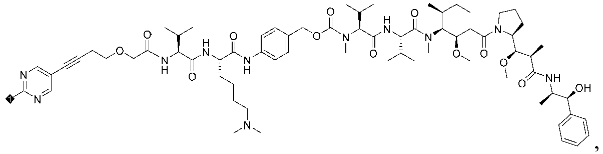 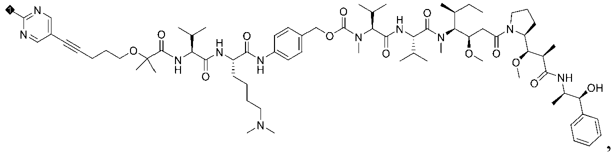 and

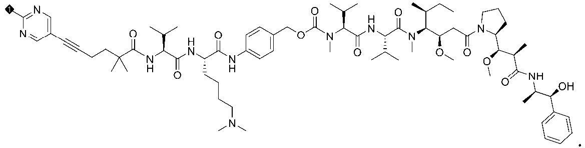

7. The drug conjugate or the stereoisomer, the prodrug, the pharmaceutically acceptable salt, or the solvate thereof according to any one of claims 1-6, wherein one or more of the following conditions are met:
(1) n1 and n2 are each independently selected from any numerical value between 1 and 8, and n3 is 0;
D₁ is a camptothecin derivative;
D₂ is eribulin and a derivative thereof;
T, L₁, and L₂ are as defined in any one of claims 1-6;
(2) n1 and n3 are each independently selected from any numerical value between 1 and 8, and n2 is 0;
D₁ is a camptothecin derivative;
D₃ is MMAE/MMAF and a derivative thereof;
T, L₁, and L₃ are as defined in any one of claims 1-6;
(3) n2 and n3 are each independently selected from any numerical value between 1 and 8, and n1 is 0;
D₂ is MMAE/MMAF and a derivative thereof;
D₃ is eribulin and a derivative thereof;
T, L₂, and L₃ are as defined in any one of claims 1-6;
(4) n1 and n2 are each independently selected from any numerical value between 1 and 8, and n3 is 0;
D₁ is a camptothecin derivative;
D₂ is duocarmycin or calicheamicin and a derivative thereof;
T, L₁, and L₂ are as defined in any one of claims 1-6.

8. The drug conjugate or the stereoisomer, the prodrug, the pharmaceutically acceptable salt, or the solvate thereof according to any one of claims 1-7, wherein the drug conjugate is selected from:

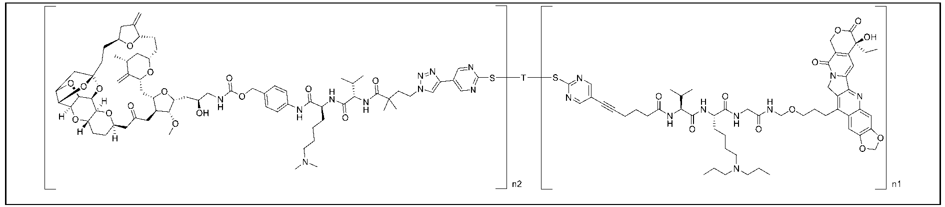

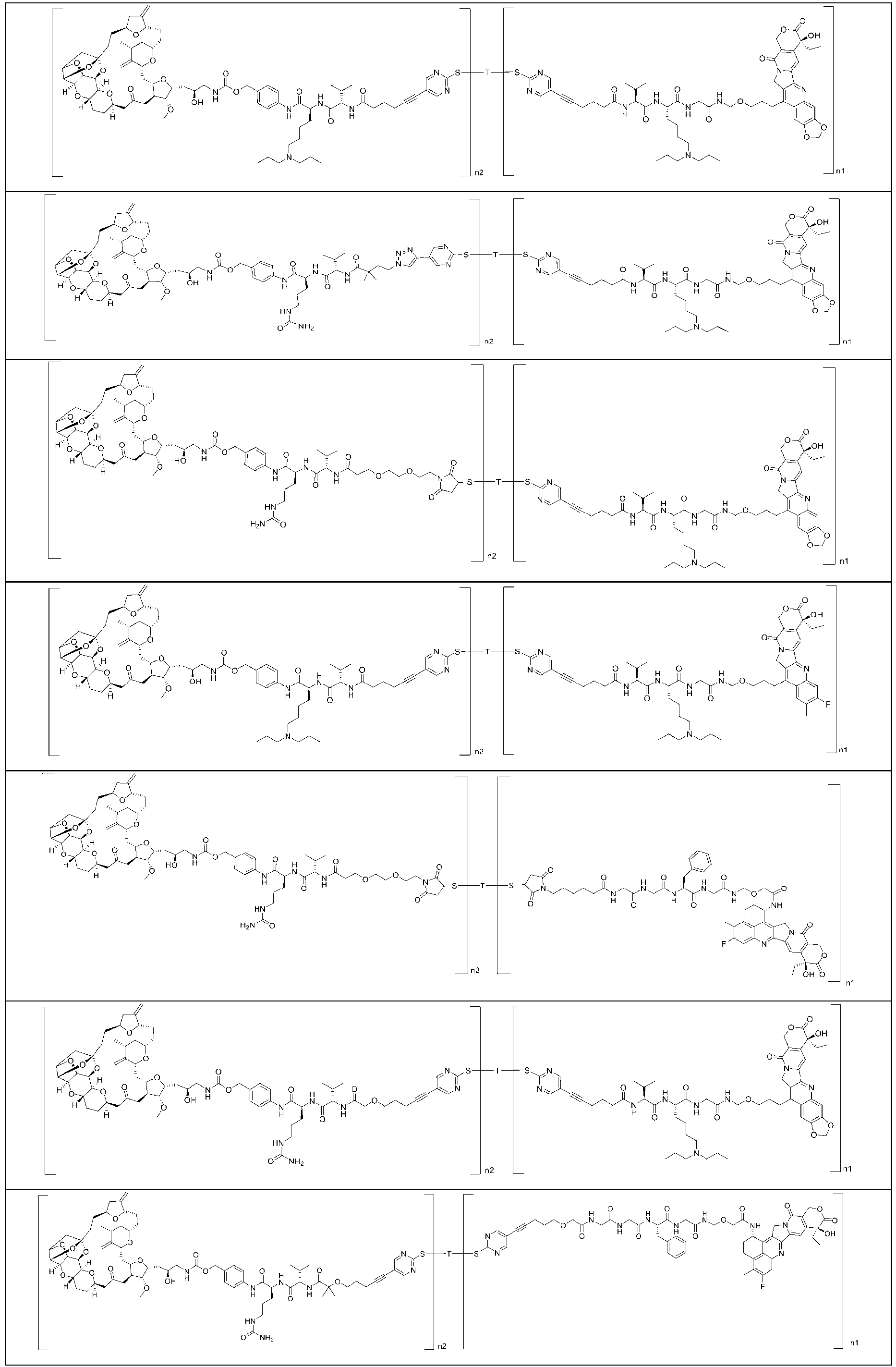

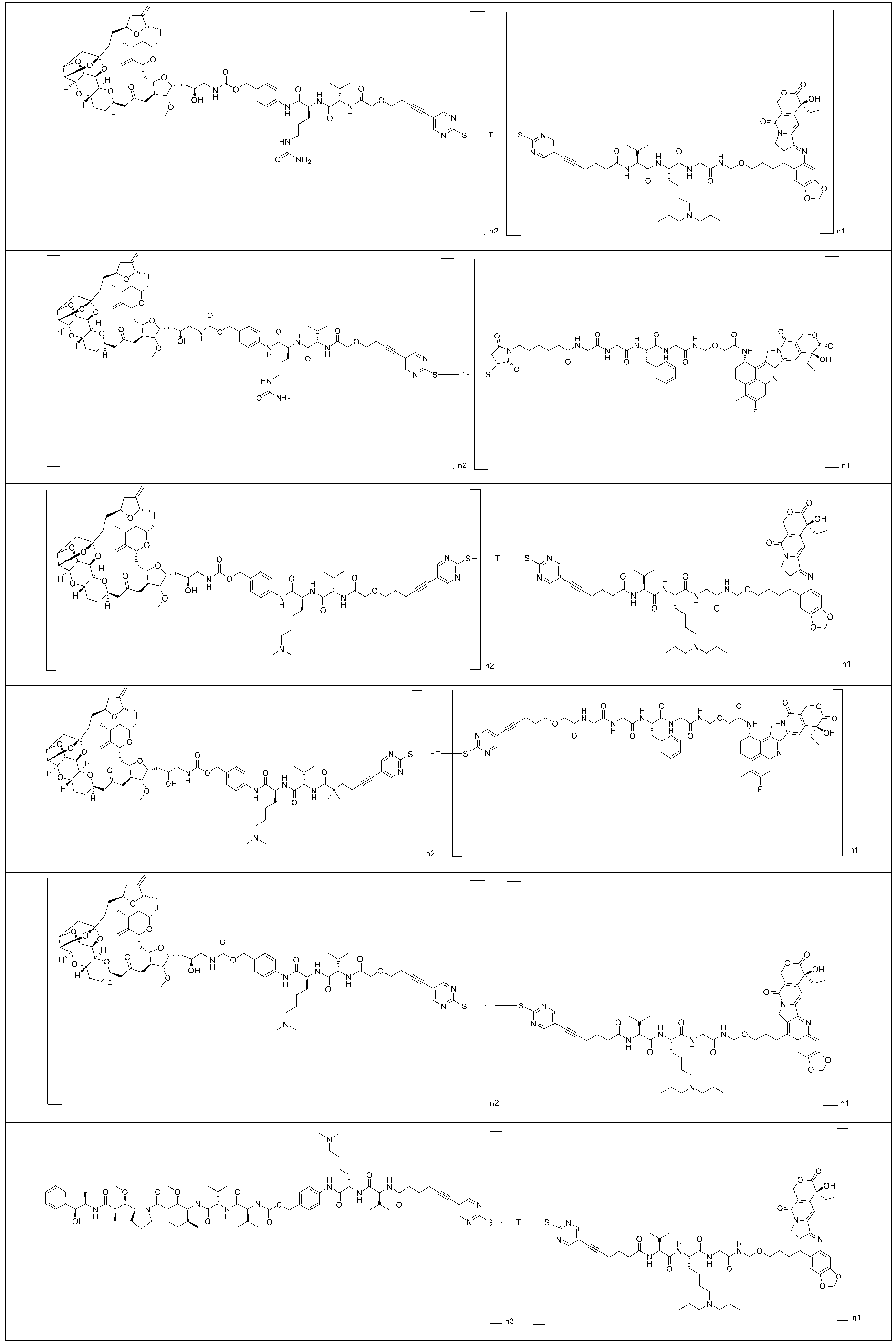

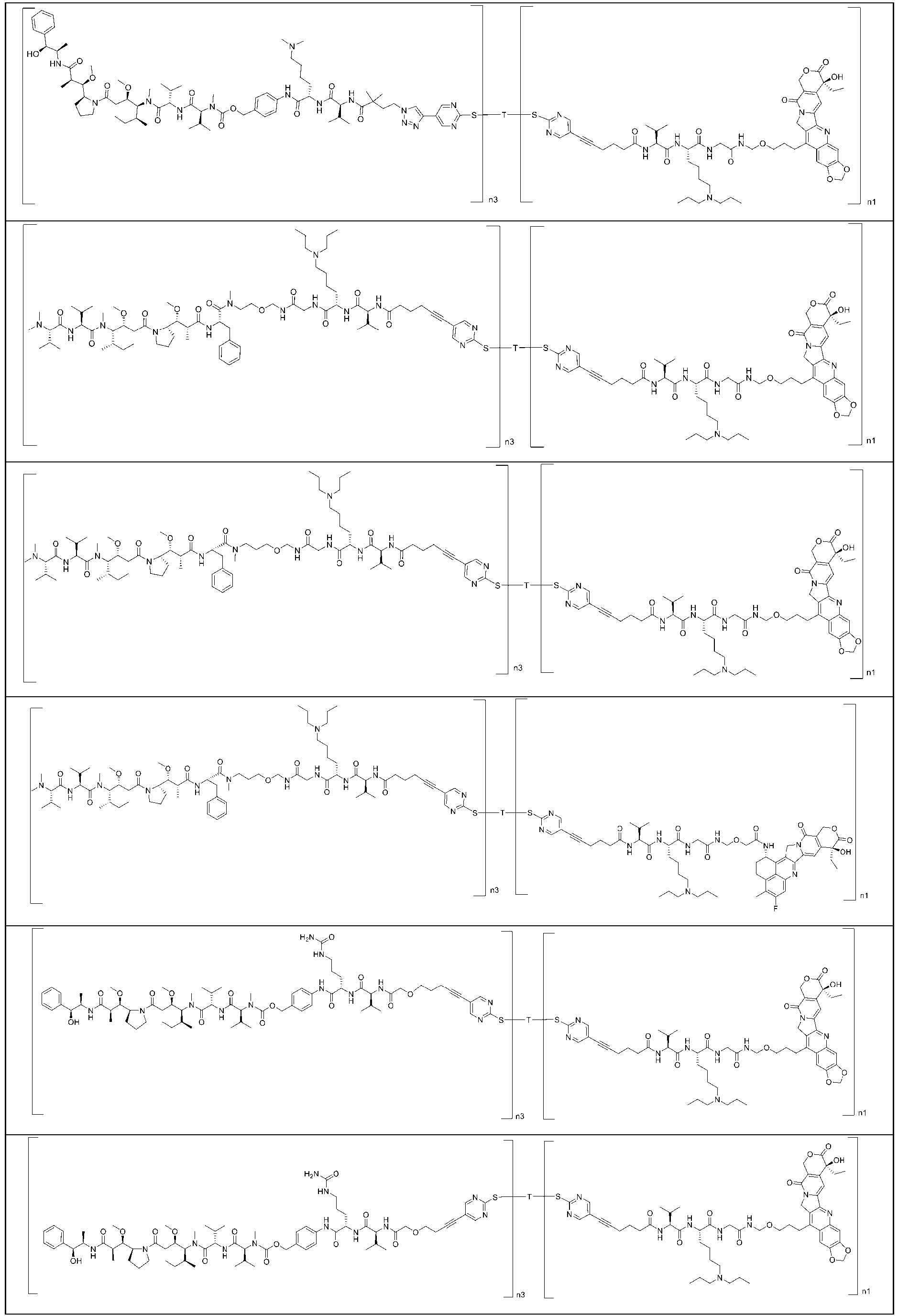

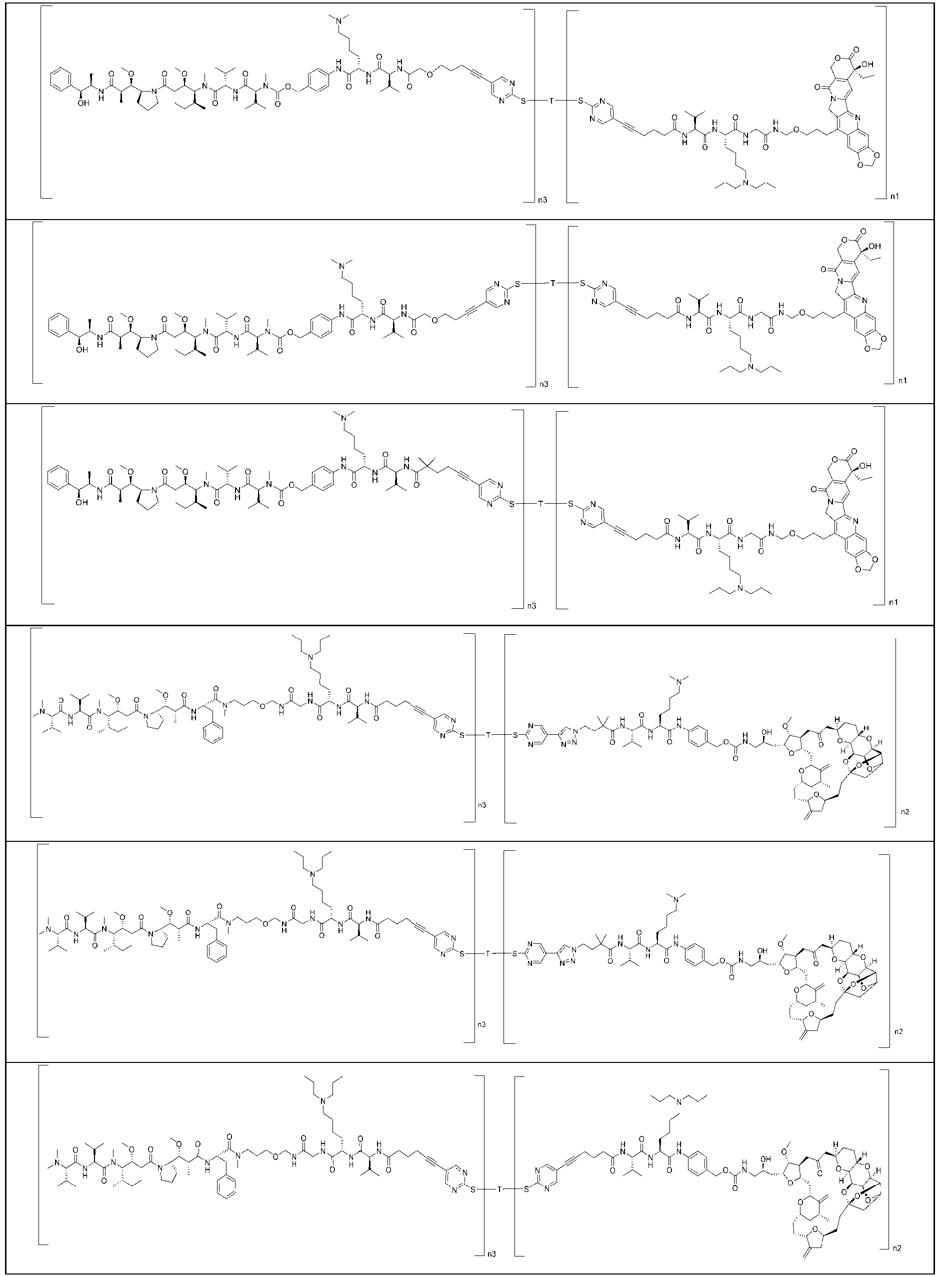

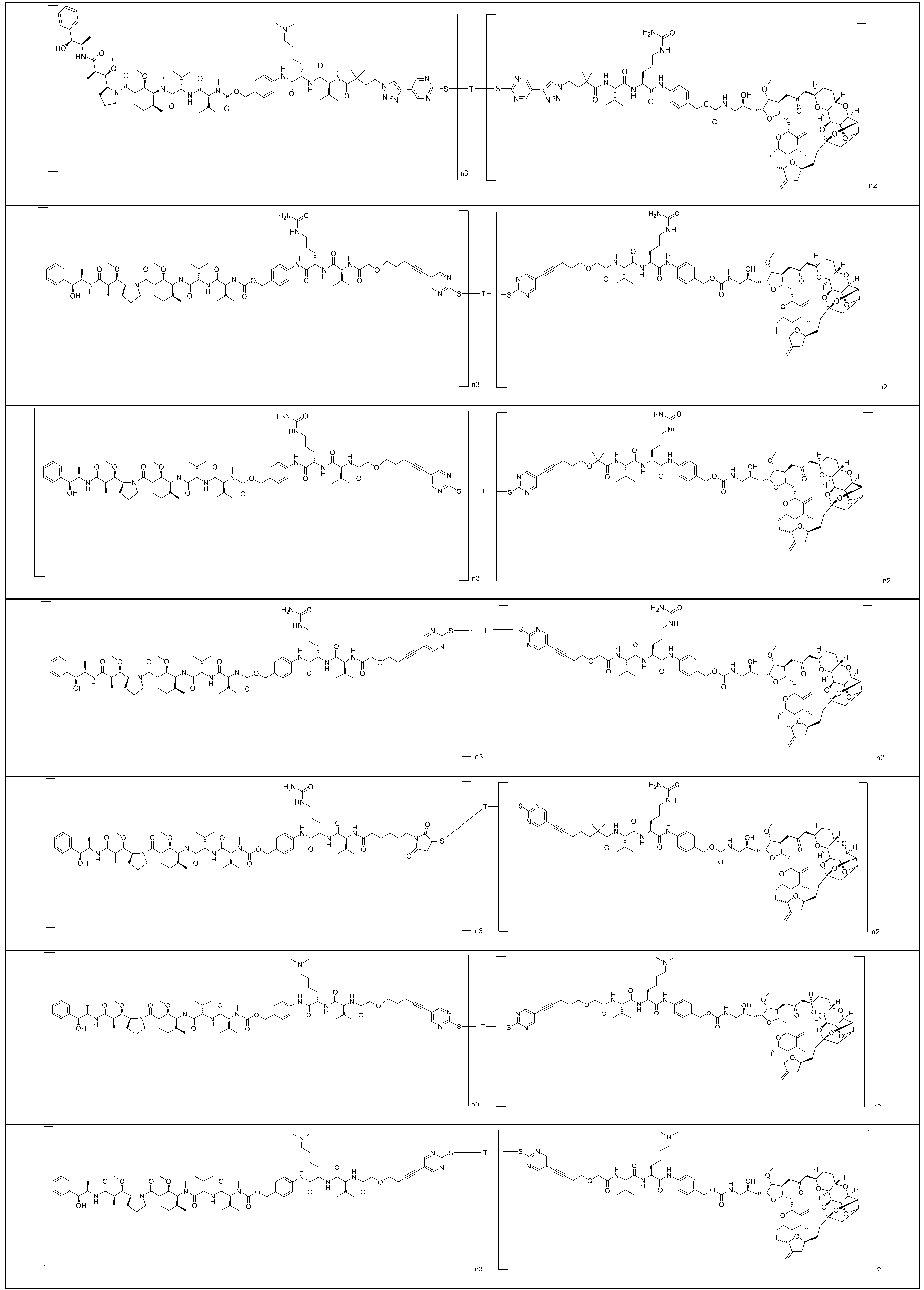

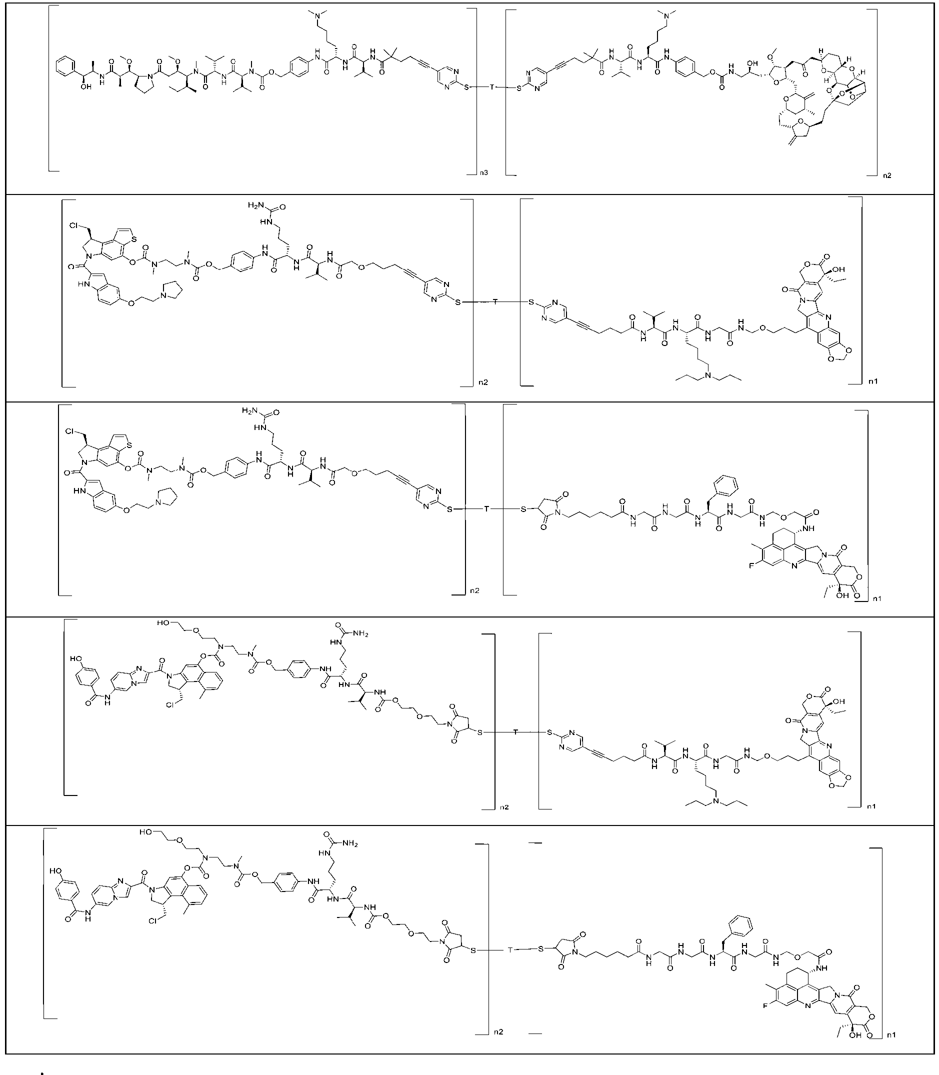

9. A preparation method for the drug conjugate or the stereoisomer, the prodrug, the pharmaceutically acceptable salt, or the solvate thereof according to claim 1, comprising: subjecting a mixture of an antibody T(SH)n with an opened interchain disulfide bond and optionally Lg-L₁-D₁, Lg-L₂-D₂, and Lg-L₃-D₃ in a determined ratio to a conjugation reaction in a suitable solvent,
wherein:
when the linkers L₁, L₂, and L₃ comprise 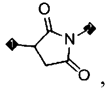 Lg-L₁, Lg-L₂, and Lg-L₃ comprise 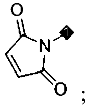
when the linkers L₁, L₂, and L₃ comprise 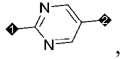 Lg is a leaving group when reacting with T(SH)n; preferably, Lg is selected from halogen, sulfonyl, tertiary amine salt groups (Me₃N⁺ and Et₃N⁺), diazonium salt groups, -OMs, MeSO₂-, and CF₃SO₃-; more preferably, Lg is selected from F and MeSO₂-;
L₁, D₁, L₂, D₂, L₃, D₃, n1, n2, and n3 are as defined in any one of claims 1-10;
preferably, the preparation method satisfies one or more of the following conditions:
(1) the conjugation reaction is performed in water and/or an organic solvent; the organic solvent is preferably selected from N,N-dimethylformamide, dimethyl sulfoxide, N-methylpyrrolidone, nitriles (for example, acetonitrile), alcohols (for example, methanol and ethanol), and any combination thereof;
(2) the preparation method comprises: subjecting a mixture of the antibody T(SH)n with an opened interchain disulfide bond and Lg-L₁-D₁ and Lg-L₂-D₂ in a determined ratio to the conjugation reaction in a suitable solvent, wherein the determined ratio of Lg-L₁-D₁ to Lg-L₂-D₂ is preferably 1, 1.5, 2, 2.5, 3, 3.5, 4, 4.5, 5, 5.5, 6, 6.5, or 7.5, and more preferably 1.5, 2, 2.5, 3, 3.5, 4, 4.5, 5, 5.5, 6, 6.5, or 7.5; the preparation method preferably comprises: adding Lg-L₁-D₁ and Lg-L₂-D₂ in a determined ratio, sequentially or simultaneously, to the antibody T(SH)n with an opened interchain disulfide bond in a suitable solvent, and performing the conjugation reaction;
or the preparation method comprises: subjecting a mixture of the antibody T(SH)n with an opened interchain disulfide bond and Lg-L₁-D₁ and Lg-L₃-D₃ in a determined ratio to the conjugation reaction in a suitable solvent, wherein the determined ratio of Lg-L₁-D₁ to Lg-L₃-D₃ is preferably 1, 1.5, 2, 2.5, 3, 3.5, 4, 4.5, 5, 5.5, 6, 6.5, or 7.5, and more preferably 1.5, 2, 2.5, 3, 3.5, 4, 4.5, 5, 5.5, 6, 6.5, or 7.5; the preparation method preferably comprises: adding Lg-L₁-D₁ and Lg-L₃-D₃ in a determined ratio, sequentially or simultaneously, to the antibody T(SH)n with an opened interchain disulfide bond in a suitable solvent, and performing the conjugation reaction.

10. A drug-linker conjugate or a stereoisomer, a prodrug, a pharmaceutically acceptable salt, or a solvate thereof, having the following structures: 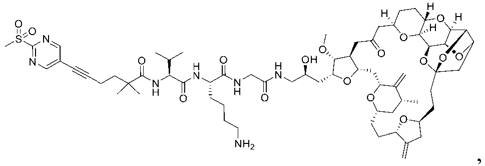 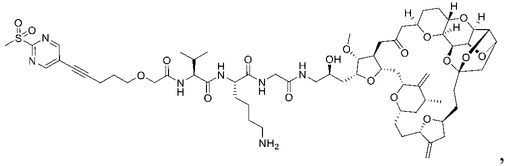 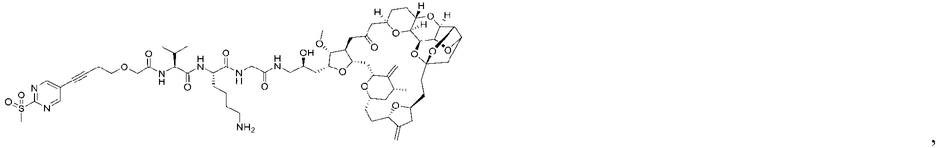 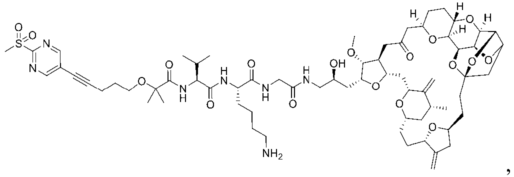 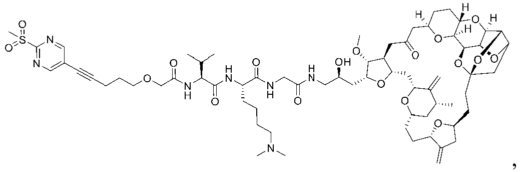 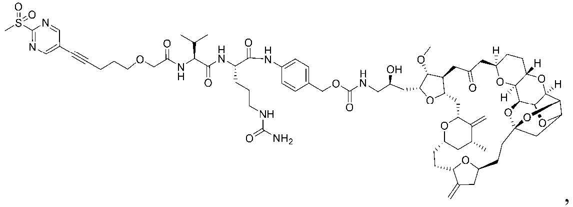 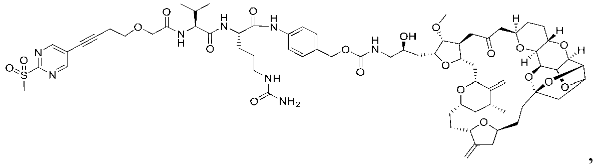 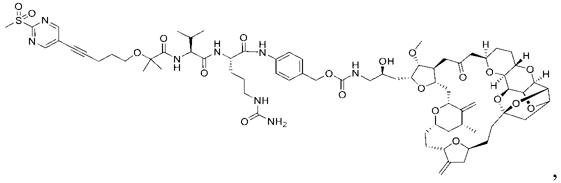 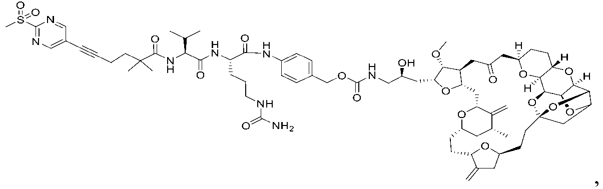 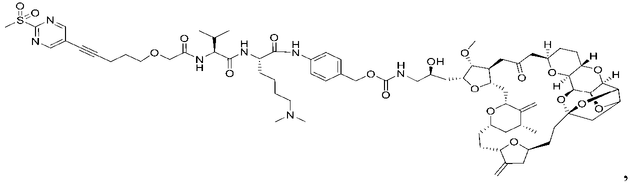 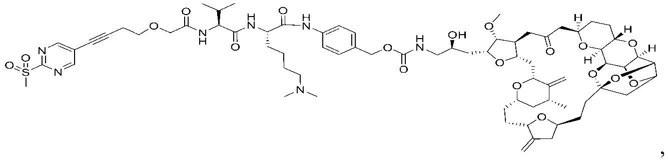 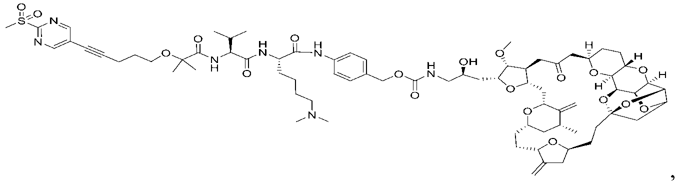 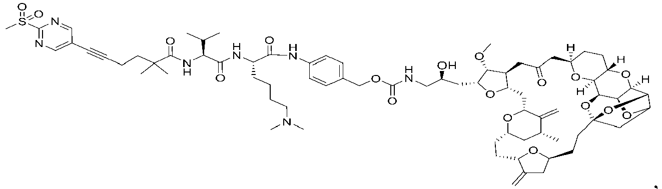 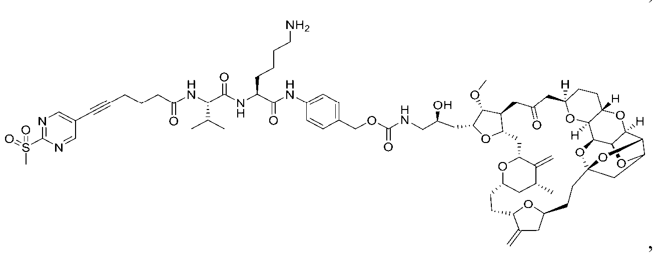 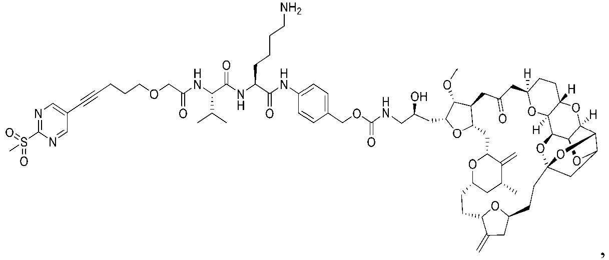 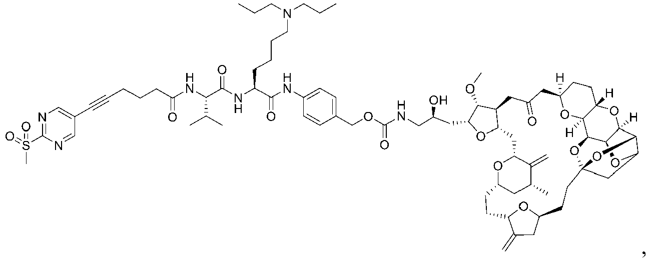 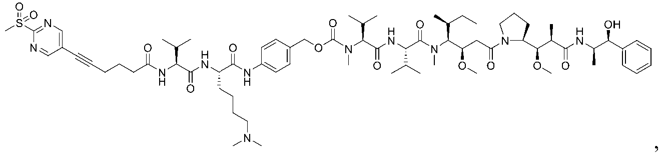 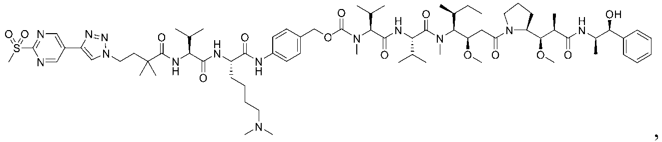 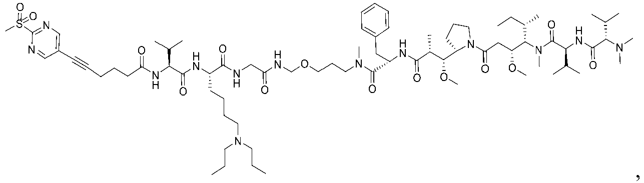 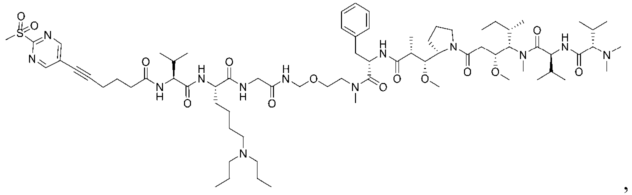 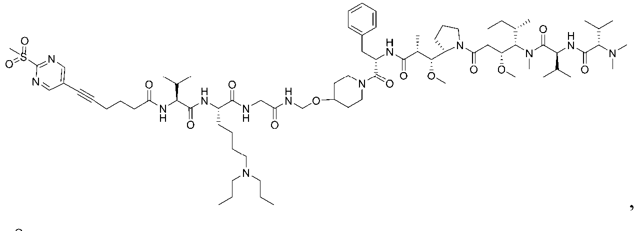 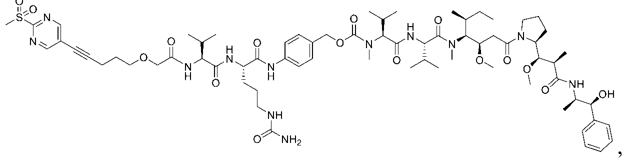 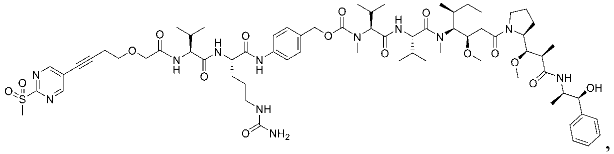 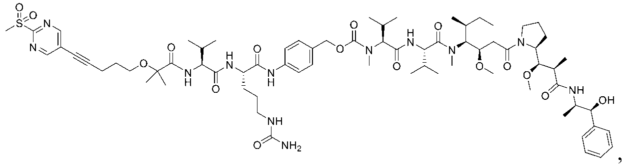 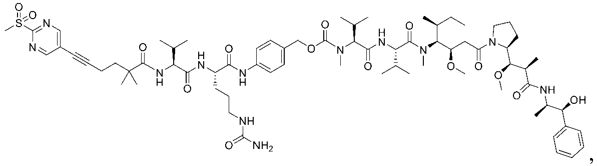 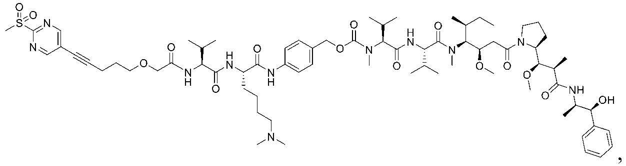 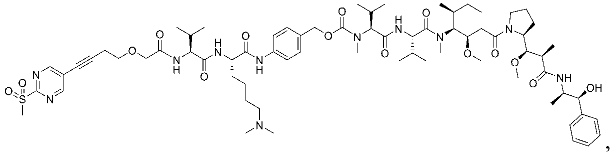 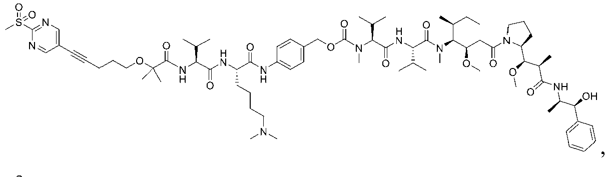 and

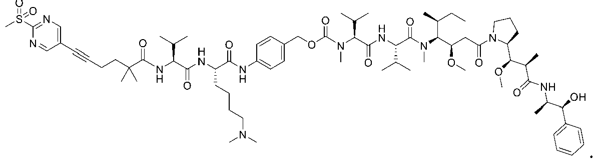

11. A pharmaceutical composition, comprising the drug conjugate or the stereoisomer thereof, the prodrug thereof, the pharmaceutically acceptable salt thereof, or the pharmaceutically acceptable solvate thereof according to any one of claims 1-8, and optionally one or more pharmaceutical adjuvants.

12. Use of the drug conjugate or the stereoisomer thereof, the prodrug thereof, the pharmaceutically acceptable salt thereof, or the pharmaceutically acceptable solvate thereof according to any one of claims 1-8, or the pharmaceutical composition according to claim 11 in the manufacture of a medicament for treating and/or preventing a disease associated with abnormal cell activity (for example, a cancer disease),
wherein preferably, the cancer disease is a solid tumor and/or a hematological tumor;
more preferably, the cancer disease is selected from esophageal cancer (for example, esophageal adenocarcinoma and esophageal squamous cell carcinoma), brain tumor, lung cancer (for example, small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, urothelial cancer, non-Hodgkin's lymphoma, central nervous system tumors (for example, neuroglioma, glioblastoma multiforme, glioma, or sarcoma), prostate cancer, and thyroid cancer; preferably selected from gastric cancer, non-small cell lung cancer, breast cancer, and breast adenocarcinoma;
or the cancer disease is selected from a tumor with high HER2 expression and a tumor with low HER2 expression; the tumor with high HER2 expression is preferably breast adenocarcinoma, and the tumor with low HER2 expression is preferably gastric cancer and/or breast cancer;
or the cancer disease is selected from tumors with drug resistance; the tumor with drug resistance is selected from esophageal cancer (for example, esophageal adenocarcinoma and esophageal squamous cell carcinoma), brain tumor, lung cancer (for example, small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, urothelial cancer, non-Hodgkin's lymphoma, central nervous system tumors (for example, neuroglioma, glioblastoma multiforme, glioma, or sarcoma), prostate cancer, and thyroid cancer; preferably, an ABCB 1 or ABCG2 transporter is overexpressed in the tumor with drug resistance; the tumor with drug resistance is more preferably Enhertu-resistant gastric cancer.
